# EUROPEAN PATENT SPECIFICATION

(11) **EP 1 863 811 B1**
(45) Date of publication and mention of the grant of the patent: **12.03.2014**
(21) Application number: 06717188.4
(22) Date of filing: 20.03.2006
(51) Int. Cl.: C07D 471/04, A61K 31/437, A61P 35/00

(54) **IMIDAZO[1,2-A]PYRIDINE DERIVATIVES: PREPARATION AND PHARMACEUTICAL APPLICATIONS**
IMIDAZO[1,2-A]PYRIDINDERIVATE: HERSTELLUNG UND PHARMAZEUTISCHE ANWENDUNGEN
DERIVES IMIDAZO[1,2-A]PYRIDINE : PREPARATION ET APPLICATIONS PHARMACEUTIQUES

(30) Priority: 21.03.2005 US 663265 P; 18.01.2006 US 759544 P
(43) Date of publication of application: 12.12.2007
(73) Proprietor: MEI Pharma, Inc., San Diego, CA 92130 (US)
(72) Inventor: LEE, Ken Chi Lik, Singapore 670627 (SG); SUN, Eric T., Singapore 269265 (SG)
(74) Representative: Schrell, Andreas
(86) International application number: PCT/SG2006/000064
(87) International publication number: WO 2006/101455

(56) References cited:
- WO-A-2004/082638
- WO-A1-99/55705
- WO-A1-99/59587
- WO-A2-2004/021989
- WO-A2-2005/090358
- US-A1- 2005 054 701
- MARKS P A ET AL: "Histone deacetylase inhibitors: inducers of differentiation or apoptosis of transformed cells." JOURNAL OF THE NATIONAL CANCER INSTITUTE 2 AUG 2000, vol. 92, no. 15, 2 August 2000 (2000-08-02), pages 1210-1216, XP002495887 ISSN: 0027-8874
- PARIS M ET AL: "Histone deacetylase inhibitors: From bench to clinic" JOURNAL OF MEDICINAL CHEMISTRY 20080327 US, vol. 51, no. 6, 27 March 2008 (2008-03-27), pages 1505-1529, XP002495888 ISSN: 0022-2623

## Description

### FIELD OF THE INVENTION

The present invention relates to hydroxamate compounds that are inhibitors of histone deacetylase (HDAC). More particularly, the present invention relates to imidazo[1,2-a]pyridine containing compounds and methods for their preparation. These compounds may be useful as medicaments for the treatment of proliferative disorders as well as other diseases involving, relating to or associated with enzymes having histone deacetylase (HDAC) activities.

### BACKGROUND OF THE INVENTION

Local chromatin architecture is generally recognized as an important factor in the regulation of gene expression. The architecture of chromatin, a protein-DNA complex, is strongly influenced by post-translational modifications of the histones which are the protein components. Reversible acetylation of histones is a key component in the regulation of gene expression by altering the accessibility of transcription factors to DNA. In general, increased levels of histone acetylation are associated with increased transcriptional activity, whereas decreased levels of acetylation are associated with repression of gene expression [Wade P.A. Hum. Mol. Genet. 10, 693-698 (2001), De Ruijter A.J.M. et al, Biochem. J., 370, 737-749 (2003)]. In normal cells, histone deacetylases (HDACs) and histone acetyl transferase together control the level of acetylation of histones to maintain a balance. Inhibition of HDACs results in the accumulation of acetylated histones, which results in a variety of cell type dependent cellular responses, such as apoptosis, necrosis, differentiation, cell survival, inhibition of proliferation and cytostasis.

Inhibitors of HDAC have been studied for their therapeutic effects on cancer cells. For example, suberoylanilide hydroxamic acid (SAHA) is a potent inducer of differentiation and/or apoptosis in murine erythroleukemia, bladder, and myeloma cell lines [Richon V.M. et al, Proc. Natl. Acad. Sci. USA, 93: 5705-5708 (1996), Richon V.M. et al, Proc. Natl. Acad. Sci. USA, 95: 3003-3007 (1998)]. SAHA has been shown to suppress the growth of prostate cancer cells *in vitro* and *in vivo* [Butler L.M. et al, Cancer Res. 60, 5165-5170 (2000)]. Other inhibitors of HDAC that have been widely studied for their anti-cancer activities are trichostatin A (TSA) and trapoxin B [Yoshida M. et al, J. Biol. Chem., 265, 17174 (1990), Kijima M. et al, J. Biol. Chem., 268, 22429 (1993)]. Trichostatin A is a reversible inhibitor of mammalian HDAC. Trapoxin B is a cyclic tetrapeptide, which is an irreversible inhibitor of mammalian HDAC. However, due to the in vivo instability of these compounds they are less desirable as anti-cancer drugs. Recently, other small molecule HDAC inhibitors have become available for clinical evaluation [US6,552,065]. Additional HDAC inhibiting compounds have been reported in the literature [Bouchain G. et al, J. Med. Chem., 46, 820-830 (2003)] and patents [WO 03/066579A2, WO 01/38322 A1]. The in vivo activity of such inhibitors can be directly monitored by their ability to increase the amount of acetylated histones in the biological sample. HDAC inhibitors have been reported to interfere with neurodegenerative processes, for instance, HDAC inhibitors arrest polyglutamine-dependent neurodegeneration [Nature, 413(6857): 739-43, 18 October, 2001]. In addition, HDAC inhibitors have also been known to inhibit production of cytokines such as TNF, IFN, IL-1 which are known to be implicated in inflammatory diseases and/or immune system disorders. [ J. Biol. Chem. 1990; 265(18): 10230-10237; Science, 1998; 281: 1001-1005; Dinarello C.A. and Moldawer L.L. Proinflammatory and antiinflammatory cytokines in rheumatoid arthritis. A primer for clinicians. 2nd Edition, Amergen Inc., 2000].

P.A. Marx et al. (Journal of National Cancer Institute, 2000, 92 (15), 1210 to 1216) discloses histone deacetylase inhibitors being inducers of differentiation or apoptosis of transformed cells.

N. Paris et al. (Journal of Medicinal Chemistry, 2008, 51 (6), 1505 to 1529) and WO 2004/082638 A2 disclose also histone deacetylase inhibitors.

Nevertheless, there is still a need to provide further HDAC inhibitors that would be expected to have useful, improved pharmaceutical properties in the treatment of diseases such as cancer, neurodegenerative diseases, disorders involving angiogenesis and inflammatory and/or immune system disorders.

The compound m-carboxy-cinnamic acid bishydroxamic acid (CBHA) is a hydroxamic acid based histone deacetylase (HDAC) inhibitor. CBHA has been shown to induce apoptotic cell death of neuroblastoma cell lines such as LAI-55n, KCN-69n, and SK-N-ER. (Marks, P.A.; Richon, V. M.; Rifkind, R. A.; J. Natl. Cancer Inst. 2000, 92(15), 1210-1216).

Thus, one technical problem of the present invention can be seen in the provision of alternative, preferably improved, histone deacetylase inhibitors in comparision to CBHA.

### SUMMARY OF THE INVENTION

The present invention is characterized by the subject matter of the independent claims.

According to the present invention a compound of the formula (1c) is provided: 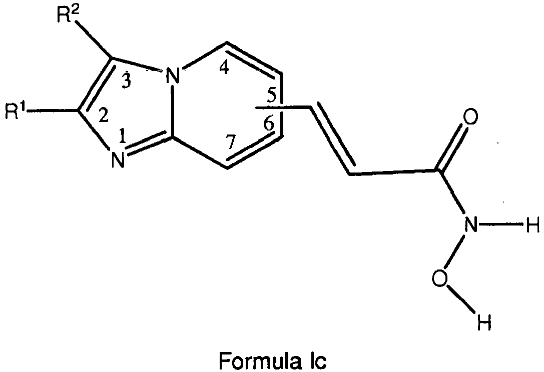 wherein:
R¹ is selected from the group consisting of: C₁-C₁₄alkyl, arylC₁-C₁₄alkyl, aryl and CO₂H;
R² is L where L is -(CR²⁰R²¹)ₘ-(C²²R²³)ₙ-(CR²⁴R²⁵)ₒ-NR²⁶R²⁷;
wherein
each R²⁰, R²¹, R²², R²³, R²⁴ and R²⁵ is independently selected from the group consisting of H and C₁-C₁₄alkyl; or
R²⁰ and R²¹ when taken together may form a group of formula =O or =S, and/or
R²² and R²³ when taken together may form a group of formula =O or =S, and/or
R²⁴ and R²¹ when taken together may form a group of formula =O or =S;
each R²⁶ and R²⁷ is independently selected from the group consisting of: H, C₁-C₁₄alkyl, C₂-C₁₄heteroalkyl, C₅-C₁₀cycloalkyl, optionally substituted aryl, arylC₁-C₁₄alkyl, C₅-C₁₀cycloalkylC₁-C₁₄alkyl and G, or
R²⁶ and R²⁷ when taken together with the nitrogen atom to which they are attached form a heterocycloalkyl which may be optionally substituted;
wherein the optional substituents on the heterocycloalkyl are selected from the group consisting of H, methyl, ethyl and benzyl,
wherein the optional substituents on aryl are selected from the group consisting of H, methoxy, methylendioxy, and piperidinyl,
m, n and o are each integers that are independently selected from the group consisting of 0, 1, 2, 3 and 4;
G is a group of formula:

   -L³W³

   wherein
   L³ is C₁-C₅alkylene;
   W³ is selected from the group consisting of -OR¹², -N(R¹²)₂, and -C(O)N(R¹²)₂,
R¹² is selected from the group consisting of H, -CN, C₁-C₁₄alkyl, C₂-C₁₄alkynyl, C₁-C₁₄haloalkyl, C₂-C₁₄heteroalkyl, hydroxy, and hydroxyC₁-C₁₄alkyl,
or a pharmaceutically acceptable salt thereof.

In a preferred embodiment of the present invention the double bond is attached at ring position 5 or 6, preferably at ring position 6, of the compound.

In a preferred embodiment of the present invention R¹ is C₁-C₁₄ alkyl.

In a preferred embodiment of the present invention R¹ is selected from the group consisting of methyl, ethyl, 2-carboxy-ethyl, propyl, isopropyl, 2,2-dimethyl-propyl, butyl, isobutyl, tert-butyl, pentyl, 2,4,4-trimethyl-pentyl, hexyl, 2-phenyl-ethyl, phenyl and 4-fluoro-phenyl.

In a preferred embodiment of the present invention the compound has the formula 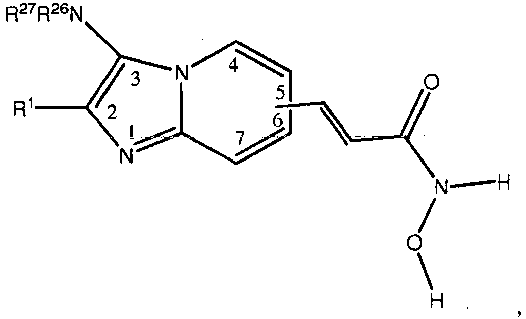 wherein R¹, R²⁶ and R²⁷ are as defined above.

In a preferred embodiment of the present invention the compound has the formula: 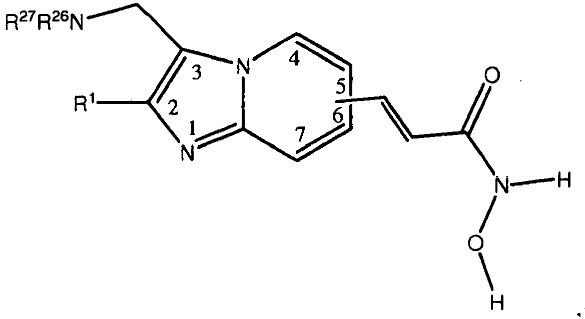 wherein R¹, R²⁶, and R²⁷ are as defined above.

The compounds as specified in the claims are based on the group of compounds of the formula (I): 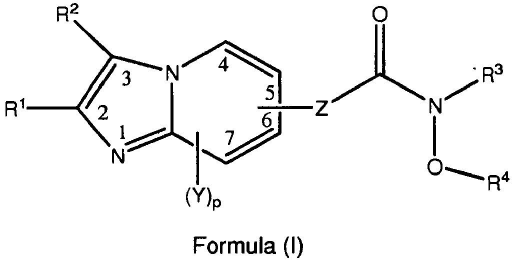 wherein:
R¹ is selected from the group consisting of: H, halogen, -CN, -NO₂, -CF₃, -OCF₃, alkyl, alkenyl, alkynyl, haloalkyl, haloalkenyl, heteroalkyl, cycloalkyl, cycloalkenyl, heterocycloalkyl, heterocycloalkenyl, aryl, heteroaryl, cycloalkylalkyl, heterocycloalkylalkyl, arylalkyl, heteroarylalkyl, arylalkenyl, cycloalkylheteroalkyl, arylheteroalkyl, heterocycloalkylheteroalkyl, heteroarylheteroalkyl, hydroxy, hydroxyalkyl, alkoxy, alkoxyalkyl, alkoxyaryl, alkenyloxy, alkynyloxy, cycloalkylkoxy, heterocycloalkyloxy, aryloxy, heteroaryloxy, arylalkyloxy, phenoxy, benzyloxy, amino, alkylamino, aminoalkyl, acylamino, arylamino, sulfonylamino, sulfinylamino, -COOH, -COR⁵, -COOR⁵, -CONHR⁵, -NHCOR⁵, -NHCOOR⁵, -NHCONHR⁵, C(=NOH)R⁵, -alkylNCOR⁵, alkoxycarbonyl, alkylaminocarbonyl, sulfonyl, alkylsulfonyl, alkylsulfinyl, arylsulfonyl, arylsulfinyl, aminosulfonyl, SR⁶ and acyl, each of which may optionally be substituted;
   or R¹ = L;
R² is selected from the group consisting of: H, halogen, -CN, -NO₂, -CF₃, -OCF₃, alkyl, alkenyl, alkynyl, haloalkyl, haloalkenyl, heteroalkyl, cycloalkyl, cycloalkenyl, heterocycloalkyl, heterocycloalkenyl, aryl, heteroaryl, cycloalkylalkyl, heterocycloalkylalkyl, arylalkyl, heteroarylalkyl, arylalkenyl, cycloalkylheteroalkyl, arylheteroalkyl, heterocycloalkylheteroalkyl, heteroarylheteroalkyl, hydroxy, hydroxyalkyl, alkoxy, alkoxyalkyl, alkoxyaryl, alkenyloxy, alkynyloxy, cycloalkylkoxy, heterocycloalkyloxy, aryloxy, heteroaryloxy, arylalkyloxy, phenoxy, benzyloxy, amino, alkylamino, aminoalkyl, acylamino, arylamino, sulfonylamino, sulfinylamino, -COOH, -COR⁵, -COOR⁵, -CONHR⁵, -NHCOR⁵, -NHCOOR⁵, -NHCONHR⁵, C(=NOH)R⁵, -alkylNCOR⁵, alkoxycarbonyl, alkylaminocarbonyl, sulfonyl, alkylsulfonyl, alkylsulfinyl, arylsulfonyl, arylsulfinyl, aminosulfonyl, SR⁶ and acyl, each of which may optionally be substituted;
   or R² = L;
R³ is selected from the group consisting of H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl each of which may be optionally substituted;
R⁴ is selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl each of which may be optionally substituted;
each Y is independently selected from the group consisting of: H, halogen, -CN, -NO₂, -CF₃, -OCF₃, alkyl, alkenyl, alkynyl, haloalkyl, haloalkenyl, haloalkynyl, heteroalkyl, cycloalkyl, cycloalkenyl, heterocycloalkyl, heterocycloalkenyl, aryl, heteroaryl, hydroxy, hydroxyalkyl, alkoxy, alkoxyalkyl, alkoxyaryl, alkoxyheteroaryl, alkenyloxy, alkynyloxy, cycloalkyloxy, cycloalkenyloxy, heterocycloalkyloxy, heterocycloalkenyloxy, aryloxy, heteroaryloxy, arylalkyl, heteroarylalkyl, arylalkyloxy, amino, alkylamino, acylamino, aminoalkyl, arylamino, sulfonyl, alkylsulfonyl, arylsulfonyl, aminosulfonyl, aminoalkyl, alkoxyalky, -COOH -C(O)OR⁶, -COR⁶, -SH, -SR⁷, -OR⁷, acyl and -NR⁸R⁹ each of which may be optionally substituted.;
each R⁵ is independently selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl each of which may be optionally substituted;
each R⁶ is independently selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl each of which may be optionally substituted;
each R⁷ is independently selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl each of which may be optionally substituted;
each R⁸ and R⁹ is independently selected from the group consisting of: H, alkyl, alkenyl, alkynyl, haloalkyl, heteroalkyl, cycloalkyl, heterocycloalkyl, aryl, heteroaryl, cycloalkylalkyl, heterocycloalkylalkyl, arylalkyl, heteroarylalkyl and acyl each of which may be optionally substituted;
p is an integer selected from the group consisting of 0, 1, 2, and 3;
L is selected from the group consisting of:
   a) Cy-L'-W-
   b) Cy-L¹-W-L²-;
   c) Cy-(CH₂)ₖ-W-;
   d) L¹-W-L²-;
   e) Cy-L¹-;
   f) R¹²-W¹-L¹-W-; and
   g) -(CR²⁰R²¹)ₘ-(CR²²R²³)ₙ-(CR²⁴R²⁵)ₒ-NR²⁶R²⁷;
   wherein
   Cy is selected from the group consisting of C₁-C₁₅ alkyl, aminoalkyl, heteroalkyl, heterocycloalkyl, cycloalkyl, aryl, aryloxy and heteroaryl, each of which may be optionally substituted;
   L' is selected from the group consisting of a bond, C₁-C₅ alkyl and C₂-C₅ alkenyl, each of which may be optionally substituted;
   L² is selected from the group consisting of C₁-C₅ alkyl and C₂-C₅ alkenyl, each of which may be optionally substituted;
   k is 0, 1, 2, 3, 4 or 5;
   W is selected from the group consisting of a bond, -O-, -S-, -S(O)-, -S(O)₂-, -N(R¹⁰)-, -C(O)N(R¹⁰)-, -SO₂N(R¹⁰)-, -N(R¹⁰)C(O)-, -N(R¹⁰)SO₂-, -N(R¹⁰)C(O)N(R¹¹)-, -C(O)N(R¹⁰)C(O)N(R¹¹)- and -N(R¹⁰)C(O)N(R¹¹C(O)-;
   W¹ is selected from the group consisting of a bond, -O-, -S-, -S(O)-, -S(O)₂-, -N(R¹⁰)-, -C(O)N(R¹⁰)-, -SO₂N(R¹⁰)-, -N(R¹⁰)C(O)-, -N(R¹⁰)SO₂-, -N(R¹⁰)C(O)N(R¹¹)-, -C(O)N(R¹⁰)C(O)N(R¹¹)- and -N(R¹⁰)C(O)N(R¹¹)C(O)-;
   Each R²⁰, R²¹, R²², R²³, R²⁴ and R²⁵ is independently selected from the group consisting of: H, halogen, -CN, -NO₂, -CF₃, -OCF₃, alkyl, alkenyl, alkynyl, haloalkyl, haloalkenyl, haloalkynyl, heteroalkyl, cycloalkyl, cycloalkenyl, heterocycloalkyl, heterocycloalkenyl, aryl, heteroaryl, cycloalkylalkyl, heterocycloalkylalkyl, arylalkyl, heteroarylalkyl, arylalkenyl, cycloalkylheteroalkyl, heterocycloalkylheteroalkyl, heteroarylheteroalkyl, arylheteroalkyl, hydroxy, hydroxyalkyl, alkoxy, alkoxyalkyl, alkoxyaryl, alkoxyheteroaryl, alkenyloxy, alkynyloxy, cycloalkylkoxy, heterocycloalkyloxy, aryloxy, arylalkyloxy, phenoxy, benzyloxy heteroaryloxy, amino, alkylamino, acylamino, aminoalkyl, arylamino, alkoxycarbonyl, alkylaminocarbonyl, sulfonyl, alkylsulfonyl, aminosulfonyl, arylsulfonyl, arylsulfinyl -COOH, -C(O)OR⁵, -COR⁵, -SH, -SR⁶, -OR⁶ and acyl, each of which may be optionally substituted; or
   R²⁰ and R²¹ when taken together may form a group of formula =O or =S, and/or
   **R²²** and R²³ when taken together may form a group of formula =O or =S, and/or
   R²⁴ and R²⁵ when taken together may form a group of formula =O or =S;
   Each R²⁶ and R²⁷ is independently selected from the group consisting of: H, halogen, alkyl, alkenyl, alkynyl, haloalkyl, haloalkenyl, heteroalkyl, cycloalkyl, cycloalkenyl, heterocycloalkyl, heterocycloalkenyl, aryl, heteroaryl, cycloalkylalkyl, heterocycloalkylalkyl, arylalkyl, heteroarylalkyl, arylalkenyl, cycloalkylheteroalkyl, heterocycloalkylheteroalkyl, heteroarylheteroalkyl, arylheteroalkyl, hydroxy, hydroxyalkyl, alkoxy, alkoxyalkyl, alkoxyaryl, alkenyloxy, alkynyloxy, cycloalkylkoxy, heterocycloalkyloxy, aryloxy, arylalkyloxy, heteroaryloxy, amino, alkylamino, aminoalkyl, acylamino, arylamino, phenoxy, benzyloxy, COOH, alkoxycarbonyl, alkylaminocarbonyl, sulfonyl, alkylsulfonyl, alkylsulfinyl, arylsulfonyl, arylsulfinyl, aminosulfonyl, SR⁵, acyl and G, each of which may be optionally substituted, or
   R²⁶ and R²¹ when taken together with the nitrogen atom to which they are attached form a heterocycloalkyl or heteroaryl group, each of which may be optionally substituted;
m, n and o are each integers that are independently selected from the group consisting of 0, 1, 2, 3 and 4;
G is a group of formula:

   -L³W³

   wherein
   L³ is selected from the group consisting of C₁-C₅ alkyl and C₂-C₅ alkenyl, each of which may be optionally substituted;
   W³ is selected from the group consisting of a bond, -OR¹², -SR¹², -S(O)R¹², -S(O)₂R¹², -N(R¹²)₂, -C(O)N(R¹²)₂, -SO₂N(R¹²)₂, -NR¹²C(O)-, -NR¹²SO₂R¹², -NR¹²C(O)N(R¹²)₂, -C(O)NR¹²C(O)N(R¹²)₂ and -N(R¹²)C(O)N(R¹²)C(O)R¹²;
R¹⁰ and R¹¹ are the same or different and are independently selected from H, C₁-C₆ alkyl, C₁-C₆alkenyl, C₁-C₁₀ heteroalkyl, C₄-C₉ cycloalkyl, C₄-C₉ heterocycloalkyl, aryl, heteroaryl, arylalkyl, heteroarylalkyl and acyl each of which may be optionally substituted;
R¹² is selected from the group consisting H, halogen, -CN, -NO₂, -CF₃, -OCF₃, alkyl, alkenyl, alkynyl, haloalkyl, haloalkenyl, heteroalkyl, cycloalkyl, cycloalkenyl, heterocycloalkyl, heterocycloalkenyl, aryl, heteroaryl, cycloalkylalkyl, heterocycloalkylalkyl, arylalkyl, heteroarylalkyl, arylalkenyl, cycloalkylheteroalkyl, arylheteroalkyl, heterocycloalkylheteroalkyl, heteroarylheteroalkyl, hydroxy, hydroxyalkyl, alkoxy, alkoxyalkyl, alkoxyaryl, alkenyloxy, alkynyloxy, cycloalkylkoxy, heterocycloalkyloxy, aryloxy, heteroaryloxy, arylalkyloxy, phenoxy, benzyloxy, amino, alkylamino, aminoalkyl, acylamino, arylamino, sulfonylamino, sulfinylamino, -COOH, -COR⁵, -COOR⁵, -CONHR⁵, -NHCOR⁵, -NHCOOR⁵, -NHCONHR⁵, C(=NOH)R⁵, -alkylNCOR⁵, alkoxycarbonyl, alkylaminocarbonyl, sulfonyl, alkylsulfonyl, alkylsulfinyl, arylsulfonyl, arylsulfinyl, aminosulfonyl, SR⁶ and acyl, each of which may optionally be substituted;
Z is selected from -CH₂-, -CH,CH₂-, -CH=CH- and C₃-C₆ cycloalkyl each of which may be optionally substituted;
or a pharmaceutically acceptable salt thereof.

One suitable genus of hydroxamic compounds are those in which R³ is H: 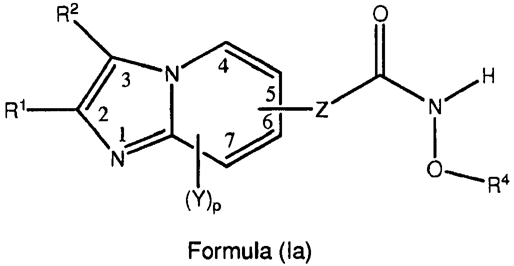 wherein R¹, R², R⁴, Y, p and Z are as described above.

Another group of useful compounds are those wherein both R³ and R⁴ are H: 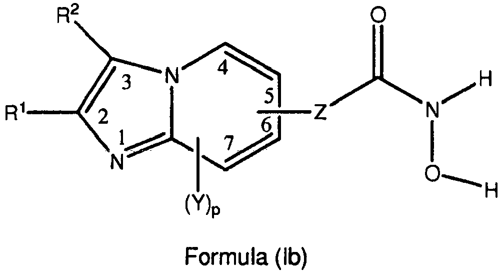 wherein R¹, R², Y, p and Z are as described above.

As with any group of structurally related compounds which possess a particular utility, certain groups are preferred for the compounds of the Formula (I), (Ia) and (Ib) in their end use application.

R¹ can be selected from the group consisting of H, -COOH, C₁-C₁₀-alkyl, alkenyl, heteroalkyl, haloalkyl, alkynyl, aryl, cycloalkyl, heterocycloalkyl, heteroaryl, C₄-C₉ heterocycloalkylalkyl, cycloalkylalkyl, arylalkyl, and heteroarylalkyl, each of which may be substituted as previously stated.

In one embodiment R¹ is preferably C₁-C₁₀ alkyl, even more preferably C₁-C₆ alkyl, each of which may be optionally substituted. Examples of specific values of alkyl are methyl, ethyl, 2-carboxy-ethyl, propyl, isopropyl, 2,2-dimethyl-propyl, butyl, isobutyl, tert-butyl, pentyl, 2,4,4-trimethyl-pentyl, and hexyl, each of which may be optionally substituted.

Accordingly, the compounds as specified in the claims are based on the group of compounds of formula (II). 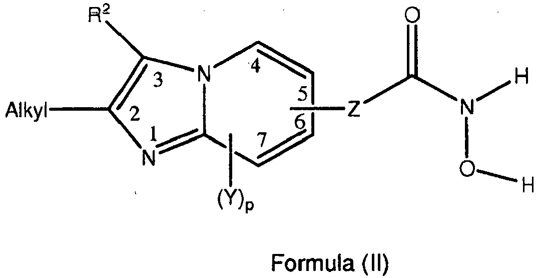 wherein R², Y, p and Z are as defined above for formula (I).

R¹ can be arylalkyl. The arylalkyl group may be of any suitable type. In general the aryl portion of the arylalkyl group is a monocyclic or bicyclic aryl moiety such as phenyl or naphthyl. The alkyl portion is generally C₁-C₁₀ alkyl, more generally C₁-C₆ alkyl. Examples of specific arylalkyl moieties include phenylhexyl, phenylpentyl, phenyl butyl, phenylpropyl, phenylethyl and phenylmethyl. In each of these groups either the aryl or the alkyl group may be optionally further substituted.

R¹ can be Ar, wherein Ar is aryl or heteroaryl. In one form the aryl is a monocyclic or bicyclic aryl or a monocyclic or bicyclic heteroaryl.

Accordingly, the compounds as specified in the claims are based on the group of compounds of the formula (III). 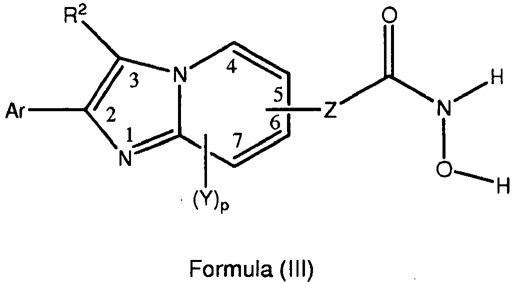 wherein Ar is aryl or heteroaryl and R², Y, p and Z are as defined above for formula (I).

When Ar is aryl, examples of suitable aryl include phenyl, napthyl, indenyl, anthracenyl and phenanthrenyl.

Ar can be phenyl leading to compounds of the formula (IIIa) 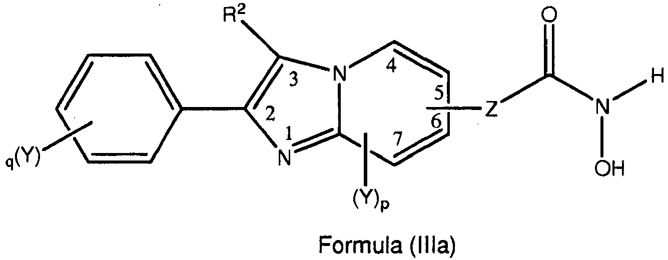 wherein R², Y, p, and Z are as defined above for formula (I) and q is an integer from 0 to 5.

R¹ can be heteroaryl. Examples of suitable heteroaryl that may be used include thiophene, benzothiophene, benzofuran, benzimidazole, benzoxazole, benzothiazole, benzisothiazole, naphtho[2,3-b]thiophene, furan, isoindolizine, xantholene, phenoxatine, pyrrole, imidazole, pyrazole, pyridine, pyrazine, pyrimidine, pyridazine, indole, isoindole, 1H-indazole, purine, quinoline, isoquinoline, phthalazine, naphthyridine, quinoxaline, cinnoline, carbazole, phenanthridine, acridine, phenazine, thiazole, isothiazole, phenothiazine, oxazole, isooxazole, furazane, phenoxazine, 2-,3-or 4- pyridyl, 2-, 3-, 4-, 5-, or 8- quinolyl, 1-, 3-, 4-, or 5- isoquinolinyl, 1-, 2-, or 3-indolyl, and 2-, or 3- thienyl.

If R¹ is alkyl or heteroalkyl then it can not substituted by a cycloalkyl, aryl, heteroaryl, or heterocycloalkyl moiety.

Specific values of R¹ can be : H; methyl; carboxyl, (pyridin-2-yl)methyl; (pyridin-3-yl)methyl; ethyl; 2-hydroxy-ethyl; 2-(pyridin-2-yl)ethyl; 2-(pyridin-3-yl)ethyl; 2-phenyl-ethyl; 2-carboxy-ethyl; 2-(morpholin-4-yl)-ethyl; 2-(piperidin-1-yl)-ethyl; 2-(pyrollidin-1-yl)-ethyl; 2-diethylamino-ethyl; propyl; isopropyl, 2,3-di-hydroxy-propyl; 3-hydroxy-propyl; 3-methoxy-propyl; 3-isopropoxy-propyl; 2,2-dimethyl-propyl; 3-dimethylamino-propyl; 3-dimethylamino-2,2-dimethyl-propyl; 3-(2-oxo-pyrollidin-1-yl)-propyl; 3-(morpholin-4-yl)-propyl; 3-(imadazol-1-yl)-propyl; 3-(4-methyl-piperidin-1-yl)-propyl; 3-(pyrollidin-1-yl)-propyl; butyl, 4-dimethylamino-butyl; 5-hydroxy-pentyl; allyl; phenyl, 4-fluoro-phenyl, benzyl; 3,4,5-trimethoxybenzyl; 2,2-dimethyl butyl; 2-methylpropyl; 2-methyl butyl; norbornyl-1-methyl; bicyclo[3.3.0]octane-3-methyl, pentyl, 2,4,4-trimethyl pentyl, and hexyl.

R² can be selected from the group consisting of H, halogen, C₁-C₁₀ alkyl, alkenyl, heteroalkyl, haloalkyl, alkynyl, aryl, cycloalkyl, heterocycloalkyl, heteroaryl, C₄-C₉ heterocycloalkylalkyl, cycloalkylalkyl, arylalkyl, and heteroarylalkyl each of which may be substituted as previously stated.

R² can be selected from the group consisting of H, alkyl, arylalkyl, aryl, heteroaryl, heteroalkyl, cycloalkyl, and L, each of which may be substituted as previously stated.

R² can be a heteroalkyl group. The heteroalkyl group can contain 2 to 10 atoms in the normal chain, more preferably 4 to 6 atoms in the normal chain. The heteroalkyl group can contain only one heteroatom in the normal chain, with a nitrogen atom being the preferred heteroatom. The heteroalkyl group can contain at least two heteroatoms in the normal chain. There can be two heteroatoms in the normal chain, one being a nitrogen atom and the other being selected from the group consisting of O, N and S.

In one specific embodiment the heteroalkyl group is selected from the group consisting of:

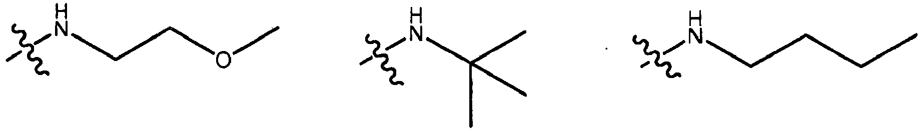

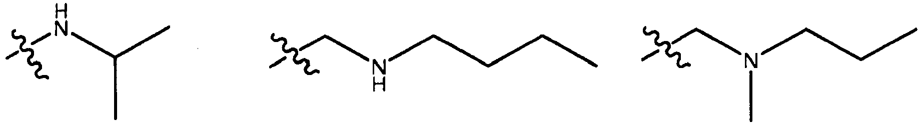

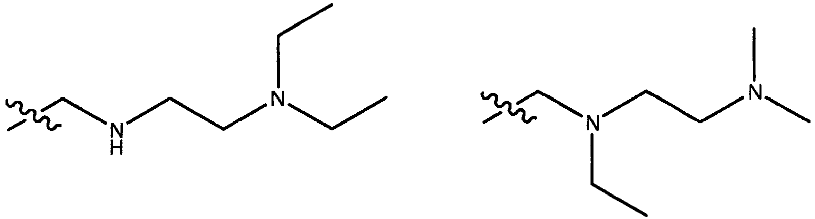

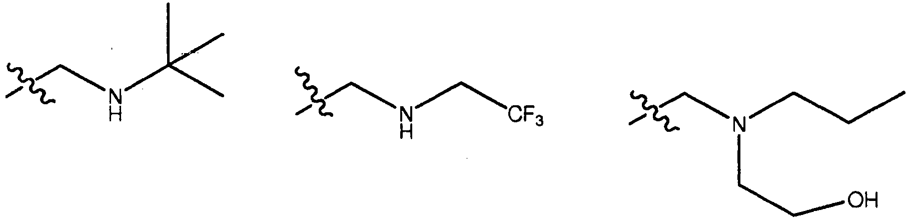

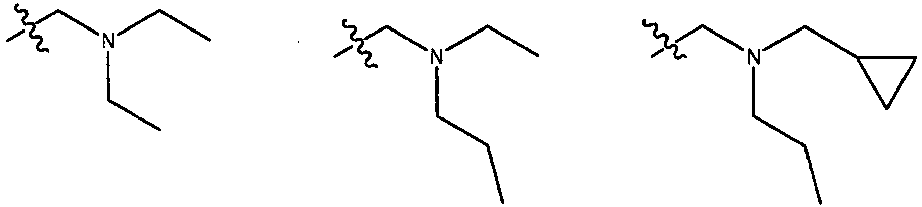

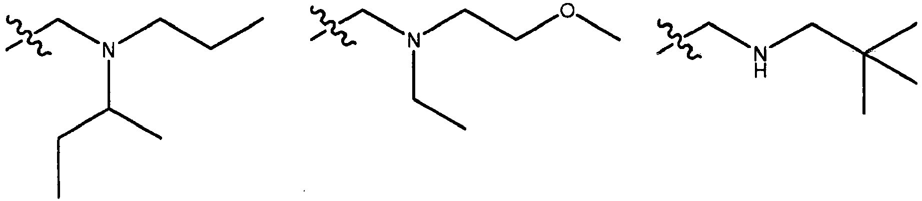

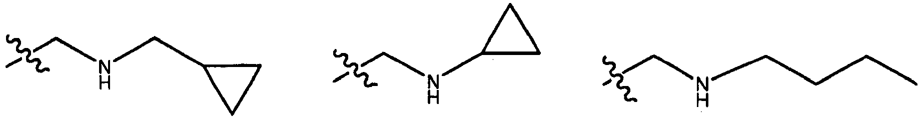

R² can be selected from the group consisting of H, hydroxyalkyl, alkyl, alkoxyalkyl, and aminoalkyl each of which may be substituted as previously stated.

If R² is alkyl or heteroalkyl then it is not substituted by a cycloalkyl, aryl, heteroaryl, or heterocycloalkyl.

Specific values of R² can be: H; methyl; benzylamino-methyl; dibenzylamino-methyl; [2-(4-fluoro-phenyl)-acetylamino]-methyl; [2-(4-methoxy-phenyl)-acetylamino]-methyl; 4-methoxy-benzylamino-methyl; benzyloxy-methyl; phenylacetylamino-methyl; 1-amino-2-phenyl-ethyl; 2-benzylamino-ethyl; 2-(3-methoxy-phenyl)-ethyl; 2-(pyridin-3-yl)ethyl; 2-(2-phenoxyacetylamino)-ethyl; 2-benzenesulphonylamino-ethyl; 2-phenyl-ethyl; isopropyl; 2-phenyl-propyl; 3-phenyl-propyl; 3-phenoxy-propyl; 3-(1H-indol-3-yl)-propyl; 4-methoxy-phenyl; 4-fluoro-phenyl; 4-benzyloxy-3-methoxy-phenyl; isobutyl; cyclohexyl; octyl; benzyl; pyridin-2-yl; pyridin-4-yl; thiophen-3-yl; (2-methoxy-ethyl)-amine, cyclohexyl-amine, t-butyl-amine, butylamine, isopropylamine, (4-piperidinyl-phenyl)-amine, (3,4,5-trimethoxyphenyl)-amine, (3,4-methylenedioxy-benzyl) amine, (3,4-methylenedioxy-phenyl) amine benzylsulfanyl, and 2-phenylmethansulfanyl.

In another embodiment R² is L and thus the compounds of the invention are based on the group of compounds of formula (IV). 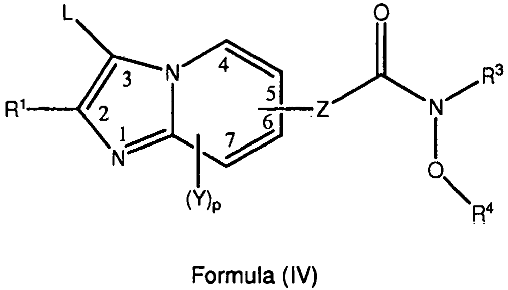 wherein R¹, R³, R⁴, L, Y, p and Z are as defined above for the compounds of formula (I).

In one form R³ is H providing compounds of formula (IVa) 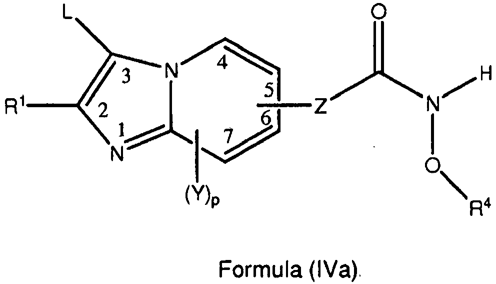 wherein R¹, R⁴, L, Y, p and Z are as defined above for the compounds of formula (I).

In a further form R⁴ is H providing compounds of formula (IVb). 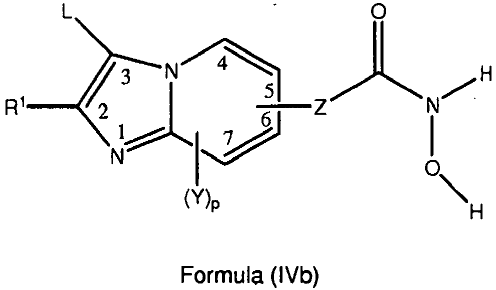 wherein R¹, L, Y, p and Z are as defined above for the compounds of formula (I).

In the compounds of formula (IV), (IVa) and (IVb) the preferred values of R¹ are alkyl, cycloalkyl, aryl, arylalkyl and heteroaryl, each of which may be optionally substituted. The compounds of the invention are based on the group of compounds of formula (IVc). 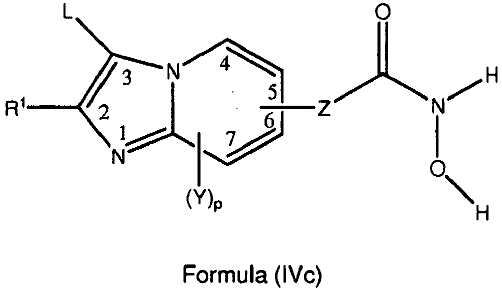 wherein R¹ is selected from the group consisting of alkyl, cycloalkyl, aryl, arylalkyl and heteroaryl, each of which may be optionally substituted; and L, Z, Y and p are as defined for compounds of formula I.

In one form R¹ is Ar providing compound of formula (IVd). 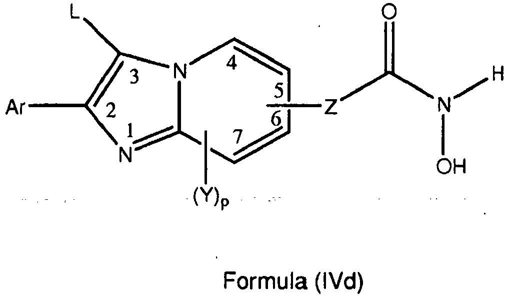 wherein Ar is aryl or heteroaryl and Y, p, L and Z are as defined above for formula (I).

Ar can be phenyl providing compounds of formula (IVe) 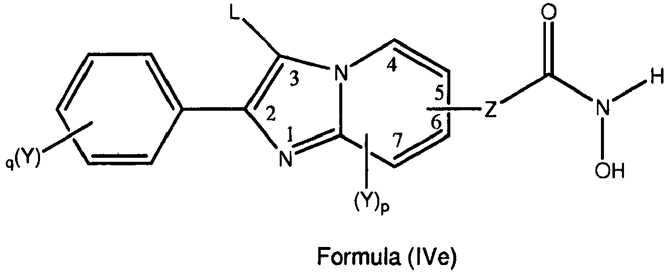 wherein Y, p, L and Z are as defined above for formula (I) and q is an integer from 0 to 5.

R¹ can be alkyl providing compounds of formula (IVf) 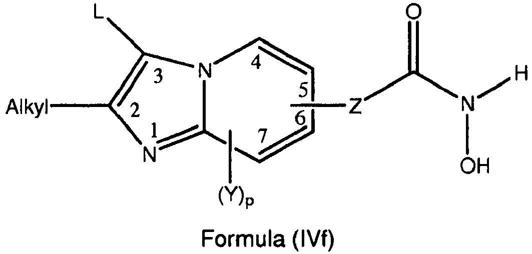 wherein Y, p, L and Z are as defined above for formula (I).

In the compounds on which the compounds of the present invention are based and in particular the compounds of formula (IV), (IVa), (IVb), (IVc), (IVd), (IVe) and (IVf) there are a number of specific values of L (and hence specific values of R²).

R² can be L which is a group of formula: 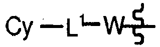 wherein
Cy is selected from the group consisting of C₁-C₁₅ alkyl, aminoalkyl, heterocycloalkyl, cycloalkyl, aryl, aryloxy and heteroaryl, each of which may be optionally substituted;
L¹ is selected from the group consisting of a bond, C₁-C₅ alkyl, and C₂-C₅ alkenyl, each of which may be optionally substituted;
W is selected from the group consisting of a bond, -O-, -S-, -S(O)-, -S(O)₂-, -N(R¹⁰)-, -C(O)N(R¹⁰)-, -SO₂N(R¹⁰)-, -N(R¹⁰)C(O)-, -N(R¹⁰)SO₂-, -N(R¹⁰)C(O)N(R¹¹)-, -C(O)N(R¹⁰)C(O)N(R¹¹)- and -N(R¹⁰)C(O)N(R¹¹)C(O)-;

This provides compounds of formula (V): 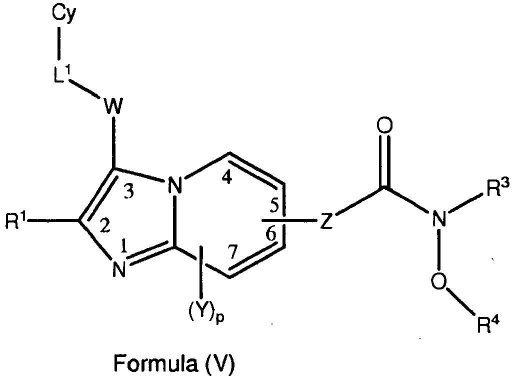 wherein R¹, R³, R⁴, Y, p and Z are as defined above for the compounds of formula (I), and W, L¹ and Cy are as defined immediately above. In a further form W is a group of formula

-NR¹⁰-,

most preferably a group -NH-.

In this form R² is L which is a group of formula

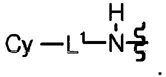

In one form L¹ is selected from the group consisting of a bond or methyl.

In one form Cy is aryl, or cycloalkyl, each of which may be substituted. Examples of typical values of Cy include phenyl, cyclopentyl and cyclohexyl. Specific values of Cy include 3,4,5-trimethoxy-phen-1-yl; 3,4-methylenedioxy pheny-1-yl; 4-piperidin-1-yl-phen-1-yl, and cyclohexyl.

W can be a bond.

In this form R₂ is a group of formula 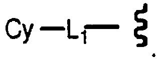
L₁ is selected from the group consisting of C₁ - C₅ alkyl and C₁-C₅ alkenyl, each of which may be optionally substituted;
In one form Cy is selected from C₁-C₁₅ alkyl, aminoalkyl, heterocycloalkyl, cycloalkyl, aryl, aryloxy or heteroaryl, each of which may be optionally substituted,

In the group of compounds on which the compounds of the invention are based R² can be L wherein:

L= L¹-W-L²-

wherein L¹ and L² are the same or different and are independently selected from C₁-C₅alkyl and C₂-C₅alkenyl each which may be optionally substituted; and
W is selected from the group consisting of a bond, -O-, -S-, -S(O)-, -S(O)₂-, -N(R¹⁰)-, -C(O)N(R¹⁰)-, -SO₂N(R¹⁰)-, -N(R¹⁰)C(O)-, -N(R¹⁰)SO₂-, -N(R¹⁰)C(O)N(R¹¹)-, -C(O)N(R¹⁰)C(O)N(R¹¹)- and -N(R¹⁰)C(O)N(R¹¹)C(O);

This provides compounds of formula (VI): 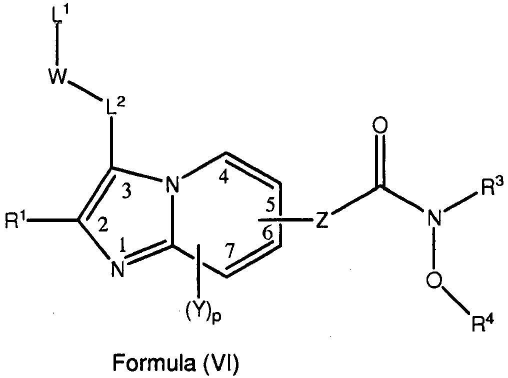 wherein R¹, R³, R⁴, Y, p and Z are as defined above for the compounds of formula (I), and W, L¹ and L² are as defined immediately above.

In one form W is a group of formula -N(R¹⁰)-, such that L is a group of formula:

L¹-N(R¹⁰)-L²-.

In another form R¹⁰ is selected from H or alkyl. Suitable specific values of R¹⁰ include H, methyl, ethyl, propyl, isopropyl, butyl, isobutyl, pentyl or hexyl.

L² can be methyl or ethyl, and L¹ can be H or alkyl. Suitable examples of L¹ include H, methyl, ethyl, propyl, isopropyl, butyl, isobutyl, pentyl and hexyl.

In the group of the compounds on which the compounds of the invention are based the group L can be chosen such that:

L= R¹²-W¹-L¹-W-

wherein L¹ is selected from the group consisting of C₁ - C₅ alkyl, or C₁-C₅ alkenyl, each of which may be optionally substituted;
W and W¹ are each independently selected from the group consisting of a bond, -O-, -S-, -S(O)-, -S(O)₂-, -N(R¹⁰)-, -C(O)N(R¹⁰)-, -SO₂N(R¹⁰)-, -N(R¹⁰)C(O)-, -N(R¹⁰)SO₂-, -N(R¹⁰)C(O)N(R¹¹)-, -C(O)N(R¹⁰)C(O)N(R¹¹)- and -N(R¹⁰)C(O)N(R¹¹)C(O)-;
R¹² can be selected from the group consisting H, halogen, -CN, -NO₂, -CF₃,-OCF₃, alkyl, alkenyl, alkynyl, haloalkyl, haloalkenyl, heteroalkyl, cycloalkyl, cycloalkenyl, heterocycloalkyl, heterocycloalkenyl, aryl, heteroaryl, cycloalkylalkyl, heterocycloalkylalkyl, arylalkyl, heteroarylalkyl, arylalkenyl, cycloalkylheteroalkyl, arylheteroalkyl, heterocycloalkylheteroalkyl, heteroarylheteroalkyl, hydroxy, hydroxyalkyl, alkoxy, alkoxyalkyl, alkoxyaryl, alkenyloxy, alkynyloxy, cycloalkylkoxy, heterocycloalkyloxy, aryloxy, heteroaryloxy, arylalkyloxy, phenoxy, benzyloxy, amino, alkylamino, aminoalkyl, acylamino, arylamino, sulfonylamino, sulfinylamino, -COOH, -COR⁵, -COOR⁵, -CONHR⁵, -NHCOR⁵, -NHCOOR⁵, -NHCONHR⁵, C(=NOH)R⁵, -alkylNCOR⁵, alkoxycarbonyl, alkylaminocarbonyl, sulfonyl, alkylsulfonyl, alkylsulfinyl, arylsulfonyl, arylsulfinyl, aminosulfonyl, SR⁶ and acyl, each of which may optionally be substituted;

This provides compounds of formula (VII): 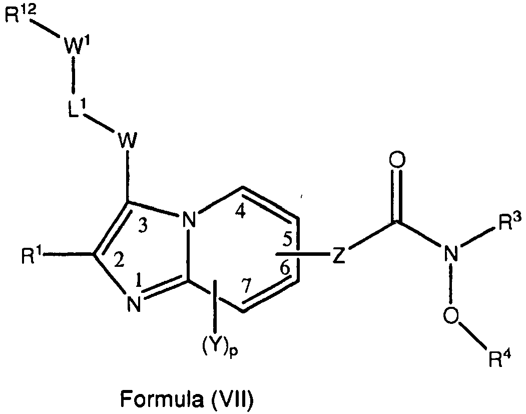 wherein R¹, R³, R⁴, Y, p and Z are as defined above for the compounds of formula (I), and W, L¹ and W² and R¹² are as defined immediately above.

In one form W= -N(R¹⁰)- such that L is a group of formula:

R¹²-W¹-L¹-N(R¹⁰)-,

R¹⁰ can be H such that L is a group of formula

R¹²-W¹-L¹-NH-,

In one form W¹ is -N(R¹⁰)C(O)- such that L is a group of formula

R¹²-N(R¹⁰)C(O)-L¹-NH-,

R¹⁰ can be selected from the group consisting of H and alkyl. Specific values of R¹⁰ include H, methyl, ethyl, propyl and isopropyl.

W¹ can be -O- such that L is a group of formula:

R¹²-O-L¹-NH-

L¹ can be C₁-C₅ alkyl. Specific examples include methyl, ethyl or propyl.

R¹² can be alkyl, heteroalkyl, and alkynyl, each of which may be substituted.

Specific values of R¹² include methyl, ethyl, 2-(dimethylamino)-ethyl, propyl, isopropyl, butyl, tert-butyl, 2,2,2-trifluoroethyl, 2-methoxy-ethyl, 2-methylsulfanyl-ethyl, 2-propynyl, 2-(diethylamino)-ethyl, 2-cyano-methyl, 2-hydroxy-ethyl, (3-dimethyl-amino-2,2-dimethyl)-propyl, and 3-methyl-butanl-ol-2-yl.

In the compounds of the invention the group L is chosen such that: L is a group of formula:

-(CR²⁰R²¹)ₘ-(CR²²R²³)ₙ-(CR²⁴R²⁵)ₒ-NR²⁶R²⁷;

Each R²⁰, R²¹, R²², R²³, R²⁴ and R²⁵ can be independently selected from the group consisting of: H, halogen, -CN, -NO₂, -CF₃, -OCF₃, alkyl, alkenyl, alkynyl, haloalkyl, haloalkenyl, haloalkynyl, heteroalkyl, cycloalkyl, cycloalkenyl, heterocycloalkyl, heterocycloalkenyl, aryl, heteroaryl, cycloalkylalkyl, heterocycloalkylalkyl, arylalkyl, heteroarylalkyl, arylalkenyl, cycloalkylheteroalkyl, heterocycloalkylheteroalkyl, heteroarylheteroalkyl, arylheteroalkyl, hydroxy, hydroxyalkyl, alkoxy, alkoxyalkyl, alkoxyaryl, alkoxyheteroaryl, alkenyloxy, alkynyloxy, cycloalkylkoxy, heterocycloalkyloxy, aryloxy, arylalkyloxy, phenoxy, benzyloxy, heteroaryloxy, amino, alkylamino, acylamino, aminoalkyl, arylamino, alkoxycarbonyl, alkylaminocarbonyl, sulfonyl, alkylsulfonyl, aminosulfonyl, arylsulfonyl, arylsulfinyl, - COOH, -C(O)OR⁵, -COR⁵, -SH, -SR⁶, -OR⁶ and acyl, each of which may be optionally substituted; or
R²⁰ and R²¹ when taken together may form a group of formula =O or =S, and/or
R²² and R²³ when taken together may form a group of formula =O or =S, and/or
R²⁴ and R²⁵ when taken together may form a group of formula =O or =S;
Each R²⁶ and R²⁷ can be independently selected from the group consisting of: H, halogen, alkyl, alkenyl, alkynyl, haloalkyl, haloalkenyl, heteroalkyl, cycloalkyl, cycloalkenyl, heterocycloalkyl, heterocycloalkenyl, aryl, heteroaryl, cycloalkylalkyl, heterocycloalkylalkyl, arylalkyl, heteroarylalkyl, arylalkenyl, cycloalkylheteroalkyl, heterocycloalkylheteroalkyl, heteroarylheteroalkyl, arylheteroalkyl, hydroxy, hydroxyalkyl, alkoxy, alkoxyalkyl, alkoxyaryl, alkenyloxy, alkynyloxy, cycloalkyloxy, heterocycloalkyloxy, aryloxy, arylalkyloxy, heteroaryloxy, amino, alkylamino, aminoalkyl, acylamino, arylamino, phenoxy, benzyloxy, COOH, alkoxycarbonyl, alkylaminocarbonyl, sulfonyl, alkylsulfonyl, alkylsulfinyl, arylsulfonyl, arylsulfinyl, aminosulfonyl, SR⁵, acyl and G, each of which may be optionally substituted, or
R²⁶ and R²⁷ when taken together with the nitrogen atom to which they are attached form a heterocycloalkyl or can form a heteroaryl group, each of which may be optionally substituted;
each m, n and o are each integers independently selected from the group consisting of 0, 1, 2, 3 and 4;
G is a group of formula:

   -L³W³

   wherein
   L³ can be C₁-C₅ alkyl or C₂-C₅ alkenyl, each of which may be optionally substituted;
   W³ is selected from the group consisting of a bond, -OR¹², -SR¹², -S(O)R¹², -S(O)₂R¹², -N(R¹²)₂, -C(O)N(R¹²)₂, -SO₂ N(R¹²)₂, -NR¹²C(O)-, -NR¹²SO₂R¹², -NR¹²C(O)N(R¹²)₂, -C(O)NR¹²C(O)N(R¹²)₂ and -N(R¹²)C(O)N(R¹²)C(O)R¹²;

This provides compounds of the formula (VIII). In the compounds of formula (VIII) the sum of m+n+o can be 0 which provides compounds of formula (VIIIa). 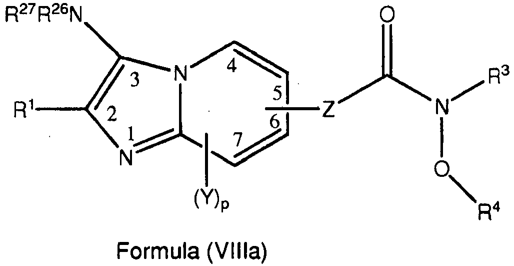 wherein wherein R¹, R³, R⁴, R²⁶ and R²⁷ Y, p and Z are as defined above for the compounds of formula I,

The sum of m+n+o can be 1 which provides compounds of formula (VIIb). 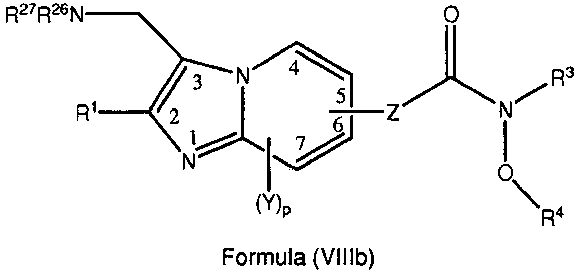 wherein wherein R¹, R³, R⁴, R²⁶ and R²⁷ Y, p and Z are as defined above for the compounds of formula (I).

The sum of m+n+o can be 2 which provides compounds of formula (VIIIc). 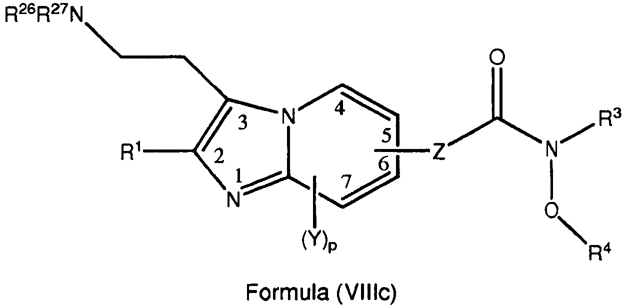 wherein wherein R¹, R³, R⁴, R²⁶ and R²⁷ Y, p and Z are as defined above for the compounds of formula (I).

In the compounds of formula (VIII) to (VIIIc) R²⁶ and R²⁷ can be independently selected from the group consisting of H, alkyl, hydroxyalkyl, heteroalkyl, cycloalkyl, aryl, arylalkyl, cycloalkylalkyl and G. Examples of specific values of R²⁶ and R²⁷, preferably of the present invention, include H, 3,4,5-trimethoxyphenyl, 3,4-methylenedioxybenzyl, 4-piperidin-1yl-phenyl, 3,4-methylenedioxyphenyl, 2-methoxy-ethyl, cyclopropyl, cyclohexyl, methyl, cyclopropyl-methyl, ethyl, propyl, isopropyl, 2,2-dimethyl-propyl, butyl, t-butyl, sec-butyl, 2-(diethylamino)-ethyl. 2-(dimethylamino)-ethyl and 2,2,2 triflouroethyl.

In one embodiment R²⁶ is G and R²⁷ is H or alkyl.

In one form of this embodiment G is a group of formula: -(CH₂)₂-C(O)N(R¹²)₂

In one form of this embodiment each R¹² can be independently selected from the group consisting of H, alkyl, hydroxyalkyl, heteroalkyl; and alkynyl.

In specific embodiments G is selected from the group consisting of:

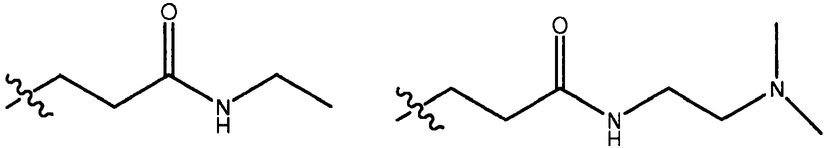

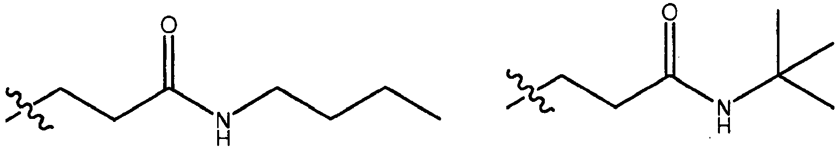

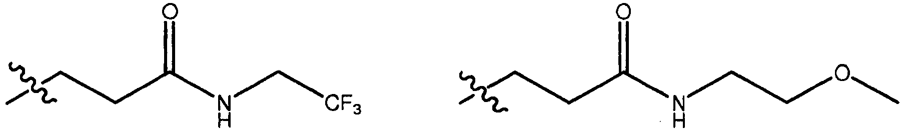

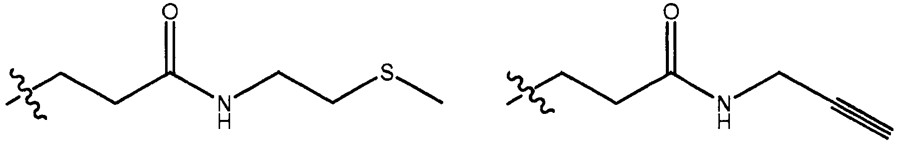

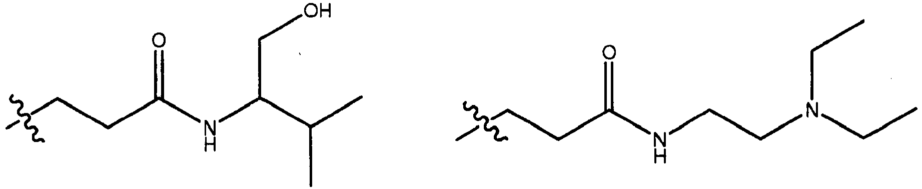

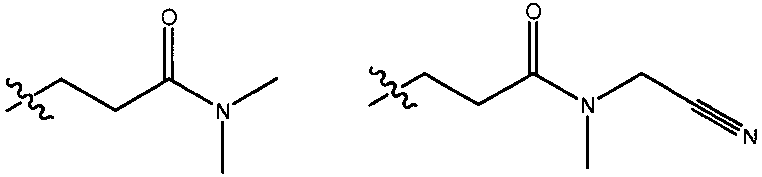

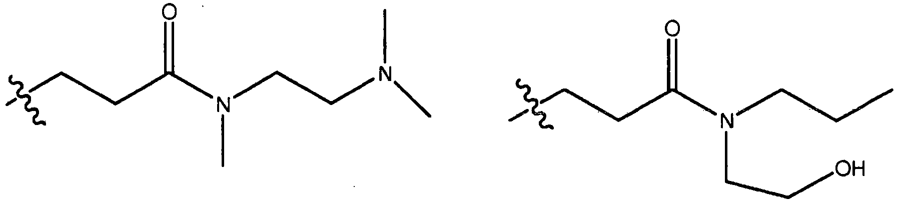

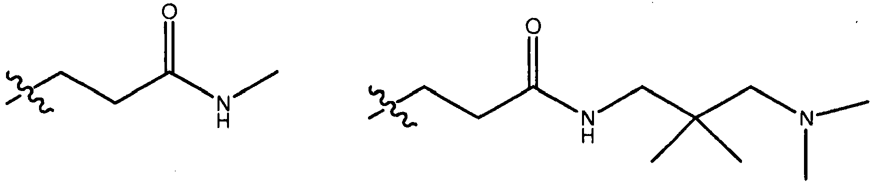

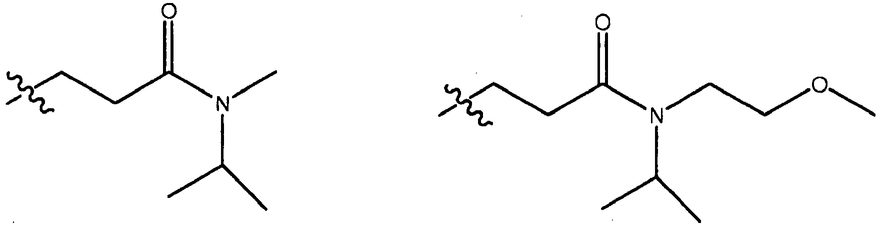

In the compounds of formula (VIII) to (Vlllc) R²⁶ and R²⁷ when taken together with the nitrogen atom to which they are attached form a heterocycloalkyl group. In one embodiment the heterocycloalkyl group is a C₅ or C₆ heterocycloalkyl group. Specific values include a piperidinyl, a piperazinyl or a morpholinyl group, each of which may be optionally substituted.

In the compounds of formula (VIII) to (Vlllc), R¹ can be alkyl, aryl, heteroaryl or arylalkyl.

There are a number of specific values of other substituents that are common to the compound of formula I to VIII.

In the group of compounds on which the compounds of the invention are based, if a group such as R¹ or R² is substituted, particularly preferred substituents are selected from the group consisting of: halogen, =O, =S, -CN, -NO₂ alkyl, alkenyl, methylendioxy, heteroalkyl, haloalkyl, alkynyl, aryl, cycloalkyl, heterocycloalkyl, heteroaryl, hydroxy, hydroxyalkyl, alkoxy, alkylamino, aminoalkyl, acylamino, phenoxy, alkoxyalkyl, benzyloxy, alkylsulfonyl, arylsulfonyl, aminosulfonyl, -C(O)OR₅, COOH, SH, and acyl.

If Y is present it is preferably at the 4 or 7 positions of the aromatic ring.
p is preferably 0.
R³ is preferably H, C₁-C₆ alkyl, or acyl, more preferably H or C₁-C₄ alkyl, most preferably H;
R⁴ is preferably H or C₁-C₄ alkyl, most preferably H;
R⁵ is preferably C₁-C₄ alkyl, heteroalkyl, or acyl, most preferably methyl;
R⁶ is preferably C₁-C₄ alkyl, heteroalkyl or acyl, most preferably C₁-C₄ alkyl;
R⁷ is preferably C₁-C₄ alkyl, heteroalkyl or acyl, most preferably C₁-C₄ alkyl;
R⁸ and R⁹ are preferably selected from the group consisting of H, C₁-C₆ alkyl, C₄-C₉cycloalkyl, C₄-C₉heterocycloalkyl, aryl, heteroaryl, arylalkyl, and heteroarylalkyl

The Z moiety can be a group of formula -CH=CH-. The moiety can be in the "E" configuration and can be at the 5 or 6 position. The Z moiety can be at the 5 position. The Z moiety can be at the 6 position.

In addition to compounds of the invention as described above the embodiments disclosed are also directed to pharmaceutically acceptable salts. Such compounds, including salts, are at times collectively referred to herein as "HDAC inhibiting agents" or "HDAC inhibitors". In certain embodiments the compounds disclosed are used to modify deacetylase activity, in some cases histone deacetylase activity and in some cases HDAC 8, or HDAC 1 activity.

The embodiments disclosed also relate to pharmaceutical compositions each comprising a therapeutically effective amount of a HDAC inhibiting agent of the embodiments described with a pharmaceutically acceptable carrier or diluent for treating cellular proliferative ailments. The term "effective amount" as used herein indicates an amount necessary to administer to a host to achieve a therapeutic result, e.g., inhibition of proliferation of malignant cancer cells, benign tumor cells or other proliferative cells.

The invention also relates to pharmaceutical compositions including a compound of the invention with a pharmaceutically acceptable carrier, diluent or excipient.

The invention also provides agents for the treatment of a disorder caused by, associated with or accompanied by disruptions of cell proliferation and/or angiogenesis including a compound of formula (Ic) as disclosed herein. In one embodiment the agent is an anti-cancer agent. In another embodiment, the agent is an anti-angiogenesis agent.

The invention also relates to the use of compounds of formula: (Ic) in the preparation of a medicament for the treatment of a disorder caused by, associated with or accompanied by disruptions of cell proliferation and/or angiogenesis. In one embodiment he disorder is a proliferative disorder, such as a cancer.

The compounds of the present invention surprisingly show low toxicity, together with a potent anti-proliferative activity.

In yet a further embodiment the invention relates to the use of the compounds of formula (Ic) for the treatment of a disorder, disease or condition that can be treated by the inhibition of histone deacetylase including administration of a therapeutically effective amount of a compound of formula (Ic).

In one embodiment the disorder is selected from the group consisting of but not limited to Proliferative disorders (e.g. cancer); Neurodegenerative diseases including Huntington's Disease, Polyglutamine diseases, Parkinson's Disease, Alzheimer's Disease, Seizures, Striatonigral degeneration, Progressive supranuclear palsy, Torsion dystonia, Spasmodic torticollis and dyskinesis, Familial tremor, Gilles de la Tourette syndrome, Diffuse Lewy body disease, Pick's disease, Intracerebral haemorrhage Primary lateral sclerosis, Spinal muscular atrophy, Amyotrophic lateral sclerosis, Hypertrophic interstitial polyneuropathy, Retinitis pigmentosa, Hereditary optic atrophy, Hereditary spastic paraplegia, Progressive ataxia and Shy-Drager syndrome; Metabolic diseases including Type 2 diabetes; Degenerative Diseases of the Eye including Glaucoma, Age-related macular degeneration, macular myopic degeneration, Rubeotic glaucoma, Interstitial keratitis, Diabetic retinopathy, Peter's anomaly retinal degeneration, Cellophane Retinopathy; Cogan's Dystrophy; Corneal Dystrophy; Iris Neovascularization (Rubeosis); Neovascularization of the Cornea; Retinopathy of Prematurity; Macular Edema; Macular Hole; Macular Pucker; Marginal Blepharitis, Myopia, nonmalignant growth of the conjunctiva; Inflammatory diseases and/or Immune system disorders including Rheumatoid Arthritis (RA), Osteoarthritis, Juvenile chronic arthritis, Graft versus Host disease, Psoriasis, Asthma, Spondyloarthropathy, Crohn's Disease, inflammatory bowel disease, Colitis Ulcerosa, Alcoholic hepatitis, Diabetes, Sjoegrens's syndrome, Multiple Sclerosis, Ankylosing spondylitis, Membranous glomerulopathy, Discogenic pain, Systemic Lupus Erythematosus, allergic contact dermatitis; Disease involving angiogenesis including cancer, psoriasis, rheumatoid arthritis; Psychological disorders including bipolar disease, schizophrenia, depression and dementia; Cardiovascular Diseases including Heart failure, restenosis, cardiac hypertrophy and arteriosclerosis; Fibrotic diseases including liver fibrosis, lung fibrosis, cystic fibrosis and angiofibroma; Infectious diseases including Fungal infections, such as *Candida Albicans,* Bacterial infections, Viral infections, such as Herpes Simplex, Protozoal infections, such as Malaria, Leishmania infection, Trypanosoma brucei infection, Toxoplasmosis and coccidiosis, and Haematopoietic disorders including thalassemia, anemia and sickle cell anemia.

The invention also provides agents for the treatment of a disorder, disease or condition that can be treated by the inhibition of histone deacetylase including a compound of formula (Ic) as disclosed herein. In one embodiment the agent is an anti-cancer agent.

The invention also relates to the use of compounds of formula (Ic) in the preparation of a medicament for the treatment of a disorder, disease or condition that can be treated by the inhibition of histone deacetylase.

The invention also provides agents for inhibiting cell proliferation including a compound of formula (Ic) as disclosed herein.

The invention also relates to the use of compounds of formula (Ic) in the preparation of a medicament for inhibiting cell proliferation.

The invention also provides agents for the treatment of neurodegenerative disorder including a compound of formula (Ic) as disclosed herein. In one embodiment the agent is an anti-Huntington's disease agent.

The invention also relates to the use of compounds of formula (Ic) in the preparation of a medicament for the treatment of a neurodegenerative disorder. In one embodiment the neurodegenerative disorder is Huntington's Disease.

The invention also provides agents for the treatment of inflammatory disease and/or immune system disorder including a compound of formula (Ic) as disclosed herein.

The invention also relates to the use of compounds of formula (Ic) in the preparation of a medicament for the treatment of inflammatory disease and/or immune system disorder. In one embodiment the inflammatory disease and/or immune system disorder is rheumatoid arthritis. In another embodiment the inflammatory disease and/or immune system disorder is Systemic Lupus Erythematosus.

The invention also provides agents for the treatment of eye disease mediated by HDAC inhibition including a compound of formula (Ic). In one embodiment, the eye disease is macular degeneration. In another embodiment, the eye disease is glaucoma. In another embodiment, the eye disease is retinal degeneration.

The invention also relates to the use of compounds of formula (Ic) in the preparation of a medicament for the treatment of eye disease mediated by HDAC inhibition. The compound used can be a compound of formula (Ia), or a compound of formula (Ib). In one embodiment, the eye disease is macular degeneration. In another embodiment, the eye disease is glaucoma. In another embodiment, the eye disease is retinal degeneration.

### DETAILED DESCRIPTION OF THE EMBODIMENTS

There are disclosed hydroxamate compounds, for example imidazo[1,2-a]pyridine containing hydroxamic acid in one of the substituents, that may be inhibitors of deacetylases, including but not limited to inhibitors of histone deacetylases. The hydroxamate compounds may be suitable for prevention or treatment of a disorder caused by, associated with or accompanied by disruptions of cell proliferation and/or angiogenesis when used either alone or together with a pharmaceutically acceptable carrier, diluent or excipient. An example of such a disorder is cancer.

As used herein the term 'cancer' is a general term intended to encompass the vast number of conditions that are characterised by uncontrolled abnormal growth of cells.

It is anticipated that the compounds of the invention will be useful in treating various cancers including but not limited to bone cancers including Ewing's sarcoma, osteosarcoma, chondrosarcoma and the like, brain and CNS tumours including acoustic neuroma, neuroblastomas, glioma and other brain tumours, spinal cord tumours, breast cancers, colorectal cancers, advanced colorectal adenocarcinomas, colon cancers, endocrine cancers including adenocortical carcinoma, pancreatic cancer, pituitary cancer, thyroid cancer, parathyroid cancer, thymus cancer, multiple endocrine neoplasma, gastrointestinal cancers including stomach cancer, esophageal cancer, small intestine cancer, Liver cancer, extra hepatic bile duct cancer, gastrointestinal carcinoid tumour, gall bladder cancer, genitourinary cancers including testicular cancer, penile cancer, prostate cancer, gynaecological cancers including cervical cancer, ovarian cancer, vaginal cancer, uterus/endometrium cancer, vulva cancer, gestational trophoblastic cancer, fallopian tube cancer, uterine sarcoma, head and neck cancers including oral cavity cancer, lip cancer, salivary gland cancer, larynx cancer, hypopharynx cancer, orthopharynx cancer, nasal cancer, paranasal cancer, nasopharynx cancer, leukemias including childhood leukemia, acute lymphocytic leukemia, acute myeloid leukemia, chronic lymphocytic leukemia, chronic myeloid leukemia, hairy cell leukemia, acute promyelocytic leukemia, plasma cell leukemia, myelomas, haematological disorders including myelodysplastic syndromes, myeloproliferative disorders, aplastic anemia, Fanconi anemia, Waldenstroms Macroglobulinemia, lung cancers including small cell lung cancer, non-small cell lung cancer, lymphomas including Hodgkin's disease, non-Hodgkin's lymphoma, cutaneous T-cell lymphoma, peripheral T-cell lymphoma, AIDS related Lymphoma, B-cell lymphoma, Burkitt's lymphoma; eye cancers including retinoblastoma, intraocular melanoma, skin cancers including melanoma, non-melanoma skin cancer, merkel cell cancer, soft tissue sarcomas such as childhood soft tissue sarcoma, adult soft tissue sarcoma, Kaposi's sarcoma, urinary system cancers including kidney cancer, Wilms tumour, bladder cancer, urethral cancer, and transitional cell cancer.

Exemplary cancers that may be treated by the compounds of the present invention are breast cancer, lung cancer, ovarian cancer, prostate cancer, head and neck cancer, renal cancer (e.g. renal cell carcinoma), gastric cancer, colon cancer, colon cancer, colorectal cancer and brain cancer.

Exemplary cancers that may be treated by compounds of the present invention include but are not limited to B-cell lymphoma (e.g. Burkitt's lymphoma), leukemias (e.g. Acute promyelocytic leukemia), cutaneous T-cell lymphoma (CTCL) and peripheral T-cell lymphoma.

Exemplary cancers that may be treated by compounds of the present invention include solid tumors and hematologic malignancies.

The compounds may also be used in the treatment of a disorder involving, relating to or, associated with dysregulation of histone deacetylase (HDAC).

There are a number of disorders that have been implicated by or known to be mediated at least in part by HDAC activity, where HDAC activity is known to play a role in triggering disease onset, or whose symptoms are known or have been shown to be alleviated by HDAC inhibitors. Disorders of this type that would be expected to be amenable to treatment with the compounds of the invention include the following but not limited to: Proliferative disorders (e.g. cancer); Neurodegenerative diseases including Huntington's Disease, Polyglutamine diseases, Parkinson's Disease, Alzheimer's Disease, Seizures, Striatonigral degeneration, Progressive supranuclear palsy, Torsion dystonia, Spasmodic torticollis and dyskinesis, Familial tremor, Gilles de la Tourette syndrome, Diffuse Lewy body disease, Pick's disease, Intracerebral haemorrhage Primary lateral sclerosis, Spinal muscular atrophy, Amyotrophic lateral sclerosis, Hypertrophic interstitial polyneuropathy, Retinitis pigmentosa, Hereditary optic atrophy, Hereditary spastic paraplegia, Progressive ataxia and Shy-Drager syndrome,; Metabolic diseases including Type 2 diabetes; Degenerative Diseases of the Eye including Glaucoma, Age-related macular degeneration, macular myopic degeneration, Rubeotic glaucoma, Interstitial keratitis, Diabetic retinopathy, Peter's anomaly retinal degeneration, Cellophane Retinopathy; Cogan's Dystrophy; Corneal Dystrophy; Iris Neovascularization (Rubeosis); Neovascularization of the Cornea; Retinopathy of Prematurity; Macular Edema; Macular Hole; Macular Pucker; Marginal Blepharitis, Myopia, nonmalignant growth of the conjunctiva; Inflammatory diseases and/or Immune system disorders including Rheumatoid Arthritis (RA), Osteoarthritis, Juvenile chronic arthritis, Graft versus Host disease, Psoriasis, Asthma, Spondyloarthropathy, Crohn's Disease, inflammatory bowel disease, Colitis Ulcerosa, Alcoholic hepatitis, Diabetes, Sjoegrens's syndrome, Multiple Sclerosis, Ankylosing spondylitis, Membranous glomerulopathy, Discogenic pain, Systemic Lupus Erythematosus, allergic contact dermatitis; Disease involving angiogenesis including cancer, psoriasis, rheumatoid arthritis; Psychological disorders including bipolar disease, schizophrenia, depression and dementia; Cardiovascular Diseases including Heart failure, restenosis, cardiac hypertrophy and arteriosclerosis; Fibrotic diseases including liver fibrosis, lung fibrosis, cystic fibrosis and angiofibroma; Infectious diseases including Fungal infections, such as *Candida Albicans,* Bacterial infections, Viral infections, such as Herpes Simplex, Protozoal infections, such as Malaria, Leishmania infection, Trypanosoma brucei infection, Toxoplasmosis and coccidiosis, and Haematopoietic disorders including thalassemia, anemia and sickle cell anemia.

As used herein, the term unsubstituted means that there is no substituent or that the only substituents are hydrogen.

The term "optionally substituted" as used throughout the specification denotes that the group may or may not be further substituted or fused (so as to form a condensed polycyclic system), with one or more non-hydrogen substituent groups. Preferably the substituent groups are one or more groups independently selected from the group consisting of halogen, =O, =S, -CN, -NO₂, -CF₃, -OCF₃, alkyl, alkenyl, alkynyl, haloalkyl, haloalkenyl, haloalkynyl, heteroalkyl, cycloalkyl, cycloalkenyl, heterocycloalkyl, heterocycloalkenyl, aryl, heteroaryl, cycloalkylalkyl, heterocycloalkylalkyl, heteroarylalkyl, arylalkyl, cycloalkylalkenyl, heterocycloalkylalkenyl, arylalkenyl, heteroarylalkenyl, cycloalkylheteroalkyl, heterocycloalkylheteroalkyl, arylheteroalkyl, heteroarylheteroalkyl, hydroxy, hydroxyalkyl, alkoxy, alkoxyalkyl, alkoxycycloalkyl, alkoxyheterocycloalkyl, alkoxyaryl, alkoxyheteroaryl, alkoxycarbonyl, alkylaminocarbonyl, alkenyloxy, alkynyloxy, cycloalkyloxy, cycloalkenyloxy, heterocycloalkyloxy, heterocycloalkenyloxy, aryloxy, phenoxy, benzyloxy, heteroaryloxy, arylalkyloxy, arylalkyl, heteroarylalkyl, cycloalkylalkyl, heterocycloalkylalkyl, arylalkyloxy, amino, alkylamino, acylamino, aminoalkyl, arylamino, sulfonylamino, sulfinylamino, sulfonyl, alkylsulfonyl, arylsulfonyl, aminosulfonyl, sulfinyl, alkylsulfinyl, arylsulfinyl, aminosulfinylaminoalkyl, -COOH, -COR⁶, -C(O)OR⁶, CONHR⁶, NHCOR⁶, NHCOOR⁶, NHCONHR⁶, C(=NOH)R⁶, -SH, -SR⁶, -OR⁶ and acyl. Substituent groups themselves may be further optionally substituted.

"Halogen" represents chlorine, fluorine, bromine or iodine.

"Alkyl" as a group or part of a group refers to a straight or branched aliphatic hydrocarbon group, preferably a C₁-C₁₄ alkyl, more preferably C₁-C₁₀ alkyl, most preferably C₁-C₆ unless otherwise noted. Examples of suitable straight and branched C₁-C₆ alkyl substituents include methyl, ethyl, n-propyl, 2-propyl, n-butyl, sec-butyl, t-butyl, hexyl, and the like.

"Alkylamino" includes both monoalkylamino and dialkylamino, unless specified. "Monoalkylamino" means a -NH-Alkyl group, in which alkyl is as defined above. "Dialkylamino" means a -N(alkyl)₂ group, in which each alkyl may be the same or different and are each as defined herein for alkyl. The alkyl group is preferably a C₁-C₆ alkyl group.

"Arylamino" includes both mono-arylamino and di-arylamino unless specified. Mono-arylamino means a group of formula aryl NH- in which aryl is as defined herein, di-arylamino means a group of formula (aryl₂)N- where each aryl may be the same or different and each are as defined herein for aryl.

"Acyl" means an alkyl-CO- group in which the alkyl group is as described herein. Examples of acyl include acetyl and benzoyl. The alkyl group is preferably a C₁-C₆ alkyl group.

"Alkenyl" as group or part of a group denotes an aliphatic hydrocarbon group containing at least one carbon-carbon double bond and which may be straight or branched preferably having 2-14 carbon atoms, more preferably 2-12 carbon atoms, most preferably 2-6 carbon atoms, in the chain. The group may contain a plurality of double bonds in the normal chain and the orientation about each is independently E or Z. Exemplary alkenyl group include, but are not limited to, ethenyl and propenyl.

"Alkoxy" refers to an -O-alkyl group in which alkyl is defined herein. Preferably the alkoxy is a C₁-C₆alkoxy. Examples include, but are not limited to, methoxy and ethoxy.

"Alkenyloxy" refers to an -O- alkenyl group in which alkenyl is as defined herein. Preferred alkenyloxy groups are C₁-C₆ alkenyloxy groups.

"Alkynyloxy" refers to an -O-alkynyl group in which alkynyl is as defined herein. Preferred alkynyloxy groups are C₁-C₆ alkynyloxy groups.

"Alkoxycarbonyl" refers to an -C(O)-O-alkyl group in which alkyl is as defined herein. The alkyl group is preferably a C₁-C₆ alkyl group. Examples include, but not limited to, methoxycarbonyl and ethoxycarbonyl.

"Akylsulfinyl" means a -S(O)-alkyl group in which alkyl is as defined above. The alkyl group is preferably a C₁-C₆ alkyl group. Exemplary alkylsulfinyl groups include, but not limited to, methylsulfinyl and ethylsulfinyl.

"Alkylsulfonyl" refers to a -S(O)₂-alkyl group in which alkyl is as defined above. The alkyl group is preferably a C₁-C₆ alkyl group. Examples include, but not limited to methylsulfonyl and ethylsulfonyl.

"Alkynyl" as a group or part of a group means an aliphatic hydrocarbon group containing a carbon-carbon triple bond and which may be straight or branched preferably having from 2-14 carbon atoms, more preferably 2-12 carbon atoms in the chain, preferably 2-6 carbon atoms in the chain. Exemplary structures include, but are not limited to, ethynyl and propynyl.

"Alkylaminocarbonyl" refers to an alkylamino-C(O)- group in which alkylamino is as defined above.

"Cycloalkyl" refers to a saturated or partially saturated, monocyclic or fused or spiro polycyclic, carbocycle preferably containing from 3 to 9 carbons per ring, such as cyclopropyl, cyclobutyl, cyclopentyl, cyclohexyl and the like, unless otherwise specified. It includes monocyclic system such as cyclohexyl, bicyclic systems such as decalin, and polycyclic systems such as adamantane.

"Cycloalkylalkyl" means a cycloalkyl-alkyl- group in which the cycloalkyl and alkyl moieties are as previously described. Exemplary monocycloalkylalkyl groups include cyclopropylmethyl, cyclopentylmethyl, cyclohexylmethyl and cycloheptylmethyl.

"Heterocycloalkyl" refers to a saturated or partially saturated monocyclic, bicyclic or polycyclic ring containing at least a heteroatom selected from nitrogen, sulfur, oxygen, preferably from 1 to 3 heteroatoms in at least one ring. Each ring is preferably from 3 to 10 membered, more preferably 4 to 7 membered. Examples of suitable heterocycloalkyl substituents include pyrrolidyl, tetrahydrofuryl, tetrahydrothiofuranyl, piperidyl, piperazyl, tetrahydropyranyl, morphilino, 1,3-diazapane, 1,4-diazapane, 1,4-oxazepane, and 1,4-oxathiapane.

"Heterocycloalkenyl" refers to a heterocycloalkyl as described above but containing at least one double bond.

"Heterocycloalkylalkyl" refers to a heterocycloalkyl-alkyl group in which the heterocycloalkyl and alkyl moieties are as previously described. Exemplary heterocycloalkylalkyl groups include (2-tetrahydrofuryl)methyl, (2-tetrahydrothiofuranyl)methyl.

"Heteroalkyl" refers to a straight- or branched-chain alkyl group preferably having from 2 to 14 carbons, more preferably 2 to 10 atoms in the chain, one or more of which has been replaced by a heteroatom selected from S, O, and N. Exemplary heteroalkyls include alkyl ethers, secondary and tertiary alkyl amines, alkyl sulfides, and the like.

"Aryl" as a group or part of a group denotes (i) an optionally substituted monocyclic, or fused polycyclic, aromatic carbocycle (ring structure having ring atoms that are all carbon) preferably having from 5 to 12 atoms per ring. Examples of aryl groups include phenyl, naphthyl, and the like; (ii) an optionally substituted partially saturated bicyclic aromatic carbocyclic moiety in which a phenyl and a C₅₋₇ cycloalkyl or C₅₋₇ cycloalkenyl group are fused together to form a cyclic structure, such as tetrahydronaphthyl, indenyl or indanyl.

"Arylalkenyl" means an aryl-alkenyl- group in which the aryl and alkenyl are as previously described. Exemplary arylalkenyl groups include phenylallyl.

"Arylalkyl" means an aryl-alkyl- group in which the aryl and alkyl moieties are as previously described. Preferred arylalkyl groups contain a C₁₋₅ alkyl moiety. Exemplary arylalkyl groups include benzyl, phenethyl and naphthelenemethyl.

"Cycloalkenyl" means an optionally substituted non-aromatic monocyclic or polycyclic ring system containing at least one carbon-carbon double bond and preferably having from 5-10 carbon atoms per ring. Exemplary monocyclic cycloalkenyl rings include cyclopentenyl, cyclohexenyl or cycloheptenyl.

The term "heteroaryl" either alone or part of another group refers to groups containing an aromatic ring (preferably a 5 or 6 membered aromatic ring) having 1 or more heteroatoms as ring atoms in the aromatic ring with the remainder of the ring atoms being carbon atoms. Suitable heteroatoms include oxygen, sulfur, and nitrogen. Examples of heteroaryl include thiophene, benzothiophene, benzofuran, benzimidazole, benzoxazole, benzothiazole, benzisothiazole, naphtho[2,3-b]thiophene, furan, isoindolizine, xantholene, phenoxatine, pyrrole, imidazole, pyrazole, pyridine, pyrazine, pyrimidine, pyridazine, indole, isoindole, 1 H-indazole, purine, 4H-quinolidine, isoquinoline, quinoline, phthalazine, naphthyridine, quinoxaline, quinazoline, cinnoline, carbazole, phenanthridine, acridine, phenazine, thiazole, isothiazole, phenothiazine, oxazole, isoxazole, furazane, phenoxazine, 2-, 3-, or 4-pyridyl, 2-, 3-, 4-, 5-, or 8-quinolyl, 1-, 3-, 4-, or 5-isoquinolyl, 1-, 2-, or 3-indolyl, 2-benzothiazolyl, 2-benzo[b]thienyl, benzo[b]furanyl, 2- or 3-thienyl, or the like. More preferred examples include 2- or 3-thienyl, 2-, 3-, or 4-pyridyl, 2- or 3-quinolyl, 1-isoquinolyl, 1- or 2-indolyl, 2-benzothiazolyl, and the like.

"Heteroarylalkyl" means a heteroaryl-alkyl group in which the heteroaryl and alkyl moieties are as previously described. Preferred heteroarylalkyl groups contain a C₁ to C₆ alkyl moiety. Exemplary heteroarylalkyl groups include pyridylmethyl.

"Lower alkyl" as a group means unless otherwise specified, an aliphatic hydrocarbon group which may be straight or branched having 1 to 6 carbon atoms in the chain, more preferably 1 to 4 carbons such as methyl, ethyl, propyl (n-propyl or isopropyl) or butyl (n-butyl, isobutyl or tertiary-butyl).

In Formula (I), as well as in Formulae (1a)-(Ic) defining sub-sets of compounds within Formula (I), there is shown an imidazo[1,2-a]pyridine ring system. Within this ring system, there are substitutable positions at the 4-,5-, 6-, and 7-ring positions. In each of Formulae (I), (1a), (Ib) and (Ic), there is a requirement for attachment of an acidic moiety at one of the ring positions. This acidic moiety may be provided by but is not limited to groups containing, a hydroxamic acid or salt derivatives of such acid which when hydrolyzed would provide the acidic moiety. In some embodiments the acidic moiety may be attached to the ring position through an alkylene group such as -CH₂- or -CH₂CH₂-, or an alkenyl group such as -CH=CH-. Preferred positions for attachment of the acidic moiety are the 5- and 6-ring positions.

It is understood that included in the family of compounds of Formula (I), preferably Formula (Ic), are isomeric forms including diastereoisomers, enantiomers, tautomers, and geometrical isomers in "E" or "Z" configurational isomer or a mixture of E and Z isomers. It is also understood that some isomeric forms such as diastereomers, enantiomers, and geometrical isomers can be separated by physical and/or chemical methods and by those skilled in the art.

Some of the compounds of the disclosed embodiments may exist as single stereoisomers, racemates, and/or mixtures of enantiomers and/or diastereomers. All such single stereoisomers, racemates and mixtures thereof are intended to be within the scope of the subject matter described and claimed.

Additionally, Formula (I), preferably Formula (Ic), is intended to cover, where applicable, solvated as well as unsolvated forms of the compounds. Thus, each formula includes compounds having the indicated structure, including the hydrated as well as the non-hydrated forms.

In addition to compounds of the Formula (I), preferably Formula (Ic), the HDAC inhibiting agents of the various embodiments include pharmaceutically acceptable salts.

The term "Pharmaceutically acceptable salts" refers to salts that retain the desired biological activity of the above-identified compounds, and include pharmaceutically acceptable acid addition salts and base addition salts. Suitable pharmaceutically acceptable acid addition salts of compounds of Formula (I), preferably Formula (Ic), may be prepared from an inorganic acid or from an organic acid. Examples of such inorganic acids are hydrochloric, sulfuric, and phosphoric acid. Appropriate organic acids may be selected from aliphatic, cycloaliphatic, aromatic, heterocyclic carboxylic and sulfonic glasses of organic acids, examples of which are formic, acetic, propionic, succinic, glycolic, gluconic, lactic, malic, tartaric, citric, fumaric, maleic, alkyl sulfonic, arylsulfonic. Suitable pharmaceutically acceptable base addition salts of compounds of Formula (I) include metallic salts made from lithium, sodium, potassium, magnesium, calcium, aluminium, and zinc, and organic salts made from organic bases such as choline, diethanolamine, morpholine. Other examples of organic salts are: ammonium salts, quaternary salts such as tetramethylammonium salt; amino acid addition salts such as salts with glycine and arginine. Additional information on pharmaceutically acceptable salts can be found in Remington's Pharmaceutical Sciences, 19th Edition, Mack Publishing Co., Easton, PA 1995. In the case of agents that are solids, it is understood by those skilled in the art that the inventive compounds, agents and salts may exist in different crystalline or polymorphic forms, all of which are intended to be within the scope of the present invention and specified formulae.

Preferred HDAC inhibiting agents include those having an IC₅₀ value of 10 µM or less.

Administration of compounds within Formula (I), preferably Formula (Ic), to humans can be by any of the accepted modes for enteral administration such as oral or rectal, or by parenteral administration such as subcutaneous, intramuscular, intravenous and intradermal routes. Injection can be bolus or via constant or intermittent infusion. The active compound is typically included in a pharmaceutically acceptable carrier or diluent and in an amount sufficient to deliver to the patient a therapeutically effective dose. In various embodiments the inhibitor compound may be selectively toxic or more toxic to rapidly proliferating cells, e.g. cancerous tumors, than to normal cells.

The term "therapeutically effective amount" or "effective amount" is an amount sufficient to effect beneficial or desired results. An effective amount can be administered in one or more administrations. An effective amount is typically sufficient to palliate, ameliorate, stabilize, reverse, slow or delay the progression of the disease state. A therapeutically effective amount can be readily determined by a skilled practitioner by the use of conventional techniques and by observing results obtained in analogous circumstances. In determining the effective amount a number of factors are considered including the species of the patient, its size, age, general health, the specific disease involved, the degree or severity of the disease, the response of the individual patient, the particular compound administered, the mode of administration, the bioavailability of the compound, the dose regimen selected, the use of other medication and other relevant circumstances.

In using the compounds of the invention they can be administered in any form or mode which makes the compound bioavailable. One skilled in the art of preparing formulations can readily select the proper form and mode of administration depending upon the particular characteristics of the compound selected, the condition to be treated, the stage of the condition to be treated and other relevant circumstances. We refer the reader to Remingtons Pharmaceutical Sciences, 19th edition, Mak Publishing Co. (1995) for further information.

The compounds of the present invention can be administered alone or in the form of a pharmaceutical composition in combination with a pharmaceutically acceptable carrier, diluent or excipient. The compounds of the invention, while effective themselves, are typically formulated and administered in the form of their pharmaceutically acceptable salts as these forms are typically more stable, more easily crystallised and have increased solubility.

The compounds are, however, typically used in the form of pharmaceutical compositions which are formulated depending on the desired mode of administration. As such in a further embodiment the present invention provides a pharmaceutical composition including a compound of Formula (Ic), and a pharmaceutically acceptable carrier, diluent or excipient. The compositions are prepared in manners well known in the art.

A pharmaceutical pack or kit comprising one or more containers filled with one or more of the ingredients of the pharmaceutical compositions of the invention can be provided. In such a pack or kit can be found a container having a unit dosage of the agent(s). The kits can include a composition comprising an effective agent either as concentrates (including lyophilized compositions), which can be diluted further prior to use or they can be provided at the concentration of use, where the vials may include one or more dosages. Conveniently, in the kits, single dosages can be provided in sterile vials so that the physician can employ the vials directly, where the vials will have the desired amount and concentration of agent(s). Associated with such container(s) can be various written materials such as instructions for use, or a notice in the form prescribed by a governmental agency regulating the manufacture, use or sale of pharmaceuticals or biological products, which notice reflects approval by the agency of manufacture, use or sale for human administration.

The compounds of the invention may be used or administered in combination with one or more additional drug (s) that include chemotherapeutic drugs or HDAC inhibitor drugs and/or procedures (e.g. surgery, radiotherapy) for the treatment of the disorder/diseases mentioned. The components can be administered in the same formulation or in separate formulations. If administered in separate formulations the compounds of the invention may be administered sequentially or simultaneously with the other drug (s).

In addition to being able to be administered in combination with one or more additional drugs that include chemotherapeutic drugs or HDAC inhibitor drugs the compounds of the invention may be used in a combination therapy. When this is done the compounds are typically administered in combination with each other. Thus one or more of the compounds of the invention may be administered either simultaneously (as a combined preparation) or sequentially in order to achieve a desired effect. This is especially desirable where the therapeutic profile of each compound is different such that the combined effect of the two drugs provides an improved therapeutic result.

Pharmaceutical compositions of this invention for parenteral injection comprise pharmaceutically acceptable sterile aqueous or nonaqueous solutions, dispersions, suspensions or emulsions as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol, and the like), and suitable mixtures thereof, vegetable oils (such as olive oil), and injectable organic esters such as ethyl oleate. Proper fluidity can be maintained, for example, by the use of coating materials such as lecithin, by the maintenance of the required particle size in the case of dispersions, and by the use of surfactants.

These compositions may also contain adjuvants such as preservative, wetting agents, emulsifying agents, and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents such as sugars, sodium chloride, and the like. Prolonged absorption of the injectable pharmaceutical form may be brought about by the inclusion of agents that delay absorption such as aluminium monostearate and gelatin.

If desired, and for more effective distribution, the compounds can be incorporated into slow release or targeted delivery systems such as polymer matrices, liposomes, and microspheres.

The injectable formulations can be sterilized, for example, by filtration through a bacterial-retaining filter, or by incorporating sterilizing agents in the form of sterile solid compositions that can be dissolved or dispersed in sterile water or other sterile injectable medium just prior to use.

Solid dosage forms for oral administration include capsules, dragees, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like.

The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

If desired, and for more effective distribution, the compounds can be incorporated into slow release or targeted delivery systems such as polymer matrices, liposomes, and microspheres.

The active compounds can also be in microencapsulated form, if appropriate, with one or more of the above-mentioned excipients.

Liquid dosage forms for oral administration include pharmaceutically acceptable emulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, and perfuming agents.

Suspensions, in addition to the active compounds, may contain suspending agents as, for example, ethoxylated isostearyl alcohols, polyoxyethylene sorbitol and sorbitan esters, microcrystalline cellulose, aluminium metahydroxide, bentonite, agar-agar, and tragacanth, and mixtures thereof.

Compositions for rectal or vaginal administration are preferably suppositories which can be prepared by mixing the compounds of this invention with suitable non-irritating excipients or carriers such as cocoa butter, polyethylene glycol or a suppository wax which are solid at room temperature but liquid at body temperature and therefore melt in the rectum or vaginal cavity and release the active compound.

Dosage forms for topical administration of a compound of this invention include powders, patches, sprays, ointments and inhalants. The active compound is mixed under sterile conditions with a pharmaceutically acceptable carrier and any needed preservatives, buffers, or propellants which may be required.

A preferred dosage will be a range from about 0.01 to 300 mg per kilogram of body weight per day. A more preferred dosage will be in the range from 0.1 to 100 mg per kilogram of body weight per day, more preferably from 0.2 to 80 mg per kilogram of body weight per day, even more preferably 0.2 to 50 mg per kilogram of body weight per day. A suitable dose can be administered in multiple sub-doses per day.

As discussed above, the compounds of the embodiments disclosed inhibit histone deacetylases. The enzymatic activity of a histone deacetylase can be measured using known methodologies [Yoshida M. et al, J. Biol. Chem., 265, 17174 (1990), J. Taunton et al, Science 1996 272: 408]. In certain embodiments, the histone deacetylase inhibitor interacts with and/or reduces the activity of more than one known histone deacetylase in the cell, which can either be from the same class of histone deacetylase or different class of histone deacetylase. In some other embodiments, the histone deacetylase inhibitor interacts and reduces the activity of predominantly one histone deacetylase, for example HDAC-1, HDAC-2, HDAC-3 or HDAC-8, which belongs to Class I HDAC enzymes [De Ruijter A.J.M. et al, Biochem. J., 370, 737-749 (2003)]. HDACs can also target non-histone substrates to regulate a variety of biological functions implicated in disease pathogenesis. These non-histone substrates include Hsp90, α-tubulin, p53, NFkb and HIF1a [Drummond et al., Annu. Rev. Pharmacol. Toxicol. 45:495 (2004)].. Certain preferred histone deacetylase inhibitors are those that interact with, and/or reduce the activity of a histone deacetylase which is involved in tumorigenesis, and these compounds may be useful for treating proliferative diseases. Examples of such cell proliferative diseases or conditions include cancer (include any metastases), psoriasis, and smooth muscle cell proliferative disorders such as restenosis. The inventive compounds may be particularly useful for treating tumors such as breast cancer, colon cancer, lung cancer, ovarian cancer, prostate cancer, head and/or neck cancer, or renal, gastric, pancreatic cancer and brain cancer as well as hematologic malignancies such as lymphoma and leukemias. In addition, the inventive compounds may be useful for treating a proliferative disease that is refractory to the treatment with other chemotherapeutics; and for treating hyperproliferative condition such as leukemias, psoriasis and restenosis. In other embodiments, compounds of this invention can be used to treat pre-cancer conditions or hyperplasia including familial adenomatous polyposis, colonic adenomatous polyps, myeloid dysplasia, endometrial dysplasia, endometrial hyperplasia with atypia, cervical dysplasia, vaginal intraepithelial neoplasia, benign prostatic hyperplasia, papillomas of the larynx, actinic and solar keratosis, seborrheic keratosis and keratoacanthoma.

Additionally compounds of the various embodiments disclosed herein may be useful for treating neurodegenerative diseases, and inflammatory diseases and/or immune system disorders.

The disorder is preferably selected from the group consisting of cancer, inflammatory diseases and/or immune system disorders (e.g. rheumatoid arthritis, systemic lupus erythematosus), angiofibroma, cardiovascular diseases, fibrotic diseases, diabetes, autoimmune diseases, chronic and acute neurodegenerative disease like Huntington's disease, Parkinson's disease, disruptions of nerval tissue and infectious diseases like fungal, bacterial and viral infections. In another embodiment the disorder is a proliferative disorder.

The histone deacetylase inhibitors of the invention have significant antiproliferative effects and promote differentiation, cell cycle arrest in the G1 or G2 phase, and induce apoptosis.

### SYNTHESIS OF DEACETYLASE INHIBITORS

The agents of the various embodiments may be prepared using the reaction routes and synthesis schemes as described below, employing the techniques available in the art using starting materials that are readily available. The preparation of particular compounds of the embodiments is described in detail in the following examples, but the artisan will recognize that the chemical reactions described may be readily adapted to prepare a number of other agents of the various embodiments. For example, the synthesis of non-exemplified compounds may be successfully performed by modifications apparent to those skilled in the art, e.g. by appropriately protecting interfering groups, by changing to other suitable reagents known in the art, or by making routine modifications of reaction conditions. A list of suitable protecting groups in organic synthesis can be found in T.W. Greene and P. G. M. Wuts' Protective Groups in Organic Synthesis, 3rd Edition, Wiley-InterScience, 1999. Alternatively, other reactions disclosed herein or known in the art will be recognized as having applicability for preparing other compounds of the various embodiments.

Reagents useful for synthesizing compounds may be obtained or prepared according to techniques known in the art.

In the examples described below, unless otherwise indicated, all temperatures in the following description are in degrees Celsius and all parts and percentages are by weight, unless indicated otherwise.

Various starting materials and other reagents were purchased from commercial suppliers, such as Aldrich Chemical Company or Lancaster Synthesis Ltd., and used without further purification, unless otherwise indicated. Tetrahydrofuran (THF) and N,N-dimethylformamide (DMF) were purchased from Aldrich in SureSeal bottles and used as received. All solvents were purified by using standard methods in the art, unless otherwise indicated.

The reactions set forth below were performed under a positive pressure of nitrogen, argon or with a drying tube, at ambient temperature (unless otherwise stated), in anhydrous solvents, and the reaction flasks are fitted with rubber septa for the introduction of substrates and reagents via syringe. Glassware was oven-dried and/or heat-dried. Analytical thin-layer chromatography was performed on glass-backed silica gel 60 F 254 plates (E Merck (0.25 mm)) and eluted with the appropriate solvent ratios (v/v). The reactions were assayed by TLC and terminated as judged by the consumption of starting material.

The TLC plates were visualized by UV absorption or with a p-anisaldehyde spray reagent or a phosphomolybdic acid reagent (Aldrich Chemical, 20wt% in ethanol) which was activated with heat, or by staining in iodine chamber. Work-ups were typically done by doubling the reaction volume with the reaction solvent or extraction solvent and then washing with the indicated aqueous solutions using 25% by volume of the extraction volume (unless otherwise indicated). Product solutions were dried over anhydrous sodium sulfate prior to filtration, and evaporation of the solvents was under reduced pressure on a rotary evaporator and noted as solvents removed in vacuo. Flash column chromatography [Still et al, J. Org. Chem., 43, 2923 (1978)] was conducted using E Merck-grade flash silica gel (47-61 mm) and a silica gel:crude material ratio of about 20:1 to 50:1, unless otherwise stated. Hydrogenolysis was done at the pressure indicated or at ambient pressure.

1 H NMR spectra was recorded on a Bruker instrument operating at 400 MHz, and ¹³C-NMR spectra was recorded operating at 100 MHz. NMR spectra are obtained as CDCl₃ solutions (reported in ppm), using chloroform as the reference standard (7.25 ppm and 77.00 ppm) or CD₃OD (3.4 and 4.8 ppm and 49.3 ppm), or an internal tetramethylsilane standard (0.00 ppm) when appropriate. Other NMR solvents were used as needed. When peak multiplicities are reported, the following abbreviations are used: s = singlet, d = doublet, t = triplet, m = multiplet, br = broadened, dd = doublet of doublets, dt = doublet of triplets. Coupling constants, when given, are reported in Hertz.

Mass spectra were obtained using LC/MS either in ESI or APCI. All melting points are uncorrected.

All final products had greater than 90% purity (LC/PDA: Xterra 1S column, 4.6 x 20mm 3.5µ column; 2.0 ml/min, gradient 5-95% B over 6 min, Solvent A: H₂O with 0.1 % TFA; Solvent B: acetonitrile with 0.1 TFA; UV 254)).

The following examples are intended to illustrate the embodiments disclosed and are not to be construed as being limitations thereto. Additional compounds, other than those described below, may be prepared using the following described reaction scheme or appropriate variations or modifications thereof.

### SYNTHESIS

Scheme I illustrates the procedure used for preparing compounds of Formula VIIIa, wherein (Y)_{P} are hydrogens. Specifically, Scheme I illustrates the reaction of 6-membered amino heterocycles (reactant I, 4-bromo-2-amino pyridine) with an aldehyde and an isonitrile to form fused 3-amino imidazo heterocycles [Tet Lett, 1998, 39, 3635*;* Angew. Chem Int Ed English, 1998, 2234]. Other 6-membered amino heterocycles can be used to form fused heterocycles. By analogy, appropriate 5-membered amino heterocycles can be reacted with an appropriate aldehyde and an isonitrile to form 5,5-fused imidazo heterocycles.
As illustrated in Scheme **I,** an amino heterocycle 4-bromo-2-amino pyridine **(I)** was reacted with an aldehyde **II,** and an isonitrile **III,** in one pot reaction under acid catalyzed condition to furnish a fused imidazo heterocycle bearing secondary amine disposed on the 3-position of the fused ring. The halogen substituent (Y = halogen, p=1) on the fused ring can then be reacted with ethyl acrylate **V** under Heck conditions to produce an α,β-unsaturated ester which can subsequently be converted to a hydroxamic acid **VII.**

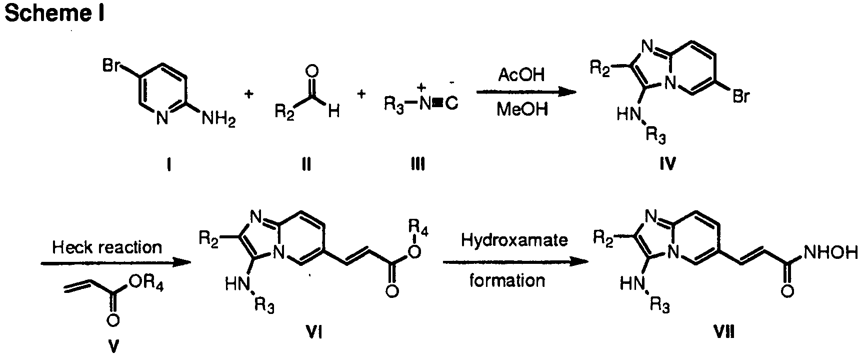

Alternatively, compounds of **VII** can be prepared by first introducing the α,β-unsaturated ester on amino heterocycle I to form the intermediate **VIII.** This is followed by a three component one pot reaction that fuses **VIII,** aldehyde **II,** and isonitrile III to furnish the fused ring **VI.** The ester **Vl** can be converted to an hydroxamic acid by methods known in the literature. This alternative method of preparation is illustrated in Scheme **II**. 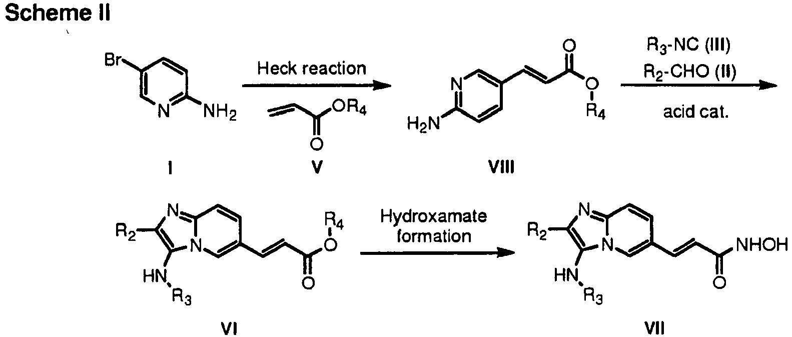 The preparations of 6-substituted alkenoyl hydroxamates are illustrated in Schemes III - IX.
Scheme III illustrates the procedure used for preparing compounds of Formula VIIIa (wherein (Y)ₚ are hydrogens) in which the hydroxamic acid bearing substituent is at the 6-position. Using 4-cyano-2-amino pyridine as one of the starting materials of the three component reaction, the imidazopyridine structure could be constructed. Further elaborations by converting the nitrile group to the corresponding aldehyde could be achieved by DIBAL-H reduction. The aldehyde could be reacted with an appropriate Wittig reagent to provide the desired alkenyl ester which could be converted to the desired hydroxamate. 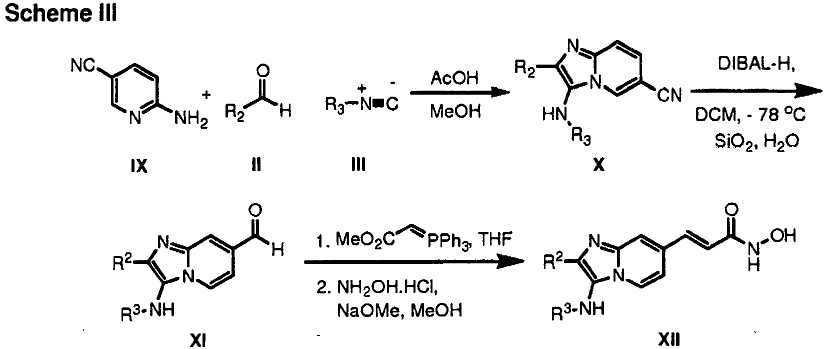 Scheme IV illustrates yet another procedure to prepare compounds of Formula VIIIa (wherein (Y)ₚ are hydrogens), by using 4-bromo-2-aminopyridine as the starting material. The synthetic steps are quite similar to those illustrated in Scheme I. Both schemes utilize Heck reaction to introduce the alkenyl ester functionality which was eventually converted to hydroxamic acid. 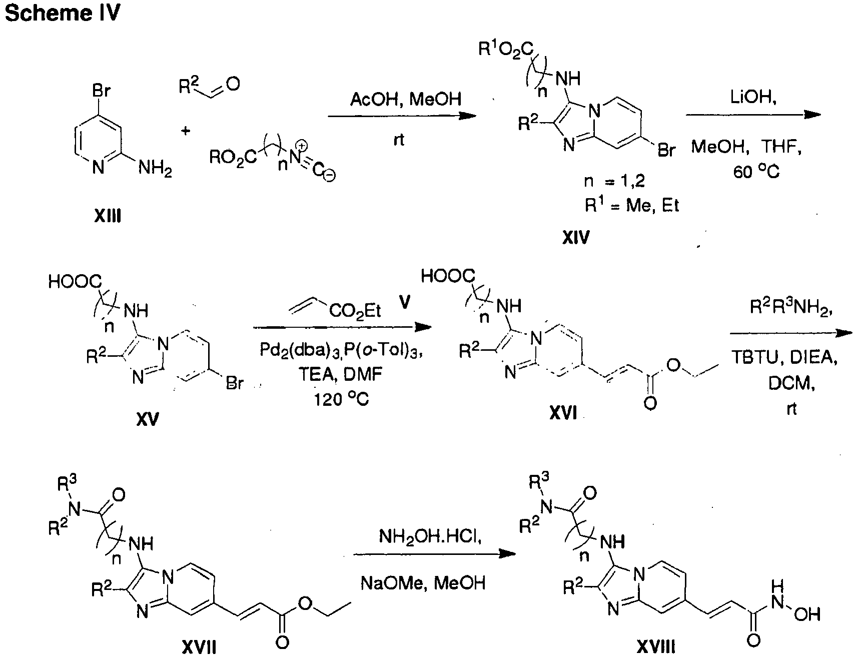 Scheme V illustrates yet another method in preparing compounds of Formula VIIIb, wherein (Y)ₚ are hydrogens. The imidazopyridine core structure was constructed by a condensation reaction using 4-bromo-2-aminopyridine as one of the starting materials [J. Med. Chem. 1998, 41, 5108]. The methanol group was introduced at the 3 position by reacting with formaldehyde to give the intermediate **XX.** This intermediate **XX** was then subjected to Heck reaction condition in which the alkenyl ester was produced. The alcohol group in **XXI** was then oxidized to the aldehyde which was further converted to an aminoalkyl group under reductive amination conditions using sodium acetoxyborohydride. The hydroxamic acid was formed as described in the previous schemes. 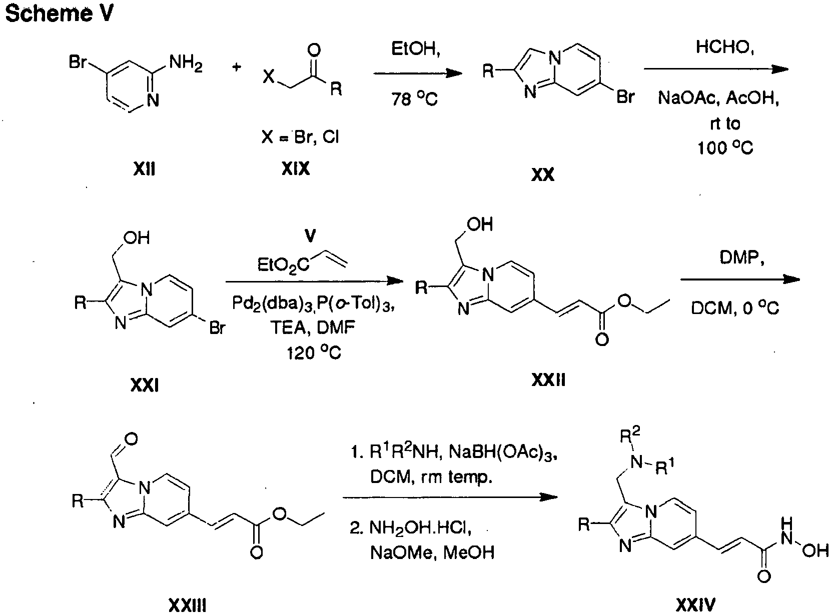 Scheme VI illustrates yet another method in preparing compounds of Formula Vlllb, wherein (Y)ₚ are hydrogens. The imidazolpyridine core structure was constructed by the condensation reaction using 4-bromo-2-aminopyridine as one of the starting material. The alkenyl ester group was introduced at the 6-position by the Heck reaction. This intermediate **XXV** was then subjected to a Mannich reaction in which the aminoalkyl group was introduced [J. Org. Chem. 1965, 30, 2403]. Without further workups and purifications, the crude material was converted into the hydroxamic acid as described in the previous schemes. 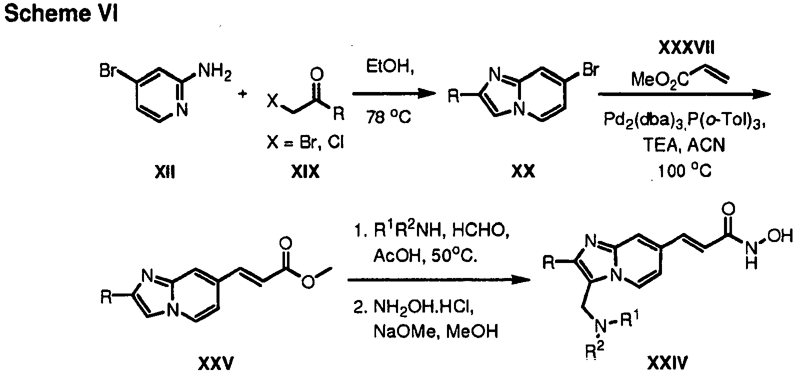 Scheme VII illustrates yet another method of preparing compounds of Formula Vlllb, wherein (Y)ₚ are hydrogens. The imidazolpyridine core structure was constructed by the condensation reaction using 4-cyano-2-aminopyridine as one of the starting material. The alkenyl ester group was introduced at the 6-position by a series of common organic transformations (basic hydrolysis; esterification; DIBAL-H reduction; DMP oxidation and the Wittig reaction). The intermediate **XXV** was then subjected to a Mannich reaction in which the aminoalkyl group was introduced [J. Org. Chem. 1965, 30, 2403]. Without further workups and purifications, the crude material was converted into the hydroxamic acid as described in the previous schemes. 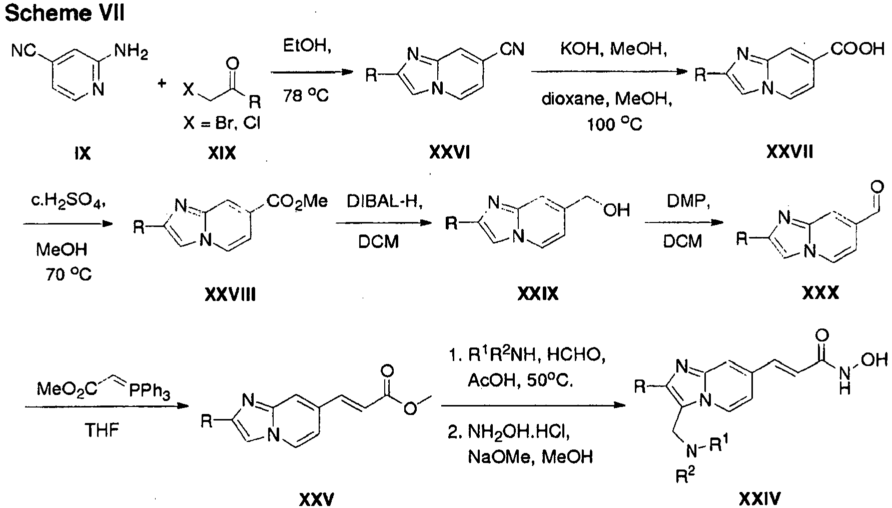 Scheme VIII illustrates yet another method of preparing compounds of Formula la & Ib. The imidazolpyridine core structure was constructed by the condensation reaction using 4-bromo-2-aminopyridine and the appropriate bromoketoamides **XXX** [J. Med. Chem. 2005, 48, 292]. The amide was reduced to the corresponding amines before introducing the alkenyl ester group at the 6-position by the Heck reaction. The intermediate **XXXII** was then converted into the hydroxamic acid as described in the previous schemes. 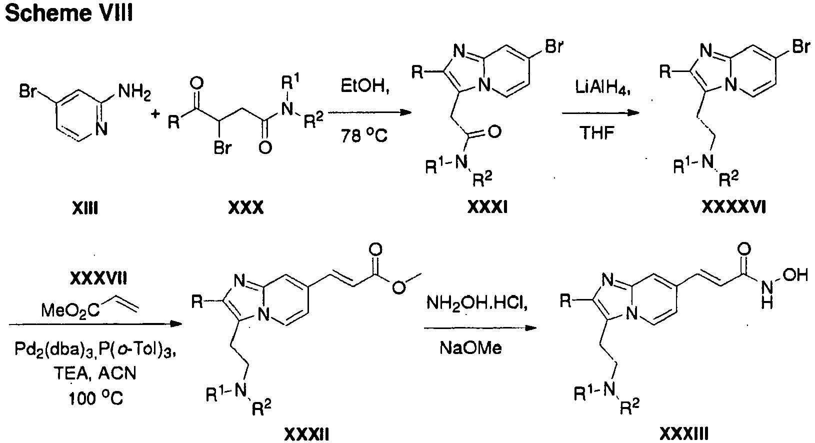 Scheme IX illustrates yet another method of preparing compounds of Formula Ia & Ib. The imidazopyridine core structure was constructed by a condensation reaction using 4-bromo-2-aminopyridine as one of the starting materials. The methanol group was introduced at the 3-position by reacting with formaldehyde to give the intermediate **XX.** This intermediate **XX** was then subjected to chlorination and subsequent reaction with NaCN to furnish the cyano-intermediate [*Eur. Pat. Appl.* 266890]. Further reduction with LiAlH₄ and followed by a reductive amination of the former gave the intermediate **XXXVI.** The alkenyl ester group was introduced at the 6-position by the Heck reaction. The intermediate **XXXII** was then converted into the hydroxamic acid as described in the previous schemes. 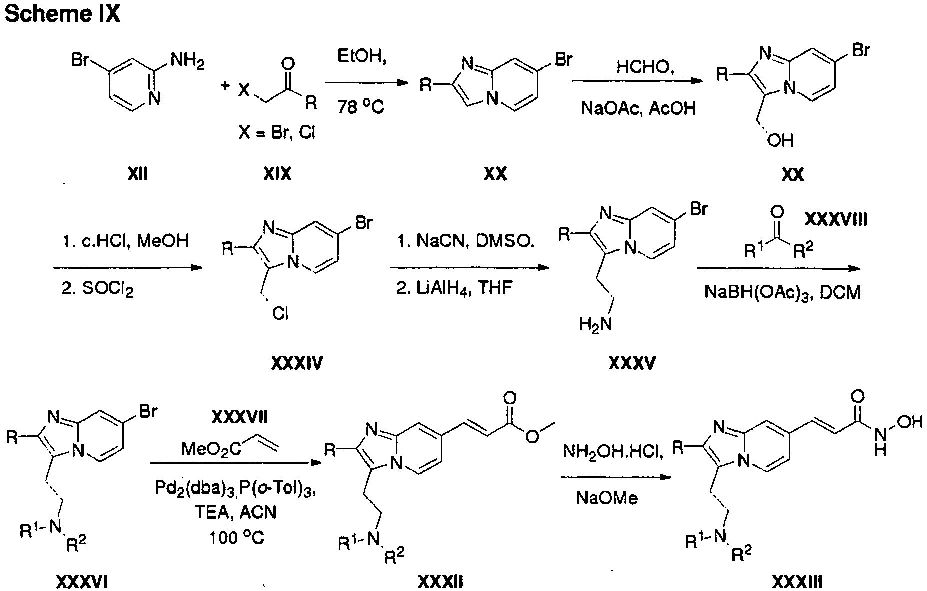 Scheme X illustrates yet another method of preparing compounds of Formula la & Ib. The imidazolpyridine core structure was constructed by the condensation reaction using 4-bromo-2-aminopyridine and the appropriate bromoketoamides **XXXIX.** The alkenyl ester group was introduced at the 6-position by the Heck reaction. Further deprotection and followed by reductive amination provided the intermediate **XXXII.** The intermediate **XXXII** was then converted into the hydroxamic acid as described in the previous schemes. 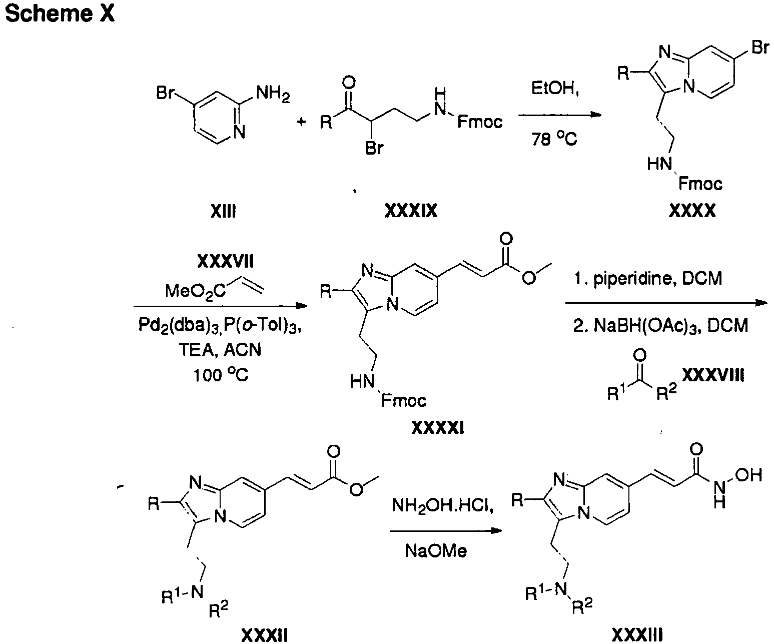 Based on Scheme VIII, Scheme IX and Scheme X, and by varying the starting materials used in the synthesis, a wide variety of compounds of Formula Ib, preferably Formula Ic, (where p = 0; Z = CHCH alkene; R² = -CH₂CH₂NR²⁶R²⁷) could be prepared, including, but not limited to, those in Table 1:

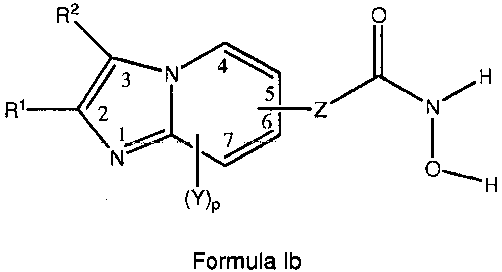

**Table 1**

| **Compound No.** | **R¹** | **R²⁶** | **R²⁷** |
|---|---|---|---|
| **65** | Ph | -CH₂CH₃ | H |
| **66** | Ph | -CH₂CH₂CH₃ | H |
| **67** | Ph | -C(CH₃)₃ | H |
| **68** | Ph | -CH₂C(CH₃)₃ | H |
| **69** | Ph | -CH₂CH₃ | -CH₂CH₃ |
| **70** | Ph | -CH₂CH₂CH₃ | -CH₃ |
| **71** | Ph | -CH(CH₃)₂ | -CH₃ |
| **72** | 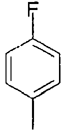 | -CH₂CH₃ | H |
| **73** | 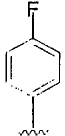 | -CH₂CH₂CH₃ | H |
| **74** | 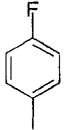 | -C(CH₃)₃ | H |
| **75** | 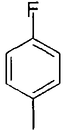 | -CH₂C(CH₃)₃ | H |
| **76** | 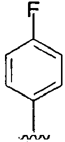 | -CH₂CH₃ | -CH₂CH₃ |
| **77** | 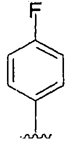 | -CH₂CH₂CH₃ | -CH₃ |
| **78** | 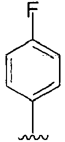 | -CH(CH₃)₂ | -CH₃ |
| **79** | 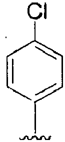 | -CH₂CH₃ | H |
| **80** | 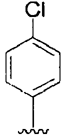 | -CH₂CH₂CH₃ | H |
| **81** | 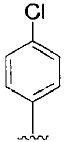 | -C(CH₃)₃ | H |
| **82** | 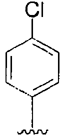 | -CH₂C(CH₃)₃ | H |
| **83** | 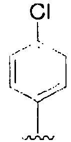 | -CH₂CH₃ | -CH₂CH₃ |
| **84** | 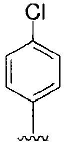 | --CH₂CH₂CH₃ | -CH₃ |
| **85** | 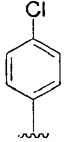 | -CH(CH₃)₂ | -CH₃ |
| **86** | 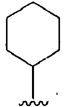 | -CH₂CH₃ | H |
| **87** | 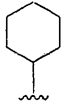 | -CH₂CH₂CH₃ | H |
| **88** | 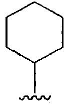 | -C(CH₃)₃ | H |
| **89** | 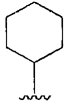 | -CH₂C(CH₃)₃ | H |
| **90** | 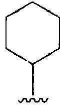 | -CH₂CH₃ | -CH₂CH₃ |
| **91** | 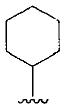 | -CH₂CH₂CH₃ | -CH₃ |
| **92** | 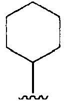 | -CH(CH₃)₂ | -CH₃ |
| **93** | 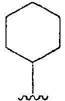 | -C(CH₃)₃ | -CH₃ |
| **94** | 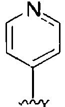 | -CH₂CH₂CH₃ | H |
| **95** | 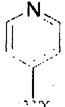 | -C(CH₃)₃ | H |
| **96** | 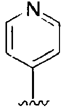 | -CH₂C(CH₃)₃ | H |
| **97** | 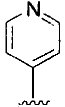 | -CH₂CH₃ | -CH₂CH₃ |
| **98** | 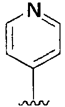 | -CH₂CH₂CH₃ | -CH₃ |
| **99** | 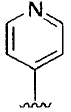 | -CH(CH₃)₂ | -CH₃ |
| **100** | 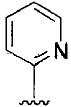 | -CH₂CH₂CH₃ | H |
| **101** | 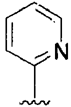 | -C(CH₃)₃ | H |
| **102** | 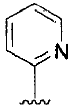 | -CH₂C(CH₃)₃ | H |
| **103** | 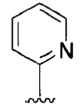 | -CH₂CH₃ | -CH₂CH₃ |
| **104** | 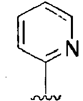 | -CH₂CH₂CH₃ | -CH₃ |
| **105** | 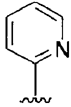 | -CH(CH₃)₂ | -CH₃ |
| **106** | -(CH₂)₃CH₃ | -CH₂CH₃ | -CH₂CH₃ |
| **107** | -(CH₂)₃CH₃ | -CH₂CH₂CH₃ | -CH₃ |
| **108** | -(CH₂)₃CH₃ | -CH(CH₃)₂ | -CH₃ |
| **109** | -(CH₂)₃CH₃ | -C(CH₃)₃ | -CH₃ |
| **110** | -(CH₂)₃CH₃ | -C(CH₃)₃ | -CH₂CH₃ |
| **111** | -(CH₂)₃CH₃ | -CH₂C(CH₃)₃ | H |
| **112** | 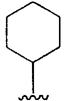 | -C(CH₃)₃ | -CH₂CH₃ |
| **113** | 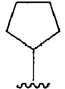 | -CH₂CH₃ | H |
| **114** | 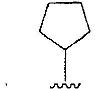 | -CH₂CH₂CH₃ | H |
| **115** | 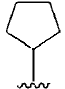 | -C(CH₃)₃ | H |
| **116** | 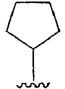 | -CH₂C(CH₃)₃ | H |
| **117** | 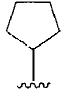 | -CH₂CH₃ | -CH₂CH₃ |
| **118** | 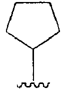 | -CH₂CH₂CH₃ | -CH₃ |
| **119** | 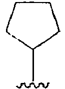 | -CH(CH₃)₂ | -CH₃ |
| **120** | 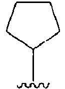 | -C(CH₃)₃ | -CH₃ |
| **121** | 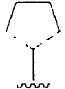 | -C(CH₃)₃ | -CH₂CH₃ |
| **122** | -C(CH₃)₃ | -CH₂CH₃ | H |
| **123** | -C(CH₃)₃ | -CH₂CH₂CH₃ | H |
| **124** | -C(CH₃)₃ | -C(CH₃)₃ | H |
| **125** | -C(CH₃)₃ | -CH₂C(CH₃)₃ | H |
| **126** | -C(CH₃)₃ | -CH₂CH₃ | -CH₂CH₃ |
| **127** | -C(CH₃)₃ | -CH₂CH₂CH₃ | -CH₃ |
| **128** | -C(CH₃)₃ | -CH(CH₃)₂ | -CH₃ |
| **129** | -C(CH₃)₃ | -C(CH₃)₃ | -CH₃ |
| **130** | (CH₃)₃ | -C(CH₃)₃ | -CH₂CH₃ |
| **131** | -CH₃ | -CH₂CH₃ | H |
| **132** | -CH₃ | -CH₂CH₂CH₃ | H |
| **133** | -CH₃ | -C(CH₃)₃ | H |
| **134** | -CH₃ | -CH₂C(CH₃)₃ | H |
| **135** | -CH₃ | -CH₂CH₃ | -CH₂CH₃ |
| **136** | -CH₃ | -CH₂CH₂CH₃ | -CH₃ |
| **137** | -CH₃ | -CH(CH₃)₂ | -CH₃ |
| **138** | -CH₃ | -C(CH₃)₃ | -CH₃ |
| **139** | -CH₃ | -C(CH₃)₃ | -CH₂CH₃ |
| **140** | 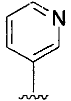 | -CH₂CH₂CH₃ | H |
| **141** | 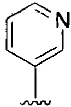 | -C(CH₃)₃ | H |
| **142** | 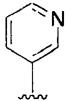 | -CH₂C(CH₃)₃ | H |
| **143** | 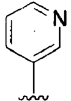 | -CH₂CH₃ | -CH₂CH₃ |
| **144** | 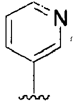 | -CH₂CH₂CH₃ | -CH₃ |
| **145** | 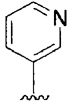 | -CH(CH₃)₂ | -CH₃ |
| **146** | 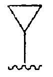 | -CH₂CH₃ | H |
| **147** | 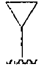 | -CH₂CH₂CH₃ | H |
| **148** | 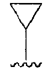 | -C(CH₃)₃ | H |
| **149** | 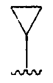 | -CH₂C(CH₃)₃ | H |
| **150** | 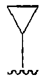 | -CH₂CH₃ | -CH₂CH₃ |
| **151** | 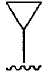 | -CH₂CH₂CH₃ | -CH₃ |
| **152** | 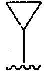 | -CH(CH₃)₂ | -CH₃ |
| **153** | 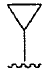 | -C(CH₃)₃ | -CH₃ |
| **154** | 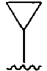 | -C(CH₃)₃ | -CH₂CH₃ |
| **155** | -(CH₂)₃CH₃ | -CH₂CH₃ | H |
| **156** | -(CH₂)₃CH₃ | -CH₂CH₂CH₃ | H |
| **157** | -(CH₂)₃CH₃ | -C(CH₃)₃ | H |

The following preparation and examples are given to enable those skilled in the art to more clearly understand and to practice the subject matter hereof. They should not be considered as limiting the scope of the disclosure, but merely as being illustrative and representative thereof.

### Example 1

### Preparation of N-Hydroxy-3-[2-phenethyl-3-(3,4,5-trimethoxy-ahenylamino)-imidazo[1,2-a]pyridin-6-yl]-acrylamide (Compound 1)

### Step 1: 3-Component Reaction

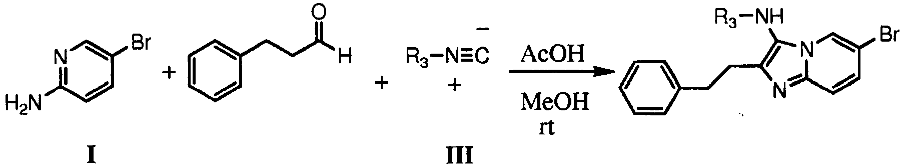

To a solution of the amine (3.04 mmol) in MeOH (10.0 mL) was added the aldehyde (3.04 mmol), the isonitrile (3.04 mmol) and AcOH (6.08 mmol) at room temp. The reaction was stirred overnight. When LCMS had shown the full depletion of the starting material amine, 1 M HCl (25 mL) was added till pH ∼ 1 before being concentrated *in vacuo.* NaHCO₃ (30 mL) was then added and ethyl acetate (4 x 20 mL) was used to extract the aqueous layer. The combined organic extracts were then washed with NaHCO₃ (2 x 20 mL) and brine (2 x 20 mL), before being died in Na₂SO₄. The mixture was then filtered and concentrated *in vacuo.* The crude product was used immediately for the next step without further purification.

### (6-Bromo-2-phenethyl-imidazo[1,2-a]pyridin-3-yl)-(3,4,5-trimethoxy-phenyl)-amine

HPLC: 87.5 %; t_{R} = 2.741 min; LCMS (ESI) Calcd for C₂₄H₂₄BrN₃O₃ [M⁺]: 481.1001, found 482.03 [MH]⁺.

### Step2: Heck Reaction

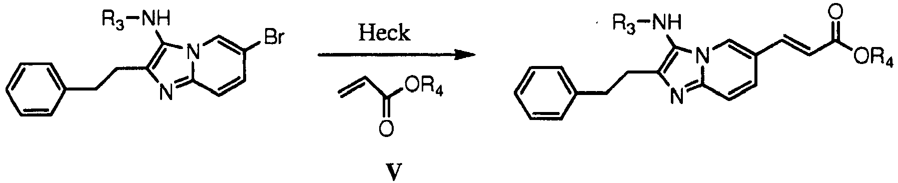

Ethyl acrylate (1.5 equiv) was added into a stirred suspension of the amine (1 equiv), Pd₂(dba)₃ (0.1 equiv), P(*o*-tol)₃ (0.18 equiv), Et₃N (1.54 equiv) and DMF (0.32 M) at room temp. The reaction was heated to reflux at ∼ 120°C. When the starting material had fully depleted (monitored by LCMS), the reaction mixture was diluted with ethyl acetate (20 mL). The organic layer was then washed with NaHCO3 (2x10 mL) and brine (2x10 mL).
The organic layer was dried in Na₂SO₄ before being filtered and concentrated *in vacuo.* The crude product was purifed by flash column chromatography.

### 3-[2-Phenethyl-3-(3,4,5-trimethoxy-phenylamino)-imidazo[1,2-a]pyridin-6-yl]-acrylic acid ethyl ester

R_{f} = 0.44 [Hexane : ethyl acetate (1:3)]HPLC: 95.6 %; t_{R} = 2.532 min; LCMS (ESI) Calcd for C₂₉H₃₁ N₃O₅ [M⁺]: 501.2264, found 502.17 [MH]⁺.

### Step 3: Hydroxamic acid formation

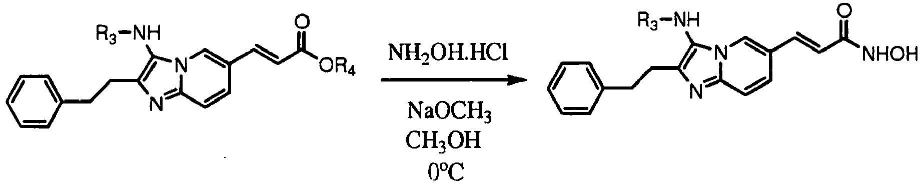

NH₂OH.HCl (10 equiv) was added into a solution of the ester (1 equiv) and MeOH (0.25 M) at room temperature. The reaction mixture was cooled to 0 °C before NaOCH₃ (20 equiv; 25% wt solution in MeOH) was introduced. When LCMS had shown the full depletion of the starting material, the reaction mixture was poured into ice-water and extracted with ethyl acetate (4 x 15 mL). The organic extracts were then washed with NaHCO₃ (2 x 20 mL) and brine (2 x 20 mL), before being dried in Na₂SO₄. The mixture was then filtered and concentrated *in vacuo.* The crude product was purifed by the Bison system.

### N-Hydroxy-3-[2-phenethyl-3-(3,4,5-trimethoxy-phenylamino)-imidazo[1,2-a]pyridin-6-yl]-acrylamide (Compound 1)

HPLC: 98.8 %; t_{R} = 2.847 min; LCMS (ESI) Calcd for C₂₇H₂₈N₄O₅ [M⁺]: 488.2060, found 489.14 [MH]⁺; ¹H NMR (400 MHz, d₆-DMSO ₃): δ 10.86 (brs, 1H), 8.62 (s, 1 H), 8.09 (s, 1H), 8.02 (d, *J* = 9.38 Hz, 1 H), 7.94 (d, *J* = 9.35 Hz, 1 H), 7.63 (d, *J* = 15.83 Hz, 1H), 7.26 - 7.23 (m, 2H), 7.18-7.15 (m, 3H), 6.61 (d, *J* = 15.84 Hz, 1 H), 3.63 (s, 6H), 3.57 (s, 3H), 2.99 (s, 4H); ¹³C NMR (100.5 MHz, d₆-DMSO): δ 153.6, 141.3, 140.2, 137.4, 133.4, 132.8, 131.0, 128.4, 128.4, 126.2, 124.5, 124.1, 121.9, 121.7, 113.5, 91.6, 60.0, 55.7, 33.3, 26.1.

### Example 2

### Preparation of 3-{3-[(Benzo[1,3]dioxol-5-ylmethyl)-amino]-2-phenethyl-imidazo[1,2-a]pyridin-6-yl}-N-hydroxy-acrylamide (Compound 2)

The titled compound was prepared according to the procedures described in Example 1,by using appropriate starting materials.

HPLC: 98.26 %; t_{R} = 2.368 min; LCMS (ESI) Calcd for C₂₆H₂₄N₄O₄ [M⁺]: 456.1798, found 457.13 [MH]⁺; ¹H NMR (400 MHz, d₆-DMSO): δ 10.95 (brs, 1 H), 8.74 (s, 1 H), 8.02 (d, *J* = 9.29 Hz, 1 H), 7.88 (d, *J* = 9.33 Ha, 1H), 7.66 (d, *J* = 15.79 Hz, 1 H), 7.31-7.27 (m, 2H), 7.22 - 7.16 (m, 3H), 6.97 (d, *J* = 1.34 Hz, 1H), 6.78 (d, *J* = 7.88 Hz, 1), 6.67 - 6.63 (m, 2H), 5.93 (s, 2h), 5.62 (brs, 1 H), 3.90 (d, *J* = 7.92 Hz, 2H), 2.87 - 2.84

(m, 2H), 2.79 - 2.75 (m, 2H);¹³C NMR (100.5 MHz, d₆-DMSO): 147.2, 146.4, 140.3, 135.8, 133.2, 128.4, 128.1, 127.8, 126.2, 125.0, 124.1, 121.8, 112.4, 109.0, 107.9, 100.8, 50.7, 33.9, 25.5.

### Example 3

### Preparation of N-Hydroxy-3-[2-phenethyl-3-(4-piperidin-1-yl-phenylamino)-imidazo[1,2-a]pyridin-6-yl]-acrylamide (Compound 3)

The titled compound was prepared according to the procedures described in Example 1, by using appropriate starting materials.

HPLC: 100 %; t_{R} = 1.604 min; LCMS (ESI) Calcd for C₂₉H₃₁N₅O₂ [M⁺]: 481.2478, found 482.21 [MH]⁺; ¹H NMR (400 MHz, d₆-DMSO ₃): δ 8.65 (d, *J* = 10.78 Hz, 2H), 8.09 (d, *J* = 9.44 Hz, 1H), 8.01 (d, *J* = 9.38 Hz, 1H), 7.61 (d, *J* = 15.82 Hz, 1H), 7.45 (d, *J* = 8.53 Hz, 2H), 7.24 - 7.13 (m, 6H), 6.70 - 6.62 (m, 3H), 3.45 (brs, 4H), 2.99 (s, 4H), 1.89-1.79 (m, 4H), 1.51 (brs, 2H);¹³C NMR (100.5 MHz, d₆-DMSO): δ 161.8, 140.0, 137.5, 133.2, 132.8,128.9, 128.4, 128.1, 126.2, 124.7, 124.6, 122.2, 122.0, 121.2, 117.9, 115.0, 114.3, 113.4, 55.9, 33.2, 25.9, 23.4, 20.7.

### Example 4

### Preparation of 3-[3-(Benzo[1,3]dioxol-5-ylamino)-2-phenethyl-imidazo[1,2-a]pyridin-6-yl]-(Compound 4)

### Step 1: Heck Reaction

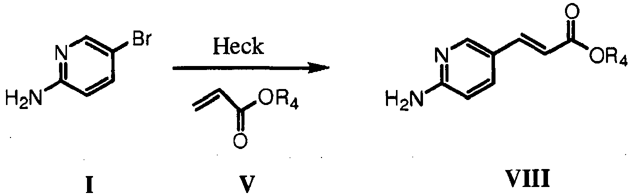

Ethyl acrylate (0.47 mL, 4.33 mmol) was added into a stirred suspension of the amine (0.50 g, 2.89 mmol), Pd₂(dba)₃ (0.2646 g, 0.289 mmol), P(*o*-tol)₃ (0.1583 g, 0.52 mmol), Et₃N (0.62 mL, 4.45 mmol) and CH₂Cl₂ (12 mL) at room temp. The reaction was heated to reflux at ∼ 80°C. When the starting material had fully depleted (monitored by LCMS), the reaction mixture was diluted with ethyl acetate (20 mL). The organic layer was then washed with NaHCO3 (2x10 mL) and brine (2x10 mL). The organic layer was dried in Na₂SO₄ before being filtered and concentrated *in vacuo.* The crude product was purifed by the Bison system and was isolated as a light yellow solid [trifluoroacetic acid (TFA) salt] (75%, 0.63 g).

### 3-(2-Amino-pyridin-4-yl)-acrylic acid ethyl ester

HPLC: 97.5 %; t_{R} = 1.114 min; LCMS (ESI) Calcd for C₁₀H₁₂N₂O₂ [M⁺]: 192.0899, found 193.08 [MH]⁺; ¹H NMR (400 MHz, CDCl₃): δ 8.36 (brs, 1 H), 8.29 (s, 1H), 8.26 (dd, *J*= 2.00, 9.27 Hz, 1H), 7.56 (dd, *J* = 16.02 Hz, 1 H), 6.92 (d, *J* = 9.20 Hz, 1H), 6.55 (d, *J*= 16.00 Hz, 1 H), 4.17 (q, *J* = 7.08 Hz, 2H), 1.24 (t, *J* = 7.10 Hz, 3H);¹³C NMR (100.5 MHz, CDCl₃): δ 165.9, 155.3, 139.9, 139.5, 139.3, 119.1, 118.1, 117.2, 115.2, 113.1, 60.0, 14.1.

### Step 2: 3-Component Reaction

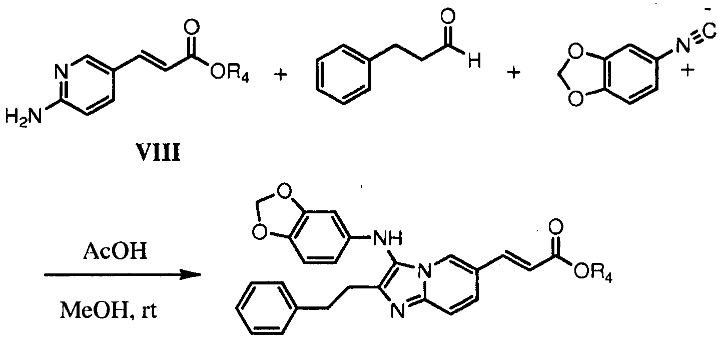

To a solution of the amine (0.48 g, 1.67 mmol) in MeOH (6.0 mL) was added (2) (0.22 mL, 1.67 mmol), (3) (0.27 g, 1.67 mmol) and AcOH (0.19 mL, 3.35 mmol) at room temperature. The reaction was stirred overnight. When LCMS had shown the full depletion of the starting material amine, 1 M HCl (15 mL) was added till pH ∼ 1 before being concentrated *in vacuo.* NaHCO₃ (20 mL) was then added and ethyl acetate (3 x 20 mL) was used to extract the aqueous layer. The combined organic extracts were then washed with brine (2 x 20 mL), before being died in Na₂SO₄. The mixture was then filtered and concentrated *in vacuo.* The crude product was purified by flash coloumn chromatography and the product was isolated as a viscous dark brown oil (72%, 0.55 g).

### 3-[3-(Benzo[1,3]dioxol-5-ylamino-2-phenethyl-imidazo[1,2-a]pyridin-6-yl]-acrylic acid ethyl ester

R_{f} = 0.33 [Hexane : ethyl acetate (1:1)]HPLC: 100 %; t_{R} = 3.057 min;
LCMS (ESI) Calcd for C₂₇H₂₅N₃O₄ [M⁺]: 455.1845, found 456.16 [MH]⁺;¹H NMR (400 MHz, CDCl₃): δ 7.79 (s, 1 H), 7.54 (s, 1H), 7.51 (d, *J* = 7.21 Hz, 1 H), 7.37 (d, *J* = 9.38 Hz, 1 H), 7.26 - 7.21 (m, 2H), 7.08 (dd, *J* = 1.85, 7.85 Hz, 2H), 6.57 (d, *J* = 8.28 Hz, 1H). 6.35 (d, *J* = 15.87 HZ, 1 H), 5.89 (d, *J* = 2.31 Hz, 1 H), 5.85 (s, 2H), 5.74 (dd, *J* = 2.36, 8.29 Hz, 1 H), 4.60 (brs, 1H), 4.23 (q, *J* = 7.12 Hz, 2H), 3.02 - 2.97 (m, 4H), 1.31 (t, *J*= 7.12 Hz, 3H); ¹³C NMR (100.5 MHz, CDCl₃): δ 166.6, 148.6, 142.3, 142.2, 141.7, 141.1, 140.7, 140.2, 128.7, 128.3, 126.0, 124.2, 121.5, 120.8, 120.1, 118.2, 117.5, 108.7, 104.8, 100.9, 95.9, 60.6, 35.4, 29.5, 14.3.

### Step 3: Hydroxamic acid formation

NH₂OH.HCl (0.12 g, 1.7 mmol) was added into a solution of the ester (75.1 mg, 0.17 mmol) and MeOH (5 mL) at room temperature. The reaction mixture was cooled to 0 °C before NaOCH₃ (0.78 mL, 3.40 mmol; 25% wt solution in MeOH) was introduced. When LCMS had shown the full depletion of the starting material, the reaction mixture was poured into ice-water and extracted with ethyl acetate (4 x 15 mL). The organic extracts were then washed with NaHCO₃ (2 x 20 mL) and brine (2 x 20 mL), before being dried in Na₂SO₄. The mixture was then filtered and concentrated *in vacuo.* The crude product was purifed by the Bison system and was isolated as a light yellow solid [trifluoroacetic acid (TFA) salt] (62%, 67.1 mg).

### 3-[3-(Benzo[1,3]dioxol-5-ylamino)-2-phenethyl-imidazo[1,2-a]pyridin-6-yl]-N-hydroxy-acrylamide (Compound 4)

HPLC: 100 %; t_{R} = 2.332 min; LCMS (ESI) Calcd for C₂₅H₂₂N₄O₄ [M⁺]: 442.1641, found 443.13 [MH]⁺; ¹H NMR (400 MHz, CDCl₃): δ 8.55 (s, 1 H), 8.02 - 7.96 (m, 2H), 7.91 (d, *J* = 9.28 Hz, 1H), 7.62 (d, *J* = 15.82 Hz, 1 H), 7.26 - 7.13 (m, 7H), 6.68 (d, *J* = 8.29 Hz, 1H), 6.58 (d, *J* = 15.82 Hz, 1 H), 6.29 (d, *J* = 2.12 Hz, 1H), 5.98 (dd, *J* = 2.17, 8.28 Hz, 1 H), 5.89 (s, 2H), 2.97 (s, 4H);¹³C NMR (100.5 MHz, CDCl₃): δ 148.0, 140.5, 140.2, 140.0, 129.1, 128.9, 128.6, 128.4, 128.1, 126.2, 124.4, 122.4, 121.6, 108.6, 105.3, 100.6, 99.4, 96.5, 33.4, 26.1.

### Example 5

### N-Hydroxy-3-[3-(2-methoxy-ethylamino)-2-phenethyl-imidazo[1,2-a]pyridin-6-yl]-acrylamide (Compound 5)

The titled compound was prepared according to the procedures described in Example 1, by using appropriate starting materials.
HPLC: 100 %; t_{R} = 1.806 min; LCMS (ESI) Calcd for C₂₁H₂₄N₄O₃ [M⁺]: 380.1848, found 380.98 [MH]⁺; ¹H NMR (400 MHz, d₆-DMSO): δ 10.95 (brs, 1H), 8.82 (s, 1 H), 8.02 (d, *J* = 9.37 Hz, 1 H), 7.88 (d, *J* = 9.33 Hz, 1 H), 7.65 (d, *J* = 15.81 Hz, 1 H), 7.32 - 7.15 (m, 5H), 6.65 (d, *J* = 15.83 Hz, 1 H), 5.19 (brs, 1H). 3.35 (t, *J* = 5.28 Hz, 2H), 3.21 (s, 3H), 3.07 - 3.06 (m, 2H), 3.02 - 2.99 (m, 4H).

### Example 6

### 3-(3-Cyclohexylamino-2-phenethyl-imidazo[1,2-a]pyridin-6-yl)-N-hydroxy-acrylamide (Compound 6)

The titled compound was prepared according to the procedures described in Example 1, by using appropriate starting materials.
HPLC: 100 %; t_{R} = 1.806 min; LCMS (ESI) Calcd for C₂₄H₂₈N₄O₂ [M⁺]: 404.2212, found 405.04 [MH]⁺; ¹H NMR (400 MHz, d₆-DMSO): δ 10.94 (brs, 1H), 8.80 (s, 1H), 8.01 (d, *J* = 9.48 Hz, 1H), 7.88 (d, *J* = 9.34 Hz, 1 H), 7.75 (d, *J* = 15.82 Hz, 1H), 7.32 - 7.19 (m, 5H), 6.64 (d, *J* = 15.85 Hz, 1H), 4.97 (brs, 1 H), 3.10 - 2.99 (m, 4H), 2.73 (brs, 1 H), 1.79 - 1.76 (m, 2H), 1.66 - 1.65 (m, 2H), 1.20 - 1.10 (m, 6H);¹³C NMR (100.5 MHz, d₆-DMSO): δ 140.4, 135.7, 133.4, 128.7, 128.6, 128.4, 128.2, 127.5, 126.2, 124.9, 124.1, 121.8, 118.5, 115.5, 112.4, 109.5, 71.4, 57.9, 46.8, 40.1, 39.9, 39.7, 39.5, 39.3, 39.1, 38.9, 33.9, 25.6.

### Example 7

### N-Hydroxy-3-[2-isopropyl-3-(2-methoxy-ethylamino)-imidazo[1,2-a]pyridin-6-yl]-acrylamide (Compound 7)

The titled compound was prepared according to the procedures described in Example 1, by using appropriate starting materials.
HPLC: 97.27 %; t_{R} = 1.164 min; LCMS (ESI) Calcd for C₁₆H₂₂N₄O₃ [M⁺]: 318.1692, found 319.13 [MH]⁺; ¹H NMR (400 MHz, d₆-DMSO): δ 10.95 (brs, 1H), 8.84 (s, 1H), 8.02 (d, *J* = 9.32 Hz, 1 H), 7.85 (d, *J* = 9.32 Hz, 1 H), 7.67 (d, *J* = 15.82 Hz, 1 H), 6.65 (d, *J* = 15.83 Hz, 1 H), 5.25 (brs, 1 H), 3.43 (t, *J* = 5.43 Hz, 2H), 3.39 - 3.27 (m, 1 H), 3.23 (s, 3H), 3.15 (t, *J* = 5.16 Hz, 2H), 1.32 (d, *J* = 6.97 Hz, 6H).

### Example 8

### 3-[2-(2,2-Dimethyl-propyl)-3-(2-methoxy-ethylamino)-imidazo[1,2-a]pyridin-6-yl]-N-hydroxy-acrylamide (Compound 8)

The titled compound was prepared according to the procedures described in Example 1, by using appropriate starting materials.
HPLC: 100 %; t_{R} = 1 1.601 min; LCMS (ESI) Calcd for C₁₈H₂₆N₄O₃ [M⁺]: 346.2005, found 347.11 [MH]⁺; ¹H NMR (400 MHz, d₆-DMSO): δ 8.88 (s, 1 H), 8.04 (d, *J* = 9.42 Hz, 1 H), 7.87 (d, *J* = 9.34 Hz, 1 H), 7.67 (d, *J =* 15.81 Hz, 1 H), 6.67 (d, *J* = 15.82 Hz, 1 H), 5.07(brs, 1H). 3.48 (t, *J* = 5.33 Hz, 2H), 3.27 (s, 3H), 3.18 (t, *J* = 5.20 Hz, 2H), 2.72 (s, 2H), 0.99 (s, 9H).

### Example 9

### N-Hydroxy-3-[3-(2-methoxy-ethylamino)-2-pentyl-imidazo[1,2-a]pyridin-6-yl]-acrylamide (Compound 9)

The titled compound was prepared according to the procedures described in Example 1, by using appropriate starting materials.
HPLC: 100 %; t_{R} = 1.787 min; LCMS (ESI) Calcd for C₁₈H₂₆N₄O₃ [M⁺]: 346.2005, found 347.16 [MH]⁺; ¹H NMR (400 MHz, MeOD): δ 8.77 (s, 1 H), 8.04 (d, *J* = 9.32 Hz, 1 H), 7.71 (d, *J* = 9.32 Hz, 1 H), 7.62 (d, *J* = 15.77 Hz, 1H), 6.61 (d, *J* = 15.76 Hz, 1H), 3.46 (t, *J* = 5.08 Hz, 2H), 3.29 (s, 3H), 3.19 (t, *J* = 5.10 Hz, 2H), 1.37 - 1.23 (m, 6H), 0.90-0.86 (m, 3H).

### Example 10

### 3-[6-(2-Hydroxycarbamoyl-vinyl)-3-(2-methoxy-ethylamino)-imidazo[1,2-a]pyridin-2-yl]-propionic acid (Compound 10)

The titled compound was prepared according to the procedures described in Example 1, by using appropriate starting materials.
HPLC: 100 %; t_{R} = 1.524 min; LCMS (ESI) Calcd for C₁₆H₂₀N₄O₅ [M⁺]: 348.1434, found 350.06 [MH]⁺; ¹H NMR (400 MHz, d₆-DMSO): δ 10.91 (s, 1 H), 8.81 (s, 1H), 7.96 (d, *J* = 9.48 Hz, 1H), 7.77 (d, *J* = 9.28 Hz, 1H), 7.65 (d, *J* = 15.68 Hz, 1H), 7.26 (s, 1H), 6.61 (d, *J* = 15.80 Hz, 1H), 5.31 (brs, 1h), 4.32 (d, *J* = 5.60 Hz, 2H), 3.38 - 3.32 (masked peaks).

### Example 11

### 3-[2-Etyl-3-(2-methoxy-ethylamino-imidazo[1,2-a]pyridin-6-yl]-N-hydroxy-acrylamide (Compound 11)

The titled compound was prepared according to the procedures described in Example 1, by using appropriate starting materials.
HPLC: 87.12 %; t_{R} = 0.936 min; LCMS (ESI) Calcd for C₁₅H₂₀N₄O₃ [M⁺]: 304.1535, found 305.08 [MH]⁺; ¹H NMR (400 MHz, d₆-DMSO): δ 8.85 (s, 1 H), 8.04 (dd, *J* = 1.12, 9.44 Hz, 1 H), 7.88 (d, *J* = 9.32 Hz, 1 H), 7.66 (d, *J* = 15.88 Hz, 1 H), 6.67 (d, *J* = 15.84 Hz, 1H), 3.43 (t, *J* = 5.26 Hz, 2H), 3.23 (s, 3H), 3.16 (t, *J* = 5.18 Hz, 2H), 2.82 (q, *J* = 7.58 Hz, 2H), 1.28 (t, *J*= 7.55 Hz, 3H).

### Example 12

### 3-tert-Butylamino-6-(2-hydroxycarbamoyl-vinyl)-imidazo[1,2-a]pyridine-2-carboxylic acid (Compound 12)

The titled compound was prepared according to the procedures described in Example 1, by using appropriate starting materials.
HPLC: 99.99 %; t_{R} = 1.752 min; LCMS (ESI) Calcd for C₁₅H₁₈N₄O₄ [M⁺]: 318.1328, found 274.28 [MH - COOH]⁺; ¹H NMR (400 MHz, MeOD): δ 8.84 (s, 1H), 8.09 (dd, *J* = 1.53, 9.46 Hz, 1H), 7.83 (d, *J* = 16.04 Hz, 1 H), 7.76 (d, *J* = 9.44 Hz, 1H), 7.55 (s, 1H). 6.74 (d, *J* = 16.02 Hz, 1H), 1.32 (s, 9H).

### Example 13

### 3-(2-Butyl-3-butylamino-imidazo[1,2-a]pyridin-6-yl)-N-hydroxy-acrylamide (Compound 13)

The titled compound was prepared according to the procedures described in Example 1, by using appropriate starting materials.
HPLC: 100 %; t_{R} = 2.136 min; LCMS (ESI) Calcd for C₁₈H₂₆N₄O₂ [M⁺]: 330.2056, found 331.34 [MH]⁺; ¹H NMR (400 MHz, d₆-DMSO): δ 10.94 (brs, 1 H), 8.82 (s, 1 H), 8.01 (dd, *J* = 0.94, 8.42 Hz, 1 H), 7.85 (d, *J* = 9.34 Hz, 1H), 7.69 (d, *J* = 15.80 Hz, 1 H), 6.65 (d, *J* = 15.84 Hz, 1 H), 5.13 (brs, 1H), 2.99 (t, *J*= 7.16 Hz, 2H), 2.79 (t, *J*= 7.45 Hz, 2H), 1.71 -1.63 (m, 1H), 1.56 -1.49 (m, 2H), 1.42 -1.32 (m, 4H), 0.94 - 0.88 (m, 6H); ¹³C NMR (100.5 MHz, d₆-DMSO): 161.9, 135.5, 133.4, 128.8, 128.5, 127.4, 124.9, 124.3, 121.9, 118.4, 112.3, 47.2, 32.0, 30.3, 23.1, 21.7, 19.5, 13.8, 13.6.

### Example 14

### N-Hydroxy-3-(2-isoaropyl-3-isopropylamino-imidazo[1,2-a]pyridin-6-yl)-acrylamide (Compound 14)

The titled compound was prepared according to the procedures described in Example 1, by using appropriate starting materials.
HPLC: 99.21 %; t_{R} = 1.432 min; LCMS (ESI) Calcd for C₁₆H₂₂N₄O₂ [M⁺]: 302.1743, found 303.30 [MH]⁺; ¹H NMR (400 MHz, d₆-DMSO): δ 8.86 (s, 1H), 8.05 (dd, *J* = 1.04, 9.41 Hz, 1 H), 7.90 (d, *J*= 9.34 Hz, 1 H), 7.75 (d, *J* = 15.84 Hz, 1H), 6.68 (d, *J* = 15.85 Hz, 1 H), 3.38 - 2.27 (m, 2H), 1.32 (d, *J* = 6.98 Hz, 6H), 1.13 (d, *J* = 6.28 Hz, 6H); ¹³C NMR (100.5 MHz, d₆-DMSO): 161.9, 136.2, 134.0, 133.5, 128.8, 126.2, 125.3, 124.5, 121.9, 121.1, 112.3, 48.3, 23.7, 22.8, 21.5.

### Example 15

### (E)-N-hydroxy-3-(3-(2-methoxyethylamino)-2-(2,4,4-trimethylpentyl)imidazo[1,2-a]pyridin-6-yl)acrylamide (Compound 15)

The titled compound was prepared according to the procedures described in Example 1, by using appropriate starting materials.

### Example 16

### (E)-N-hydroxy-3-(3-(2-methoxyethylamino)-2-(2,4,4-trimethylpentyl)imidazo[1,2-a]pyridin-8-yl)acrylamide (Compound 16)

The titled compound was prepared according to the procedures described in Scheme III by using appropriate starting materials.

### Example 17

### Preparation of (E)-N-hydroxy-3-(3-(2-methoxyethylamino)-2-pentylimidazo[1,2-a]pyridin-7-yl)acrylamide (Compound 17)

### Step 1:Multi-Component Reaction

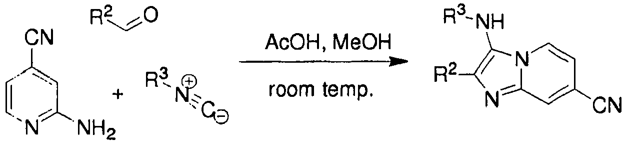

To a solution of 4-cyano-2-aminopyridine (0.27 mg, 2.24 mmol) in MeOH (7.45 mL) was added the aldehyde (2.24 mmol), the isonitrile (2.24 mmol) and AcOH (260 µL, 4.48 mmol) at room temp. The reaction was stirred overnight and monitored by LCMS/TLC. When the reaction has completed, 1 *N* hydrochloric acid was added till pH ∼ 1. The mixture was then evaporated. Saturated sodium bicarbonate solution was then added and ethyl acetate was used to extract. The combined organic extracts were then washed with brine, before drying in anhydrous sodium sulfate. The mixture was then filtered and concentrated. The crude product was used immediately without further purification
(Tetrahedron Letters, 1998, 39, 3635).

### Step 2: Reduction of Nitrile

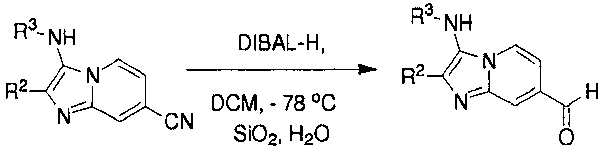

DIBAL-H (1.70 mL, 1.70 mmol) was added slowly into a stirred pre-dried solution of the nitrile (0.33 g, 1.13 mmol) and DCM (5 mL) at - 78 °C and the reaction was allowed to warm up to 40 °C over 1 h. Hydrolysis was effected by slowly adding a homogenous mixture of silica gel and water. After stirring for 1h at 0 °C, anhydrous potassium carbonate and magnesium sulfate solids were added, the solids were filtered off and rinsed thoroughly with DCM. The solvents were evaporated and the crude product was purified by flash column chromatography
(European Journal of Organic Chemistry, 1999, 2609-2621).

### Step 3: Wittig Reaction

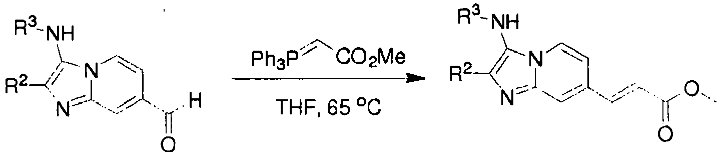

The Wittig reagent (0.12 g, 0.37 mmol) )was added slowly into a stirred solution of the aldehyde (0.11 g, 0.37 mmol) and THF (4 mL) and the reaction was allowed to warm up to 65°C overnight. When LCMS has indicated the completion of the reaction, the reaction mixture was concentrated and purified by flash column chromatography.

### Step 4: Hydroxamic Acid Formation

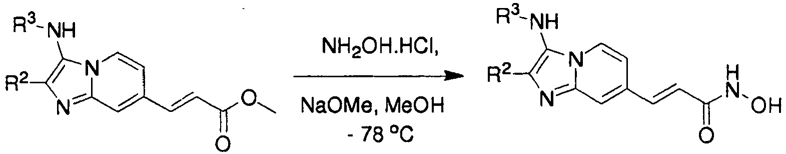

To a stirred solution of the ester (27.6 mg, 0.08 mmol), NH₂OH.HCl (55.4 mg, 0.80 mmol), MeOH (159 µL) at - 78°C was added NaOMe (365 µL, 1.60 mmol). The mixture was then allowed to warm up to room temperature. The reaction was monitored by LCMS. After the completion of the reaction, the mixture was cooled to - 78°C before 1*N* hydrochloric acid was added slowly to solubilize the mixture. Small amounts of H₂O and MeOH were added if necessary to dissolve the mixture. The crude was purified immediately by reversed phase prep-HPLC.

### (E)-N-hydroxy-3-(3-(2-methoxyethylamino)-2-)pentylimidazo[1,2-a]pyridin-7-yl)acrylamide (Compound 17)

HPLC: 98.86 %; t_{R} = 1.697 min; LCMS (ESI) Calcd for C₁₈H₂₆N₄O₃ [M⁺]: 346.431; found 347.12 [MH]⁺) ; ¹H NMR (400 MHz, MeOD): δ 8.60 (d, *J* = 7.2 Hz, 1H), 7.82 (s, 1H), 7.67 (d, *J* = 15.7 Hz, 1 H), 7.62 (dd, *J* = 7.4, 1.4 Hz, 1H), 6.76 (d, *J* = 15.7 Hz, 1H), 3.51 (t, *J* = 4.8 Hz, 2H), 3.36 (s, 3H), 3.25 (t, *J* = 5.1 Hz, 2H), 2.88 (d, *J* = 7.7 Hz, 2H), 1.80-1.75 (m, 2H), 1.45 - 1.38 (m, 4H), 0.97 (t, *J* = 7.0 Hz, 3H); ¹³C NMR (100.5 MHz, MeOD): 140.1, 137.8, 137.2, 130.8, 130.3, 126.6, 124.8, 114.9, 113.3, 111.9, 73.2, 59.2, 59.0, 32.6, 29.4, 24.8, 23.4, 14.2.

### Example 18

### Preparation of (E)-3-(3-(3-(ethylamino)-3-oxopropylamino)-2-hexylimidazo[1,2-a]pyridin-6-yl)-N-hydroxyacrylamide (Compound 18)

### Step 1: Ester Hydrolysis

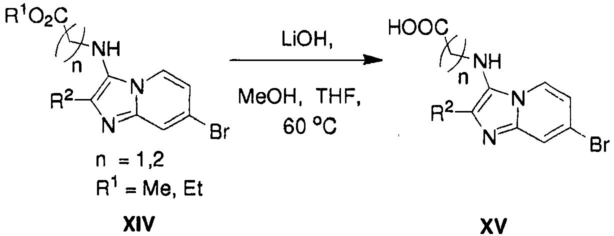

To a stirred solution of the ester (0.39 g, 1.06 mmol), MeOH (1.6 mL) and THF (8.4 mL) was added LiOH (45.7 mg, 1.9 mmol) and the reaction was stirred at 65°C for 4 h. When the reaction is completed, the reaction mixture was evaporated. The crude product was used immediately without further purification.

### Step 2: Acylation

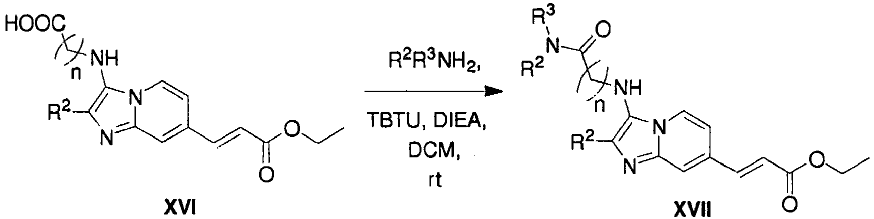

To a solution of the acid (40.0 mg, 0.103 mmol), DCM (2.0 mL) and DIEA (25.6 µL, 0.155 mmol) at room temperature was added TBTU (49.7 mg, 0.155 mmol). After stirring for ∼ 0.5h, the amine (0.155 mmol) was added. When the starting material has fully depleted, ethyl aceate (20 mL) was added to dilute the mixture. The organic contents were washed with saturated sodium bicarbonate solution and brine, before drying in anhydrous sodium sulfate. The mixture was then filtered and concentrated in vacuo. The crude product was used immediately without further purification.

### (E)-3-(3-(3-(ethylamino)-3-oxopropylamino)-2-hexylimidazo[1,2-a]pyridin-6-yl)-N-hydroxyacrylamide (Compound 18)

HPLC: 96.26 %; t_{R} = 1.845 min; LCMS (ESI) Calcd for C₂₀H₃₁N₅O₃ [M⁺]: 401.50, found 402.16 [MH]⁺; ¹H NMR (400 MHz, MeOD): δ 8.75 (s, 1H), 8.00 (d, *J* = 9.19 Hz, 1H), 7.67 (d, *J* = 9.3 Hz, 1H), 7.61 (d, *J* = 15.8 Hz, 1H), 6.61 (d, *J* = 15.7 Hz, 1H), 3.27 - 3.24 (m, 2H), 3.10 (q, *J* = 7.3 Hz, 2H), 2.78 (t, *J* = 7.7 Hz, 2H), 2.39 (t, *J* = 6.0 Hz, 2H), 1.71 - 1.63 (m, 2H), 1.34 - 1.31 (masked peaks), 1.29 - 1.25 (masked peaks), 1.01 (t, *J* = 7.3 Hz, 3H), 0.82 (t, *J* = 7.1 Hz, 3H); ¹³C NMR (100.5 MHz, MeOD): δ 173.8, 162.5, 137.6, 131.1, 130.3, 130.0, 126.6, 126.3, 122.5, 112.9, 45.2, 37.3, 35.3, 32.6, 30.1, 29.7, 24.9, 23.6, 14.8 14.4.

### Example 19

### (E)-3-(3-(3-(2-(dimethylamino)ethylamino)-3-oxopropylamino)-2-hexylimidazo[1,2-a]pyridin-6-yl)-N-hydroxyacrylamide (Compound 19)

The titled compound was prepared according to the procedures described in Example 18 by using appropriate starting materials.
HPLC: 99.95 %; t_{R} = 1.494 min; LCMS (ESI) Calcd for C₂₃H₃₆N₆O₃ [M⁺]: 444.57, found 445.18 [MH]⁺; ¹H NMR (400 MHz, MeOD): δ 8.96 (s, 1H), 8.11 (d, *J*= 9.0 Hz, 1H), 7.81 (d, *J* = 12.1 Hz, 1H ), 7.70 (d, *J* = 15.8 Hz, 1H), 6.71 (d, *J* = 15.8 Hz, 1H), 3.62 (t, *J* = 5.9 Hz, 2H), 3.35 (t, *J* = 6.6 Hz, 2H), 2.96 (s, 6H), 2.86 (t, *J* = 7.8 Hz, 2H), 2.59 (t, *J* = 6.1 Hz, 2H), 1.8 - 1.72 (m, 2H), 1.43 - 1.4 (m, 2H), 1.37 - 1.34 (m, 4H), 0.91 (t, *J* = 4.6 Hz, 3H); ¹³C NMR (100.5 MHz, d₄-MeOD): δ 175.4, 165.0, 163.0, 137.6, 135.7, 130.8, 130.2, 130.0, 126.8, 126.5, 122.5, 112.9, 58.6, 44.8, 43.9, 36.8, 35.8, 32.6, 30.0, 29.8, 24.8, 23.6, 14.3.

### Example 20

### 3-{3-[2-(2-Dimethylamino-ethylcarbamoyl)-ethylamino]-2-hexyl-imidazo[1,2-a]pyridin-7-yl}-N-hydroxy-acrylamide (Compound 20)

The titled compound was prepared according to the procedures described in Example 18 by using appropriate starting materials.
HPLC: 97.59 %; t_{R} = 1.524 min; LCMS (ESI) Calcd for C₂₃H₃₆N₆O₃ [M⁺]: 444.58; found 445.13 [MH]⁺; ¹H NMR (400 MHz, MeOD): δ 8.66 (d, *J*= 7.1 Hz, 1H), 7.86 (s, 1H), 7.69 - 7.64 (m, 2H), 6.79 (d, *J* = 15.8 Hz, 1 H), 3.59 (t, *J* = 5.9 Hz, 2H), 3.36 - 3.32 (m, 2H), 3.28 - 3.25 (m, 2H), 2.94 (s, 6H), 2.89 (t, *J* = 7.8 Hz, 2H), 2.58 '(t, *J* = 6.4 Hz, 2H), 1.81 - 1.74 (m, 2H), 1.44 - 1.31 (m, 6H), 0.92 (t, *J* = 6.9 Hz, 3H); ¹³C NMR (100.5 MHz, d₄-MeOD): δ 175.1, 164.5, 140.3, 137.9, 137.1, 130.5, 130.0, 126.3, 125.0, 115.2, 111.8, 58.4, 44.9, 43.8, 37.1, 35.7, 32.6, 30.1, 29.8, 24.9, 23.6, 14.3.

### Example 21

### 3-[3-(2-Butylcarbamoyl-ethylamino)-2-hexyl-imidazo[1,2-a]pyridin-7-yl]-N-hydroxy-acrylamide (Compound 21)

The titled compound was prepared according to the procedures described in Example 18 by using appropriate starting materials.
HPLC: 99.99 %; t_{R} = 2.301 min; LCMS (ESI) Calcd for C₂₃H₃₅N₅O₃ [M⁺]: 429.565; found 430.12 [MH]⁺; ¹H NMR (400 MHz, MeOD): δ 8.65 (d, *J*= 7.2 Hz, 1H), 7.83 (s, 1H), 7.70 - 7.64 (m, 2H), 6.77 (d, *J* = 15.7 Hz, 1 H), 3.33 - 3.33 (m, 2H), 3.19 - 3.16 (m, 2H), 2.89 - 2.86 (m, 2H), 2.49 (t, *J* = 6.2 Hz, 2H), 1.81 - 1.73 (m, 2H), 1.51 - 1.33 (m, 12H), 0.95 - 0.90 (m, 6H).

### Example 22

### 3-[3-(2-tert-Butylcarbamoyl-ethylamino)-2-hexyl-imidazo[1,2-a]pyridin-7-yl]-N-hydroxy-acrylamide (Compound 22)

The titled compound was prepared according to the procedures described in Example 18 by using appropriate starting materials.
HPLC: 99.99 %; t_{R} = 2.331 min; LCMS (ESI) Calcd for C₂₃H₃₆N₅O₃ [M⁺]: 429.565; found 430.12 [MH]⁺.

### Example 23

### 3-{2-Hexyl-3-[2-(2,2,2-trifluoro-ethylcarbamoyl)-ethylamino]-imidazo[1,2-a]pyridin-7-yl}-N-hydroxy-acrylamide (Compound 23)

The titled compound was prepared according to the procedures described in Example 18 by using appropriate starting materials.
HPLC: 99.99 %; t_{R} = 2.228 min; LCMS (ESI) Calcd for C₂₁H₂₈F₃N₅O₃ [M⁺]: 455.481; found 456.07 [MH]⁺; ¹H NMR (400 MHz, MeOD): δ 8.64 (d, *J* = 7.0 Hz, 1 H), 7.85 (s, 1 H), 7.69 - 7.63 (m, 2H), 6.79 (d, *J* = 15.7 Hz, 1 H), 5.48 (s, 2H), 3.98 - 3.91 (m, 2H), 3.41 - 3.33 (m, 2H), 2.93 - 2.81 (m, 2H), 1.78 - 1.73 (m, 2H), 1.44 - 1.30 (m, 6H), 0.92 (t, *J* = 6.9 Hz, 3H); ¹³C NMR (100.5 MHz, d₄-MeOD): δ 174.5, 164.5, 140.2, 137.9,137.1, 130.6, 129.9, 126.3, 124.9, 121.3, 115.0, 111.9, 54.8, 44.8, 36.9, 32.6, 30.0, 29.7, 24.9, 23.6, 14.3.

### Example 24

### 3-{2-Hexyl-3-[2-(2-methoxy-ethylcarbamoyl)-ethylamino]-imidazo[1,2-a]pyridin-7-yl}-N-hydroxy-acrylamide (Compound 24)

The titled compound was prepared according to the procedures described in Example 18 by using appropriate starting materials.
HPLC: 99.99 %; t_{R} = 1.875 min; LCMS (ESI) Calcd for C₂₂H₃₃N₅O₄ [M⁺]: 431.537; found 432.13 [MH]⁺; ¹H NMR (400 MHz, MeOD): δ 8.64 (d, *J* = 7.1 Hz, 1H), 7.85 (s, 1H), 7.69 - 7.63 (m, 2H), 6.78 (d, *J* = 15.7 Hz, 1 H), 3.46 (t, *J* = 5.4 Hz, 2H), 3.39 - 3.34 (m, 2H), 2.88 (t, *J* = 7.6 Hz, 2H), 2.51 (t, *J* = 6.1 Hz, 2H), 1.81 - 1.73 (m, 2H), 1.44 - 1.30 (m, 6H), 0.92 (t, *J* = 6.9 Hz, 3H); ¹³C NMR (100.5 MHz, d₄-MeOD): δ 174.1, 164.5, 140.2, 137.9, 137.2, 130.6, 130.0, 126.4, 124.9, 115.0, 111.9, 71.9, 58.9, 45.1, 40.3, 37.2, 32.6, 30.1, 29.8, 24.9, 23.6, 14.4.

### Example 25

### 3-{2-Hexyl-3-[2-(2-methylsulfanyl-ethylcarbamoyl)-ethylamino]-imidazo[1,2-a]pyridin-7-yl}-N-hydroxy-acrylamide (Compound 25)

The titled compound was prepared according to the procedures described in Example 18 by using appropriate starting materials.
HPLC: 98.07 %; t_{R} = 2.109 min; LCMS (ESI) Calcd for C₂₂H₃₃N₅O₃S [M⁺]: 447.602; found 448.07 [MH]⁺; ¹H NMR (400 MHz, MeOD): δ 8.64 (d, *J* = 7.1 Hz, 1H), 7.85 (s, 1 H), 7.69 - 7.63 (m, 2H), 6.78 (d, *J* = 15.7 Hz, 1 H), 3.46 (t, *J* = 5.4 Hz, 2H), 3.39 - 3.34 (m, 2H), 2.88 (t, *J* = 7.6 Hz, 2H), 2.51 (t, *J* = 6.1 Hz, 2H), 1.81 - 1.73 (m, 2H), 1.44-1.30 (m, 6H), 0.92 (t, *J* = 6.9 Hz, 3H); ¹³C NMR (100.5 MHz, d₄-MeOD): δ 174.1, 164.5, 140.2, 137.9, 137.2, 130.6, 130.0, 126.4, 124.9, 115.0, 111.9, 71.9, 58.9, 45.1, 40.3, 37.2, 32.6, 30.1, 29.8, 24.9, 23.6, 14.4.

### Example 26

### 3-[2-Hexyl-3-(2-prop-2-ynylcarbamoyl-ethylamino)-imidazo[1,2-a]pyridin-7-yl]-N-hydroxy-acrylamide (Compound 26)

The titled compound was prepared according to the procedures described in Example 18 by using appropriate starting materials.
HPLC: 99.08 %; t_{R} = 1.985 min; LCMS (ESI) Calcd for C₂₂H₂₉N₅O₃ [M⁺]: 411.506; found 412.11 [MH]⁺; ¹H NMR (400 MHz, MeOD): δ 8.65 (d, *J* = 7.1 Hz, 1 H), 7.84 (s, 1 H), 7.69 - 7.64 (m, 2H), 6.78 (d, *J* = 15.7 Hz, 1 H), 5.49 (s, 2H), 3.96 (d, *J* = 2.5 Hz, 2H), 3.38-3.34 (m, 2H), 2.88 (t, *J* = 7.6 Hz, 2H), 2.51 (t, *J* = 6.0 Hz, 2H), 1.81 - 1.73 (m, 2H), 1.45 - 1.30 (m, 6H), 0.92 (t, *J* = 6.9 Hz, 3H); ¹³C NMR (100.5 MHz, d₄-MeOD): δ 173.6, 164.5, 140.3, 137.9, 137.1, 130.6, 129.9, 126.4, 124.9, 115.1, 111.9, 80.6, 72.3, 44.9, 37.1, 132.6, 30.1, 29.7, 29.4, 24.9, 23.6, 14.4.

### Example 27

### 3-{2-Hexyl-3-[2-(1-hydroxymethyl-2-methyl-propylcarbamoyl)-ethylamino]-imidazo[1,2-a]pyridin-7-yl}-N-hydroxy-acrylamide (Example 27)

The titled compound was prepared according to the procedures described in Example 18 by using appropriate starting materials.
HPLC: 90.50 %; t_{R} = 2.049 min; LCMS (ESI) Calcd for C₂₄H₃₇N₅O₄ [M⁺]: 459.591; found 460.14 [MH]⁺; ¹H NMR (400 MHz, MeOD): δ 8.68 (d, *J* = 7.1 Hz, 1H), 7.84 (s, 1 H), 7.70 - 7.63 (m, 2H), 6.78 (d, *J* = 15.3 Hz, 1 H), 3.79 - 3.74 (m, 1 H), 3.64 (dd, *J* = 4.4, 11.2 Hz, 1 H), 3.55 (dd, *J* = 6.7, 11.2 Hz, 1 H), 3.36 - 3.34 (m, 2H), 2.89 (t, *J* = 7.7 Hz, 2H), 2.57 (t, *J* = 6.7 Hz, 2H), 1.91 - 1.83 (m, 1 H), 1.81 - 1.74 (m, 2H), 1.45 - 1.29 (m, 6H), 0.98 - 0.90 (m, 9H).

### Example 28

### 3-{3-[2-(2-Diethylamino-ethylcarbamoyl)-ethylamino]-2-hexyl-imidazo[1,2-a]pyridin-7-yl}-N-hydroxy-acrylamide (Compound 28)

The titled compound was prepared according to the procedures described in Example 18 by using appropriate starting materials.
HPLC: 99.99 %; t_{R} = 1.622 min; LCMS (ESI) Calcd for C₂₅H₄₀N₆O₃ [M⁺]: 472.634; found 473.14 [MH]⁺.

### Example 29

### 3-[3-(2-Ethylcarbamoyl-ethylamino)-2-hexyl-imidazo[1,2-a]pyridin-7-yl]-N-hydroxy-acrylamide (Compound 29)

The titled compound was prepared according to the procedures described in Example 18 by using appropriate starting materials.
HPLC: 91.98 %; t_{R} = 1.926 min; LCMS (ESI) Calcd for C₂₁H₃₁N₅O₃ [M⁺]: 401.511; found 402.10 [MH]⁺; ¹H NMR (400 MHz, MeOD): δ 8.48 (d, *J* = 7.2 Hz, 1 H), 7.70 (s, 1 H), 7.64 (d, *J* = 15.5 Hz, 1H), 7.45 (d, *J* = 7.8 Hz, 1 H), 6.67 (d, *J* = 15.8 Hz, 1H), 3.68 - 3.35 (m, 2H), 3.27 - 3.25 (m, 2H), 2.83 (t, *J* = 7.4 Hz, 2H), 2.46 (t, *J* = 6.2 Hz, 2H), 1.78 - 1.74 (m, 1 H), 1.42 - 1.33 (m, 2H), 1.12 (t, *J* = 7.3 Hz, 3H), 0.93 - 0.91 (m, 3H).

### Example 30

### 3-[3-(2-Dimethylcarbamoyl-ethylamino)-2-hexyl-imidazo[1,2-a]pyridin-7-yl]-N-hydroxy-acrylamide (Compound 30)

The titled compound was prepared according to the procedures described in Example 18 by using appropriate starting materials.
HPLC: 99.9 % t_{R} = 1.990 min; LCMS (ESI) Calcd for C₂₁H₃₁N₅O₃[M⁺]: 401.50, found 402.10 [MH]⁺; ¹H NMR (400 MHz, d₄-MeOD): δ 8.71 (d, *J* = 7.5 Hz, 1 H), 7.83 (s, 1 H), 7.73 (d, *J* = 19.0 Hz, 1H), 7.66 (d, *J* = 6.4 Hz, 1H), 6.77 (d, *J* = 15.6 Hz, 1H), 3.27-3.25 (m, 2H), 3.10 (d, *J*= 2.6 Hz, 3H), 2.97 (d, *J*= 2.8 Hz, 3H), 2.88 (t, *J*= 7.4 Hz, 2H), 2.71 (t, *J* = 5.9 Hz, 2H), 1.77 (brs, 2H), 1.38 - 1.30 (m, 6H), 0.92 (t, *J* = 6.8 Hz, 3H).

### Example 31

### 3-{3-[2-(Cyanomethyl-methyl-carbamoyl)-ethylamino]-2-hexyl-imidazo[1,2-a]pyridin-7-yl}-N-hydroxy-acrylamide (Compound 31)

The titled compound was prepared according to the procedures described in Example 18 by using appropriate starting materials.
HPLC: 99.9 % t_{R} = 1.958 min; LCMS (ESI) Calcd for C₂₂H₃₀N₆O₃[M⁺]: 426.51, found 430.11 [MH]⁺; ¹H NMR (400 MHz, d₄-MeOD): δ 8.56 (d, *J* = 7.6 Hz, 1H), 7.78 (s, 1H), 7.69 (d, *J* = 16.2 Hz, 1H), 7.61 (d, *J* = 7.6 Hz, 1H), 6.73 (d, *J* = 16.0 Hz, 1H), 3.43-3.43 (m, 2H), 3.07 - 3.07 (m, 2H), 2.82 (t, *J* = 7.6 Hz, 2H), 2.46 (t, *J*= 6.3 Hz, 2H), 1.72 (t, *J* = 7.3 Hz, 2H), 1.31 - 1.23 (m, 6H), 0.94 - 0.82 (m, 3H).

### Example 32

### 3-(3-{2-[(2-Dimethylamino-ethyl)-methyl-carbamoyl]-ethylamino}-2-hexyl-imidazo[1,2-a]pyridin-7-yl)-N-hydroxy-acrylamide (Compound 32)

The titled compound was prepared according to the procedures described in Example 18 by using appropriate starting materials.
HPLC: 99.9 %; t_{R} = 1.631 min LCMS (ESI) Calcd for C₂₄H₃₈N₅O₃[M⁺]: 458.61, found 459.15 [MH]⁺; ¹H NMR (400 MHz, d₄-MeOD): δ 8.64 (d, *J* = 6.5 Hz, 1H), 7.88 (s, 1 H), 7.66 (d, *J* = 15.7 Hz, 1H), 7.54 (d, *J* = 6.2 Hz, 1H), 6.73 (d, *J* = 15.7 Hz, 1H), 3.79 (brs, 2H), 3.37 - 3.35 (m, 2H), 3.27 - 3.25 (m, 2H), 3.12 (s, 3H), 2.96 (s, 6H), 2.86 (t, *J* = 7.5 Hz, 2H), 2.76 (brs, 2H), 1.79 - 1.74 (m, 2H), 1.43 - 1.30 (m, 6H), 0.92 (t, *J* = 7.2 Hz, 3H).

### Example 33

### 3-(2-Hexyl-3-{2-[(2-hydroxy-ethyl)-propyl-carbamoyl]-ethylamino}-imidazo[1,2-a]pyridin-7-yl)-N-hydroxy-acrylamide (Example 33)

The titled compound was prepared according to the procedures described in Example 18 by using appropriate starting materials.
HPLC: 99.9 %; t_{R} = 2.137 min LCMS (ESI) Calcd for C₂₄H₃₇N₅O₄[M⁺]: 459.58, found 460.13 [MH]⁺; ¹H NMR (400 MHz, d₄-MeOD): δ 8.69 (d, *J* = 7.2 Hz, 1 H), 7.80 (s, 1 H), 7.68 (d, *J* = 15.2 Hz, 1 H), 7.62 (d, *J* = 7.5 Hz, 1 H), 6.76 (d, *J* = 15.8 Hz, 1 H), 3.73-3.68 (m, 2H), 3.51 (t, *J* = 6.00 Hz, 2H), 3.41 - 3.38 (m, 2H), 3.34 - 3.33 (masked peaks), 2.87 (t, *J* = 7.69 Hz, 2H), 2.77 (t, *J* = 5.9 Hz, 1 H), 2.74 (t, *J* = 5.8 Hz, 1 H), 1.77 (t, *J* = 5.1 Hz, 2H), 1.70 - 1.64 (m, 1 H), 1.62 - 1.56 (m, 1 H), 1.45 -1.30 (m, 6H), 0.98-0.89 (m, 6H).

### Example 34

### N-Hydroxy-3-(2-phenyl-imidazo[1,2-a]pyridin-7-yl)-acrylamide (Compound 34)

The titled compound was prepared according to the procedures described in Scheme V and Example 38 by using appropriate starting materials.
HPLC: 99.99 %; t_{R} = 1.345 min; LCMS (ESI) Calcd for C₁₆H₁₃N₃O₂ [M⁺]: 279.299; found 280.01 [MH]⁺; ¹H NMR (400 MHz, MeOD): δ 8.72 (d, *J* = 7.1 Hz, 1 H), 8.54 (s, 1 H), 7.96 (s, 1 H), 7.91 - 7.89 (m, 2H), 7.72 - 7.65 (m, 2H), 7.61 - 7.53 (m, 3H), 6.80 (d, *J* = 15.7 Hz, ¹H); ¹³C NMR (100.5 MHz, d₄-MeOD): δ 164.4, 142.6, 140.9, 139.6, 137.1, 131.7, 130.7, 129.9, 128.2, 127.5, 125.2, 115.8, 112.7, 112.5.

### Example 35

### 3-{3-[2-(3-Dimethylamino-2,2-dimethyl-propylcarbamoyl)-ethylamino]-2-hexyl-imidazo[1,2-a]pyridin-7-yl}-N-hydroxy-acrylamide (Compound 35)

The titled compound was prepared according to the procedures described in Example 18 by using appropriate starting materials.
HPLC: 99.9 %; t_{R} = 1.666 min, LCMS (ESI) Calcd for C₂₆H₄₂N₆O₃[M⁺]: 486.65,found 487.19 [MH]⁺; ¹H NMR (400 MHz, d₄-MeOD): δ 8.67 (d, *J* = 7.2 Hz, 1 H), 7.87 (s, 1 H), 7.70 - 7.64 (m, 2H), 6.78 (d, *J* = 15.8 Hz, 1 H), 3.38 - 3.35 (m, 2H), 3.22 (s, 1 H), 3.02 (s, 1 H), 2.96 (s, 1 H), 2.91 - 2.87 (m, 2H), 2.62 (t, *J* = 6.45 Hz, 2H), 1.81 -1.74 (m, 2H), 1.45 - 1.35 (m, 6H), 1.10 (s, 1 H), 0.92 (t, *J* = 6.9 Hz, 3H); ¹³C NMR (100.5 MHz, d₄-MeOD): δ 173.3, 138.3, 135.1, 128.5, 128.0, 124.3, 113.3, 109.8, 64.9, 45.4, 45.1, 43.1, 35.0, 34.9, 30.6, 28.1, 27.8, 22.9, 22.7, 21.6, 12.4.

### Example 36

### 3-[2-Hexyl-3-(2-methylcarbamoyl-ethylamino)-imidazo[1,2-a]pyridin-7-yl-N-hydroxy-acrylamide (Compound 36)

The titled compound was prepared according to the procedures described in Example 18 by using appropriate starting materials.
HPLC: 99.9 %; t_{R} = 1.800 min, LCMS (ESI) Calcd for C₂₀H₂₉N₅O₃[M⁺]: 387.484, found 388.12 [MH]⁺; ¹H NMR (400 MHz, d₄-MeOD): δ 8.68 (d, *J* = 7.2 Hz, 1 H), 7.83 (s, 1 H), 7.70 - 7.65 (m, 2H), 6.78 (d, *J* = 15.8 Hz, 1 H), 3.36 - 3.34 (m, 2H), 3.22 (s, 1 H), 2.98 (t, *J* = 7.6 Hz, 2H), 2.96 (s, 1H), 2.91 - 2.87 (m, 2H), 2.62 (t, *J* = 6.5 Hz, 2H), 2.49 (t, *J* = 6.2 Hz, 2H), 1.81 - 1.73 (m, 2H), 1.45 - 1.36 (m, 6H), 0.94 (t, *J* = 6.9 Hz, 3H); ¹³C NMR (100.5 MHz, d₄-MeOD): δ 172.5, 124.4, 113.0, 110.0, 35.1, 30.6, 28.1, 27.8, 24.3, 22.9, 21.6, 12.4.

### Example 37

### 3-{2-Hexyl-3-[2-(isopropyl-methyl-carbamoyl)-ethylamino]-imidazo[1,2-a]pyridin-7-yl}-N-hydroxy-acrylamide (Compound 37)

The titled compound was prepared according to the procedures described in Example 18 by using appropriate starting materials.

HPLC: 99.9 %; t_{R} = 2.301 min, LCMS (ESI) Calcd for C₂₃H₃₅N₅O₃[M⁺]: 429.565, found 430.12 [MH]⁺; ¹H NMR (400 MHz, d₄-MeOD): δ 8.74 (d, *J* = 7.1 Hz, 1 H), 7.83 (s, 1 H), 7.70 - 7.65 (m, 2H), 6.78 (d, *J* = 16.9 Hz, 1 H), 4.24 - 4.22 (m, 1H), 3.37- 3.34 (m, 2H), 2.91 (s, 3H), 2.88 (t, *J* = 7.9 Hz, 2H), 2.76 (dt, *J* = 6.4 Hz, 28.2Hz, 2H), 1.81 - 1.74 (m, 2H), 1.45 - 1.29 (m, 6H), 1.24 (d, *J* = 6.6 Hz, 1 H), 1.13 (d, *J* = 6.8 Hz, 1 H), 0.92 (t, *J* = 6.9 Hz, 3H); ¹³C NMR (100.5 MHz, d₄-MeOD): δ 124.5, 122.9, 109.9, 30.6, 28.1, 27.8, 21.6,18.4,17.6,12.4.

### Example 38

### 3-(2-Hexyl-3-{2-[isopropyl-(2-methoxy-ethyl)-carbamoyl]-ethylamino}-imidazo[1,2-a]pyridin-7-yl)-N-hydroxy-acrylamide (Compound 38)

The titled compound was prepared according to the procedures described in Example 18 by using appropriate starting materials.
HPLC: 99.9 %; t_{R} = 2.381 min, LCMS (ESI) Calcd for C₂₅H₃₉N₅O₄[M⁺]: 473.618, found 474.15 [MH]⁺; ¹H NMR (400 MHz, d₄-MeOD): δ 8.70 (dd. *J* = 13.6, 72 Hz, 1H), 7.84 (s, 1 H), 7.70 - 7.65 (m, 2H), 6.78 (d, *J* = 15.7 Hz, 1 H), 3.53 (s, 3H), 3.49 - 3.43 (m, 2H), 3.37 - 3.33 (m, 4H), 2.88 (t, *J* = 7.6 Hz, 2H), 2.76 (dt, *J* = 6.0, 2.4 Hz, 2H), 1.81 - 1.74 (m, 2H), 1.45 - 1.36 (m, 6H), 1.24 (d, *J* = 6.7 Hz, 3H), 1.21 (d, *J* = 6.9 Hz, 3H), 0.92 (t, *J* = 7.0 Hz, 3H).

### Example 39

### Preparation of 3-(3-Butylaminomethyl-2-phenyl-imidazo[1,2-a]pyridin-7-yl-N-hydroxy-acrylamide (Compound 39)

### Step 1: Condensation reaction

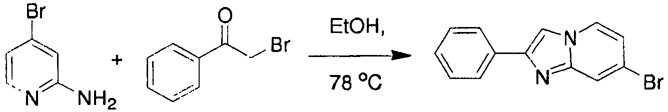

To a stirred solution of the amino-pyridine (0.10 g, 0.578 mmol) and EtOH (1.4 mL) was added the ketone (0.14 g, 0.6934 mmol) and the mixture was then stirred at 78 °C for 4 h. When the reaction has completed, the contents were evaporated. Saturated sodium carbonate solution was added and ethyl aceate was used to extract the aqueous layer. The combined organic extracts were then washed with water and followed by brine, before drying in anhydrous sodium sulfate. The contents were then filtered and concentrated. The crude product was used immediately without further purification (Journal of Medicinal Chemistry, **1998,** *41(25),* 5108).

### Step 2: Hydroxymethylation

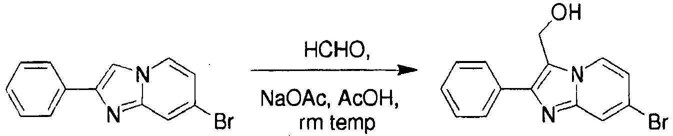

To a stirred solution of the aryl-bromide (179 mg, 0.656 mmol), HCHO (315 µL, 4.20 mmol) and AcOH (572 µL) was added NaOAc (203 mg, 2.47 mmol). When the reaction has completed, the contents were evaporated. Saturated sodium carbonate solution was added and ethyl aceate was used to extract the aqueous layer. The combined organic extracts were then washed with water and followed by brine, before drying in anhydrous sodium sulfate. The contents were then filtered and concentrated. The crude product was used immediately without further purification (Journal of Medicinal Chemistry, 1998, 41(25), 5108).

### Step 3: Heck Reaction

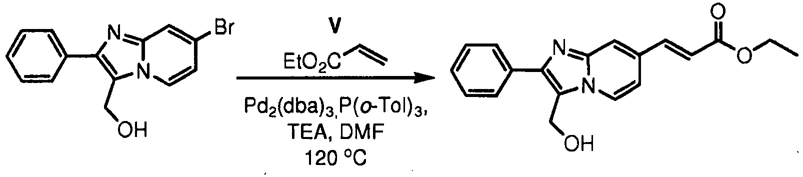

Ethyl acrylate (1.5 equiv) was added into a stirred suspension of the amine (1 equiv), Pd₂(dba)₃ (0.03 equiv), P(o-tol)₃ (0.08 equiv), Et₃N (2.0 equiv) and DMF (0.3 M) at room temp. The reaction was heated to reflux at ∼ 120 °C. When the starting material had fully depleted (monitored by LCMS), the reaction mixture was diluted with ethyl acetate (20 mL). The organic layer was then washed with NaHCO3 (2 x 10 mL) and brine (2 x 10 mL). The organic layer was dried in Na₂SO₄ before being filtered and concentrated *in vacuo.* The crude product was purifed by flash column chromatography.

### Step 4: Oxidation

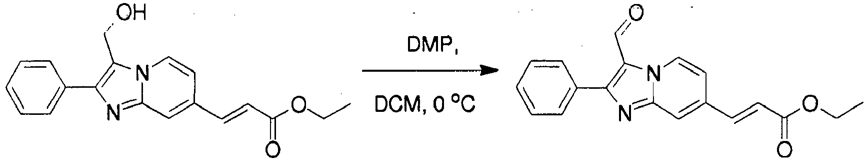

To a stirred solution of the alcohol (162 mg, 0.50 mmol) in DCM (25 mL) at 0 oC was added DMP (319 mg, 0.75 mmol). When the reaction has completed, a solution of saturated sodium bicarbonate and saturated sodium sulfate (1:1 mixture) was added. DCM was used to extract the aqueous layer. The combined organic extracts were dried in anhydrous sodium sulfate before being filtered and concentrated. The crude product was purified by flash column chromatography.

### Step 5: Reductive Amination

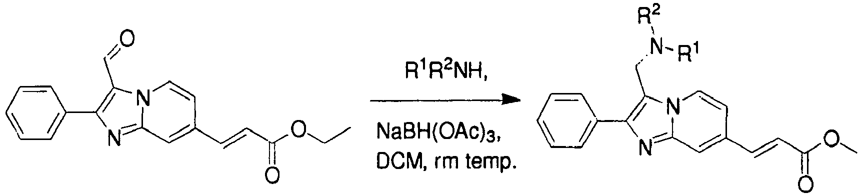

To a stirred solution of the aldehyde (43.9 mg, 0.137 mmol) and amine (0.821 mmol) in DCM (10 mL) was added NABH(OAc)₃ (120 mg, 0.548 mmol). When the reaction has completed, the contents were diluted with DCM. The organic contents were washed with saturated sodium bicarbonate, water and brine, before drying in anhydrous sodium sulfate. The contents were then filtered and concentrated. The crude product was used immediately without further purification.

### 3-(3-Butylaminomethyl-2-phenyl-imidazo[1,2-a]pyridin-7-yl)-N-hydroxy-acrylamide (Compound 39)

The titled compound was prepared according to the procedures described in Example 39 by using appropriate starting materials.
HPLC: 99.99 %; t_{R} = 1.000 min; LCMS (ESI) Calcd for C₂₁H₂₄N₄O₂ [M⁺]: 364.449; found 365.07 [MH]⁺; ¹H NMR (400 MHz, MeOD): δ 8.76 (d, *J* = 6.7 Hz, 1 H), 7.88 (s, 1 H), 7.81 - 7.79 (m, 2H), 7.64 - 7.52 (m, 5H), 6.71 (d, *J* = 15.7 Hz, 1 H), 4.92 (masked peaks, 2H), 2.93 (t, *J* = 8.0 Hz, 2H), 1.57 - 1.51 (m, 2H), 1.29 - 1.24 (m, 2H), 0.87 (t, *J* = 7.3 Hz, 3H); ¹³C NMR (100.5 MHz, d₄-MeOD): δ 164.9, 146.8, 137.9, 137.8, 131.9, 131.0, 130.5, 130.1, 127.2, 123.2, 115.8, 114.3, 113.6, 39.8, 28.8, 20.8, 13.7.

### Example 40

### N-Hydroxy-3-{3-[(methyl-propyl-amino)-methyl]-2-phenyl-imidazo[1,2-a]pyridin-7-yl}-acrylamide (Compound 40)

The titled compound was prepared according to the procedures described in Example 39 by using appropriate starting materials.
HPLC: 99.99 %; t_{R} = 0.630 min; LCMS (ESI) Calcd for C₂₁H₂₄N₄O₂ [M⁺]: 364.449; found 365.03 [MH]⁺; ¹H NMR (400 MHz, MeOD): δ 8.81 (d, *J* = 7.0 Hz, 1H), 7.96 (s, 1 H), 7.81 - 7.79 (m, 2H), 7.69 - 7.56 (m, 5H), 6.73 (d, *J* = 15.7 Hz, 1 H), 5.03 (s, 2H), 2.96 - 2.94 (m, 2H), 2.69 (s, 3H), 1.61 - 1.57 (m, 2H), 0.79 (t, *J* = 7.3 Hz, 3H); ¹³C NMR (100.5 MHz, d₄-MeOD): δ 164.9, 147.2, 145.6, 138.3, 137.8, 131.7, 131.3, 130.7, 130.2, 127.3, 123.6, 115.6, 113.9, 113.5, 58.3, 40.2, 18.4, 10.9.

### Example 41

### N-Hydroxy-3-(2-methyl-imidazo[1,2-a]pyridin-7-yl)-acrylamide (Compound 41)

The titled compound was prepared according to the procedures described in Example 39 by using appropriate starting materials.
HPLC: 100.00 %; t_{R} = 0.348 min; LCMS (ESI) Calcd for C₁₁H₁₁N₆O₂ [M⁺]: 217.085; found 218.03 [MH]⁺; ¹H NMR (400 MHz, d₄-MeOD): δ 8.57 (d, *J* =7.1 Hz, 1H), 7.86 (s, 1H), 7.82 (s, 1 H), 7.58 (d, *J*= 15.8 Hz, 1 H), 7.54 (d, *J* = 1.5 Hz, 1H), 6.68 (d, *J*= 15.8 Hz, 1 H), 2.46 (d, *J* = 0.9 Hz, 3H).

### Example 42

### Preparation of 3-(3-Butylaminomethyl-2-methyl-imidazo[1,2-a]pyridin-7-yl)-N-hydroxy-acrylamide (Compound 42))

### Step 1: Condensation reaction

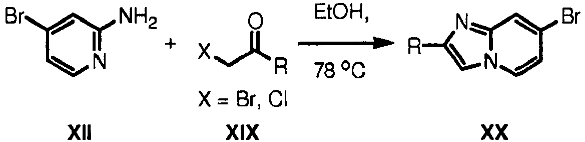

To a stirred solution of the amino-pyridine **XII** (0.10 g, 0.578 mmol) and EtOH (1.4 mL) was added the ketone **XIX** (0.14 g, 0.6934 mmol) and the mixture was then stirred at 78 °C for 4 h. When the reaction has completed, the contents were evaporated. Saturated sodium carbonate solution was added and ethyl acetate was used to extract the aqueous layer. The combined organic extracts were then washed with water and followed by brine, before drying in anhydrous sodium sulfate. The contents were then filtered and concentrated. The crude product was used immediately without further purification
(Journal of Medicinal Chemistry, 1998, 41(25), 5108).

### Step 2: Heck reaction

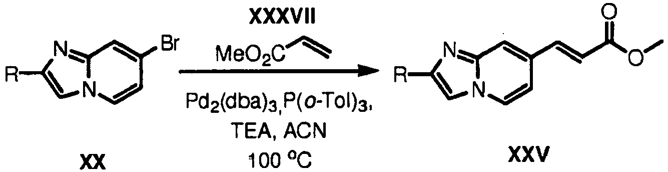

Methyl acrylate (1.5 equiv) was added into a stirred suspension of the amine **XX** (1 equiv), Pd₂(dba)₃ (0.02 equiv), P(o-Tol)₃ (0.05 equiv), Et₃N (2.0 equiv) and CH₃CN (0.3 M) at room temperature. The reaction was heated to reflux at ∼ 100 °C. When the starting material had fully depleted (monitored by LCMS), the reaction mixture was diluted with ethyl acetate. The organic layer was then washed with NaHCO₃ and brine. The organic layer was dried in Na₂SO₄ before being filtered and concentrated in vacuo. The crude product was purifed by flash column chromatography.

### Step 3: Mannich reaction and Hydroxamic acid formation

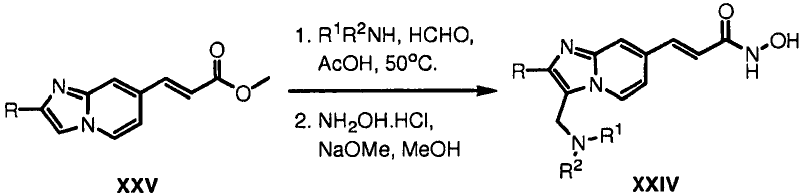

The amine (3.0 equiv) was added slowly into a stirred solution of the imidazol[1,2-α]pyridinyl methyl ester **XXV** (1.0 equiv), formaldehyde solution (3.0 equiv) and AcOH (20 equiv) and the mixture was heated up to 50 °C. When the starting material has fully depleted (monitored by LCMS), the crude product was used immediately for the next step.

To a stirred solution of the crude material from the Mannich reaction and NH₂OH.HCl (20 equiv) was added NaOMe (40 equiv) at - 78 °C. The reaction mixture was allowed to warm up slowly to room temperature. When the reaction is completed, the mixture was cooled to 0 °C before using 1 M HCl to quench the reaction. Small amounts of MeOH and H₂O were added to solubilize the mixture. The crude product was purified by reversed-phase prep-HPLC.

### 3-(3-Butylaminomethyl-2-methyl-imidazo[1,2-a]pyridin-7-yl)-N-hydroxy-acrylamide (Compound 42)

HPLC: 100.00 %; t_{R} = (LC/PDA: Xterra 1S column, 4.6 x 20mm 3.5µ column; 2.0 ml/min, gradient 1-10% B over 6 min, Solvent A: H₂O with 0.1% TFA; Solvent B: acetonitrile with 0.1 TFA; UV 254): 0.332 min; LCMS (ESI) Calcd for C₁₆H₂₂N₄O₂ [M⁺]: 302.174; found 303.11 [MH]⁺; ¹H NMR (400 MHz, d₄-MeOD): δ 8.74 (dd, *J* = 7.1, 7.2 Hz, 1H), 7.93 (d, *J* = 14.6, 1 H), 7.72 - 7.61 (m, 2H), 6.80 (d, *J* = 15.6 Hz, 1 H), 5.03 (s, 2H), 2.62 (s, 3H), 2.57 (s, 3H), 1.80 - 1.70 (m, 2H), 1.47 (q, *J* = 7.5 Hz, 2H), 1.01 (t, *J* = 3.0 Hz, 2H).

### Example 43

### 3-{2-tert-Butyl-3-[(2-diethylamino-ethylamino)-methyl]-imidazo[1,2-a]pyridin-7-yl}-N-hydroxy-acrylamide (Compound 43)

The titled compound was prepared according to the procedures described in Example 42 by using appropriate starting materials.
HPLC: 100.00 %; t_{R} = 0.628 min; LCMS (ESI) Calcd for C₂₁H₃₃N₅O₂ [M⁺]: 387.263; found 388.21 [MH]⁺; ¹H NMR (400 MHz, d₄-MeOD): δ 8.84 (d, *J* = 7.2 Hz, 1H), 7.91 (s, 1H), 7.70 - 7.67 (m, 2H), 6.82 (d, *J* = 15.7 Hz, 1 H), 4.44 (s, 2H), 3.34 - 3.33 (m, 4H), 3.27 - 3.20 (m, 4H) 1.58 (s, 9H), 1.31 (t, *J* = 7.3 Hz, 6H).

### Example 44

### 3-(3-{[(2-Dimethylamino-ethyl)-ethyl-amino]-methyl}-2-phenyl-imidazo[1,2-a]pyridin-7-yl)-N-hydroxy-acrylamide (Compound 44)

The titled compound was prepared according to the procedures described in Example 42 by using appropriate starting materials.
HPLC: 98.14%; t_{R} = 1.216 min; LCMS (ESI) Calcd for C₂₃H₂₉N₅O₂ [M⁺]: 407.518; found 408.16 [MH]⁺; ¹H NMR (400 MHz, d₄-MeOD): δ 8.86 (d, *J* = 7.1 Hz, 1 H), 8.03 (s, 1H), 7.80 - 7.75 (m, 3H), 7.71 - 7.64 (m, 4H), 6.86 (d, *J* = 15.7 Hz, 1 H), 5.09 (masked peaks), 3.27 (t, *J* = 6.6 Hz, 2H), 2.90 (t, *J* = 6.6 Hz, 2H), 2.79 (s, 6H), 1.04 (t, *J* = 7.1 Hz, 3H); ¹³C NMR (100.5 MHz, d₄-MeOD): δ 164.3, 141.6, 137.9, 136.8, 132.0, 130.7, 130.4, 130.0, 129.1, 128.6, 127.6, 125.8, 120.9, 116.0, 112.2, 55.3, 52.9, 48.3, 47.4, 46.9,43.9, 9.8.

### Example 45

### 3-[3-(tert-Butylamino-methyl)-2-phenyl-imidazo[1,2-a]pyridin-7-yl]-N-hydroxy-acrylamide (Compound 45)

The titled compound was prepared according to the procedures described in Example 42 by using appropriate starting materials.
HPLC: 98.86 %; t_{R} = 0.881 min; LCMS (ESI) Calcd for C₂₁H₂₄N₄O₂ [M⁺]: 364.449; found 365.12 [MH]⁺; ¹H NMR (400 MHz, d₄-MeOD): δ 8.82 (s, 1H), 7.96 (s, 1 H), 7.82 (d, *J* = 6.1 Hz, 2H), 7.66 - 7.59 (m, 5H), 6.74 (d, *J* = 15.7 Hz, 1 H), 4.90 (masked peaks), 1.41 (s, 9H); ¹³C NMR (100.5 MHz, d₄-MeOD): δ 164.5, 143.8, 143.5, 140.0, 137.3, 131.8, 131.5, 130.6, 130.3, 129.5, 127.7, 124.5, 119.3, 116.4, 114.9, 114.2, 59.7, 34.4, 25.6.

### Example 46

### N-Hydroxy-3-{2-phenyl-3-[(2,2,2-trifluoro-ethylamino)-methyl]-imidazo[1,2-a]pyridin-7-yl}-acrylamide (Compound 46)

The titled compound was prepared according to the procedures described in Example 42 by using appropriate starting materials.
HPLC: 92.99 %; t_{R} = 1.889 min; LCMS (ESI) Calcd for C₁₉H₁₇F₃N₄O₂ [M⁺]: 390.365; found 391.07 [MH]⁺; ¹H NMR (400 MHz, d₄-MeOD): δ 8.92 (d, *J* = 7.2 Hz, 1 H), 8.02 (s, 1 H), 7.78 - 7.76 (m, 3H), 7.71 (d, *J* = 15.7 Hz, 1 H), 7.65 - 7.63 (m, 3H), 6.86 (d, *J* = 15.7 Hz, 1 H), 4.92 (masked peaks), 4.43 (s, 2H); ¹³C NMR (100.5 MHz, d₄-MeOD): δ 164.3, 141.6, 141.4, 136.8, 136.6, 131.9, 130.6, 130.1, 129.1, 128.7, 127.7, 125.9, 122.5, 119.1, 116.2, 115.7, 111.9, 49.9 (masked peaks), 42.1.

### Example 47

### 3-{3-[(2-Diethylamino-ethylamino)-methyl]-2-phenyl-imidazo[1,2-a]pyridin-7-yl}-N-hydroxy-acrylamide (Compound 47)

The titled compound was prepared according to the procedures described in Example 42 by using appropriate starting materials.
HPLC: 97.82 %; t_{R} = 0.680 min; LCMS (ESI) Calcd for C₂₃H₂₉N₅O₂ [M⁺]: 407.518; found 408.17 [MH]⁺; ¹H NMR (400 MHz, d₄-MeOD): δ 9.09 (d, *J* = 6.3 Hz, 1H), 8.05 (s, 1H), 7.86 - 7.83 (m, 2H), 7.73 (d, *J* = 6.3 Hz, 1 H), 7.67 - 7.66 (m, 3H), 7.56 (d, *J* = 15.7 Hz, 1 H), 6.80 (d, *J* = 15.7 Hz, 1 H), 5.01 (s, 2H), 3.51 - 3.50 (m, 4H), 3.21 (t, *J* = 7.2 Hz, 4H), 1.28 (t, *J* = 7.2 Hz, 6H); ¹³C NMR (100.5 MHz, d₄-MeOD): δ 164.3, 142.3, 141.3, 140.3, 136.9, 132.1, 130.8, 130.3, 129.3, 128.8, 125.3, 122.2, 120.2, 116.4, 115.8, 112.7, 49.1, 43.5, 40.8, 8.9.

### Example 48

### N-Hydroxy-3-(3-{[(2-hydroxy-ethyl)-propyl-amino]-methyl}-2-phenyl-imidazo[1,2-a]pyridin-7-yl)-acrylamide (Compound 48)

The titled compound was prepared according to the procedures described in Example 42 by using appropriate starting materials.
HPLC: 99.99 %; t_{R} = 0.758 min; LCMS (ESI) Calcd for C₂₂H₂₆N₄O₃ [M⁺]: 394.475; found 395.11 [MH]⁺; ¹H NMR (400 MHz, d₄-MeOD): δ 8.83 (d, *J* = 7.1 Hz, 1 H), 7.85 (s, 1 H), 7.79 (d, *J* = 6.7 Hz, 2H), 7.65 - 7.59 (m, 3H), 7.56 (d, *J*= 15.7 Hz, 1 H), 7.49 (d, *J* = 6.9 Hz, 1H), 6.70 (d, *J* = 15.7 Hz, 1 H), 5.10 (s, 2H), 3.98 (t, *J* = 4.8 Hz, 2H), 3.50 - 3.43 (m, 2H), 2.78 - 2.74 (m, 2H), 1.43 - 1.39 (m, 2H), 1.35 (t, *J* = 7.3 Hz, 3H), 0.52 (t, *J* = 7.2 Hz, 3H); ¹³C NMR (100.5 MHz, d₄-MeOD): δ 164.8, 146.3, 145.0, 138.7, 137.7, 131.4, 131.1, 130.7, 130.4, 128.0, 123.8, 115.1, 114.4, 113.9, 57.4, 55.8, 55.5, 45.6, 35.1, 17.1, 10.8, 9.3.

### Example 49

### 3-(2-tert-Butyl-3-butylaminomethyl-imidazo[1,2-a]pyridin-7-yl)-N-hydroxy-acrylamide (Compound 49)

The titled compound was prepared according to the procedures described in Example 42 by using appropriate starting materials.
HPLC: 94.10 %; t_{R} = 0.706 min; LCMS (ESI) Calcd for C₁₉H₂₈N₄O₂ [M⁺]: 344.221; found 345.18 [MH]⁺; ¹H NMR (400 MHz, d₄-MeOD): δ 8.38 (d, *J* = 11.9 Hz, 1H), 7.61 (s, 1H), 7.54 (d, *J* = 15.8, 1 H), 7.21 (d, *J* = 7.2, 1H), 6.55 (d, *J* = 15.7 Hz, 1H), 4.84 (masked peaks), 3.20 - 3.12 (m, 2H), 1.73 - 1.65 (m, 2H), 1.47 (s, 9H), 1.44 - 1.38 (m, 2H), 0.95 (t, *J*= 7.3 Hz, 3H).

### Example 50

### 3-{2-tert-Butyl-3-[(methyl-propyl-amino)-methyl]-imidazo[1,2-a]pyridin-7-yl}-N-hydroxy-acrylamide (Compound 50)

The titled compound was prepared according to the procedures described in Example 42 by using appropriate starting materials.
HPLC: 100.00 %; t_{R} = (LC/PDA: Xterra 1S column, 4.6 x 20mm 3.5µ column; 2.0 ml/min, gradient 1-10% B over 6 min, Solvent A: H₂O with 0.1% TFA; Solvent B: acetonitrile with 0.1 TFA; UV 254): 0.499 min; LCMS (ESI) Calcd for C₁₉H₂₈N₄O₂ [M⁺]: 344.221; found 345.20 [MH]⁺.

### Example 51

### 3-(3-Diethylaminomethyl-2-phenyl-imidazo[1,2-a]pyridin-7-yl)-N-hydroxy-acrylamide (Compound 51)

The titled compound was prepared according to the procedures described in Example 42 by using appropriate starting materials.
HPLC: 99.16 %; t_{R} = 0.708 min;LCMS (ESI) Calcd for C₂₁H₂₄N₄O₂ [M⁺]: 364.449; found 365.13 [MH]⁺; ¹H NMR (400 MHz, d₄-MeOD): δ 8.88 (d, *J* = 6.1 Hz, 1H), 8.02 (s, 1H), 7.83 (d, *J* = 3.5 Hz, 2H), 7.71 - 7.69 (m, 4H), 7.61 (d, *J* = 15.7 Hz, 1 H), 7.68 (d, *J* = 15.7 Hz, 1 H), 5.04 (masked peaks), 3.16 - 3.15 (m, 4H), 1.12 (t, *J* = 7.1 Hz, 6H); ¹³C NMR (100.5 MHz, d₄-MeOD): δ 164.7, 145.9, 144.7, 139.4, 137.5, 131.7, 130.8, 130.5, 127.8, 124.2, 116.4, 114.8, 114.6, 113.9, 47.5, 44.6, 8.3.

### Example 52

### 3-{3-[(Ethyl-propyl-amino)-methyl]-2-phenyl-imidazo[1,2-a]pyridin-7-yl}-N-hydroxy-acrylamide (Compound 52)

The titled compound was prepared according to the procedures described in Example 42 by using appropriate starting materials.
HPLC: 99.99 %; t_{R} = 1.808 min; LCMS (ESI) Calcd for C₂₂H₂₆N₄O₂ [M⁺]: 378.476; found 379.13 [MH]⁺; ¹H NMR (400 MHz, d₄-MeOD): δ 8.88 (s, 1H), 8.02 (s, 1H), 7.84 (s, 1H), 7.70 - 7.69 (m, 4H), 7.61 (d, *J* = 15.7 Hz, 1 H), 6.81 (d, *J* = 15.7 Hz, 1 H), 5.06 (masked peaks), 3.19 (d, *J* = 6.1 Hz, 2H), 2.96 (brs, 2H), 1.55 (brs, 2H), 1.15 (t. *J* = 6.5 Hz, 3H), 0.72 (t. *J*= 7.1 Hz, 3H); ¹³C NMR (100.5 MHz, d₄-MeOD): δ 164.9, 148.4, 146.1, 138.1, 137.4, 132.4, 131.1, 130.6, 130.3, 127.2, 122.9, 116.3, 113.3, 53.8, 45.4, 17.5, 10.9, 8.6.

### Example 53

### 3-{3-[(Cyclopropylmethyl-propyl-amino)-methyl]-2-phenyl-imidazo[1,2-a]pyridin-7-yl}-N-hydroxy-acrylamide (Compound 53)

The titled compound was prepared according to the procedures described in Example 42 by using appropriate starting materials.
HPLC: 99.99 %; t_{R} = 1.136 min; LCMS (ESI) Calcd for C₂₄H₂₈N₄O₂ [M⁺]: 404.514; found 405.12 [MH]⁺; ¹H NMR (400 MHz, d₄-MeOD): δ 8.79 (d, *J* = 7.0 Hz, 1H), 8.01 (s, 1 H), 7.84 - 7.82 (m, 2H), 7.72 - 7.65 (m, 5H), 6.81 (d, *J* = 15.7 Hz, 1 H), 5.11 (s, 2H), 3.07 (d, *J* = 7.1 Hz, 2H), 2.97 - 2.93 (m, 2H), 1.49 - 1.47 (m, 2H), 1.07 (brs, 1 H), 0.73 (d, *J* = 7.6 Hz, 2H), 0.65 (t, *J* = 7.2 Hz, 3H), 0.39 (d, *J* = 4.9 Hz, 2H); ¹³C NMR (100.5 MHz, d₄-MeOD): δ 164.6, 145.4, 144.5, 139.7, 137.4, 131.8, 130.9, 130.4, 127.7, 124.5, 114.7, 114.6, 114.1, 58.7, 54.7, 44.9, 17.4, 10.9, 6.5, 5.0.

### Example 54

### 3-{3-[(sec-Butyl-propyl-amino)-methyl]-2-phenyl-imidazo[1,2-a]pyridin-7-yl}-N-hydroxy-acrylamide (Compound 54)

The titled compound was prepared according to the procedures described in Example 42 by using appropriate starting materials.
HPLC: 91.38 %; t_{R} = 1.190 min; LCMS (ESI) Calcd for C₂₄H₃₀N₄O₂ [M⁺]: 406.534; found 407.14 [MH]⁺; ¹H NMR (400 MHz, d₄-MeOD): δ 8.66 (d, *J* = 6.8 Hz, 1 H), 7.98 (s, 1 H), 7.83 - 7.78 (m, 2H), 7.68 - 7.64 (m, 6H), 6.79 (d, *J* = 15.7 Hz, 1 H), 5.01 (masked peaks), 3.23 - 3.20 (m, 1 H), 2.90 (d, *J* = 7.9 Hz, 2H), 1.72 (brs, 3H), 1.59 - 1.55 (m, 1 H), 1.17 (brs, 3H), 0.84(t, *J* = 7.2 Hz, 3H), 0.73 (brs, 3H); ¹³C NMR (100.5 MHz, d₄-MeOD): 164.9, 137.9, 137.6, 137.1, 132.4, 131.8, 131.1, 130.7, 130.4, 130.0, 128.3, 127.1, 123.2, 116.1, 113.4, 61.6, 52.8, 24.7, 19.8, 13.0, 11.3, 10.8.

### Example 55

### 3-[3-(2,6-Dimethyl-morpholin-4-ylmethyl)-2-phenyl-imidazo[1,2-a]pyridin-7-yl]-N-hydroxy-acrylamide (Compound 55)

The titled compound was prepared according to the procedures described in Example 42 by using appropriate starting materials.
HPLC: 98.36 %; t_{R} = 1.332 min;LCMS (ESI) Calcd for C₂₃H₂₆N₄O₃ [M⁺]: 406.486; found 407.08 [MH]⁺; ¹H NMR (400 MHz, d₄-MeOD): δ 9.02 (d, *J* = 7.2 Hz, 1 H), 8.02 (s, 1 H), 7.83 - 7.76 (m, 3H), 7.71 (d, *J* = 15.8 Hz, 1 H), 7.68 - 7.60 (m, 3H), 6.85 (d, *J* = 15.7 Hz, 1 H), 4.31 (d, *J* = 2.5 Hz, 2H), 4.03 - 4.00 (m, 2H), 2.73 (d, *J* = 8.7 Hz, 2H), 2.40-2.39 (m, 2H), 1.15 (d, *J* = 6.5 Hz, 6H); ¹³C NMR (100.5 MHz, d₄-MeOD): δ 164.4, 159.2, 141.9, 141.3, 138.3, 136.9, 131.9, 130.6, 130.3, 129.3, 128.1, 125.6, 120.3, 117.5, 115.5, 114.6, 112.3, 67.7, 58.8, 51.1, 18.1.

### Example 56

### 3-(3-{[Ethyl-(2-methoxy-ethyl)-amino]-methyl}-2-phenyl-imidazo[1,2-a]pyridin-7-yl)-N-hydroxy-acrylamide (Compound 56)

The titled compound was prepared according to the procedures described in Example 42 by using appropriate starting materials.
HPLC: 99.02 %; t_{R} = 1.289 min; LCMS (ESI) Calcd for C₂₃H₂₆N₄O₃ [M⁺]: 394.475; found 395.12 [MH]⁺; ¹H NMR (400 MHz, d₄-MeOD): δ 8.82 (d, *J* = 6.4 Hz, 1H), 8.04 (s, 1H), 7.83 - 7.74 (m, 3H), 7.64 (d, *J* = 15.7 Hz, 1H), 7.70 - 7.69 (m, 3H), 6.84 (d, *J* = 15.7 Hz, 1H), 5.14 (s, 2H), 3.78 (t, *J* = 4.3 Hz, 2H), 3.47 (s, 3H), 3.42 (brs, 2H), 3.04 (d, *J* = 6.8Hz, 2H), 1.00 (t, *J* = 7.0 Hz, 3H); ¹³C NMR (100.5 MHz, d₄-MeOD): δ 164.7, 145.9, 144.8, 138.9, 137.6, 131.5, 130.8, 130.7, 130.4, 127.8, 124.0, 114.9, 114.6, 114.5, 114.1, 67.8, 59.3, 25.6, 8.4.

### Example 57

### 3-[3-(4-Ethyl-piperazin-1-ylmethyl)-2-phenyl-imidazo[1,2-a]pyridin-7-yl]-N-hydroxy-acrylamide (Compound 57)

The titled compound was prepared according to the procedures described in Example 42 by using appropriate starting materials.
HPLC: 99.99 %; t_{R} = 0.899 min;LCMS (ESI) Calcd for C₂₃H₂₇N₅O₃ [M⁺]: 405.502; found 406.10 [MH]⁺; ¹H NMR (400 MHz, d₄-MeOD): δ 8.95 (d, *J* = 7.1 Hz, 1 H), 8.04 (s, 1 H), 7.78 - 7.59 (m, 7H), 6.88 (d, *J* = 15.7 Hz, 1 H), 4.20 (s, 2H), 3.52 (d, *J* = 11.6 Hz, 2H), 3.21 -3.04 (m, 6H), 2.49 (t, *J* = 11.0 Hz, 2H), 1.31 (t, *J* = 7.3 Hz, 3H); ¹³C NMR (100.5 MHz, d₄-MeOD): δ 164.4, 142.2, 141.0, 138.6, 137.0, 131.8, 130.6, 130.3, 129.3, 128.3, 125.5, 120.1, 115.5, 112.5, 52.9, 52.6, 50.4, 50.1, 9.5.

### Example 58

### 3-[3-(4-Benzyl-piperidin-1-ylmethyl)-2-phenyl-imidazo[1,2-a]pyridin-7-yl]-N-hydroxy-acrylamide (Compound 58)

The titled compound was prepared according to the procedures described in Example 42 by using appropriate starting materials.
HPLC: 97.12 %; t_{R} = 1.941 min;LCMS (ESI) Calcd for C₂₉H₃₀N₄O₂ [M⁺]: 466.585; found 467.10 [MH]⁺; ¹H NMR (400 MHz, d₄-MeOD): δ 8.86 (s, 1H), 7.92 (s, 1 H), 7.78 (d, *J* = 6.2 Hz, 2H), 7.64 - 7.59 (m, 5H), 7.25 - 7.21 (m, 2H), 7.17 - 7.13 (m, 1 H), 7.09 - 7.02 (m, 2H), 6.75 (d, *J* = 15.6 Hz, 1 H), 4.99 (s, 2H), 3.45 - 3.43 (m, 2H), 2.87 - 2.79 (m, 2H), 2.49 (d, *J*= 6.8 Hz, 2H), 1.72 - 1.68 (m, 3H), 1.40 - 1.37 (m, 2H); ¹³C NMR (100.5 MHz, d₄-MeOD): δ 164.9, 147.3, 145.6, 140.3, 138.2, 137.9, 131.7, 131.2, 130.6, 130.2, 130.1, 129.4, 127.5, 123.4, 115.6, 113.8, 113.2, 53.7, 42.6, 36.2, 33.9, 29.9.

### Example 59

### 3-{3-[(2,2-Dimethyl-propylamino)-methyl]-2-phenyl-imidazo[1,2-a]pyridin-7-yl}-N-hydroxy-acrylamide (Compound 59)

The titled compound was prepared according to the procedures described in Example 42 by using appropriate starting materials.
HPLC: 98.57 %; t_{R} = 1.196 min; LCMS (ESI) Calcd for C₂₂H₂₆N₄O₂ [M⁺]: 378.47; found 379.12 [MH]⁺; ¹H NMR (400 MHz, d₄-MeOD): δ 8.90 (s, 1H), 7.98 (s, 1H), 7.80 (d, *J* = 6.8 Hz, 2H), 7.66 - 7.67 (m, 5H), 6.80 (d, *J* = 15.7 Hz, 1 H), 5.04 (s, 2H), 2.76 (s, 2H), 0.93 (s, 9H);

### Example 60

### N-Hydroxy-3-(2-phenyl-3-pyrrolidin-1-ylmethyl-imidazo[1,2-a]pyridin-7-yl)-acrylamide (Compound 60)

The titled compound was prepared according to the procedures described in Example 42 by using appropriate starting materials.
HPLC: 98.79 %; t_{R} = 0.513 min; LCMS (ESI) Calcd for C₂₁H₂₂N₄O₂ [M⁺]: 362.42; found 363.29 [MH]⁺; ¹H NMR (400 MHz, d₆-DMSO): δ 8.92 (d, *J* = 7.2 Hz, 1 H), 7.97 (s, 1 H), 7.84 (d, *J* = 7.2 Hz, 2H), 7.49 - 7.61 (m, 4H), 7.45 (d, *J* = 7.2 Hz, 1H), 6.76 (d, *J* = 16.0 Hz, 1H), 5.09 (s, 2H), 3.38 (brs, 2H), 2.89 (brs, 2H), 1.79 (brs, 4H).

### Example 61

### 3-{3-[(Cyclopropylmethyl-amino)-methyl]-2-phenyl-imidazo[1,2-a]pyridin-7-yl}-N-hydroxy-acrylamide (Compound 61)

The titled compound was prepared according to the procedures described in Example 42 by using appropriate starting materials.
HPLC: 97.36 %; t_{R} = 0.846 min; LCMS (ESI) Calcd for C₂₁H₂₂N₄O₂ [M⁺]: 362.42; found 363.28 [MH]⁺; ¹H NMR (400 MHz, d₄-MeOD): δ 8.84 (d, *J* = 6.4 Hz, 1H), 7.94 (s, 1 H), 7.82 (d, *J* = 6.4 Hz, 2H), 7.58 - 7.66 (m, 5H), 6.76 (d, *J* = 15.7 Hz, 1 H), 4.94 (masked peaks), 2.95 (d, *J* = 7.4 Hz, 2H), 1.02 - 1.06 (m, 1 H), 0.63 - 0.68 (m, 2H), 0.33 - 0.37 (m, 2H).

### Example 62

### 3-(3-Cyclopropylaminomethyl-2-phenyl-imidazo[1,2-a]pyridin-7-yl)-N-hydroxy-acrylamide (Compound 62)

The titled compound was prepared according to the procedures described in Example 42 by using appropriate starting materials.
HPLC: 94.09 %; t_{R} = 0.595 min; LCMS (ESI) Calcd for C₂₀H₂₀N₄O₂ [M⁺]: 348.40; found 349.17 [MH]⁺; ¹H NMR (400 MHz, d₄-MeOD): δ 8.87 (s, 1 H), 8.03 (s, 1H), 7.82 (d, *J* = 7.04 Hz, 2H), 7.65 - 7.68 (m, 5H), 6.79 (d, *J* = 15.6 Hz, 1 H), 5.04 (s, 2H), 2.62 - 2.63 (q, 1H), 0,78 (s, 2H), 0.64 (d, *J* = 6.5 Hz, 2H).

### Example 63

### 3-[3-Butylaminomethyl-2-(4-fluoro-phenyl)-imidazo[1,2-a]pyridin-7-yl]-N-hydroxyacrylamide (Compound 63)

The titled compound was prepared according to the procedures described in Example 42 by using appropriate starting materials.
HPLC: 99.99 %; t_{R} = 1.151 min;LCMS (ESI) Calcd for C₂₁H₂₃FN₄O₂ [M⁺]: 382.439; found 383.28 [MH]⁺; ¹H NMR (400 MHz, MeOD): δ 8.77 (d, *J* = 7.1 Hz, 1 H), 7.89 (s, 1 H), 7.85 - 7.82 (m, 2H), 7.62 (d, *J* = 15.7 Hz, 1 H), 7.54 (d, *J* = 7.0 Hz, 1 H), 7.40 - 7.35 (m, 3H), 6.72 (d, *J* = 15.8 Hz, 1H), 4.91 (masked peaks), 3.00 - 2.96 (m, 2H), 1.62 - 1.54 (m, 2H), 1.33 - 1.27 (m, 2H), 0.90 (t, *J* = 7.3 Hz, 3H);

### Example 64

### 3-[3-(tert-Butylamino-methyl)-2-(4-fluoro-phenyl)-imidazo[1,2-a]pyridin-7-yl]-N-hydroxy-acrylamide (Compound 64)

The titled compound was prepared according to the procedures described in Example 42 by using appropriate starting materials.
HPLC: 95.45 %; t_{R} = 0.887 min;LCMS (ESI) Calcd for C₂₁H₂₃FN₄O₂ [M⁺]: 382.439; found 383.28 [MH]⁺; ¹H NMR (400 MHz, d₄-MeOD): δ 8.69 (brs, 1 H), 7.87 - 7.83 (m, 4H), 7.60 - 7.54 (m, 2H), 7.38 - 7.34 (m, 2H), 6.62 (d, *J* = 15.6 Hz, 1 H), 4.92 (masked peaks), 1.42 (s, 9H);

The following compounds (Table 2) are some representative examples prepared by methods disclosed or analogous to those disclosed in above Examples 1-64:

**Table 2**

| **Compound No.** | **Structure** | **m/z [MH]⁺** |
|---|---|---|
| **1** | 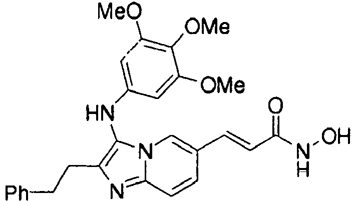 | 489 |
| **2** | 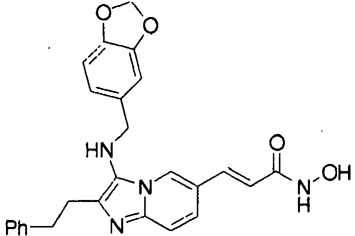 | 457 |
| **3** | 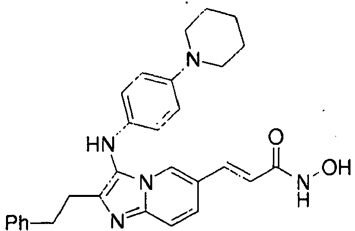 | 482 |
| **4** | 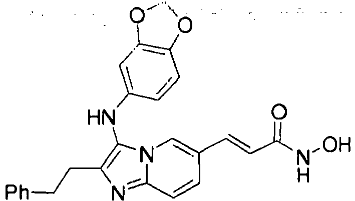 | 443 |
| **5** | 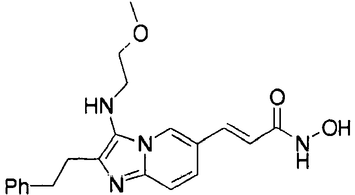 | 381 |
| **6** | 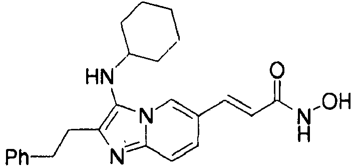 | 405 |
| **7** | 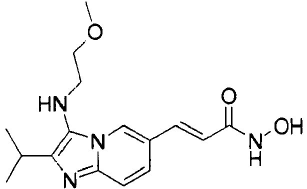 | 319 |
| **8** | 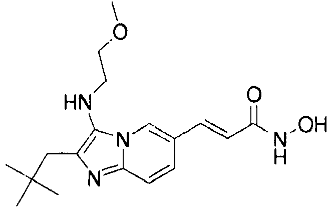 | 347 |
| **9** | 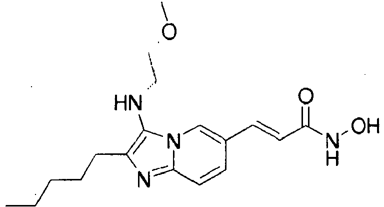 | 347 |
| **10** | 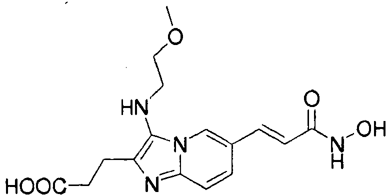 | 350 |
| **11** | 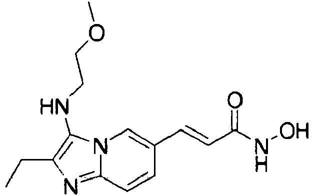 | 305 |
| **12** | 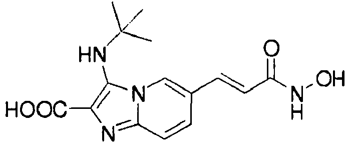 | 274 [M-COOH]⁺ |
| **13** | 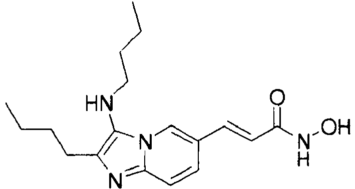 | 331 |
| **14** | 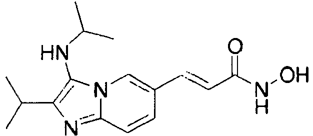 | 303 |
| **15** | 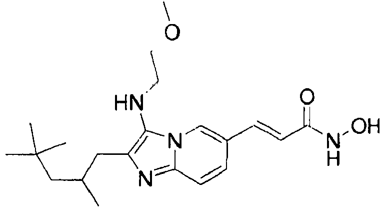 | 389 |
| **16** | 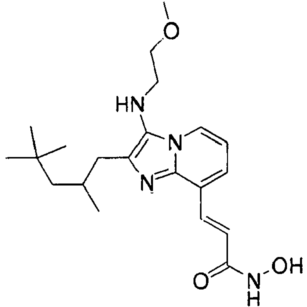 | 389 |
| **17** | 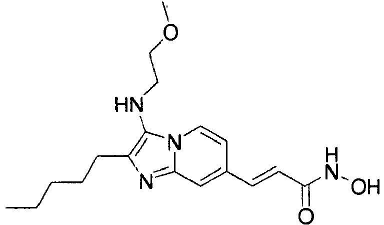 | 347 |
| **18** | 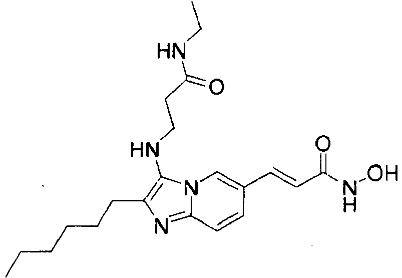 | 402 |
| **19** | 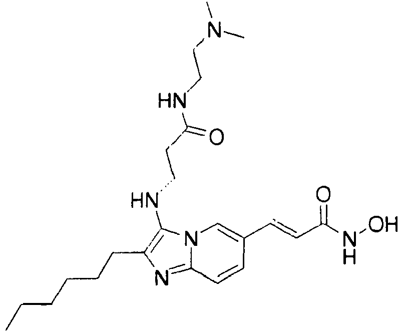 | 445 |
| **20** | 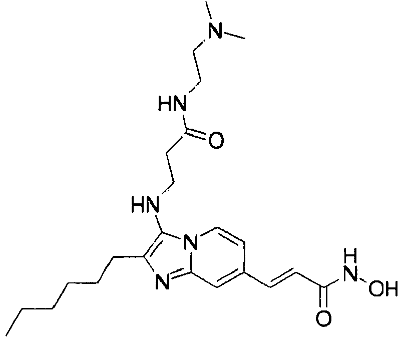 | 445 |
| **21** | 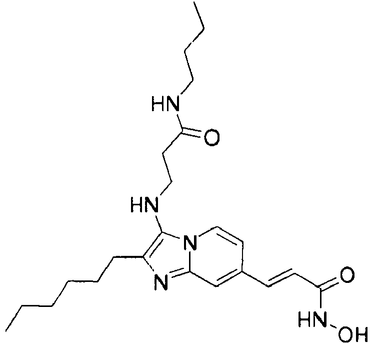 | 430 |
| **22** | 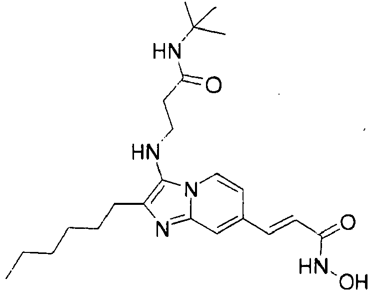 | 430 |
| **23** | 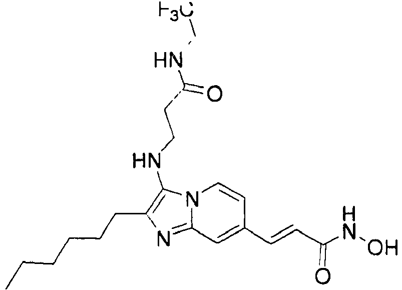 | 456 |
| **24** | 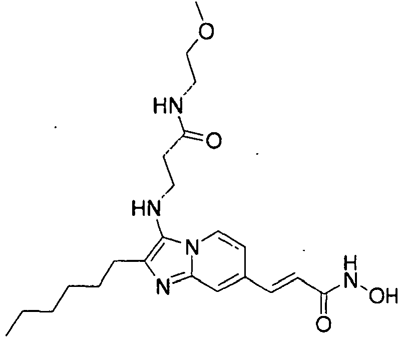 | 432 |
| **25** | 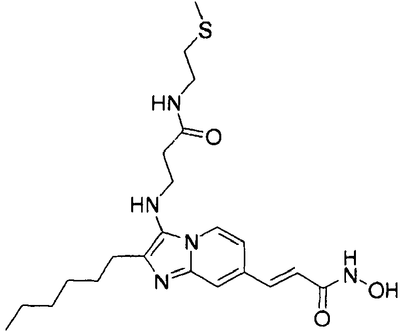 | 448 |
| **26** | 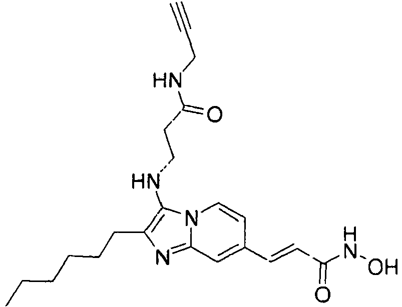 | 412 |
| **27** | 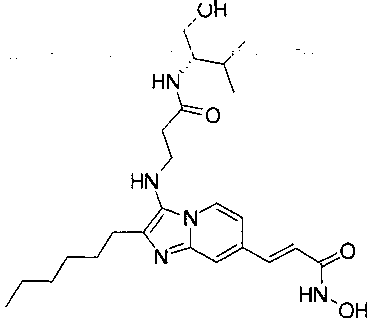 | 460 |
| **28** | 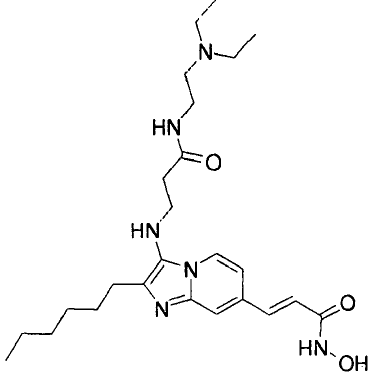 | 473 |
| **29** | 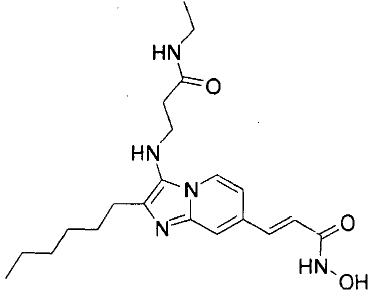 | 402 |
| **30** | 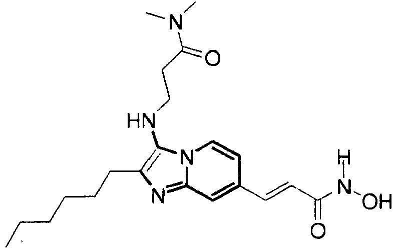 | 402 |
| **31** | 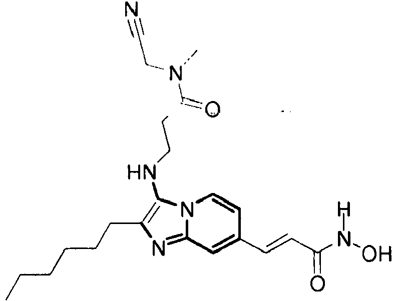 | 430 |
| **32** | 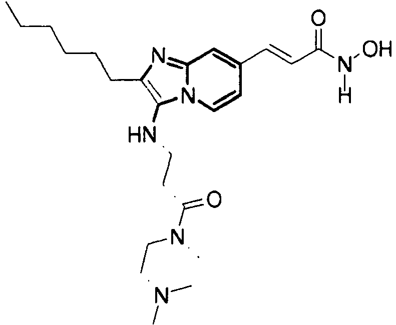 | 459 |
| **33** | 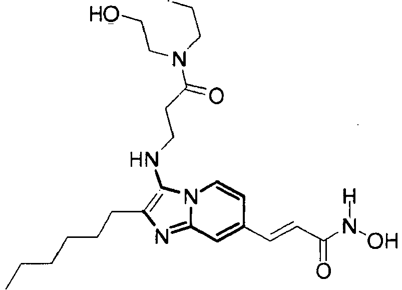 | 460 |
| **34** | 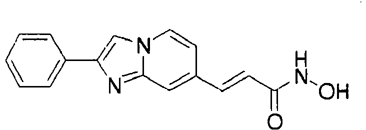 | 280 |
| **35** | 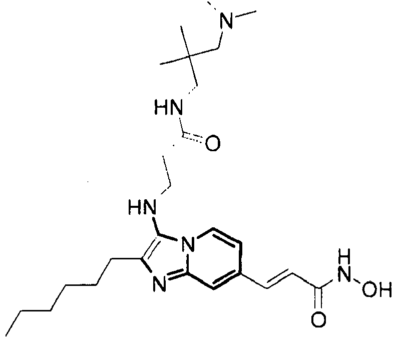 | 487 |
| **36** | 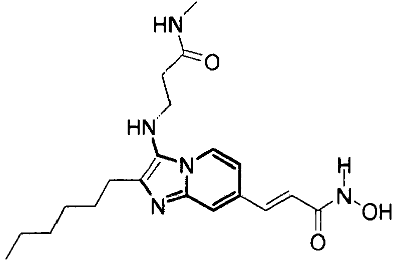 | 388 |
| **37** | 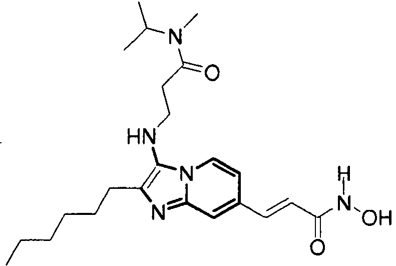 | 430 |
| **38** | 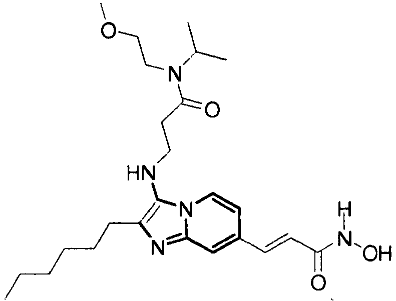 | 474 |
| **39** | 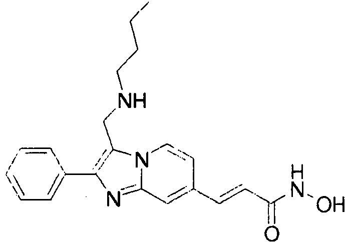 | 365 |
| **40** | 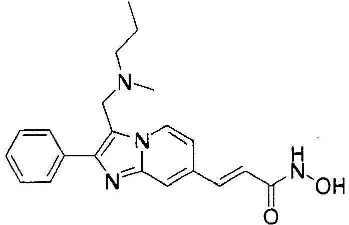 | 365 |
| **41** | 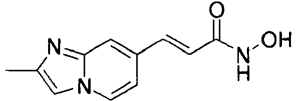 | 218 |
| **42** | 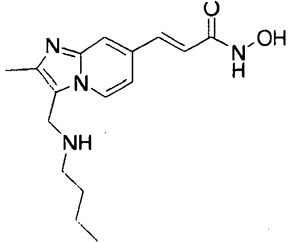 | 303 |
| **43** | 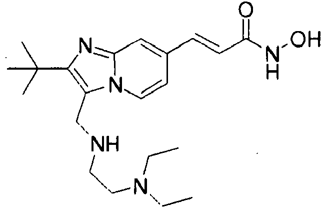 | 388 |
| **44** | 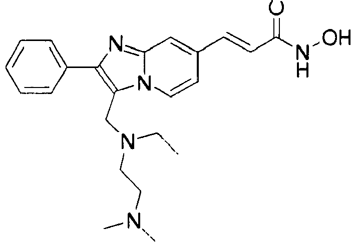 | 408 |
| **45** | 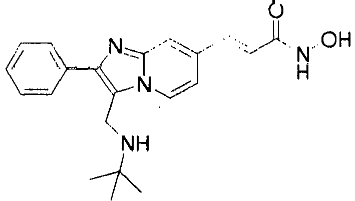 | 365 |
| **46** | 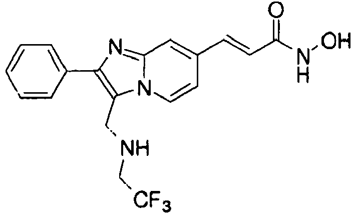 | 391 |
| **47** | 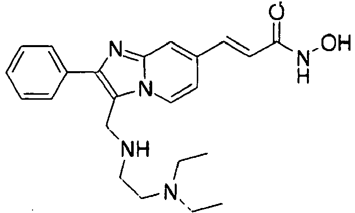 | 408 |
| **48** | 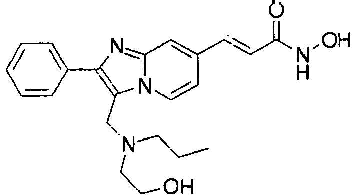 | 395 |
| **49** | 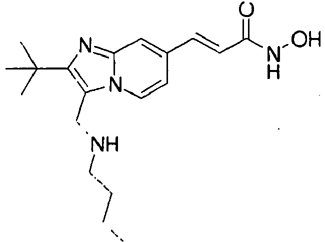 | 345 |
| **50** | 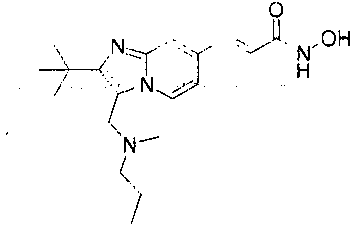 | 345 |
| **51** | 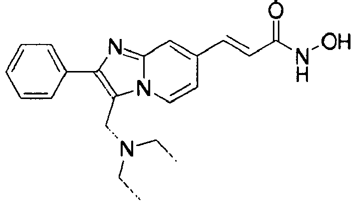 | 365 |
| **52** | 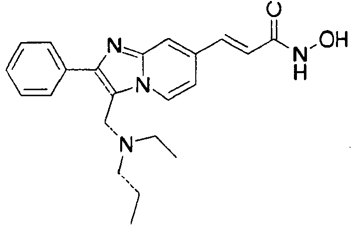 | 379 |
| **53** | 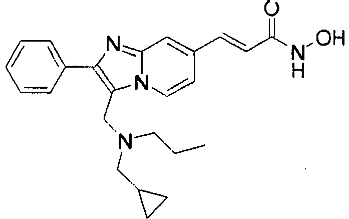 | 405 |
| **54** | 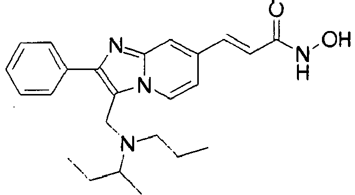 | 407 |
| **55** | 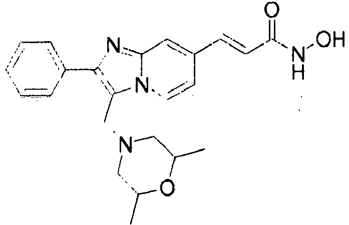 | 407 |
| **56** | 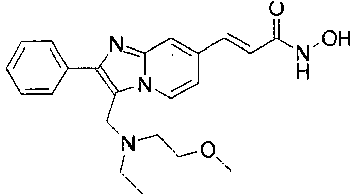 | 395 |
| **57** | 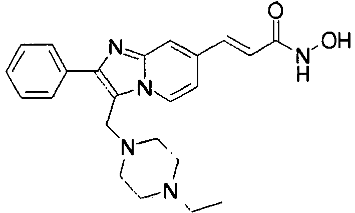 | 406 |
| **58** | 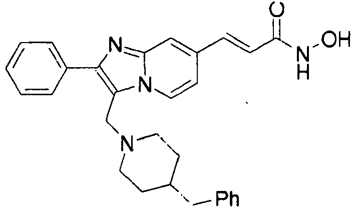 | 467 |
| **59** | 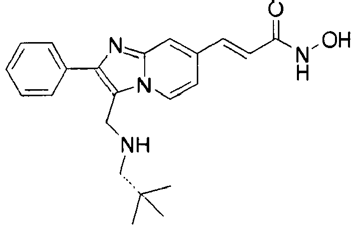 | 379 |
| **60** | 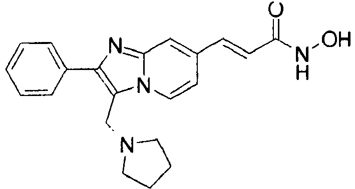 | 363 |
| **61** | 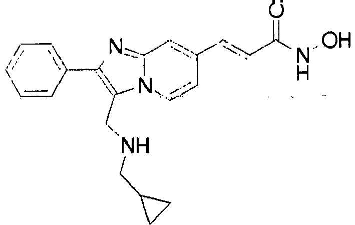 | 363 |
| **62** | 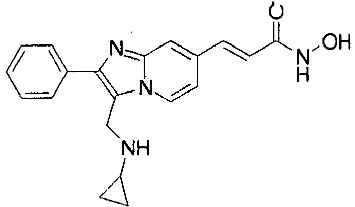 | 349 |
| **63** | 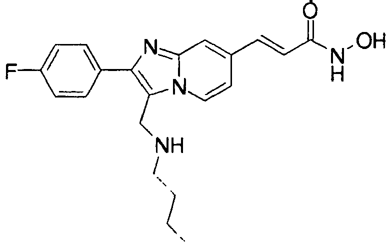 | 383 |
| **64** | 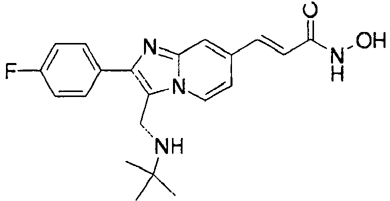 | 383 |

### BIOLOGICAL TESTING AND ENZYME ASSAYS

### Recombinant GST-HDAC Protein expression and purification

Human cDNA library was prepared using cultured SW620 cells. Amplification of human HDAC1 coding region from this cDNA library was cloned separately into the baculovirus expression pDEST20 vector (GATEWAY Cloning Technology, Invitrogen Pte Ltd). The pDEST20-HDAC1 construct was confirmed by DNA sequencing. Recombinant baculovirus was prepared using the Bac-To-Bac method following the manufacturer's instruction (Invitrogen Pte Ltd). Baculovirus titer was determined by plaque assay to be about 10⁸ PFU/ml.
Expression of GST-HDAC1 was done by infecting SF9 cells (Invitrogen Pte Ltd) with pDEST20-HDAC1 baculovirus at MOI=1 for 48 h. Soluble cell lysate was incubated with pre-equilibrated Glutathione Sepharose 4B beads (Amersham) at 4°C for 2 h. The beads were washed with PBS buffer for 3 times. The GST-HDAC1 protein was eluted by elution buffer containing 50 mM Tris, pH8.0, 150mM NaCl, 1% Triton X-100 and 10mM or 20mM reduced Glutathione. The purified GST-HDAC1 protein was dialyzed with HDAC storage buffer containing 10mM Tris, pH7.5, 100mM NaCl and 3mM MgCl₂. 20% Glycerol was added to purified GST-HDAC1 protein before storage at -80°C.

### In vitro HDAC assay for determination of IC₅₀ values

The assay has been carried out in 96 well format and the BIOMOL fluorescent-based HDAC activity assay has been applied. The reaction composed of assay buffer, containing 25 mM Tris pH 7.5, 137 mM NaCl, 2.7 mM KCl, 1 mM MgCl₂, 1 mg/ml BSA, tested compounds, an appropriate concentration of HDAC1 enzyme, 500 uM *Flur de lys* generic substrate for HDAC1 enzyme and subsequently was incubated at room temperature for 2 h. *Flur de lys* Developer was added and the reaction was incubated for 10 min. Briefly, deacetylation of the substrate sensitizes it to the developer, which then generates a fluorophore. The fluorophore is excited with 360 nm light and the emitted light (460 nm) is detected on a fluorometric plate reader (Tecan Ultra Microplate detection system, Tecan Group Ltd.).

The analytical software, Prism 3.0 (GraphPad Software Inc) has been used to generate IC₅₀ from a series of data.

The HDAC enzyme inhibition results of representative compounds are shown in Table 3 (unit in the table is micromolar).

**Table 3**

| **Compound No.** | **IC₅₀ (µM) (HDAC 1)** | **Compound No.** | **IC₅₀(µM) (HDAC 1)** |
|---|---|---|---|
| **1** | 0.15 | **33** | 0.83 |
| **2** | 0.72 | **34** | 0.87 |
| **3** | 0.79 | **35** | 0.14 |
| **4** | 0.64 | **36** | 0.39 |
| **5** | 0.35 | **37** | 0.59 |
| **6** | 1.4 | **38** | 0.36 |
| **7** | 2.7 | **39** | 0.16 |
| **8** | 1 | **40** | 0.24 |
| **9** | 1.3 | **41** | 1.3 |
| **10** | 0.32 | **42** | 0.16 |
| **11** | 6 | **43** | 2 |
| **12** | >10 | **44** | 2.9 |
| **13** | 0.72 | **45** | 0.045 |
| **14** | 2.7 | **46** | >10 |
| **15** | 0.76 | **47** | 0.65 |
| **16** | >10 | **48** | 1 |
| **17** | 0.3 | **49** | 0.16 |
| **18** | 0.63 | **50** | 0.52 |
| **19** | 0.78 | **51** | 0.2 |
| **20** | 0.099 | **52** | 0.24 |
| **21** | 0.13 | **53** | 0.60 |
| **22** | 0.14 | **54** | 1 |
| **23** | 0.16 | **55** | 2.4 |
| **24** | 0.13 | **56** | 1.6 |
| **25** | 0.073 | **57** | 4.6 |
| **26** | 0.14 | **58** | 1.5 |
| **27** | 0.18 | **59** | 0.23 |
| **28** | 0.11 | **60** | 0.084 |
| **29** | 0.38 | **61** | 0.08 |
| **30** | 0.88 | **62** | 0.31 |
| **31** | >10 | **63** | 0.063 |
| **32** | 0.24 | **64** | 0.051 |

### Cell-based proliferation assay for determination of Gl₅₀ values

Human cancer cell lines (e.g. Colo205) were obtained from ATCC. Colo205 cells were cultivated in RPMI 1640 containing 2 mM L-Glutamine, 5% FBS, 1.0 mM Na Pyruvate. Colo205 cells were seeded in 96-wells plate at 5000 cells per well. The plates were incubated at 37°C, 5% CO₂, for 24 h. Cells were treated with compounds at various concentrations for 96 h. Cell growth was then monitored using CyQUANT® cell proliferation assay (Invitrogen Pte Ltd). Dose response curves were plotted to determine Gl₅₀ values for the compounds using XL-fit (ID Business Solution, Emeryville, CA).

The cellular or growth inhibition activity results of representative compounds are shown in Table 4 below (unit in the table is micromolar). The data indicated that the compounds of this invention are active in the inhibition of tumor cell growth.

**Table 4**

| **Compound No.** | **Gl₅₀(µM) (Colo205)** | **Compound No.** | **Gl₅₀(µM) (Colo205)** |
|---|---|---|---|
| **1** | 2.1 | **38** | 3.5 |
| **2** | 3.9 | **39** | 0.44 |
| **3** | 1.9 | **40** | 0.79 |
| **4** | 2.8 | **41** | 2.5 |
| **13** | 27 | **42** | 1.5 |
| **14** | 42 | **43** | 34 |
| **15** | 9.3 | **44** | 32 |
| **16** | 14 | **45** | 0.26 |
| **17** | 5.3 | **46** | 11 |
| **18** | 39 | **47** | 30 |
| **19** | >10 | **48** | 9.2 |
| **20** | 20 | **49** | 0.59 |
| **21** | 8.9 | **50** | 2.4 |
| **22** | 9.2 | **51** | 1.1 |
| **23** | 16 | **52** | 1.2 |
| **24** | 20 | **53** | 2.9 |
| **25** | 9.9 | **54** | 4.7 |
| **26** | 12 | **55** | 9.3 |
| **27** | 24 | **56** | 9.0 |
| **28** | 28 | **57** | 14 |
| **29** | 13 | **58** | 2.9 |
| **30** | 19 | **59** | 2.1 |
| **32** | 15 | **60** | 0.48 |
| **33** | 40 | **61** | 0.56 |
| **34** | 2.1 | **62** | 2.5 |
| **35** | 7.9 | **63** | 0.54 |
| **36** | 8.5 | **64** | 0.27 |
| **37** | 7.3 | | |

### Histone acetylation assay

A hallmark of histone deacetylase (HDAC) inhibition is the increase in the acetylation level of histones. Histone acetylation, including H3, H4 and H2A can be detected by immuno-blotting (western-blot). Colo205 cells, approximately 5 x10⁵ cells, were seeded in the previously described medium, cultivated for 24 h and subsequently treated with HDAC inhibitory agents and a positive control at 10 µM final concentration. After 24 h, cells were harvested and lysed according to the instruction from Sigma Mammalian Cell Lysis Kit. The protein concentration was quantified using BCA method (Sigma Pte Ltd). The protein lysate was separated using 4-12% bis-tris SDS-PAGE gel (Invitrogen Pte Ltd) and was transferred onto PVDF membrane (BioRad Pte Ltd). The membrane was probed using primary antibody specific for acetylated histone H3 (Upstate Pte Ltd). The detection antibody, goat anti rabbit antibody conjugated with HRP was used according to the manufacturing instruction (Pierce Pte Ltd). After removing the detection antibody from the membrane, an enhanced chemiluminescent substrate for detection of HRP (Pierce Pte Ltd) was added onto the membrane. After removing the substrate, the membrane was exposed to an X-ray film (Kodak) for 1 sec - 20 mins. The X-ray film was developed using the X-ray film processor. The density of each band observed on the developed film could be qualitatively analysed using UVP Bioimaging software (UVP, Inc, Upland, CA). The values were then normalized against the density of actin in the corresponding samples to obtain the expression of the protein and compared to the values obtained from SAHA.
The results of immuno-blotting assay using acetylated histone H3 antibody are shown in Table 5 for representative compounds of this invention.

**Table 5**

| **Compound No.** | **Histone acetylation activities (Histone-3)** |
|---|---|
| **39** | active |
| **40** | active |
| **45** | active |
| **49** | active |
| **60** | active |
| **63** | active |
| **64** | active |

These data demonstrate that compounds of this invention inhibit histone deacetylases, thereby resulting in accumulation of acetylated histones.

### In vivo antineoplastic (or anti-tumor) effect of HDAC inhibiting agents:

The efficacy of the compounds of the invention can then be determined using tumor xenograft studies. The tumor xenograft model is one of the most commonly used in vivo cancer models.

In these studies Female athymic nude mice (Harlan), 12-14 weeks of age will be implanted subcutaneously in the flank with 5 x 10⁶ cells of HCT116 human colon cancer cells, or with 5 x 10⁶ cells of A2780 human ovarian cancer cells, or with 5 x 106 cells of PC3 prostate cancer cells. When the tumor reaches the size 100 mm³, the xenograft nude mice will be paired-match into various treatment groups. The selected HDAC inhibitors will be dissolved in appropriate vehicles and administered to xenograft nude mice intraperitonelly or orally daily for 21 days. The dosing volume will be 0.01 ml/ g body weight. Paclitaxol, which can be used as positive control, will be prepared for intravenous administration in an appropriate vehicle. The dosing volume for Paclitaxol will be 0.01 ml/g body weight. Tumor volume will be calculated every second day or twice-a-week of post injection using the formula: Volume (mm³) = (w² x l)/2, where w = width and I = length in mm of an HCT116, or A2780, or PC3 tumor. Compounds of this invention that are tested will show significant reduction in tumor volume relative to controls treated with vehicle only. Acetylated histone relative to vehicle treated control group when measured shall be accumulated. The result will therefore indicate that compounds of this invention are efficacious in treating a proliferative disease such as cancer.

## Claims

1. A compound of the formula (Ic): 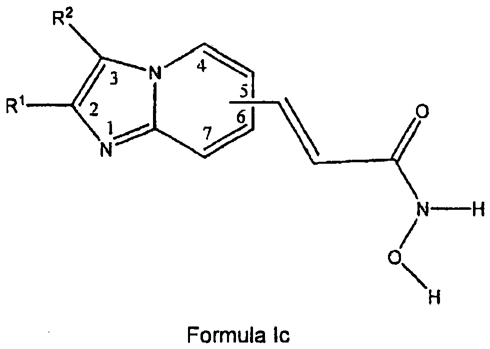 wherein:
R¹ is selected from the group consisting of: C₁-C₁₄alkyl, arylC₁-C₁₄alkyl, aryl and CO₂H;
R² is L where L is -(CR²⁰R²¹)ₘ-(CR²²R²³)ₙ-(CR²⁴R²⁵)ₒ-NR²⁵R²⁷;
wherein
each R²⁰, R²¹, R²², R²³, R²⁴ and R²⁵ is independently selected from the group consisting of H and C₁-C₁₄alkyl; or
R²⁰ and R²¹ when taken together may form a group of formula =O or =S, and/or
R²² and R²³ when taken together may form a group of formula =O or =S, and/or
R²⁴ and R²⁵ when taken together may form a group of formula =O or =S;
each R²⁶ and R²⁷ is independently selected from the group consisting of: H, C₁-C₁₄alkyl, C₂-C₁₄heteroalkyl, C₆-C₁₀cycloalkyl, optionally substituted aryl, arylC₁-C₁₄alkyl, C₅-C₁₀cycloalkylC₁-C₁₄alkyl and G, or
R²⁶ and R²⁷ when taken together with the nitrogen atom to which they are attached form a heterocycloalkyl which may be optionally substituted;
wherein the optional substituents on the heterocycloalkyl are selected from the group consisting of H, methyl, ethyl and benzyl,
wherein the optional substituents on aryl are selected from the group consisting of H, methoxy, methylendioxy, and piperidinyl,
m, n and o are each integers that are independently selected from the group consisting of 0, 1, 2, 3 and 4;
G is a group of formula:
-L³W³
wherein
L³ is C₁-C₅alkylene;
W³ is selected from the group consisting of -OR¹², -N(R¹²)₂, and -C(O)N(R¹²)₂,
R¹² is selected from the group consisting of H, -CN, C₁-C₁₄alkyl, C₂-C₁₄alkynyl, C₁-C₁₄haloalkyl, C₁-C₁₄heteroalkyl, hydroxy, and hydroxyC₁-C₁₄alkyl,
or a pharmaceutically acceptable salt thereof.

2. A compound of claim 1 wherein the double bond is attached at ring position 5 or 6.

3. A compound according to claim 1 or claim 2 wherein the double bond is attached at ring position 6.

4. A compound according to any one of claims 1 to 3 wherein R¹ is C₁-C₁₄alkyl.

5. A compound according to any one of claims 1 to 3 wherein R¹ is selected from the group consisting of methyl, ethyl, 2-carboxy-ethyl, propyl, isopropyl, 2,2-dimethyl-propyl, butyl, isobutyl, tert-butyl, pentyl, 2,4,4-trimethyl-pentyl, hexyl, 2-phenyl-ethyl, phenyl and 4-fluoro-phenyl.

6. A compound according to any one of claims 1 to 5 wherein R² is selected from the group consisting of:

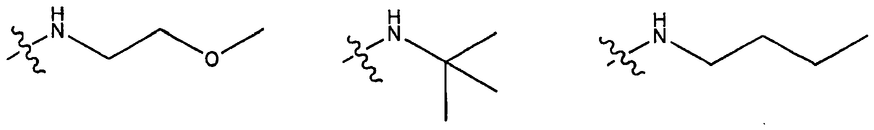

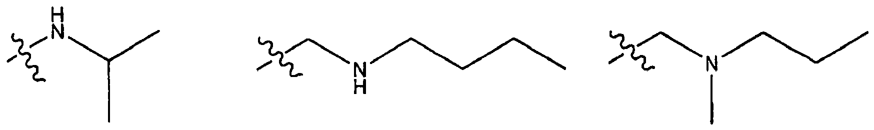

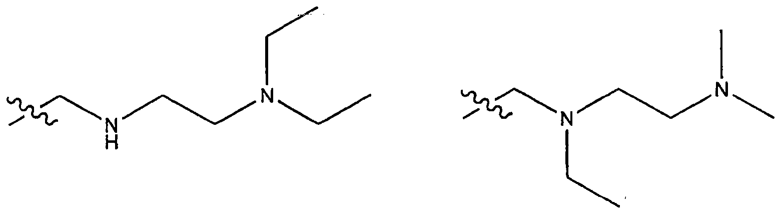

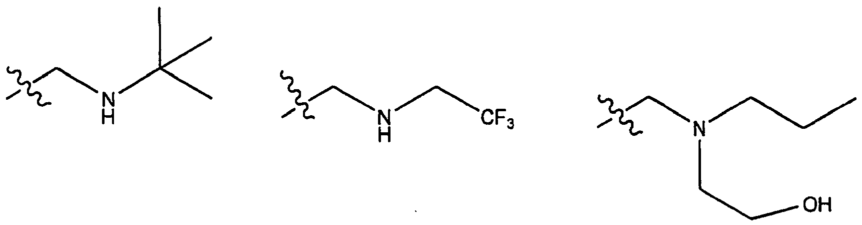

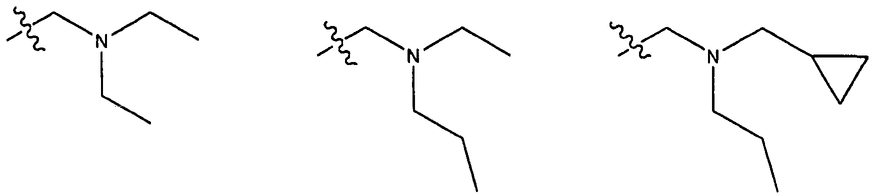

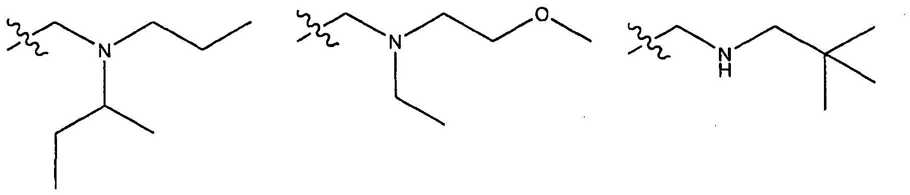

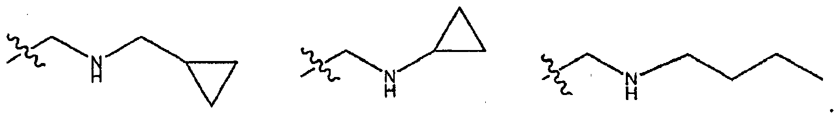

7. A compound according to claim 1 wherein the compound has the formula 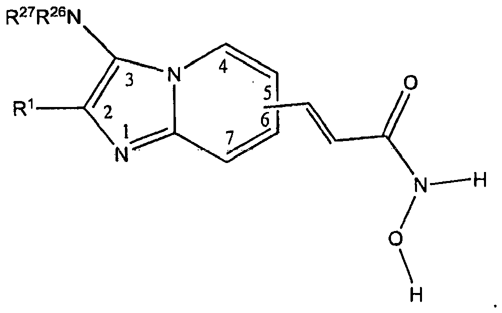 wherein R¹, R²⁶ and R²⁷ are as defined in claim 1.

8. A compound according to claim 1 wherein the compound has the formula: 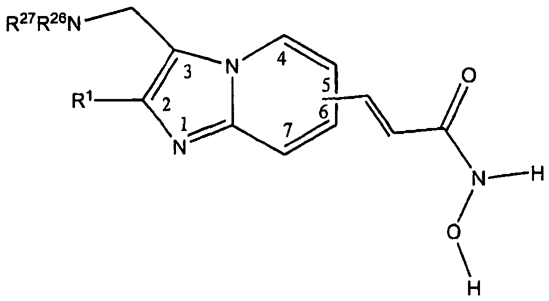 wherein R¹, R²⁶, and R²⁷ are as defined in claim 1.

9. A compound according to claim 1 wherein R²⁸ and R²⁷ are independently selected from the group consisting of H, 3,4,5-trimethoxyphenyl, 3,4-methylenedioxybenzyl, 4-piperidin-1yl-phenyl, 3,4-methylenedioxyphenyl, 2-methoxy-ethyl, cyclopropyl, cyclohexyl, methyl, cyclopropyl-methyl, ethyl, propyl, isopropyl, 2,2-dimethyl-propyl, butyl, t-butyl, sec-butyl, 2-(diethylamino)-ethyl, 2-(dimethylamino)-ethyl and 2,2,2 triflouroethyl.

10. A compound according to any one of claims 1 to 8 wherein R²⁶ is G and R²⁷ is H or alkyl wherein G is as defined in claim 1.

11. A compound according to claim 10 wherein G is a group of formula:
-(CH₂)₂-C(O)N(R¹²)_{2.}

12. A compound according to claim 11 wherein each R¹² is independently selected from the group consisting of H, C₁-C₁₄alkyl, C₂-C₁₄heteroalkyl; and C₂-C₁₄alkynyl.

13. A compound according to claim 11 wherein G is selected from the group consisting of:

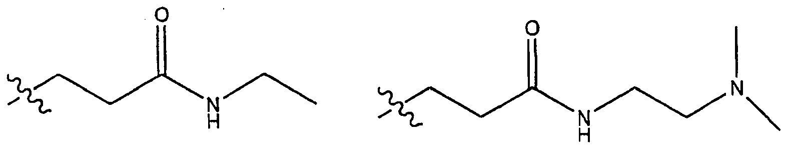

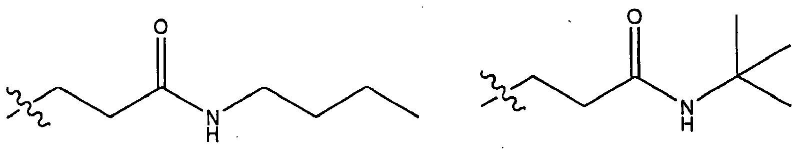

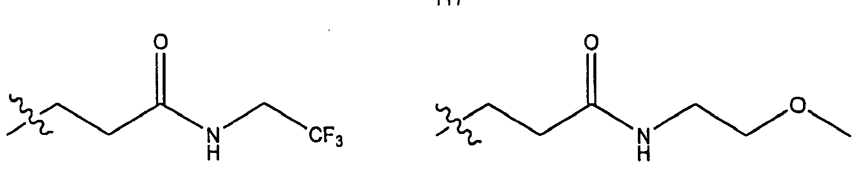

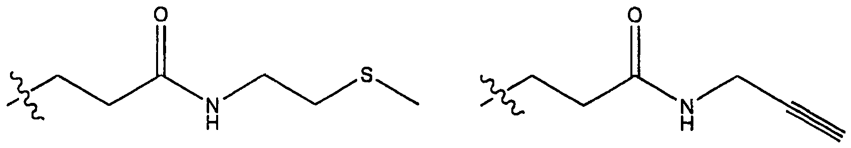

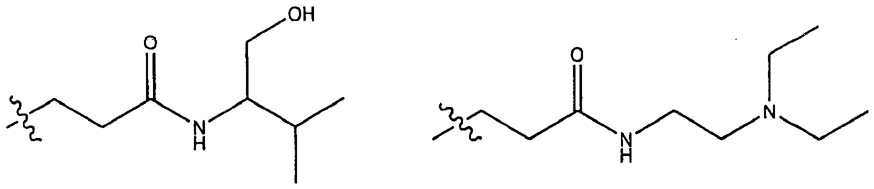

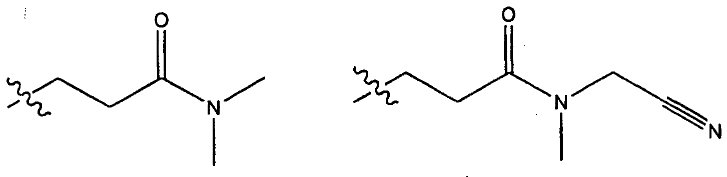

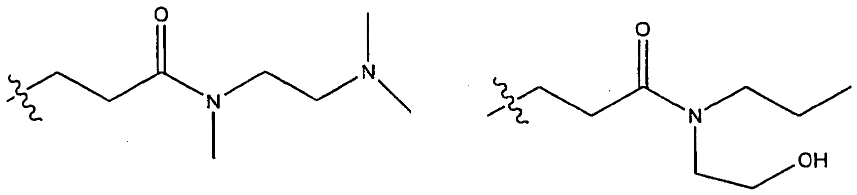

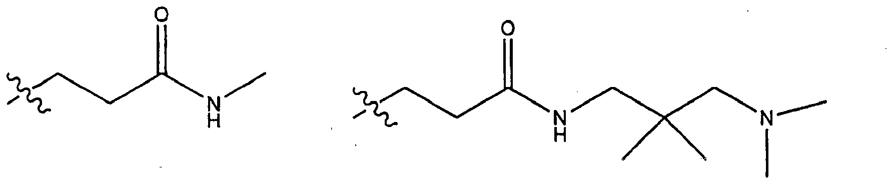

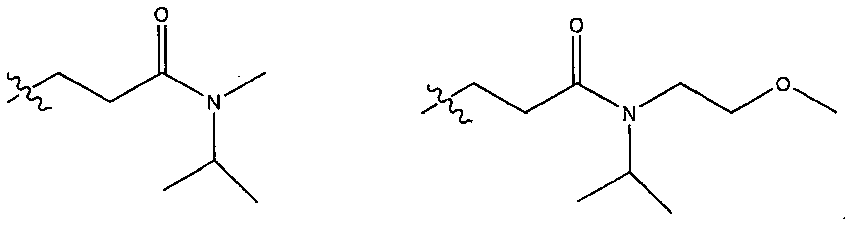

14. The compound of claim 1 wherein the compound is selected from compounds, and their pharmaceutically acceptable salts, selected from the group consisting of
| **Structure** | **Name** |
|---|---|
| 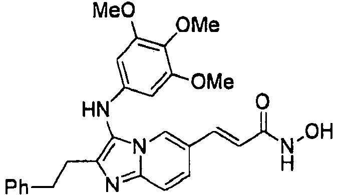 | (E)-N-hydroxy-3-(2-phenethyl-3-(3,4,5-trimethoxyphenylamino)imidazo[1,2-a]pyridin-6-yl)acrylamide |
| 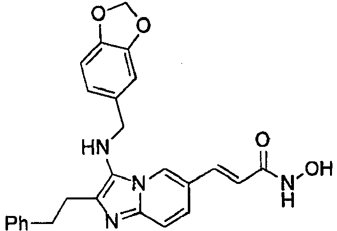 | (E)-3-(3-(benzo[d][1,3]dioxol-5-ylmethylamino)-2-phenethylimidazo[1,2-a]pyridin-6-yl)-N-hydroxyacrylamide |
| 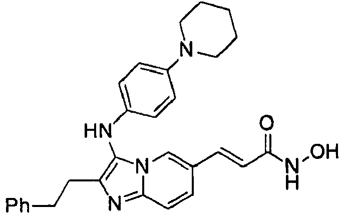 | N-Hydroxy-3-[2-phenethyl-3-(4-piperidin-1-yl-phenylamino)-imidazo[1,2-a]pyridin-6-yl]-acrylamide |
| 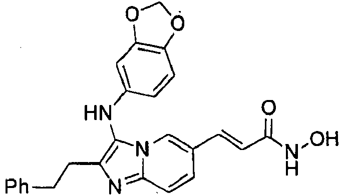 | (E)-3-(3-(benzo[d][1,3]dioxol-5-ylamino)-2-phenethylimidazo[1,2-a]pyridin-6-yl)-N-hydroxyacrylamide |
| 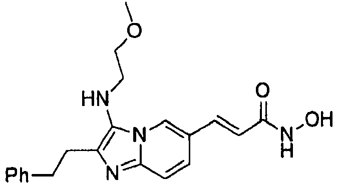 | (E)-N-hydroxy-3-(3-(2-methoxyethylamino)-2-phenethylimidazo[1,2-a]pyridin-6-yl)acrylamide |
| 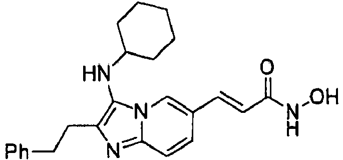 | (E)-3-(3-(cyclohexylamino)-2-phenethylimidazo[1,2-a]pyridin-6-yl)-N-hydroxyacrylamide |
| 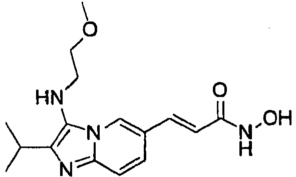 | (E)-N-hydroxy-3-(2-isopropyl-3-(2-methoxyethylamino)imidazo[1,2-a]pyridin-6-yl)acrylamide |
| 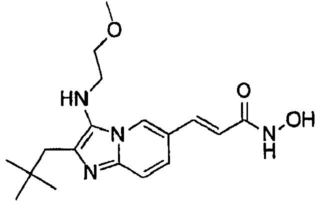 | 3-[2-(2,2-Dimethyl-propyl)-3-(2-methoxyethylamino)-imidazo[1,2-a]pyridin-6-yl]-N-hydroxy-acrylamide |
| 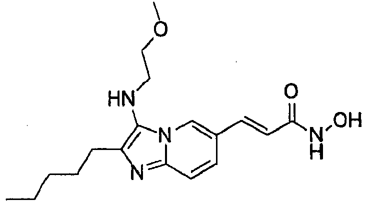 | (E)-N-hydroxy-3-(3-(2-methoxyethylamino)-2-pentylimidazo[1,2-a]pyridin-6-yl)acrylamide |
| 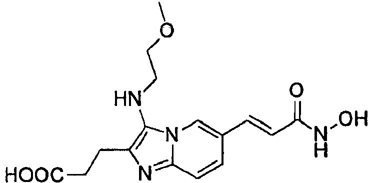 | 3-[6-(2-Hydroxycarbamoyl-vinyl)-3-(2-methoxyethylamino)-imidazo[1,2-a]pyridin-2-yl]-propionic acid |
| 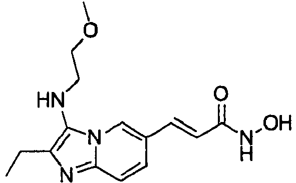 | 3-[2-Ethyl-3-(2-methoxy-ethylamino)-imidazo[1,2-a]pyridin-6-yl]-N-hydroxyacrylamide |
| 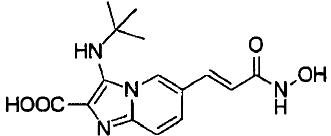 | (E)-3-(tert-butylamino)-6-(3-(hydroxyamino)-3-oxoprop-1-enyl)imidazo[1,2-a]pyridine-2-carboxylic acid |
| 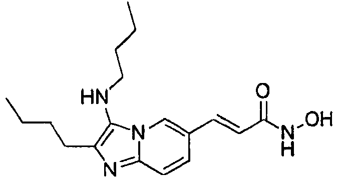 | (E)-3-(2-butyl-3-(butylamino)imidazo[1,2-a]pyridin-6-yl)-N-hydroxyacrylamide |
| 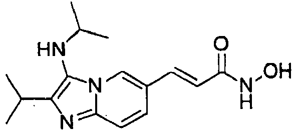 | (E)-N-hydroxy-3-(2-isopropyl-3-(isopropylamino)imidazo[1,2-a]pyridin-6-yl)acrylamide |
| 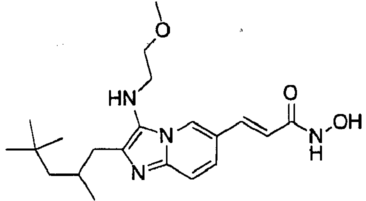 | (E)-N-hydroxy-3-(3-(2-methoxyethylamino)-2-(2,4,4-trimethylpentyl)imidazo[1,2-a]pyridin-6-yl)acrylamide |
| 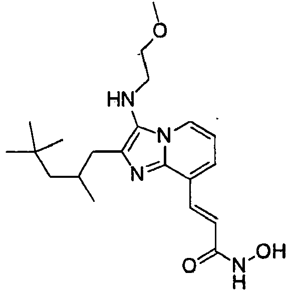 | (E)-N-hydroxy-3-(3-(2-methoxyethylamino)-2-(2,4,4-trimethylpentyl)imidazo[1,2-a]pyridin-8-yl)acrylamide |
| 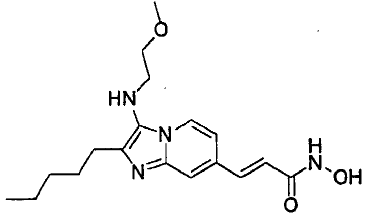 | (E)-N-hydroxy-3-(3-(2-methoxyethylamino)-2-pentylimidazo[1,2-a]pyridin-7-yl)acrylamide |
| 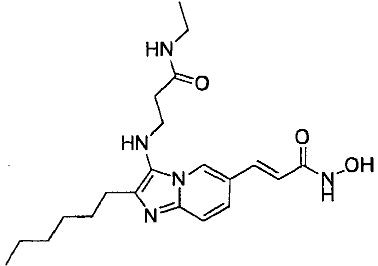 | (E)-3-(3-(3-(ethylamino)-3-oxopropylamino)-2-hexylimidazo[1,2-a]pyridin-6-yl)-N-hydroxyacrylamide |
| 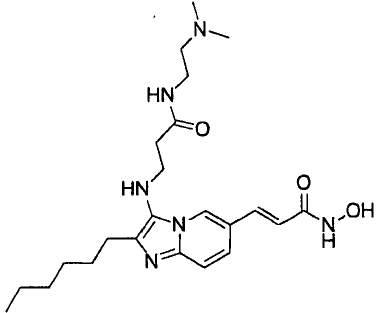 | (E)-3-(3-(3-(2-(dimethylamino)ethylamino)-3-oxopropylamino)-2-hexylimidazo[1,2-a]pyridin-6-yl)-N-hydroxyacrylamide |
| 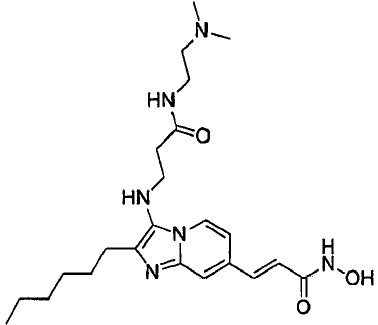 | 3-{3-[2-(2-Dimethylamino-ethylcarbamoyl)-ethylamino]-2-hexyl-imidazo[1,2-a]pyridin-7-yl}-N-hydroxy-acrylamide |
| 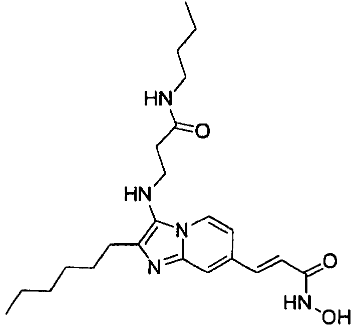 | 3-[3-(2-Butylcarbamoyl-ethylamino)-2-hexylimidazo[1,2-a]pyridin-7-yl]-N-hydroxyacrylamide |
| 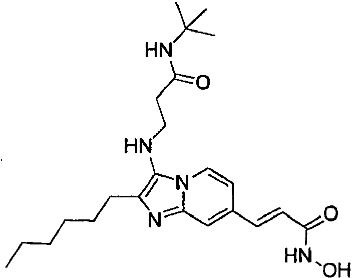 | 3-[3-(2-tert-Butylcarbamoyl-ethylamino)-2-hexyl-imidazo[1,2-a]pyridin-7-yl]-N-hydroxyacrylamide |
| 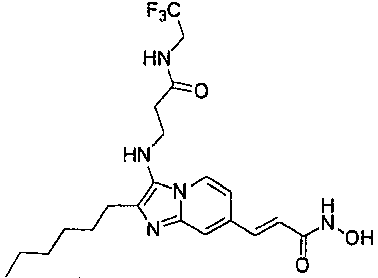 | 3-{2-Hexyl-3-[2-(2,2,2-trifluoro-ethylcarbamoyl)-ethylamino]-imidazo[1,2-a]pyridin-7-yl}-N-hydroxy-acrylamide |
| 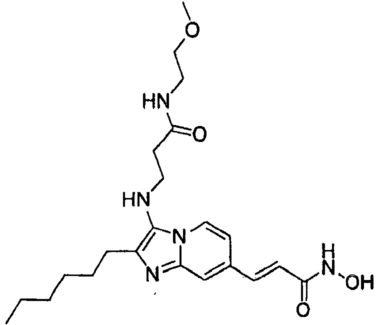 | 3-{2-Hexyl-3-[2-(2-methoxy-ethylcarbamoyl)-ethylamino]-imidazo[1,2-a]pyddin-7-yl}-N-hydroxy-acrylamide |
| 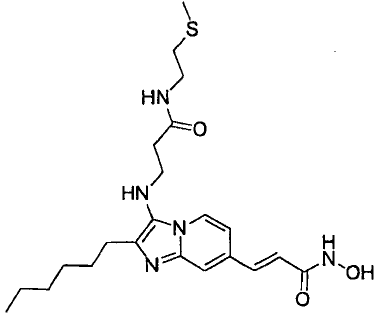 | 3-{2-Hexyl-3-[2-(2-methylsulfanylethylcarbamoyl)-ethylamino]-imidazo[1,2-a]pyridin-7-yl}-N-hydroxy-acrylamide |
| 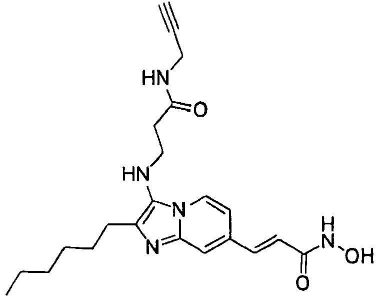 | 3-[2-Hexyl-3-(2-prop-2-ynylcarbamoylethylamino)-imidazo[1,2-a]pyridin-7-yl]-N-hydroxy-acrylamide |
| 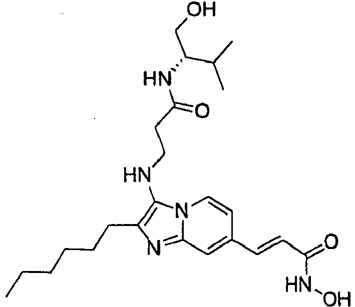 | 3-{2-Hexyl-3-[2-(1-hydroxymethyl-2-methylpropylcarbamoyl)-ethylamino]-imidazo[1,2-a]pyridin-7-yl}-N-hydroxy-acrylamide |
| 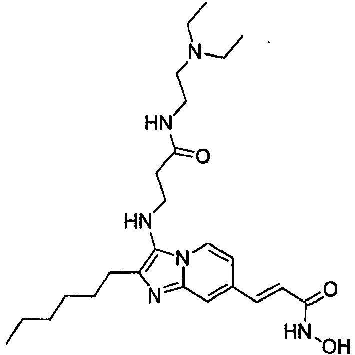 | 3-{3-[2-(2-Diethylamino-ethylcarbamoyl)-ethylamino]-2-hexyl-imidazo[1,2-a]pyridin-7-yl}-N-hydroxy-acrylamide |
| 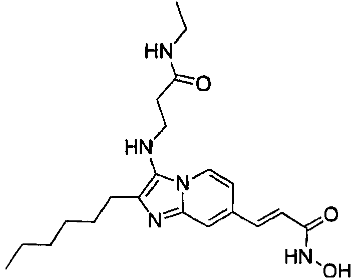 | 3-[3-(2-Ethylcarbamoyl-ethylamino)-2-hexylimidazo[1,2-a]pyridin-7-yl]-N-hydroxyacrylamide |
| 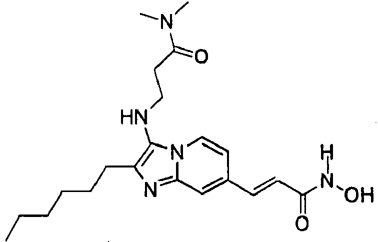 | 3-[3-(2-Dimethylcarbamoyl-ethylamino)-2-hexylimidazo[1,2-a]pyridin-7-yl]-N-hydroxyacrylamide |
| 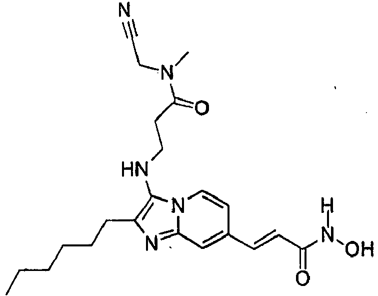 | 3-{3-[2-(Cyanomethyl-methyl-carbamoyl)-ethylamino]-2-hexyl-imidazo[1,2-a]pyridin-7-yl}-N-hydroxy-acrylamide |
| 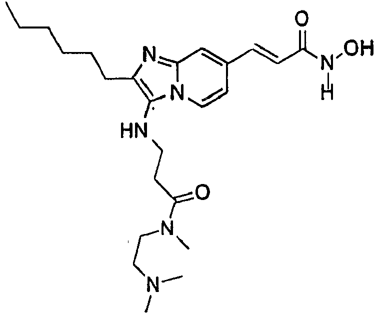 | 3-(3-{2-[(2-Dimethylamino-ethyl)-methyl-carbamoyl]-ethylamino}-2-hexyl-imidazo[1,2-a]pyridin-7-yl)-N-hydroxy-acrylamide |
| 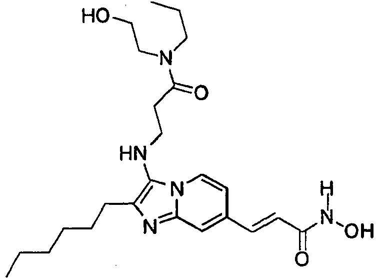 | 3-(2-Hexyl-3-{2-[(2-hydroxy-ethyl)-propylcarbamoyl]-ethylamino}-imidazo[1,2-a]pyddin-7-yl)-N-hydroxy-acrylamide |
| 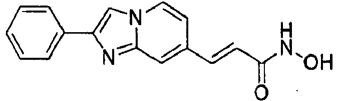 | N-Hydroxy-3-(2-phenyl-imidazo[1,2-a]pyridin-7-yl)-acrylamide |
| 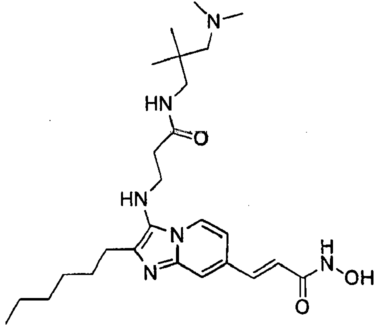 | 3-{3-[2-(3-Dimethylamino-2,2-dimethylpropylcarbamoyl)-ethylamino]-2-hexylimidazo[1,2-a]pyridin-7-yl}-N-hydroxyacrylamide |
| 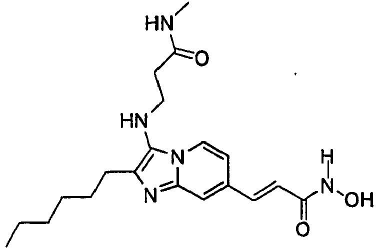 | 3-[2-Hexyl-3-(2-methylcarbamoyl-ethylamino)-imidazo[1,2-a]pyridin-7-yl]-N-hydroxyacrylamide |
| 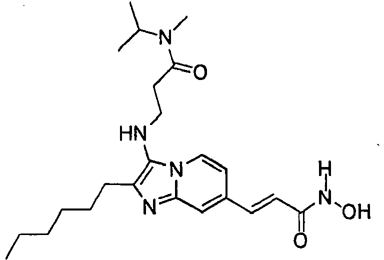 | 3-{2-Hexyl-3-[2-(isopropyl-methyl-carbamoyl)-ethylamino]-imidazo[1,2-a]pyridin-7-yl}-N-hydroxy-acrylamide |
| 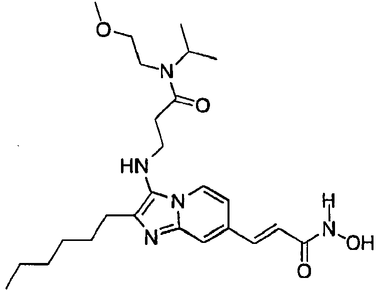 | 3-(2-Hexyl-3-{2-[isopropyl-(2-methoxy-ethyl)-carbamoyl]-ethylamino}-imidazo[1,2-a]pyridin-7-yl)-N-hydroxy-acrylamide |
| 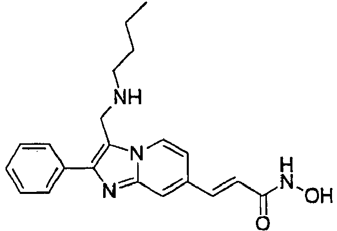 | 3-(3-Butylaminomethyl-2-phenyl-imidazo[1,2-a]pyridin-7-yl)-N-hydroxy-acrylamide |
| 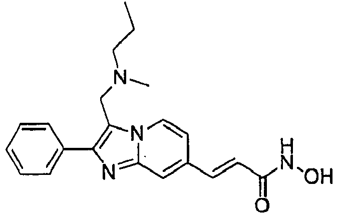 | N-Hydroxy-3-{3-[(methyl-propyl-amino)-methyl]-2-phenyl-imidazo[1,2-a]pyridin-7-yl}-acrylamide. |
| 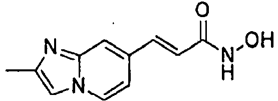 | N-Hydroxy-3-(2-methyl-imidazo[1,2-a]pyridin-7-yl)-acrylamide |
| 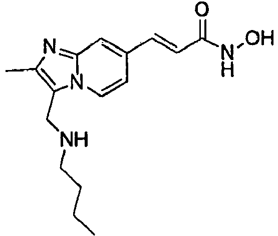 | 3-(3-Butylaminomethyl-2-methyl-imidazo[1,2-a]pyridin-7-yl)-N-hydroxy-acrylamide |
| 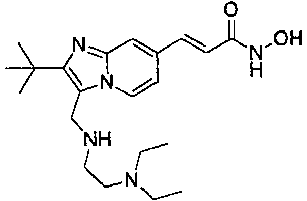 | 3-{2-tert-Butyl-3-[(2-diethylamino-ethylamino)-methyl]-imidazo[1,2-a]pyridin-7-yl}-N-hydroxy-acrylamide |
| 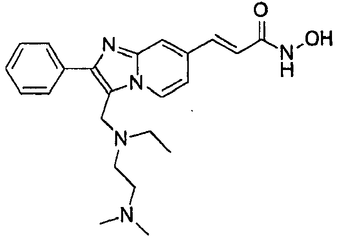 | 3-(3-{[(2-Dimethylamino-ethyl)-ethyl-amino]-methyl}-2-phenyl-imidazo[1,2-a]pyridin-7-yl)-N-hydroxy-acrylamide |
| 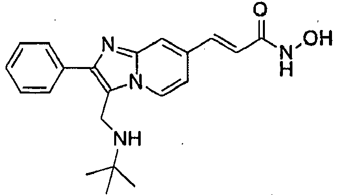 | 3-[3-(tert-Butylamino-methyl)-2-phenyl-imidazo[1,2-a]pyridin-7-yl]-N-hydroxy-acrylamide |
| 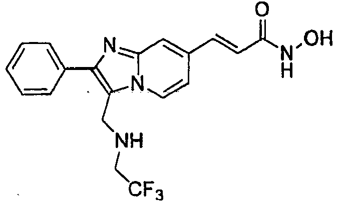 | N-Hydroxy-3-{2-phenyl-3-[(2,2,2-trifluoroethylamino)-methyl]-imidazo[1,2-a]pyridin-7-yl}-acrylamide |
| 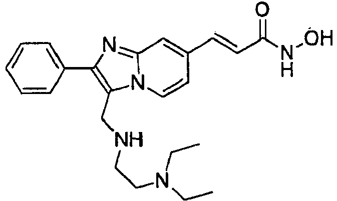 | 3-{3-[(2-Diethylamino-ethylamino)-methyl]-2-phenyl-imidazo[1,2-a]pyridin-7-yl}-N-hydroxyacrylamide |
| 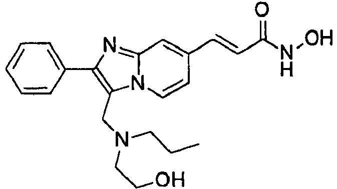 | N-Hydroxy-3-(3-{[(2-hydroxy-ethyl)-propylamino]-methyl}-2-phenyl-imidazo[1,2-a]pyridin-7-yl)-acrylamide |
| 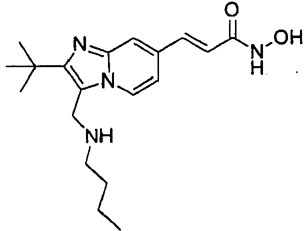 | 3-(2-tert-Butyl-3-butylaminomethyl-imidazo[1,2-a]pyridin-7-yl)-N-hydroxy-acrylamide |
| 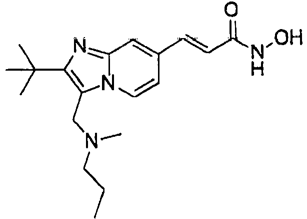 | 3-{2-tert-Butyl-3-[(methyl-propyl-amino)-methyl]-imidazo[1,2-a]pyridin-7-yl}-N-hydroxyacrylamide |
| 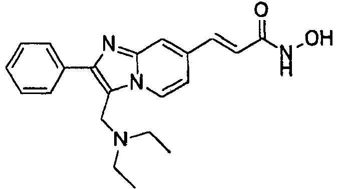 | 3-(3-Diethylaminomethyl-2-phenyl-imidazo[1,2-a]pyridin-7-yl)-N-hydroxy-acrylamide |
| 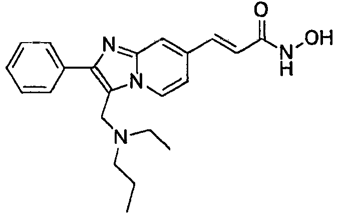 | 3-{3-[(Ethyl-propyl-amino)-methyl]-2-phenylimidazo[1,2-a]pyridin-7-yl}-N-hydroxyacrylamide |
| 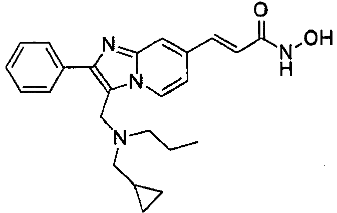 | 3-{3-[(Cyclopropylmethyl-propyl-amino)-methyl]-2-phenyl-imidazo[1,2-a]pyridin-7-yl}-N-hydroxy-acrylamide |
| 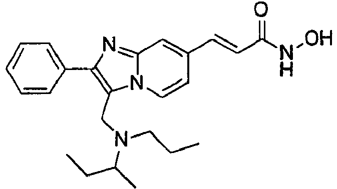 | 3-{3-[(sec-Butyl-propyl-amino)-methyl]-2-phenyl-imidazo[1,2-a]pyridin-7-yl}-N-hydroxyacrylamide |
| 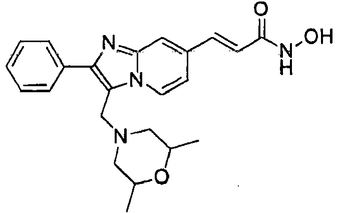 | 3-[3-(2,6-Dimethyl-morpholin-4-ylmethyl)-2-phenyl-imidazo[1,2-a]pyridin-7-yl]-N-hydroxyacrylamide |
| 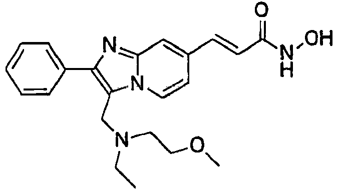 | 3-(3-{[Ethyl-(2-methoxy-ethyl)-amino]-methyl}-2-phenyl-imidazo[1,2-a]pyridin-7-yl)-N-hydroxyacrylamide |
| 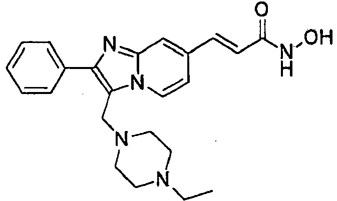 | 3-[3-(4-Ethyl-piperazin-1-ylmethyl)-2-phenylimidazo[1,2-a]pyridin-7-yl]-N-hydroxyacrylamide |
| 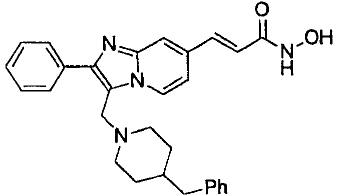 | 3-[3-(4-Benzyl-piperidin-1-ylmethyl)-2-phenylimidazo[1,2-a]pyridin-7-yl]-N-hydroxyacrylamide |
| 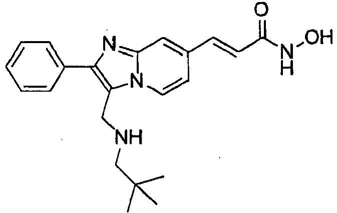 | 3-{3-[(2,2-Dimethyl-propylamino)-methyl]-2-phenyl-imidazo[1,2-a]pyridin-7-yl}-N-hydroxyacrylamide |
| 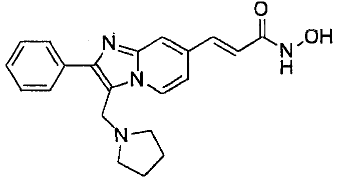 | N-Hydroxy-3-(2-phenyl-3-pyrrolidin-1-ylmethylimidazo[1,2-a]pyridin-7-yl)-acrylamide |
| 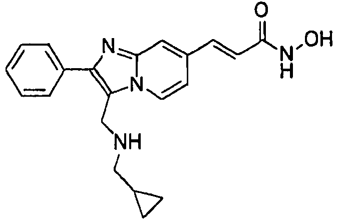 | 3-{3-[(Cyclopropylmethyl-amino)-methyl]-2-phenyl-imidazo[1,2-a]pyridin-7-yl}-N-hydroxyacrylamide |
| 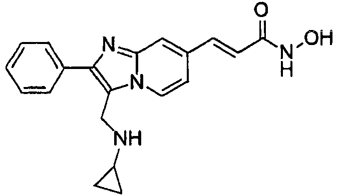 | 3-(3-Cyclopropylaminomethyl-2-phenylimidazo[1,2-a]pyridin-7-yl)-N-hydroxyacrylamide |
| 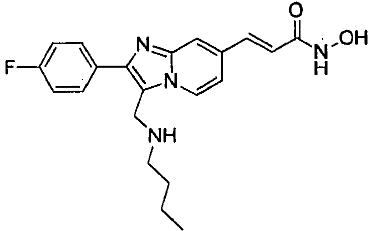 | 3-[3-Butylaminomethyl-2-(4-fluoro-phenyl)-imidazo[1,2-a]pyridin-7-yl]-N-hydroxyacrylamide |
| 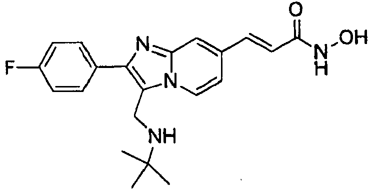 | 3-[3-(tert-Butylamino-methyl)-2-(4-fluorophenyl)-imidazo[1,2-a]pyridin-7-yl]-N-hydroxyacrylamide. |

15. A pharmaceutical composition including a compound according to any one of claims 1 to 14 and a pharmaceutically acceptable diluent, excipient or carrier.

16. A compound according to any one of claims 1 to 14 for use in the treatment of a disorder caused by, associated with or accompanied by disruptions of cell proliferation and/or angiogenesis.

17. A compound according to claim 16 wherein the proliferative disorder is cancer.

18. A compound according to any one of claims 1 to 14 for use in the treatment of a disorder that can be treated by the inhibition of histone deacetylase.

19. A compound according to claim 18 wherein the disorder is selected from the group consisting of Proliferative disorders (e.g. cancer); Neurodegenerative diseases including Huntington's Disease, Polyglutamine diseases, Parkinson's Disease, Alzheimer's Disease, Seizures, Striatonigral degeneration, Progressive supranuclear palsy, Torsion dystonia, Spasmodic torticollis and dyskinesis, Familial tremor, Gilles de la Tourette syndrome, Diffuse Lewy body disease, Pick's disease, Intracerebral haemorrhage Primary lateral sclerosis, Spinal muscular atrophy, Amyotrophic lateral sclerosis, Hypertrophic interstitial polyneuropathy, Retinitis pigmentosa, Hereditary optic atrophy, Hereditary spastic paraplegia, Progressive ataxia and Shy-Drager syndrome; Metabolic diseases including Type 2 diabetes; Degenerative Diseases of the Eye including Glaucoma, Age-related macular degeneration, macular myopic degeneration, Rubeotic glaucoma, Interstitial keratitis, Diabetic retinopathy, Peter's anomaly, retinal degeneration, Cellophane Retinopathy; Cogan's Dystrophy; Corneal Dystrophy; Iris Neovascularization (Rubeosis); Neovascularization of the Cornea; Retinopathy of Prematurity; Macular Edema; Macular Hole; Macular Pucker; Marginal Blepharitis, Myopia, nonmalignant growth of the conjunctiva; Inflammatory diseases and/or Immune system disorders including Rheumatoid Arthritis (RA), Osteoarthritis, Juvenile chronic arthritis, Graft versus Host disease, Psoriasis, Asthma, Spondyloarthropathy, Crohn's Disease, inflammatory bowel disease, Colitis Ulcerosa, Alcoholic hepatitis, Diabetes, Sjoegrens's syndrome, Multiple Sclerosis, Ankylosing spondylitis, Membranous glomerulopathy, Discogenic pain, Systemic Lupus Erythematosus, allergic contact dermatitis; Disease involving angiogenesis including cancer, psoriasis, rheumatoid arthritis; Psychological disorders including bipolar disease, schizophrenia, depression and dementia; Cardiovascular Diseases including Heart failure, restenosis, cardiac hypertrophy and arteriosclerosis; Fibrotic diseases including liver fibrosis, lung fibrosis, cystic fibrosis and angiofibroma; Infectious diseases including Fungal infections, such as Candida Albicans, Bacterial infections, Viral infections, such as Herpes Simplex, Protozoal infections, such as Malaria, Leishmania infection, Trypanosoma brucei infection, Toxoplasmosis and coccidiosis and Haematopoietic disorders including thalassemia, anemia and sickle cell anemia.

20. A compound according to claim 18 wherein the disorder is selected from the group consisting of a neurodegenerative disorder, an inflammatory disease and/or immune system disorder, and a degenerative eye disease.

21. A compound according to any one of claims 1 to 14 for use in the treatment of cancer.

22. A compound according to claim 21 wherein the cancer is a hematologic malignancy.

23. A compound according to claim 22 wherein the hematologic malignancies are selected from a group consisting of B-cell lymphoma, T-cell lymphoma and leukemia.

24. A compound according to claim 21 wherein the cancer is a solid tumor.

25. A compound according to claim 24 wherein the solid tumor is selected from the group consisting of breast cancer, lung cancer, ovarian cancer, prostate cancer, head and neck cancer, renal cancer, gastric cancer, colon cancer, pancreatic cancer and brain cancer.

## Patentansprüche

1. Verbindung der Formel (Ic): 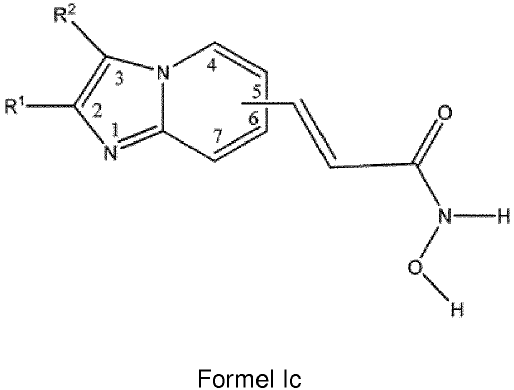 wobei
R¹ ausgewählt ist aus der Gruppe bestehend aus: C₁-C₁₄-Alkyl, Aryl-C₁-C₁₄-alkyl, Aryl und CO₂H;
R² L ist, wobei L -(CR²⁰R²¹)ₘ-(CR²²R²³)ₙ-(CR²⁴R²⁵)ₒ-NR²⁶R²⁷ ist;
wobei
jedes R²⁰, R²¹, R²², R²³, R²⁴ und R²⁵ unabhängig ausgewählt ist aus der Gruppe bestehend aus H und C₁-C₁₄-Alkyl; oder
R²⁰ und R²¹ zusammen genommen eine Gruppe der Formel =O oder =S bilden können, und/oder
R²² und R²³ zusammen genommen eine Gruppe der Formel =O oder =S bilden können, und/oder
R²⁴ und R²⁵ zusammen genommen eine Gruppe der Formel =O oder =S bilden können;
jedes R²⁶ und R²⁷ unabhängig ausgewählt ist aus der Gruppe bestehend aus: H, C₁-C₁₄-Alkyl, C₂-C₁₄-Heteroalkyl, C₅-C₁₀-Cycloalkyl, optional substituiertem Aryl, Aryl-C₁-C₁₄-Alkyl, C₅-C₁₀-Cycloalkyl-C₁-C₁₄-Alkyl und G, oder
R²⁶ und R²⁷ zusammen genommen mit dem Stickstoffatom, an welches sie gebunden sind, ein Heterocycloalkyl bilden, welches optional substituiert sein kann;
wobei die optionalen Substituenten an dem Heterocycloalkyl ausgewählt sind aus der Gruppe bestehend aus H, Methyl, Ethyl und Benzyl,
wobei die optionalen Substituenten am Aryl ausgewählt sind aus der Gruppe bestehend aus H, Methoxy, Methylendioxy, und Piperidinyl,
m, n und o jeweils ganze Zahlen sind, die unabhängig voneinander ausgewählt sind aus der Gruppe bestehend aus 0, 1, 2, 3 und 4;
G eine Gruppe der Formel :
-L³W³
ist, wobei
L³ C₁-C₅-Alkylen ist;
W³ ausgewählt ist aus der Gruppe bestehend aus -OR¹², -N(R¹²)₂, und - C(O)N(R¹²)₂,
R¹² ausgewählt ist aus der Gruppe bestehend aus H, -CN, C₁-C₁₄-Alkyl, C₂-C₁₄-Alkynyl, C₁-C₁₄-Haloalkyl, C₂-C₁₄-Heteroalkyl, Hydroxy, und Hydroxy-C₁-C₁₄-Alkyl,
oder ein pharmazeutisch verträgliches Salz davon.

2. Verbindung nach Anspruch 1, wobei die Doppelbindung an die Position 5 oder 6 im Ring gebunden ist.

3. Verbindung nach Anspruch 1 oder Anspruch 2, wobei die Doppelbindung an die Position 6 im Ring gebunden ist.

4. Verbindung nach einem der Ansprüche 1 bis 3, wobei R¹ C₁-C₁₄-Alkyl ist.

5. Verbindung nach einem der Ansprüche 1 bis 3, wobei R¹ ausgewählt ist aus der Gruppe bestehend aus Methyl, Ethyl, 2-Carboxy-ethyl, Propyl, Isopropyl, 2,2-Dimethyl-propyl, Butyl, Isobutyl, Tert-butyl, Pentyl, 2,4,4-Trimethyl-pentyl, Hexyl, 2-Phenyl-ethyl, Phenyl und 4-Fluoro-phenyl.

6. Verbindung nach einem der Ansprüche 1 bis 5, wobei R² ausgewählt ist aus der Gruppe bestehend aus:

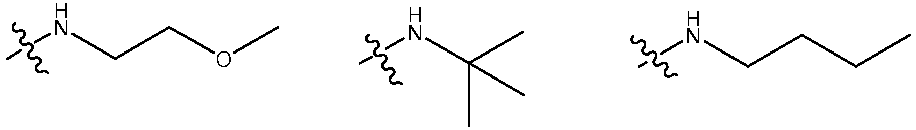

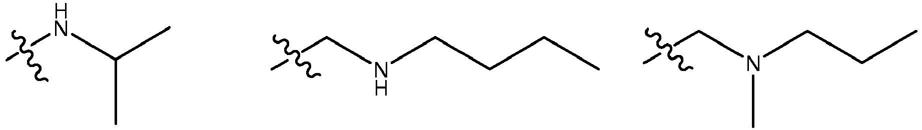

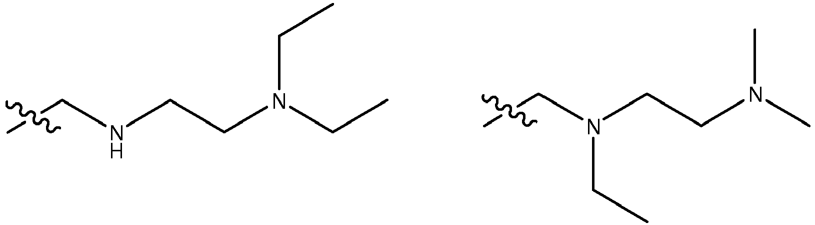

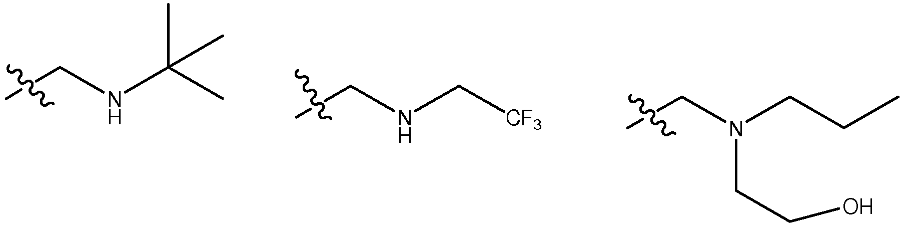

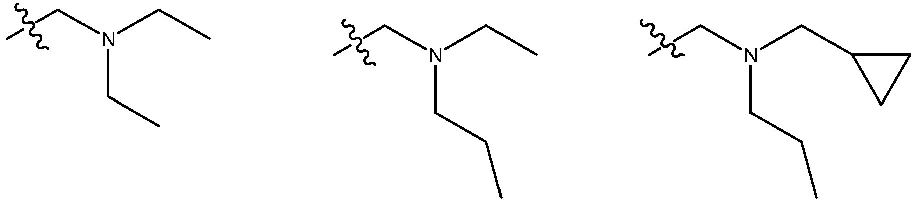

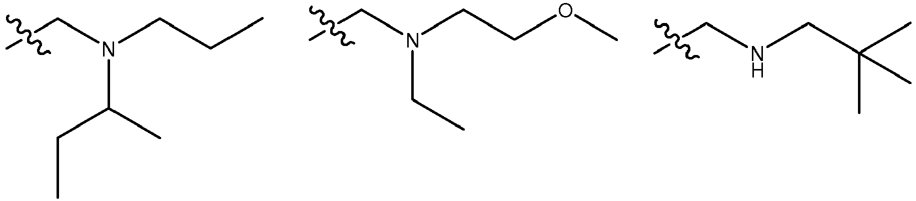

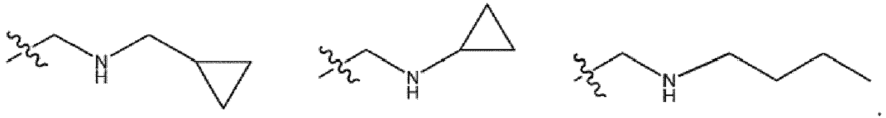

7. Verbindung nach Anspruch 1, wobei die Verbindung die Formel 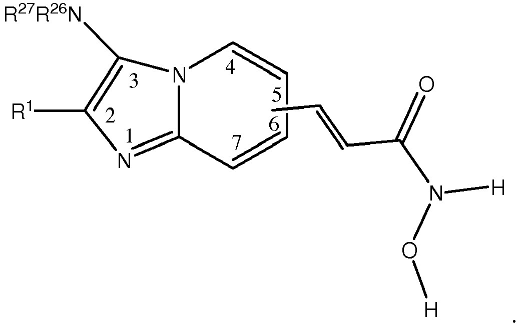 aufweist, wobei R¹ R²⁶ und R²⁷ wie in Anspruch 1 definiert sind.

8. Verbindung nach Anspruch 1, wobei die Verbindung die Formel : 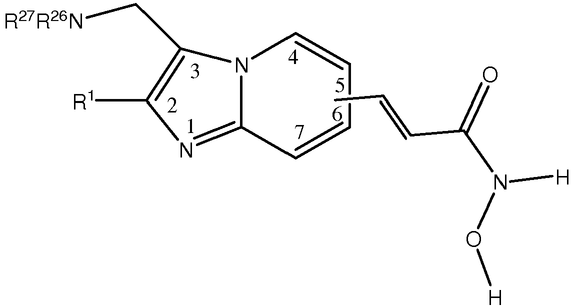 aufweist, wobei R¹, R²⁶ und R²⁷ wie in Anspruch 1 definiert sind.

9. Verbindung nach Anspruch 1, wobei R²⁶ und R²⁷ unabhängig ausgewählt sind aus der Gruppe bestehend aus H, 3,4,5-Trimethoxyphenyl, 3,4-Methylendioxybenzyl, 4-Piperidin-1-yl-phenyl, 3,4-Methylendioxyphenyl, 2-Methoxy-ethyl, Cyclopropyl, Cyclohexyl, Methyl, Cyclopropyl-methyl, Ethyl, Propyl, Isopropyl, 2,2-Dimethyl-propyl, Butyl, t-Butyl, sec-Butyl, 2-(Diethylamino)-ethyl, 2-(Dimethylamino)-ethyl und 2,2,2-Trifluoroethyl.

10. Verbindung nach einem der Ansprüche 1 bis 8, wobei R²⁶ G ist und R²⁷ H oder Alkyl ist, wobei G wie in Anspruch 1 definiert ist.

11. Verbindung nach Anspruch 10, wobei G eine Gruppe der Formel ist:
-(CH₂)₂-C(O)N(R¹²)₂.

12. Verbindung nach Anspruch 11, wobei jedes R¹² unabhängig ausgewählt ist aus der Gruppe bestehend aus H, C₁-C₁₄-Alkyl, C₂-C₁₄-Heteroalkyl und C₂-C₁₄-Alkynyl.

13. Verbindung nach Anspruch 11, wobei G ausgewählt ist aus der Gruppe bestehend aus:

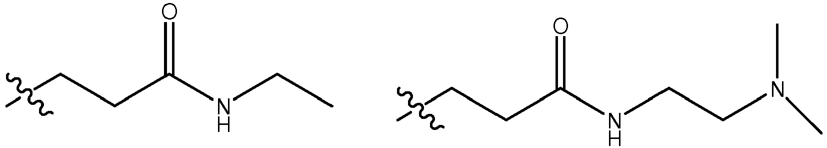

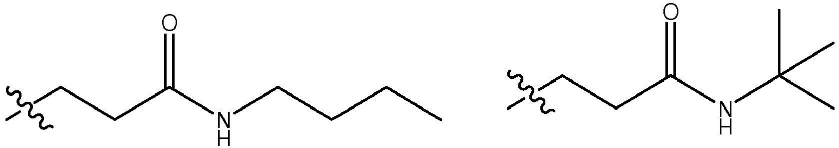

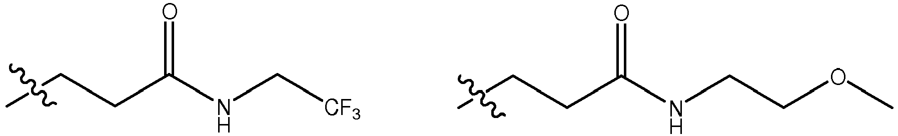

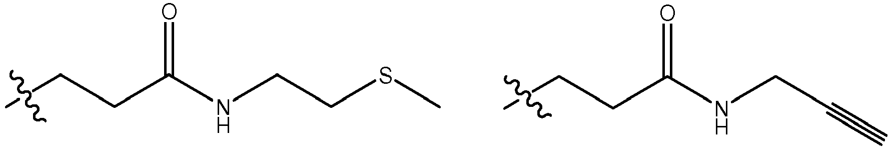

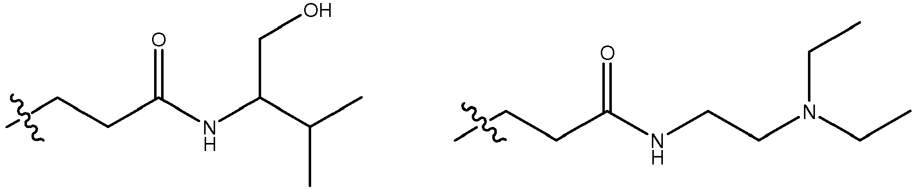

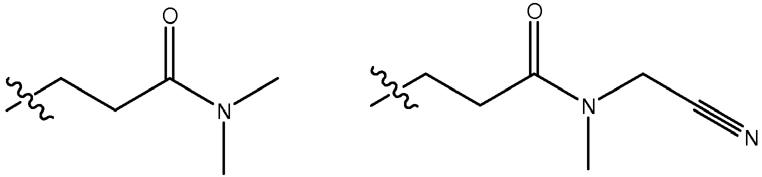

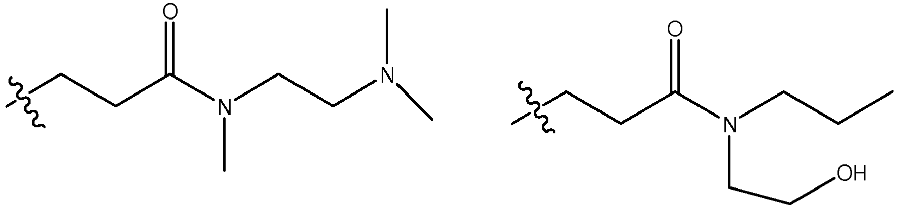

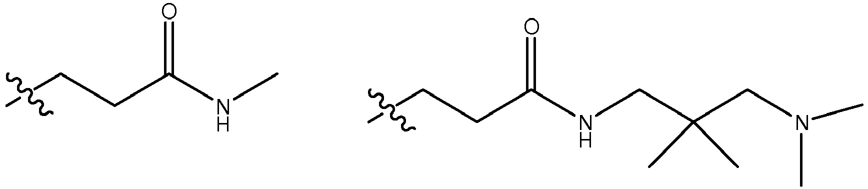

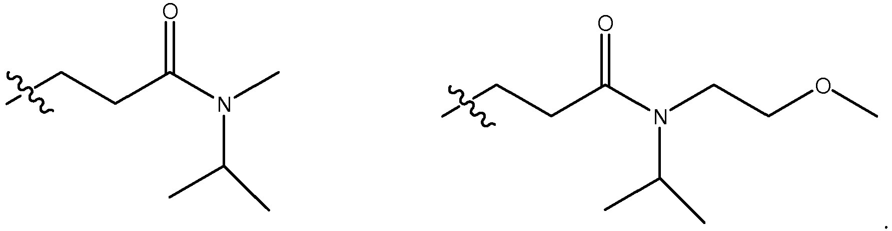

14. Verbindung nach Anspruch 1, wobei die Verbindung ausgewählt ist aus Verbindungen, und deren pharmazeutisch verträglichen Salzen, die ausgewählt sind aus der Gruppe bestehend aus
| **Struktur** | **Name** |
|---|---|
| 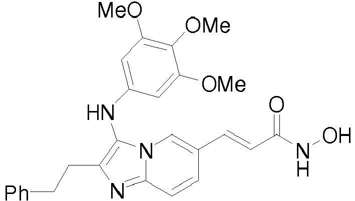 | (E)-N-hydroxy-3-(2-phenethyl-3-(3,4,5-trimethoxyphenylamino)imidazo[1,2-a]pyridin-6-yl)acrylamid |
| 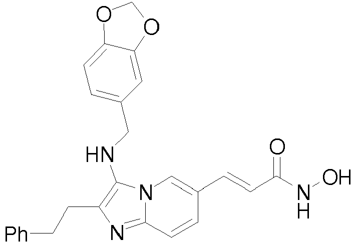 | (E)-3-(3-(benzo[d][1,3]dioxol-5-ylmethylamino)-2-phenethylimidazo[1,2-a]pyridin-6-yl)-N-hydroxyacrylamid |
| 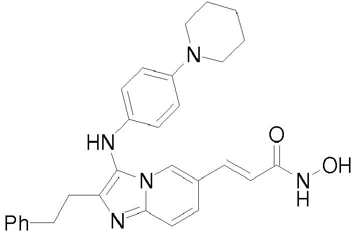 | N-Hydroxy-3-[2-phenethyl-3-(4-piperidin-1-yl-phenylamino)-imidazo[1,2-a]pyridin-6-yl]-acrylamid |
| 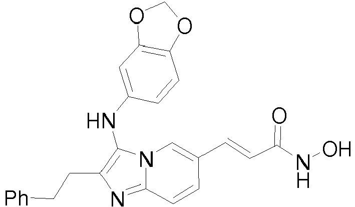 | (E)-3-(3-(benzo[d][1,3]dioxol-5-ylamino)-2-phenethylimidazo[1,2-a]pyridin-6-yl)-N-hydroxyacrylamid |
| 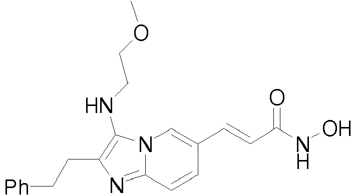 | (E)-N-hydroxy-3-(3-(2-methoxyethylamino)-2-phenethylimidazo[1,2-a]pyridin-6-yl)acrylamid |
| 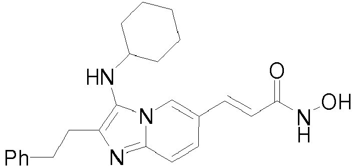 | (E)-3-(3-(cyclohexylamino)-2-phenethylimidazo[1,2-a]pyridin-6-yl)-N-hydroxyacrylamid |
| 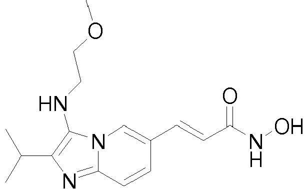 | (E)-N-hydroxy-3-(2-isopropyl-3-(2-methoxyethylamino)imidazo[1,2-a]pyridin-6-yl)acrylamid |
| 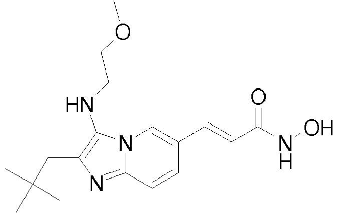 | 3-[2-(2,2-Dimethyl-propyl)-3-(2-methoxyethylamino)-imidazo[1,2-a]pyridin-6-yl]-N-hydroxyacrylamid |
| 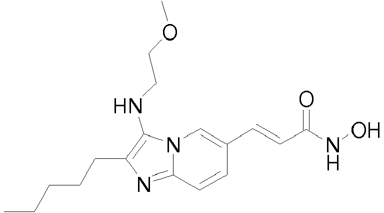 | (E)-N-hydroxy-3-(3-(2-methoxyethylamino)-2-pentylimidazo[1,2-a]pyridin-6-yl)acrylamid |
| 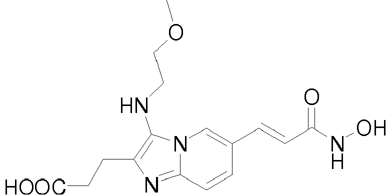 | 3-[6-(2-Hydroxycarbamoyl-vinyl)-3-(2-methoxy-ethylamino)-imidazo[1,2-a]pyridin-2-yl]-propionsäure |
| 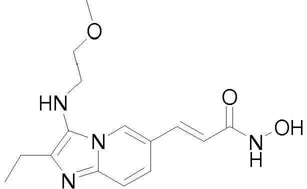 | 3-[2-Ethyl-3-(2-methoxy-ethylamino)-imidazo[1,2-a]pyridin-6-yl]-N-hydroxyacrylamid |
| 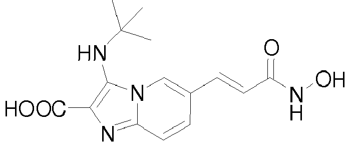 | (E)-3-(tert-butylamino)-6-(3-(hydroxyamino)-3-oxoprop-1-enyl)imidazo[1,2-a]pyridin-2-carboxylsäure |
| 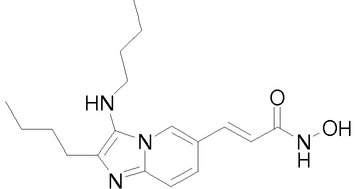 | (E)-3-(2-butyl-3-(butylamino)imidazo[1,2-a]pyridin-6-yl)-N-hydroxyacrylamid |
| 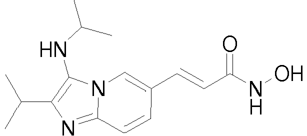 | (E)-N-hydroxy-3-(2-isopropyl-3-(isopropylamino)imidazo[1,2-a]pyridin-6-yl)acrylamid |
| 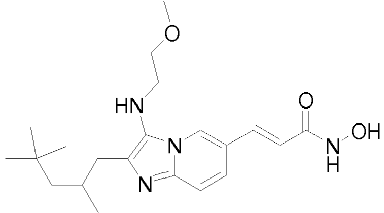 | (E)-N-hydroxy-3-(3-(2-methoxyethy!am!no)-2-(2,4,4-trimethylpentyl)imidazo[1,2-a]pyridin-6-yl)acrylamid |
| 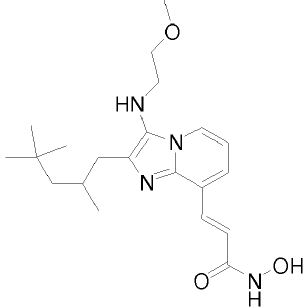 | (E)-N-hydroxy-3-(3-(2-methoxyethylamino)-2-(2,4,4-trimethylpentyl)imidazo[1,2-a]pyridin-8-yl)acrylamid |
| 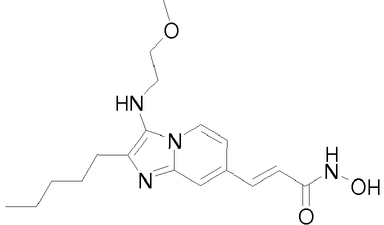 | (*E*)-N-hydroxy-3-(3-(2-methoxyethylamino)-2-pentylimidazo[1,2-a]pyridin-7-yl)acrylamid |
| 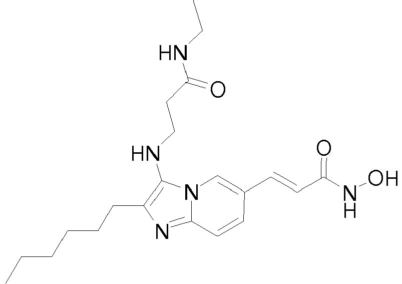 | (E)-3-(3-(3-(ethylamino)-3-oxopropylamino)-2-hexylimidazo[1,2-a]pyridin-6-yl)-N-hydroxyacrylamid |
| 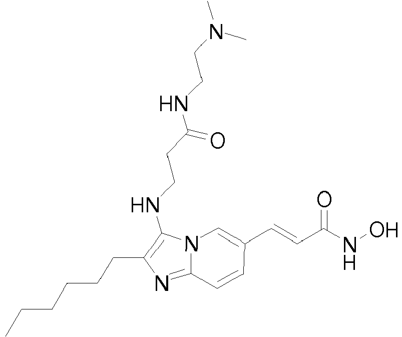 | (E)-3-(3-(3-(2-(dimethylamino)ethylamino)-3-oxopropylamino)-2-hexylimidazo[1,2-a]pyridin-6-yl)-N-hydroxyacrylamid |
| 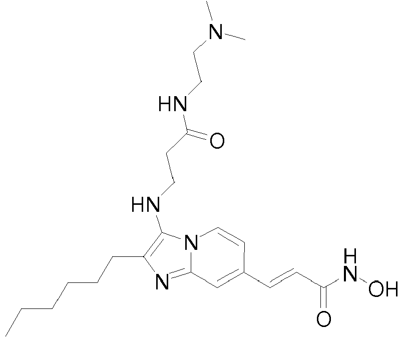 | 3-{3-[2-(2-Dimethylamino-ethylcarbamoyl)-ethylamino]-2-hexyl-imidazo[1,2-a]pyridin-7-yl}-N-hydroxyacrylamid |
| 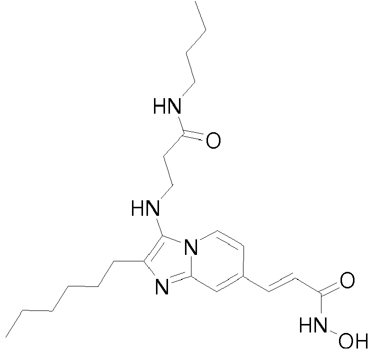 | 3-[3-(2-Butylcarbamoyl-ethylamino)-2-hexyl-imidazo[1,2-a]pyridin-7-yl]-N-hydroxyacrylamid |
| 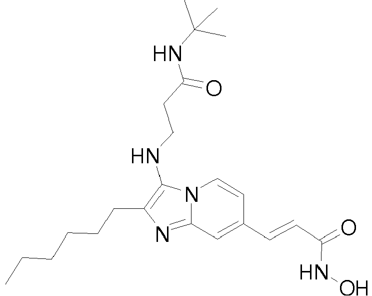 | 3-[3-(2-tert-Butylcarbamoyl-ethylamino)-2-hexyl-imidazo[1,2-a]pyridin-7-yl]-N-hydroxyacrylamid |
| 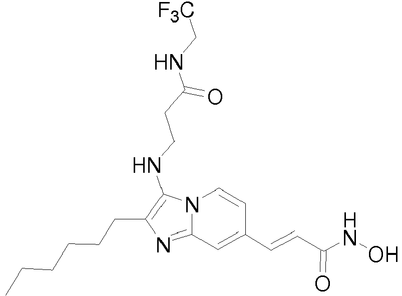 | 3-{2-Hexyl-3-[2-(2,2,2-trifluoro-ethylcarbamoyl)-ethylamino]-imidazo[1,2-a]pyridin-7-yl}-N-hydroxyacrylamid |
| 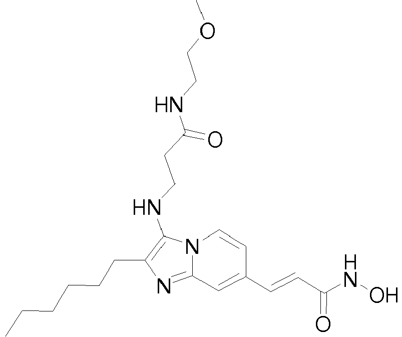 | 3-{2-Hexyl-3-[2-(2-methoxy-ethylcarbamoyl)-ethylamino]-imidazo[1,2-a]pyridin-7-yl}-N-hydroxyacrylamid |
| 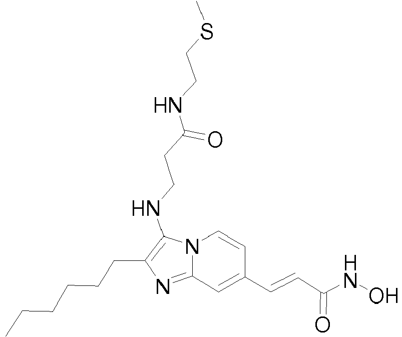 | 3-{2-Hexyl-3-[2-(2-methylsulfanyl-ethylcarbamoyl)-ethylamino]-imidazo[1,2-a]pyridin-7-yl}-N-hydroxyacrylamid |
| 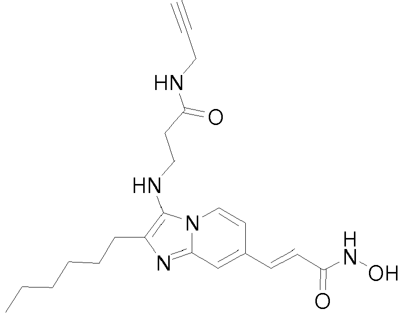 | 3-[2-Hexyl-3-(2-prop-2-ynylcarbamoyl-ethylamino)-imidazo[1,2-a]pyridin-7-yl]-N-hydroxyacrylamid |
| 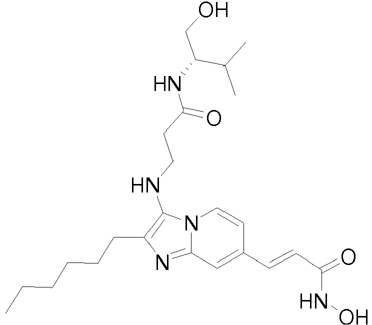 | 3-{2-Hexyl-3-[2-(1-hydroxymethyl-2-methyl-propylcarbamoyl)-ethylamino]-imidazo[1,2-a]pyridin-7-yl}-N-hydroxyacrylamid |
| 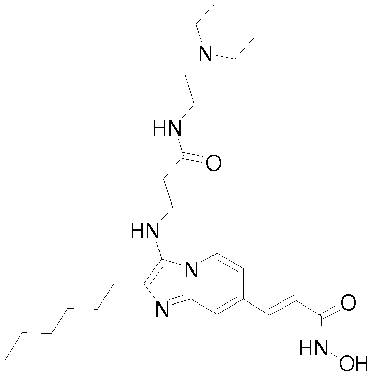 | 3-{3-[2-(2-Diethylamino-ethylcarbamoyl)-ethylamino]-2-hexyl-imidazo[1,2-a]pyridin-7-yl}-N-hydroxyacrylamid |
| 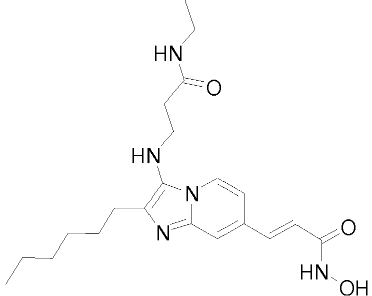 | 3-[3-(2-Ethylcarbamoyl-ethylamino)-2-hexyl-imidazo[1,2-a]pyridin-7-yl]-N-hydroxyacrylamid |
| 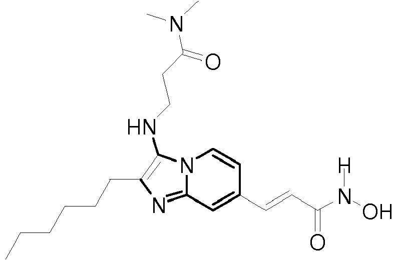 | 3-[3-(2-Dimethylcarbamoyl-ethylamino)-2-hexyl-imidazo[1,2-a]pyridin-7-yl]-N-hydroxyacrylamid |
| 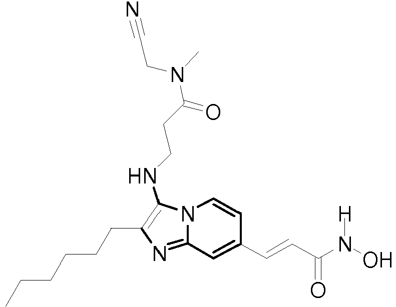 | 3-{3-[2-(Cyanomethyl-methyl-carbamoyl)-ethylamino]-2-hexyl-imidazo[1,2-a]pyridin-7-yl}-N-hydroxyacrylamid |
| 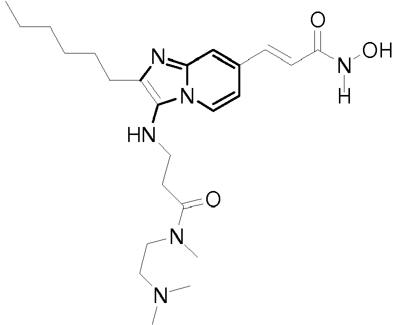 | 3-(3-{2-[(2-Dimethylamino-ethyl)-methyl-carbamoyl]-ethylamino}-2-hexyl-imidazo[1,2-a]pyridin-7-yl)-N-hydroxyacrylamid |
| 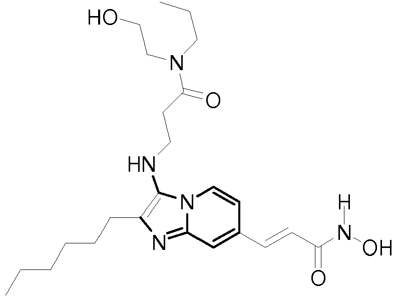 | 3-(2-Hexyl-3-{2-[(2-hydroxy-ethyl)-propyl-carbamoyl]-ethylamino}-imidazo[1,2-a]pyridin-7-yl)-N-hydroxyacrylamid |
| 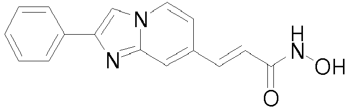 | N-Hydroxy-3-(2-phenyl-imidazo[1,2-a]pyridin-7-yl)-acrylamid |
| 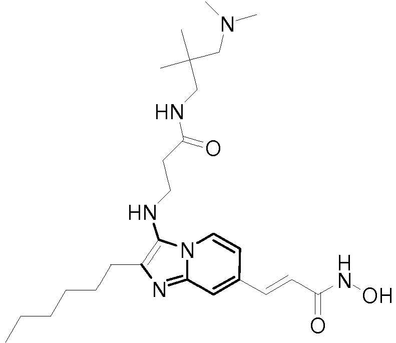 | 3-{3-[2-(3-Dimethylamino-2,2-dimethyl-propylcarbamoyl)-ethylamino]-2-hexyl-imidazo[1,2-a]pyridin-7-yl}-N-hydroxyacrylamid |
| 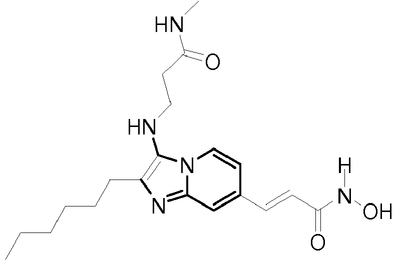 | 3-[2-Hexyl-3-(2-methylcarbamoyl-ethylamino)-imidazo[1,2-a]pyridin-7-yl]-N-hydroxyacrylamid |
| 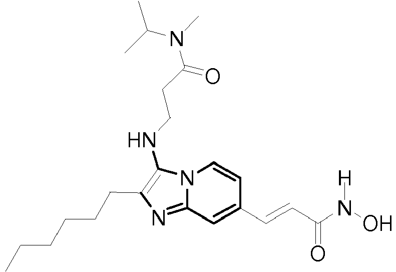 | 3-{2-Hexyl-3-[2-(isopropyl-methyl-carbamoyl)-ethylamino]-imidazo[1,2-a]pyridin-7-yl}-N-hydroxyacrylamid |
| 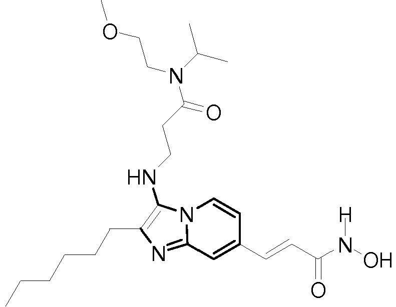 | 3-(2-Hexyl-3-{2-[isopropyl-(2-methoxy-ethyl)-carbamoyl]-ethylamino}-imidazo[1,2-a]pyridin-7-yl)-N-hydroxyacrylamid |
| 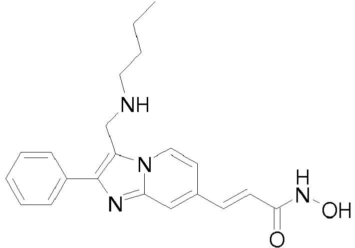 | 3-(3-Butylaminomethyl-2-phenyl-imidazo[1,2-a]pyridin-7-yl)-N-hydroxyacrylamid |
| 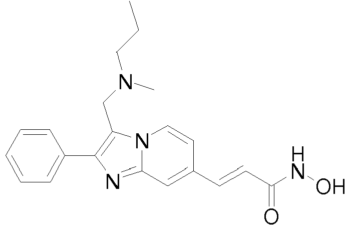 | N-Hydroxy-3-{3-[(methyl-propyl-amino)-methyl]-2-phenyl-imidazo[1,2-a]pyridin-7-yl}-acrylamid. |
| 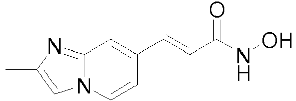 | N-Hydroxy-3-(2-methyl-imidazo[1,2-a]pyridin-7-yl)-acrylamid |
| 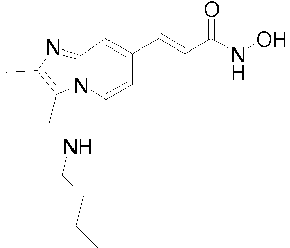 | 3-(3-Butylaminomethyl-2-methyl-imidazo[1,2-a]pyridin-7-yl)-N-hydroxyacrylamid |
| 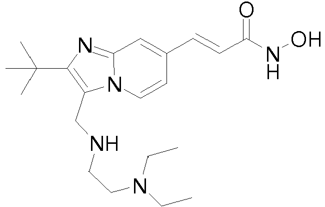 | 3-{2-tert-Butyl-3-[(2-diethylamino-ethylamino)-methyl]-imidazo[1,2-a]pyridin-7-yl}-N-hydroxyacrylamid |
| 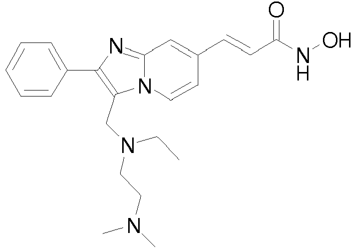 | 3-(3-{[(2-Dimethylamino-ethyl)-ethyl-amino]-methyl}-2-phenyl-imidazo[1,2-a]pyridin-7-yl)-N-hydroxyacrylamid |
| 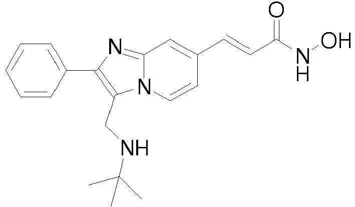 | 3-[3-(tert-Butylamino-methyl)-2-phenyl-imidazo[1,2-a]pyridin-7-yl]-N-hydroxyacrylamid |
| 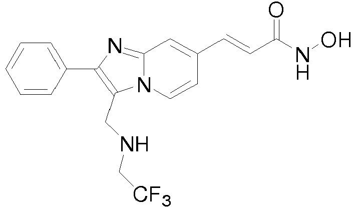 | N-Hydroxy-3-{2-phenyl-3-[(2,2,2-trifluoroethylamino)-methyl]-imidazo[1,2-a]pyridin-7-yl}-acrylamid |
| 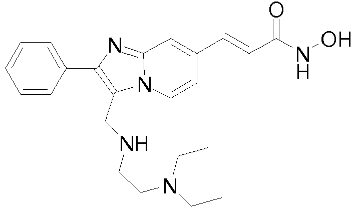 | 3-{3-[(2-Diethylamino-ethylamino)-methyl]-2-phenyl-imidazo[1,2-a]pyridin-7-yl}-N-hydroxyacrylamid |
| 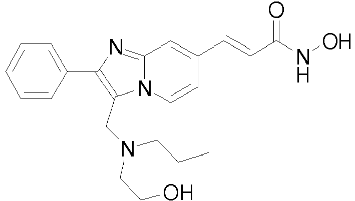 | N-Hydroxy-3-(3-{[(2-hydroxy-ethyl)-propylamino]-methyl}-2-phenyl-imidazo[1,2-a]pyridin-7-yl)-acrylamid |
| 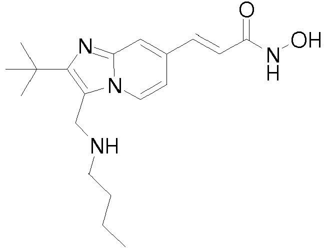 | 3-(2-tert-Butyl-3-butylaminomethylimidazo[1,2-a]pyridin-7-yl)-N-hydroxy-acrylamid |
| 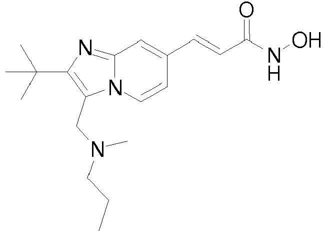 | 3-{2-tert-Butyl-3-[(methyl-propyl-amino)-methyl]-imidazo[1,2-a]pyridin-7-yl}-N-hydroxyacrylamid |
| 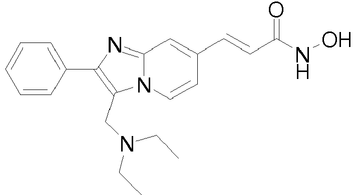 | 3-(3-Diethylaminomethyl-2-phenylimidazo[1,2-a]pyridin-7-yl)-N-hydroxyacrylamid |
| 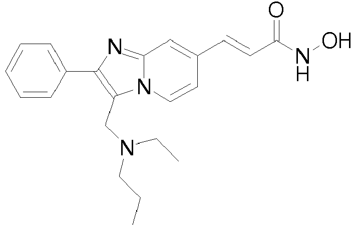 | 3-{3-[(Ethyl-propyl-amino)-methyl]-2-phenyl-imidazo[1,2-a]pyridin-7-yl}-N-hydroxyacrylamid |
| 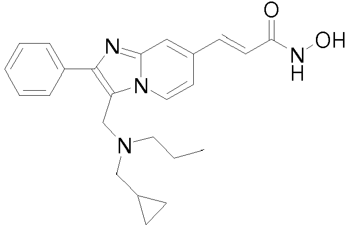 | 3-{3-[(Cyclopropylmethyl-propyl-amino)-methyl]-2-phenyl-imidazo[1,2-a]pyridin-7-yl}-N-hydroxyacrylamid |
| 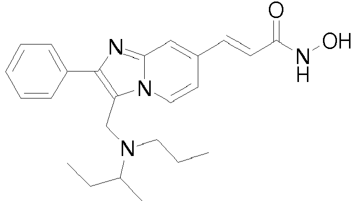 | 3-{3-[(sec-Butyl-propyl-amino)-methyl]-2-phenyl-imidazo[1,2-a]pyridin-7-yl}-N-hydroxyacrylamid |
| 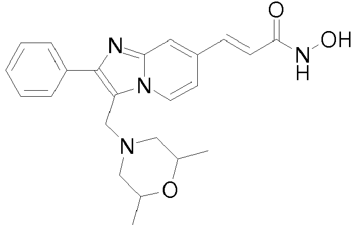 | 3-[3-(2,6-Dimethyl-morpholin-4-ylmethyl)-2-phenyl-imidazo[1,2-a]pyridin-7-yl]-N-hydroxyacrylamid |
| 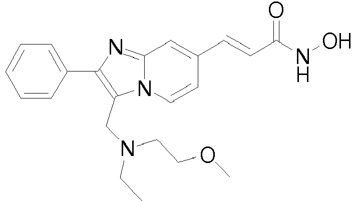 | 3-(3-{[Ethyl-(2-methoxy-ethyl)-amino]-methyl}-2-phenyl-imidazo[1,2-a]pyridin-7-yl)-N-hydroxyacrylamid |
| 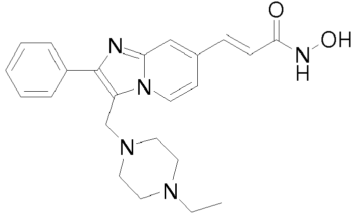 | 3-[3-(4-Ethyl-piperazin-1-ylmethyl)-2-phenyl-imidazo[1,2-a]pyridin-7-yl]-N-hydroxyacrylamid |
| 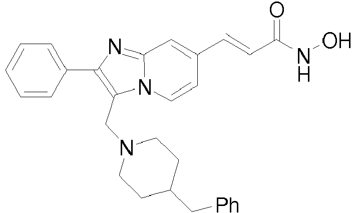 | 3-[3-(4-Benzyl-piperidin-1-ylmethyl)-2-phenyl-imidazo[1,2-a]pyridin-7-yl]-N-hydroxyacrylamid |
| 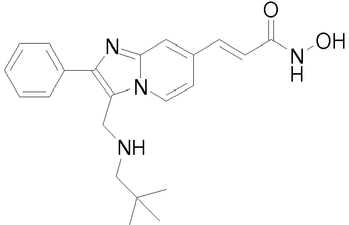 | 3-{3-[(2,2-Dimethyl-propylamino)-methyl]-2-phenyl-imidazo[1,2-a]pyridin-7-yl}-N-hydroxyacrylamid |
| 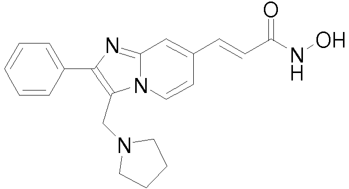 | N-Hydroxy-3-(2-phenyl-3-pyrrolidin-1-ylmethyl-imidazo[1,2-a]pyridin-7-yl)-acrylamid |
| 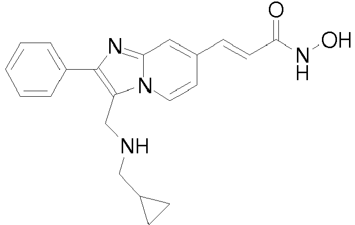 | 3-{3-[(Cyclopropylmethyl-amino)-methyl]-2-phenyl-imidazo[1,2-a]pyridin-7-yl}-N-hydroxyacrylamid |
| 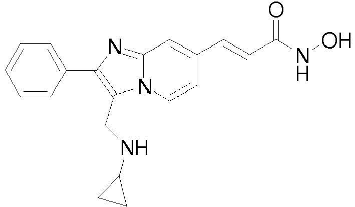 | 3-(3-Cyclopropylaminomethyl-2-phenylimidazo[1,2-a]pyridin-7-yl)-N-hydroxyacrylamid |
| 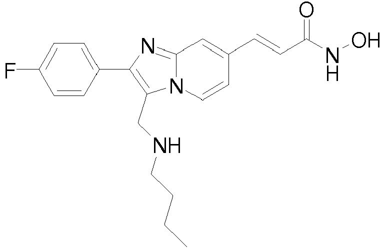 | 3-[3-Butylaminomethyl-2-(4-fluoro-phenyl)-imidazo[1,2-a]pyridin-7-yl]-N-hydroxyacrylamid |
| 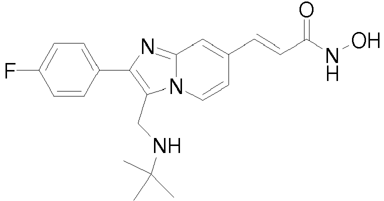 | 3-[3-(tert-Butylamino-methyl)-2-(4-fluorophenyl)-imidazo[1,2-a]pyridin-7-yl]-N-hydroxyacrylamid. |

15. Pharmazeutische Zusammensetzung, die eine Verbindung nach einem der Ansprüche 1 bis 14 sowie einen pharmazeutisch verträglichen Verdünnungsstoff, Hilfsstoff oder Trägerstoff aufweist.

16. Verbindung nach einem der Ansprüche 1 bis 14 zur Verwendung in der Behandlung einer Erkrankung, die durch Störungen der Zellproliferation und/oder durch Angiogenese verursacht wird, mit diesen assoziiert ist oder von diesen begleitet wird.

17. Verbindung nach Anspruch 16, wobei die proliferative Erkrankung Krebs ist.

18. Verbindung nach einem der Ansprüche 1 bis 14 zur Verwendung in der Behandlung einer Erkrankung, die durch Hemmung der Histon-Deacetylase behandelt werden kann.

19. Verbindung nach Anspruch 18, wobei die Erkrankung ausgewählt ist aus der Gruppe bestehend aus proliferativen Erkrankungen (z. B. Krebs); neurodegenerativen Erkrankungen einschließlich Chorea Huntington, Polyglutamin-Erkrankungen, Morbus Parkinson, Morbus Alzheimer, Krampfanfällen, striatonigraler Degeneration, progressiver supranukleärer Parese, Torsionsdystonie, Torticollis spasmodicus und Dyskinesie, familiärem Tremor, Gilles-de-la-Tourette-Syndrom, diffuser Lewy-Körperchen-Erkrankung, Pick-Krankheit, intrazerebraler Hämorrhagie, primärer Lateralsklerose, spinaler Muskelatrophie, amyotropher Lateralsklerose, hypertropher interstitieller Polyneuropathie, Retinitis pigmentosa, hereditärer Optikusatrophie, hereditärer spastischer Paraplegie, progressiver Ataxie und Shy-Drager-Syndrom; Stoffwechselerkrankungen einschließlich Typ 2-Diabetes; degenerativen Erkrankungen des Auges, einschließlich Glaukom, altersbedingter Makuladegeneration, myopischer Makuladegeneration, rubeotischem Glaukom, interstitieller Keratitis, diabetischer Retinopathie, Peters-Anomalie, retinaler Degeneration, cellophaner Retinopathie; Cogan'scher Dystrophie; Cornea-Dystrophie; Iris-Neovaskularisierung (Rubeose); Neovaskularisierung der Cornea; Retinopathia praematurorum; Makularödem; Makulaloch; epiretinale Gliose (Macula-Pucker); Blepharitis marginalis, Myopie, nicht malignem Wachstum der Konjunktiva; inflammatorischen Erkrankungen und/oder Erkrankungen des Immunsystems, einschließlich rheumatoider Arthritis (RA), Osteoarthritis, juveniler chronischer Arthritis, Graft-versus-Host-Reaktion, Psoriasis, Asthma, Spondyloarthritis, Morbus Crohn, chronisch-entzündlicher Darmerkrankungen, Colitis Ulcerosa, alkoholischer Hepatitis, Diabetes, Sjögren-Syndrom, Multipler Sklerose, Spondylitis ankylosans, membranöser Glomerulopathie, diskogenem Schmerz, systemischem Lupus Erythematodes, allergischer Kontaktdermatitis; Erkrankungen, die mit einer Angiogenese einhergehen, einschließlich Krebs, Psoriasis, rheumatoider Arthritis; psychologischen Störungen einschließlich bipolarer Störung, Schizophrenia, Depression und Demenz; kardiovaskulären Erkrankungen einschließlich Herzversagen, Restenose, Kardiohypertrophie und Arteriosklerose; fibrotischen Erkrankungen einschließlich Fibrose der Leber, Fibrose der Lunge, zystischer Fibrose und Angiofibrom; Infektionserkrankungen einschließlich Pilzinfektionen wie etwa Infektionen mit Candida Albicans, bakterieller Infektionen, viraler Infektionen, wie etwa Infektionen mit Herpes Simplex, Infektionen mit Protozoen, wie etwa Malaria, Leishmania-Infektion, Infektion mit Trypanosoma brucei, Toxoplasmose und Coccidiose, sowie hämatopoetischen Erkrankungen einschließlich Thalassämie, Anämie und Sichelzellen-Anämie.

20. Verbindung nach Anspruch 18, wobei die Erkrankung ausgewählt ist aus der Gruppe bestehend aus einer neurodegenerativen Erkrankung, einer inflammatorischen Erkrankung und/oder einer Erkrankung des Immunsystems, und einer degenerativen Augenerkrankung.

21. Verbindung nach einem der Ansprüche 1 bis 14 zur Verwendung in der Behandlung von Krebs.

22. Verbindung nach Anspruch 21, wobei der Krebs eine hämatologische Malignität ist.

23. Verbindung nach Anspruch 22, wobei die hämatologischen Malignitäten ausgewählt sind aus der Gruppe bestehend aus B-Zell-Lymphom, T-Zell-Lymphom und Leukämie.

24. Verbindung nach Anspruch 21, wobei der Krebs ein solider Tumor ist.

25. Verbindung nach Anspruch 24, wobei der solide Tumor ausgewählt ist aus der Gruppe bestehend aus Brustkrebs, Lungenkrebs, Eierstockkrebs, Prostatakrebs, Tumoren an Kopf und Nacken, Nierenkrebs, Magenkrebs, Dickdarmkrebs, Bauchspeicheldrüsenkrebs und Hirntumoren.

## Revendications

1. Composé de la formule (Ic) : 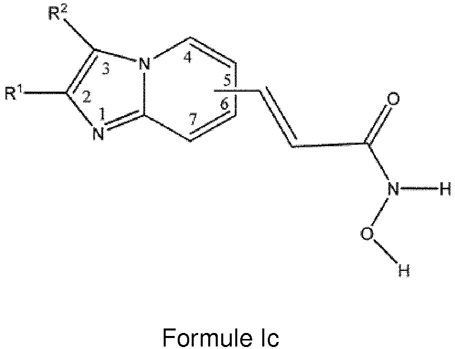 dans lequel :
R¹ est choisi dans le groupe consistant en : alkyle en C₁-C₁₄, aryle-(alkyle en C₁-C₁₄), aryle et CO₂H ;
R² est L, où L est -(CR²⁰R²¹)ₘ-(CR²²R²³)ₙ-(CR²⁴R²⁵)ₒ-NR²⁶R²⁷ ;
dans lequel
chaque R²⁰, R²¹, R²², R²³, R²⁴ et R²⁵ est indépendamment choisi dans le groupe consistant en H et alkyle en C₁-C₁₄ ; ou
R²⁰ et R²¹, lorsqu'ils sont pris ensemble, peuvent former un groupe de la formule =O ou =S, et/ou
R²² et R²³, lorsqu'ils sont pris ensemble, peuvent former un groupe de la formule =O ou =S, et/ou
R²⁴ et R²⁵, lorsqu'ils sont pris ensemble, peuvent former un groupe de la formule =O ou =S ;
chaque R²⁶ et R²⁷ est indépendamment choisi dans le groupe consistant en : H, alkyle en C₁-C₁₄, hétéroalkyle en C₂-C₁₄, cycloalkyle en C₅-C₁₀, aryle éventuellement substitué, aryle-(alkyle en C₁-C₁₄), (cycloalkyle en C₅-C₁₀)-(alkyle en C₁-C₁₄) et G, ou
R²⁶ et R²⁷, lorsqu'ils sont pris ensemble avec l'atome d'azote auquel ils sont attachés, forment un groupe hétérocycloalkyle éventuellement substitué ;
dans lequel les substituants facultatifs de l'hétérocycloalkyle sont choisis dans le groupe consistant en H, méthyle, éthyle et benzyle,
dans lequel les substituants facultatifs de l'aryle sont choisis dans le groupe consistant en H, méthoxy, méthylènedioxy et pipéridinyle,
m, n et o sont respectivement des nombres entiers qui sont indépendamment choisis dans le groupe consistant en 0, 1, 2, 3 et 4 ;
G est un groupe de la formule :
-L³W³
dans lequel
L³ est l'alkylène en C₁-C₅ ;
W³ est choisi dans le groupe consistant en -OR¹², -N(R¹²)₂ et -C(O)N(R¹²)₂,
R¹² est choisi dans le groupe consistant en H, -CN, alkyle en C₁-C₁₄, alkynyle en C₂-C₁₄, halogènoalkyle en C₁-C₁₄, hétéroalkyle en C₂-C₁₄, hydroxy, et hydroxy-(alkyle en C₁-C₁₄),
ou un sel pharmaceutiquement acceptable de celui-ci.

2. Composé selon la revendication 1, dans lequel la double liaison est attachée en position 5 ou 6 du noyau.

3. Composé selon la revendication 1 ou 2, dans lequel la double liaison est attachée en position 6 du noyau.

4. Composé selon l'une quelconque des revendications 1 à 3, dans lequel R¹ est l'alkyle en C₁-C₁₄.

5. Composé selon l'une quelconque des revendications 1 à 3, dans lequel R¹ est choisi dans le groupe consistant en méthyle, éthyle, 2-carboxy-éthyle, propyle, isopropyle, 2,2-diméthyl-propyle, butyle, isobutyle, tert-butyle, pentyle, 2,4,4-triméthyl-pentyle, hexyle, 2-phényl-éthyle, phényle et 4-fluoro-phényle.

6. Composé selon l'une quelconque des revendications 1 à 5, dans lequel R² est choisi dans le groupe consistant en :

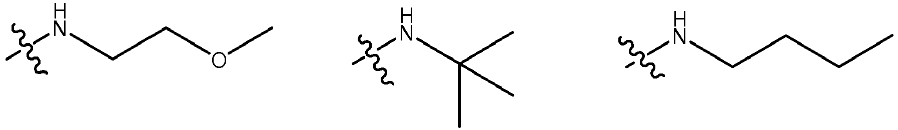

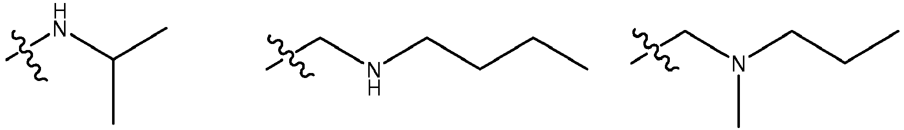

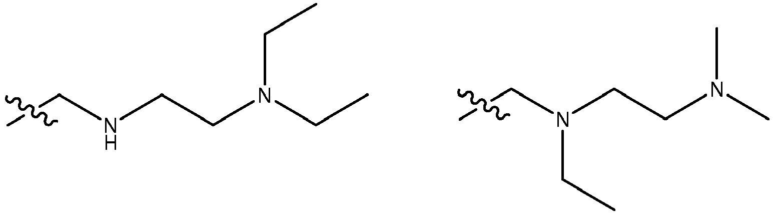

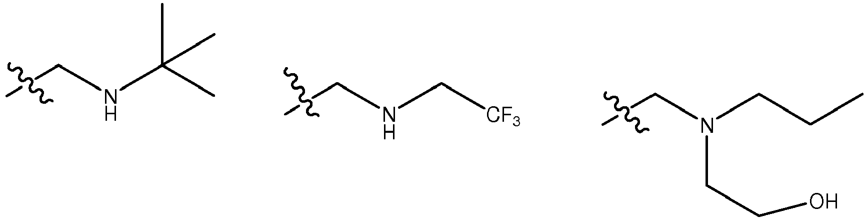

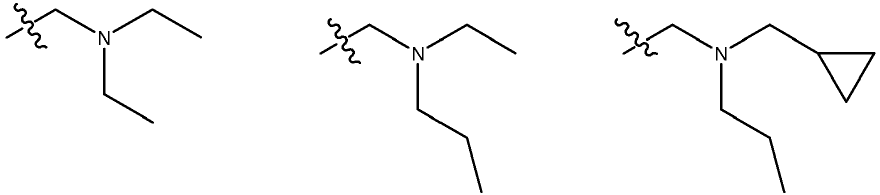

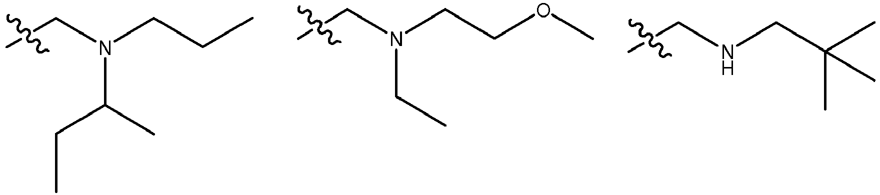

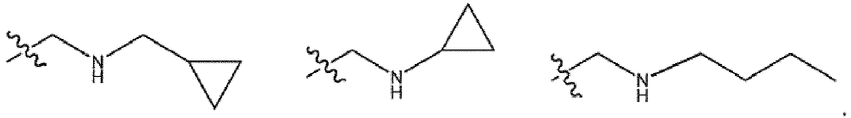

7. Composé selon la revendication 1, dans lequel le composé présente la formule : 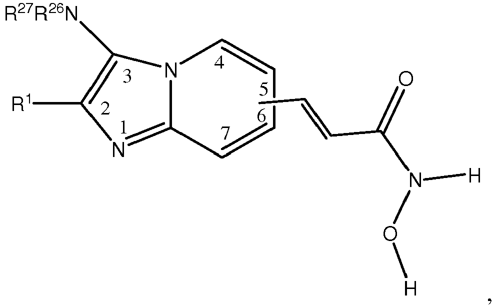 dans lequel R¹, R²⁶, et R²⁷ sont comme définis dans la revendication 1.

8. Composé selon la revendication 1, dans lequel le composé présente la formule : 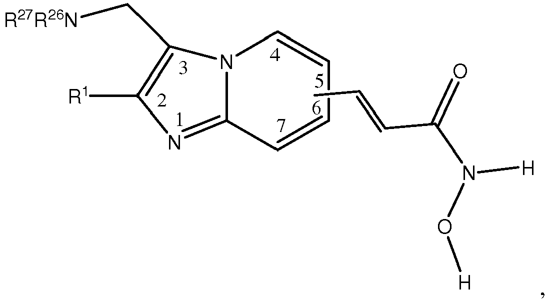 dans lequel R¹, R²⁶, et R²⁷ sont comme définis dans la revendication 1.

9. Composé selon la revendication 1, dans lequel R²⁶ et R²⁷ sont indépendamment choisis dans le groupe consistant en H, 3,4,5-triméthoxyphényle, 3,4-méthylènedioxybenzyle, 4-piperidin-1-yl-phényle, 3,4-méthylènedioxyphényle, 2-méthoxy-éthyle, cyclopropyle, cyclohexyle, méthyle, cyclopropyl-méthyle, éthyle, propyle, isopropyle, 2,2-diméthyl-propyle, butyle, t-butyle, sec-butyle, 2-(diéthylamino)-éthyle, 2-(diméthylamino)-éthyle et 2,2,2-trifluoroéthyle.

10. Composé selon l'une quelconque des revendications 1 à 8, dans lequel R²⁶ est G, et R²⁷ est H ou l'alkyle, G étant comme défini dans la revendication 1.

11. Composé selon la revendication 10, dans lequel G est un groupe de la formule :
-(CH₂)₂-C(O)N(R¹²)₂.

12. Composé selon la revendication 11, dans lequel chaque R¹² est indépendamment choisi dans le groupe consistant en H, alkyle en C₁-C₁₄, hétéroalkyle en C₂-C₁₄ ; et alkynyle en C₂-C₁₄.

13. Composé selon la revendication 11, dans lequel G est choisi dans le groupe consistant en :

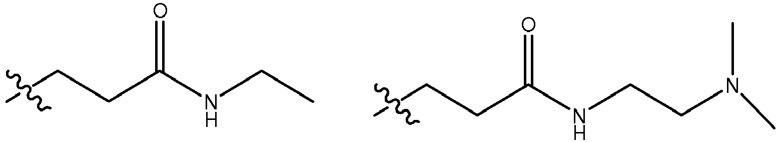

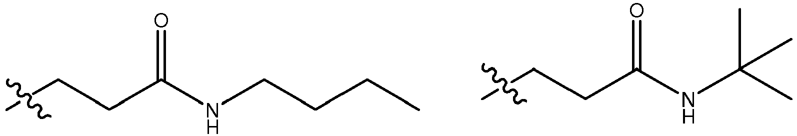

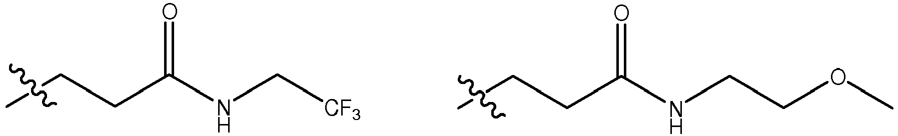

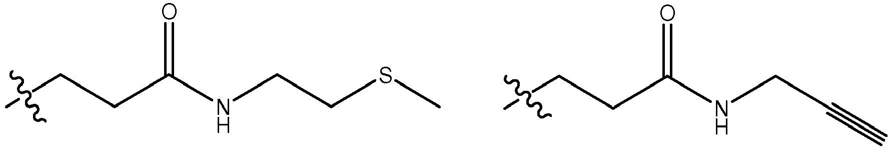

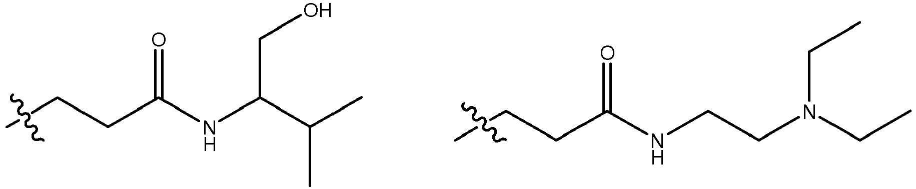

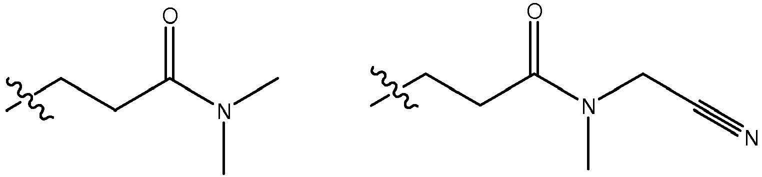

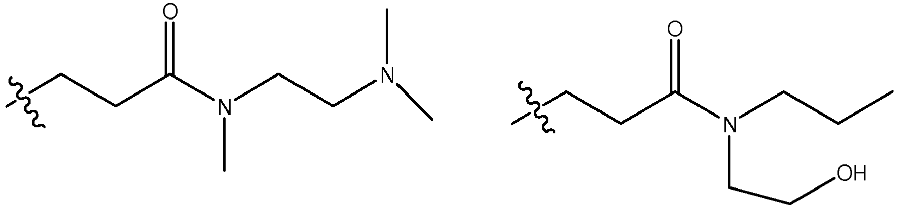

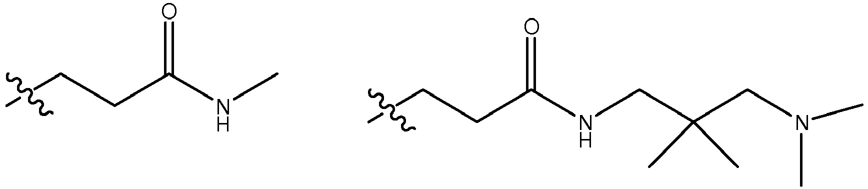

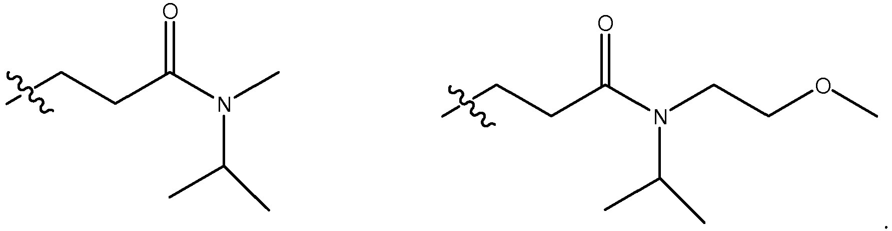

14. Composé selon la revendication 1, dans lequel le composé est choisi dans des composés, et leurs sels pharmaceutiquement acceptables, choisis dans le groupe consistant en
| **Structure** | **Nom** |
|---|---|
| 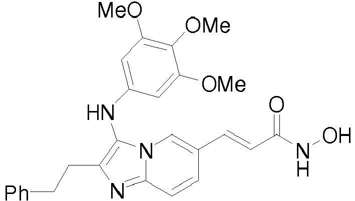 | (E)-N-hydroxy-3-(2-phénéthyl-3-(3,4,5-triméthoxyphénylamino)imidazo[1,2-a]pyridin-6-yl)acrylamide |
| 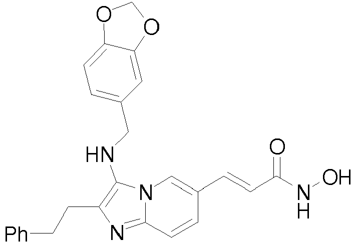 | (E)-3-(3-(benzo[d][1,3]dioxol-5-ylméthylamino)-2-phénéthylimidazo[1,2-a]pyridin-6-yl)-N-hydroxyacrylamide |
| 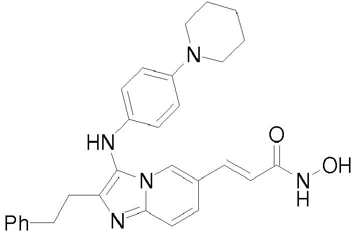 | N-hydroxy-3-[2-phénéthyl-3-(4-piperidin-1-yl-phénylamino)-imidazo[1,2-a]pyridin-6-yl]-acrylamide |
| 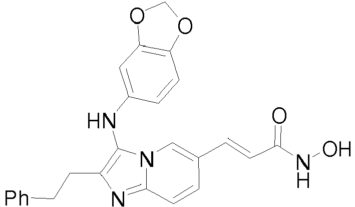 | (E)-3-(3-(benzo[d][1,3]dioxol-5-ylamino)-2-phénéthylimidazo[1,2-a]pyridin-6-yl)-N-hydroxyacrylamide |
| 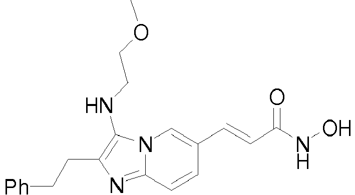 | (E)-N-hydroxy-3-(3-(2-méthoxyéthylamino)-2-phénéthylimidazo[1,2-a]pyridin-6-yl)acrylamide |
| 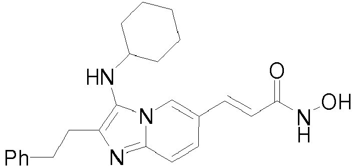 | (E)-3-(3-(cyclohexylamino)-2-phénéthylimidazo[1,2-a]pyridin-6-yl)-N-hydroxyacrylamide |
| 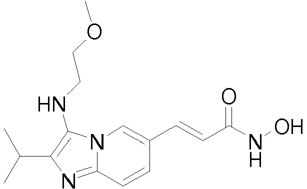 | (E)-N-hydroxy-3-(2-isopropyl-3-(2-méthoxyéthylamino)imidazo[1,2-a]pyridin-6-yl)acrylamide |
| 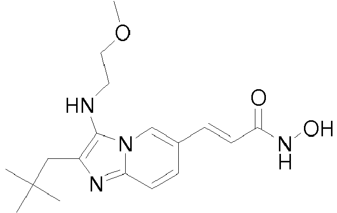 | 3-[2-(2,2-diméthyl-propyl)-3-(2-méthoxy-éthylamino)-imidazo[1,2-a]pyridin-6-yl]-N-hydroxy-acrylamide |
| 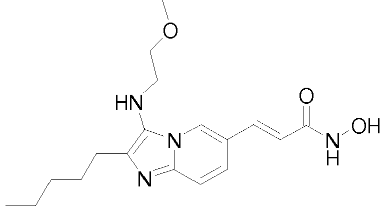 | (E)-N-hydroxy-3-(3-(2-méthoxyéthylamino)-2-pentylimidazo[1,2-a]pyridin-6-yl)acrylamide |
| 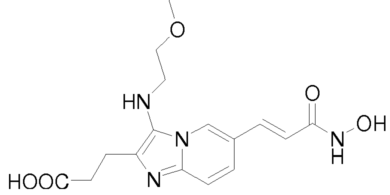 | Acide propionique de 3-[6-(2-hydroxycarbamoyl-vinyl)-3-(2-méthoxyéthylamino)-imidazo[1,2-a]pyridin-2-yle] |
| 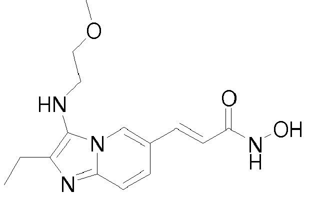 | 3-[2-éthyl-3-(2-méthoxy-éthylamino)-imidazo[1,2-a]pyridin-6-yl]-N-hydroxy-acrylamide |
| 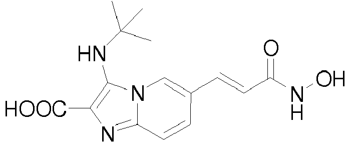 | Acide 2-carboxylique de (E)-3-(tert-butylamino)-6-(3-(hydroxyamino)-3-oxoprop-1-ènyl)imidazo[1,2-a]pyridine |
| 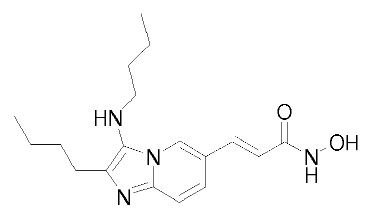 | (E)-3-(2-butyl-3-(butylamino)imidazo[1,2-a]pyridin-6-yl)-N-hydroxyacrylamide |
| 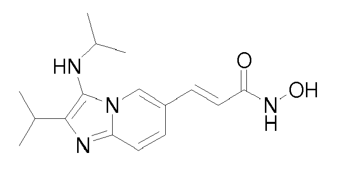 | (E)-N-hydroxy-3-(2-isopropyl-3-(isopropylamino)imidazo[1,2-a]pyridin-6-yl)acrylamide |
| 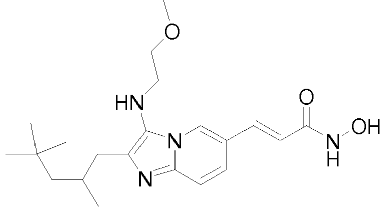 | (E)-N-hydroxy-3-(3-(2-méthoxyéthylamino)-2-(2,4,4-triméthylpentyl)imidazo[1,2-a]pyridin-6-yl)acrylamide |
| 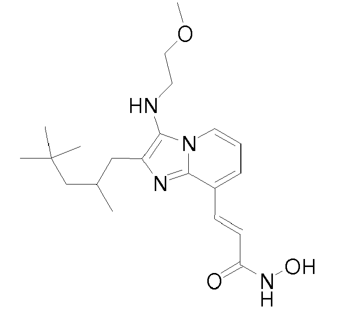 | (E)-N-hydroxy-3-(3-(2-méthoxyéthylamino)-2-(2,4,4-triméthylpentyl)imidazo[1,2-a]pyridin-8-yl)acrylamide |
| 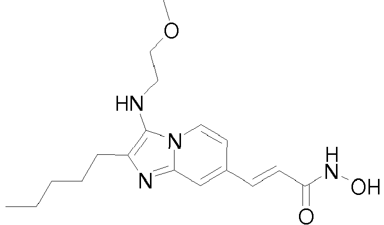 | (*E*)-N-hydroxy-3-(3-(2-méthoxyéthylamino)-2-pentylimidazo[1,2-a]pyridin-7-yl)acrylamide |
| 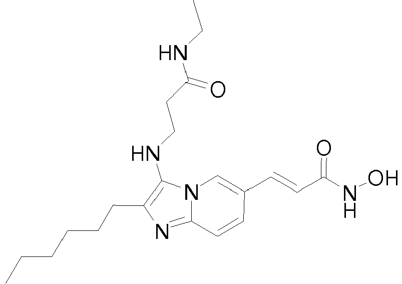 | (E)-3-(3-(3-(éthylamino)-3-oxopropylamino)-2-hexylimidazo[1,2-a]pyridin-6-yl)-N-hydroxyacrylamide |
| 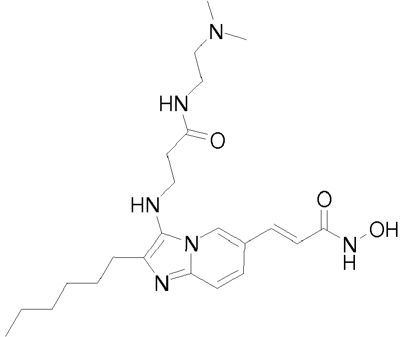 | (E)-3-(3-(3-(2-(diméthylamino)éthylamino)-3-oxopropylamino)-2-hexylimidazo[1,2-a]pyridin-6-yl)-N-hydroxyacrylamide |
| 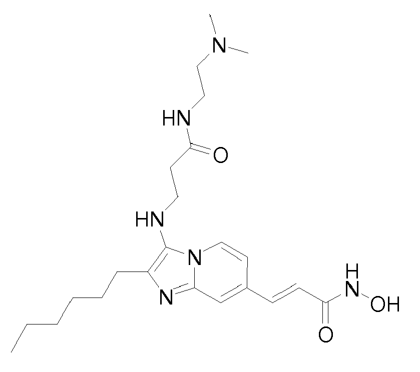 | 3-{3-[2-(2-diméthylamino-éthylcarbamoyl)-éthylamino]-2-hexyl-imidazo[1,2-a]pyridin-7-yl}-N-hydroxy-acrylamide |
| 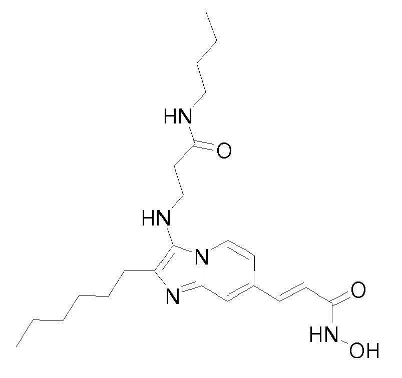 | 3-[3-(2-butylcarbamoyl-éthylamino)-2-hexyl-imidazo[1,2-a]pyridin-7-yl]-N-hydroxy-acrylamide |
| 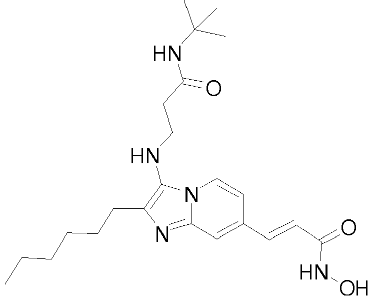 | 3-[3-(2-tert-butylcarbamoyl-éthylamino)-2-hexyl-imidazo[1,2-a]pyridin-7-yl]-N-hydroxy-acrylamide |
| 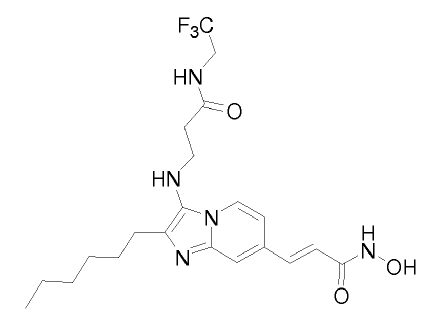 | 3-{2-hexyl-3-[2-(2,2,2-trifluoro-éthylcarbamoyl)-éthylamino]-imidazo[1,2-a]pyridin-7-yl}-N-hydroxy-acrylamide |
| 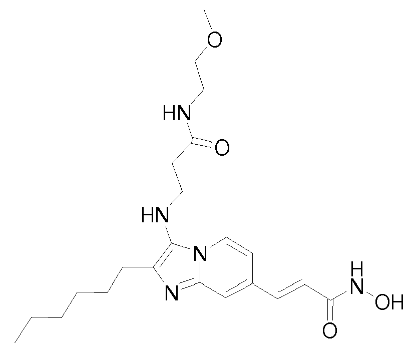 | 3-{2-hexyl-3-[2-(2-méthoxy-éthylcarbamoyl)-éthylamino]-imidazo[1,2-a]pyridin-7-yl}-N-hydroxy-acrylamide |
| 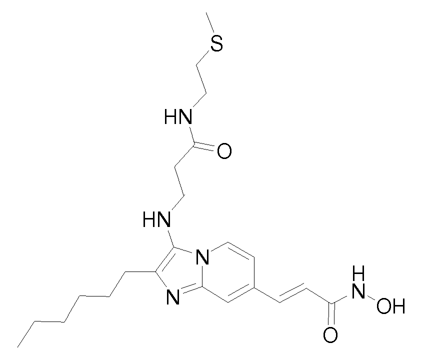 | 3-{2-hexyl-3-[2-(2-méthylsulfanyl-éthylcarbamoyl)-éthylamino]-imidazo[1,2-a]pyridin-7-yl}-N-hydroxy-acrylamide |
| 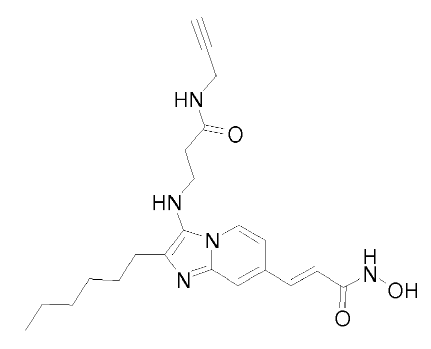 | 3-[2-hexyl-3-(2-prop-2-ynylcarbamoyl-éthylamino)-imidazo[1,2-a]pyridin-7-yl]-N-hydroxy-acrylamide |
| 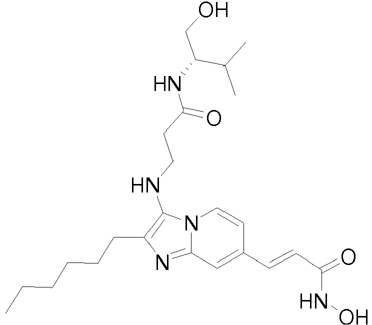 | 3-{2-hexyl-3-[2-(1-hydroxyméthyl-2-méthyl-propylcarbamoyl)-éthylamino]-imidazo[1,2-a]pyridin-7-yl}-N-hydroxy-acrylamide |
| 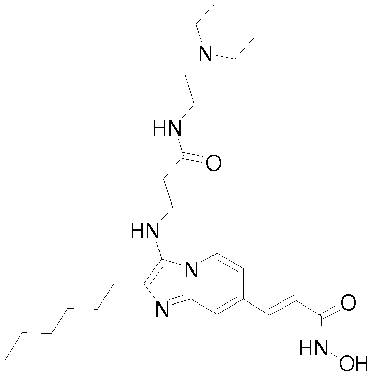 | 3-{3-[2-(2-diéthylamino-éthylcarbamoyl)-éthylamino]-2-hexyl-imidazo[1,2-a]pyridin-7-yl}-N-hydroxy-acrylamide |
| 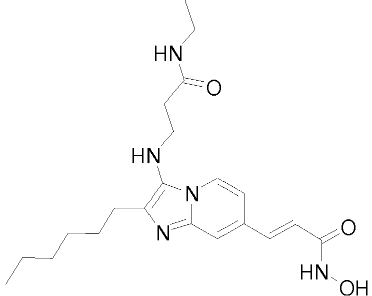 | 3-[3-(2-éthylcarbamoyl-éthylamino)-2-hexyl-imidazo[1,2-a]pyridin-7-yl]-N-hydroxy-acrylamide |
| 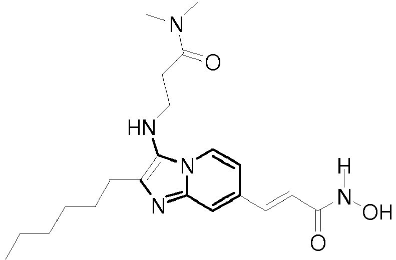 | 3-[3-(2-diméthylcarbamoyl-éthylamino)-2-hexyl-imidazo[1,2-a]pyridin-7-yl]-N-hydroxy-acrylamide |
| 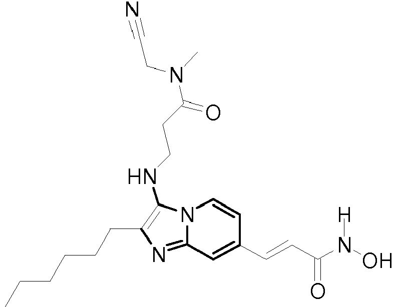 | 3-{3-[2-(cyanométhyl-méthyl-carbamoyl)-éthylamino]-2-hexyl-imidazo[1,2-a]pyridin-7-yl}-N-hydroxy-acrylamide |
| 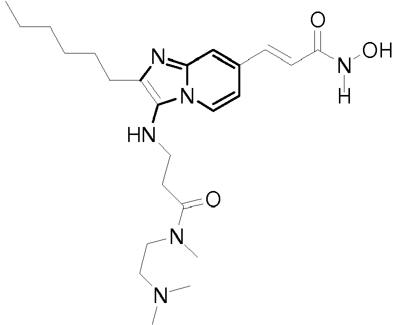 | 3-(3-{2-[(2-diméthylamino-éthyl)-méthyl-carbamoyl]-éthylamino}-2-hexyl-imidazo[1,2-a]pyridin-7-yl)-N-hydroxy-acrylamide |
| 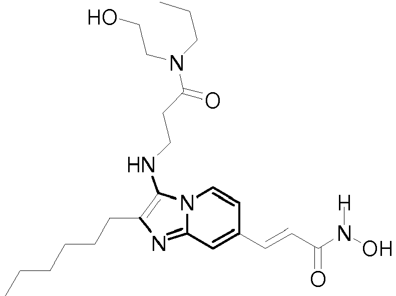 | 3-(2-hexyl-3-{2-[(2-hydroxy-éthyl)-propyl-carbamoyl]-éthylamino}-imidazo[1,2-a]pyridin-7-yl)-N-hydroxy-acrylamide |
| 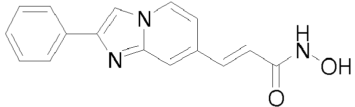 | N-hydroxy-3-(2-phényl-imidazo[1,2-a]pyridin-7-yl)-acrylamide |
| 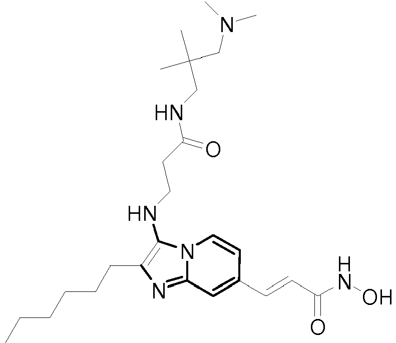 | 3-{3-[2-(3-diméthylamino-2,2-diméthylpropylcarbamoyl)-éthylamino]-2-hexylimidazo[1,2-a]pyridin-7-yl}-N-hydroxyacrylamide |
| 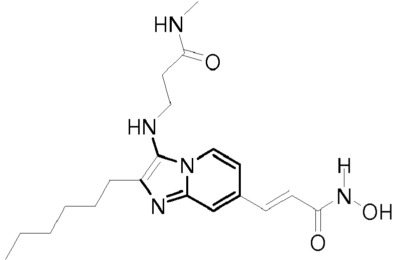 | 3-[2-hexyl-3-(2-méthylcarbamoyl-éthylamino)-imidazo[1,2-a]pyridin-7-yl]-N-hydroxy-acrylamide |
| 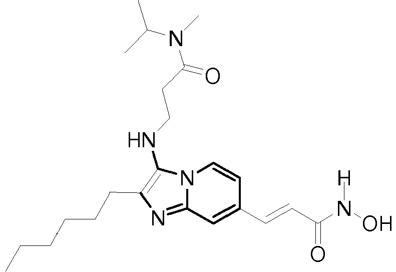 | 3-{2-hexyl-3-[2-(isopropyl-méthylcarbamoyl)-éthylamino]-imidazo[1,2-a]pyridin-7-yl}-N-hydroxy-acrylamide |
| 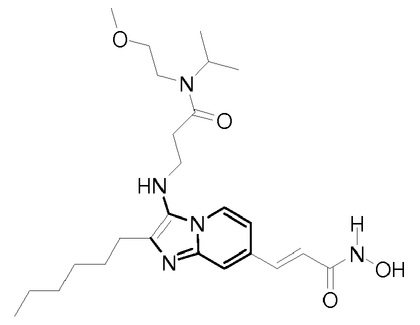 | 3-(2-hexyl-3-{2-[isopropyl-(2-méthoxyéthyl)-carbamoyl]-éthylamino}-imidazo[1,2-a]pyridin-7-yl)-N-hydroxy-acrylamide |
| 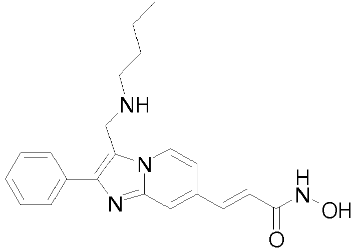 | 3-(3-butylaminométhyl-2-phénylimidazo[1,2-a]pyridin-7-yl)-N-hydroxyacrylamide |
| 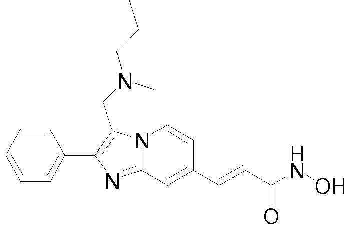 | N-hydroxy-3-{3-[(méthyl-propyl-amino)méthyl-2-phényl-imidazo[1,2-a]pyridin-7-yl}-acrylamide |
| 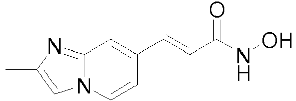 | N-hydroxy-3-(2-méthyl-imidazo[1,2-a]pyridin-7-yl)-acrylamide |
| 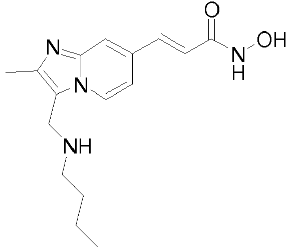 | 3-(3-butylaminométhyl-2-méthylimidazo[1,2-a]pyridin-7-yl)-N-hydroxyacrylamide |
| 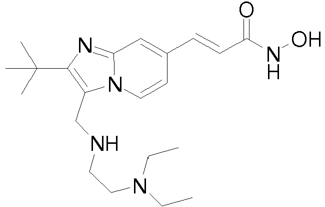 | 3-{2-tert-butyl-3-[(2-diéthylaminoéthylamino)-méthyl]-imidazo[1,2-a]pyridin-7-yl}-N-hydroxy-acrylamide |
| 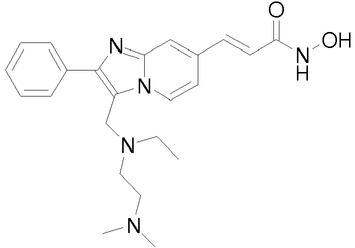 | 3-(3-{[(2-diméthylamino-éthyl)-éthyl-amino]-méthyl}-2-phényl-imidazo[1,2-a]pyridin-7-yl)-N-hydroxy-acrylamide |
| 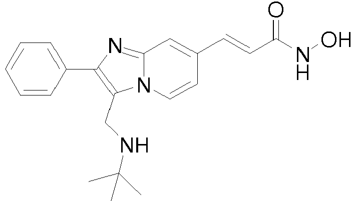 | 3-[3-(tert-butylaminométhyl)-2-phénylimidazo[1,2-a]pyridin-7-yl]-N-hydroxyacrylamide |
| 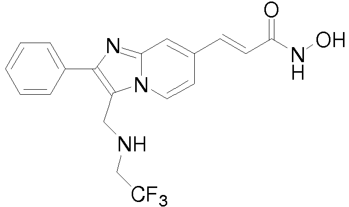 | N-hydroxy-3-{2-phényl-3-[(2,2,2-trifluoroéthylamino)-méthyl]-imidazo[1,2-a]pyridin7-yl}-acrylamide |
| 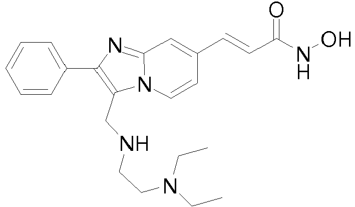 | 3-{3-[(2-diéthylamino-éthylamino)-méthyl]-2-phényl-imidazo[1,2-a]pyridin-7-yl}-N-hydroxy-acrylamide |
| 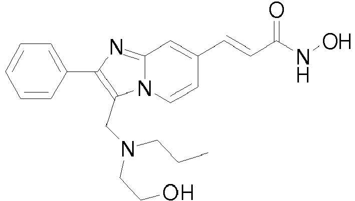 | N-hydroxy-3-(3-{[(2-hydroxy-éthyl)-propylamino]-méthyl}-2-phényl-imidazo[1,2-a]pyridin-7-yl)-acrylamide |
| 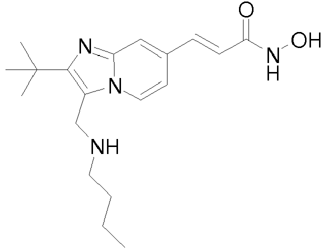 | 3-(2-tert-butyl-3-butylaminométhylimidazo[1,2-a]pyridin-7-yl)-N-hydroxyacrylamide |
| 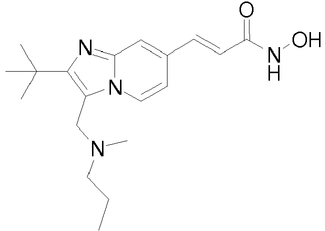 | 3-{2-tert-butyl-3-[(méthyl-propyl-amino)-méthyl-imidazo[1,2-a]pyridin-7-yl}-N-hydroxy-acrylamide |
| 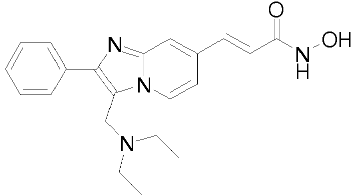 | 3-(3-diéthylaminométhyl-2-phénylimidazo[1,2-a]pyridin-7-yl)-N-hydroxyacrylamide |
| 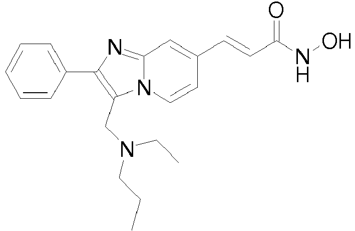 | 3-{3-[(éthyl-propyl-amino)-méthyl]-2-phénylimidazo[1,2-a]pyridin-7-yl}-N-hydroxyacrylamide |
| 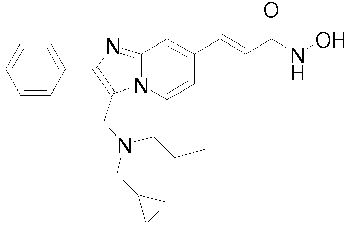 | 3-{3-[(cyclopropylméthyl-propyl-amino)-méthyl]-2-phényl-imidazo[1,2-a]pyridin-7-yl}-N-hydroxy-acrylamide |
| 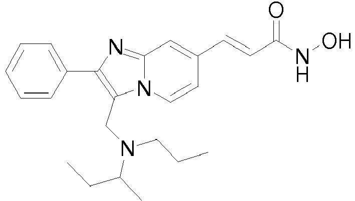 | 3-{3-[(sec-butyl-propyl-amino)-méthyl]-2-phényl-imidazo[1,2-a]pyridin-7-yl}-N-hydroxy-acrylamide |
| 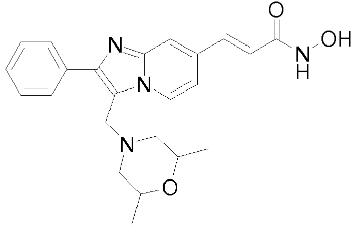 | 3-[3-(2,6-diméthyl-morpholin-4-ylméthyl)-2-phényl-imidazo[1,2-a]pyridin-7-yl]-N-hydroxy-acrylamide |
| 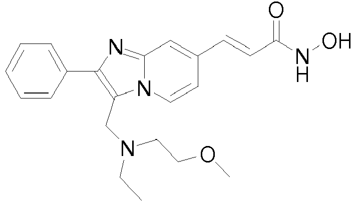 | 3-(3-{[éthyl-(2-méthoxy-éthyl)-amino]-méthyl}-2-phényl-imidazo[1,2-a]pyridin-7-yl)-N-hydroxy-acrylamide |
| 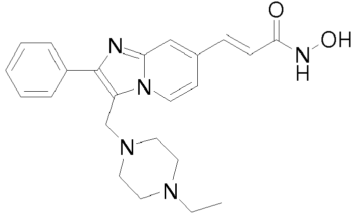 | 3-[3-(4-éthyl-piperazin-1-ylméthyl)-2-phényl-imidazo[1,2-a]pyridin-7-yl]-N-hydroxy-acrylamide |
| 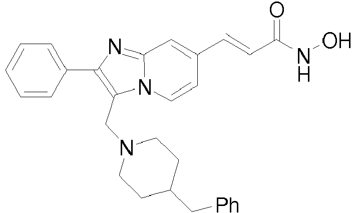 | 3-[3-(4-benzyl-piperidin-1-ylméthyl)-2-phényl-imidazo[1,2-a]pyridin-7-yl]-N-hydroxy-acrylamide |
| 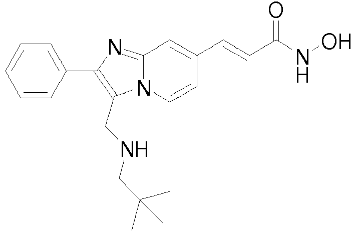 | 3-{3-[(2,2-diméthyl-propylamino)-méthyl]-2-phényl-imidazo[1,2-a]pyridin-7-yl}-N-hydroxy-acrylamide |
| 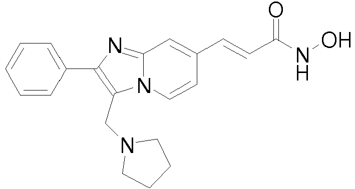 | N-hydroxy-3-(2-phényl-3-pyrrolidin-1-ylméthyl-imidazo[1,2-a]pyridin-7-yl)-acrylamide |
| 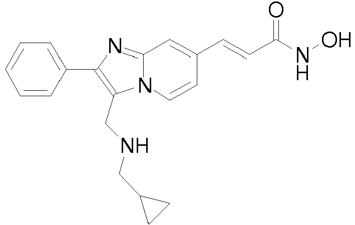 | 3-{3-[(cyclopropylméthyl-amino)-méthyl]-2-phényl-imidazo[1,2-a]pyridin-7-yl}-N-hydroxy-acrylamide |
| 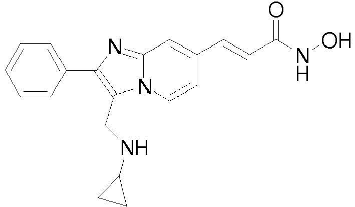 | 3-(3-cyclopropylaminométhyl-2-phényl-imidazo[1,2-a]pyridin-7-yl)-N-hydroxy-acrylamide |
| 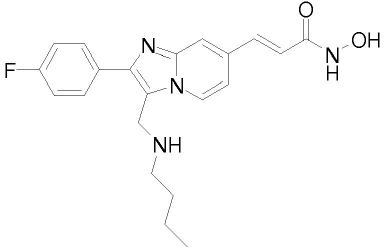 | 3-[3-butylaminométhyl-2-(4-fluoro-phényl)-imidazo[1,2-a]pyridin-7-yl]-N-hydroxy-acrylamide |
| 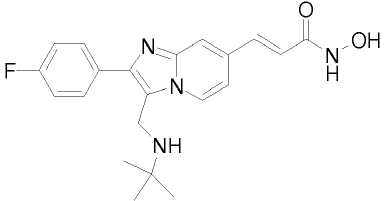 | 3-[3-(tert-butylamino-méthyl)-2-(4-fluorophényl)-imidazo[1,2-a]pyridin-7-yl]-N-hydroxy-acrylamide. |

15. Composition pharmaceutique comprenant un composé selon l'une quelconque des revendications 1 à 14 et un diluant, un excipient ou un véhicule pharmaceutiquement acceptable.

16. Composé selon l'une quelconque des revendications 1 à 14, pour l'utilisation dans le traitement des affections qui sont provoquées par, associées à ou accompagnées par des interruptions de la prolifération cellulaire et/ou angiogenèse.

17. Composé selon la revendication 16, dans lequel l'affection proliférative est le cancer.

18. Composé selon l'une quelconque des revendications 1 à 14, pour l'utilisation dans le traitement d'une affection qui peut être traitée par l'inhibition de la histone désacétylase.

19. Composé selon la revendication 18, dans lequel l'affection est choisie dans le groupe consistant en des affections prolifératives (par exemple le cancer) ; maladies neurodégénératives, y compris la chorée de Huntington, les maladies de la polyglutamine, la maladie de Parkinson, la maladie d'Alzheimer, les crises d'épilepsie, la dégénérescence striatonigrale, l'ophtalmoplégie supranucléaire progressive, la dystonie de torsion, le torticolis spasmodique et la dyskinésie, le tremblement essentiel familial, le syndrome de Gilles de la Tourette, la maladie des corps de Lewy diffus, la maladie de Pick, l'hémorragie intracérébrale, la sclérose latérale primaire, l'amyotrophie spinale, la sclérose latérale amyotrophique, la polyneuropathie interstitielle hypertrophique, la rétinite pigmentaire, l'atrophie optique héréditaire, la paraplégie spastique héréditaire, l'ataxie progressive et le syndrome de Shy-Drager ; maladies métaboliques, y compris le diabète de type 2 ; maladies dégénératives de l'oeil, y compris le glaucome, la dégénérescence maculaire liée à l'âge, la dégénérescence maculaire myopique, le glaucome rubéotique, la kératite interstitielle, la rétinopathie diabétique, la malformation de Peters, la dégénérescence rétinienne, la rétinopathie cellophane ; la dystrophie de Cogan ; la dystrophie de la cornée ; la néovascularisation de l'iris (rubéose) ; la néovascularisation de la cornée ; la rétinopathie des prématurés ; l'oedème maculaire ; le trou maculaire ; la poche maculaire ; la blépharite marginale, la myopie, la croissance non maligne de la conjonctive ; les maladies inflammatoires et/ou les troubles du système immunitaire, y compris la polyarthrite rhumatoïde (PR), l'ostéoarthrite, l'arthrite juvénile chronique, la maladie du greffon contre l'hôte, le psoriasis, l'asthme, la spondylarthrite, la maladie de Crohn, les affections intestinales inflammatoires, la rectocolite hémorragique, l'hépatite alcoolique, le diabète, le syndrome de Sjoegren, la sclérose en plaques, la spondylarthrite ankylosante, la glomérulopathie membraneuse, les douleurs discogènes, le lupus érythémateux disséminé, la dermatite de contact allergique ; les maladies mettant en jeu une angiogenèse, y compris le cancer, le psoriasis, la polyarthrite rhumatoïde ; les troubles psychologiques, y compris la maladie bipolaire, la schizophrénie, la dépression et la démence ; les maladies cardiovasculaires, y compris la défaillance cardiaque, la resténose, l'hypertrophie cardiaque et l'artériosclérose ; les maladies fibrotiques, y compris la fibrose hépatique, la fibrose pulmonaire, la mucoviscidose et l'angiofibrome ; les maladies infectieuses, y compris les infections fongiques, par exemple par Candida albicans ; les infections bactériennes, les infections virales, par exemple par herpès simplex, les infections protozoaires, telles que le paludisme, l'infection par Leishmania, l'infection par Trypanosoma brucei, la toxoplasmose et la coccidiose, et les maladies hématopoïétiques, y compris la thalassémie, l'anémie, et la drépanocytose.

20. Composé selon la revendication 18, dans lequel l'affection est choisie dans le groupe consistant en une affection neurodégénérative, une maladie inflammatoire et/ou un trouble du système immunitaire, et une maladie dégénérative de l'oeil.

21. Composé selon l'une quelconque des revendications 1 à 14 pour l'utilisation dans le traitement du cancer.

22. Composé selon la revendication 21, dans lequel le cancer est une affection hématologique maligne.

23. Composé selon la revendication 22, dans lequel les affections hématologiques malignes sont choisies dans un groupe consistant en le lymphome à cellules B, le lymphome à cellules T et la leucémie.

24. Composé selon la revendication 21, dans lequel le cancer est une tumeur solide.

25. Composé selon la revendication 24, dans lequel la tumeur solide est choisie dans le groupe consistant en le cancer du sein, le cancer des poumons, le cancer de l'ovaire, le cancer de la prostate, le cancer de la tête et du cou, le cancer du rein, cancer de l'estomac, le cancer du côlon, le cancer du pancréas et les tumeurs cérébrales.
